# EUROPEAN PATENT APPLICATION

(11) **EP 2 248 890 A1**
(43) Date of publication of application: **10.11.2010**
(21) Application number: 09305390.8
(22) Date of filing: 30.04.2009
(51) Int. Cl.: C12N 7/00, A61K 35/76, A23K 1/00, A23L 1/30

(54) **Bacteriophages specific to PAK and CHA strains of Pseudomonas aeruginosa and their applications**

(71) Applicant: Institut Pasteur, 75015 Paris (FR)
(72) Inventor: Debarbieux, Laurent, 92290 Chatenay Malabry (FR); Morello, Eric, 78280 Guyancourt (FR)
(74) Representative: Warcoin, Jacques

(57) **Abstract**

The present invention relates to isolated bacteriophage specific to PAK and CHA strains of *Pseudomonas aeruginosa* and therapeutic compositions comprising said phage. The invention is also directed to their use for the treatment or prevention of bacterial diseases caused by *P. aeruginosa,* in particular for phage therapy of lung infections. The present invention also relates to human or pet food products prepared thereof.

Sequence Listing

<110>  INSTITUT PASTEUR

<120>  Bacteriophages specific to PAK and CHA strains of Pseudomonas
       aeruginosa and their applications

<130>  67984 D27440

<160>  3

<170>  PatentIn version 3.5

<210>  1
<211>  93398
<212>  DNA
<213>  Unknown

<220>
<223>  Complete nucleic sequence of PAK-P1 phage

<400>  1
tagatttttg gtataacccc tttccgcgct accactttgc cgattgtgtt aagcgacagc      60

ttacatccgt tctcattttg caatgagaat tgcgtacagt tcatcatgcg cttcagaagt     120

gacttcactt tgcatgcgga tgcatgctat agtatacctt gtgatagctc cgctatcccg     180

ttttgcatgg cacacgaatt gcataagcat gacaagggta accctcgaaa gcggagcttt     240

caaagtgtag gctctaccct ttagtatatt gcgaccggaa gtggtgacac tgaaaacatt     300

gactctagcc cagtaaatac gttaaactaa aaggtggata gctgccgcta tcgagtaacc     360

aacattcaca caaggaggca caatgacaag caacaatcat gccaagattc acatccatta     420

cgccaagcaa atctactacg ccatgcgtaa acgtgtcatc gcaggcgatg agatatgcaa     480

agaatgggcc aagcccaaaa caggctttga agcgttctat gattgggtgg tgaatgtgtc     540

agggtacgcc gtaggctacc gcctagacaa gaacatcctc tacccgggaa acaagctcta     600

tgctccgcat acatgcgagt tcacgcctac atacatgcac actctattct ccaactgcga     660

caagacaagc aataaccgtg ccaatacgtg ggacagctat atccctctag gcgtccgcct     720

agcacgctat gacaagtacg ggatgcctgt atacgttgct cagtgtagga ccctcgggaa     780

gcaatctacc cttggatcgt ggaacgatcc gatgcaagcc catgcggcat ggcaacatgc     840

caaggtagca gctatccatg aatgtattga tctctaccag atggaggatg ttcacagtgt     900

gaacgttgtt caagcgttgc ttgagagagt ggacaaactt ggcaaggata ttgcacaggg     960

aagagagaca acccttttgc aatgatttga tggggtcgat ggtcaaccct tcgagagtgc    1020

gagaactccg ccagagtaat ccgatagcga agctatcaaa cgtatatgca tattccatgc    1080

caacatgtgg aaagatttca ccgggcaggg cgggagcccg ttggcattag ttttgcatag    1140

cttagctgtc gctaagaagg aaagaccacg gccaccatta ttttgaattt actaagggtt    1200

aggtaggcca agtgggttcg tggttaagta gctcggaaat aaatccaaaa cccagacaca    1260

gggtggccct ccattatttt cccagacata gagtaaacgc actccacaag tggtcattat    1320

tttcggcggg ggattgagta tcgtgcatac aatggcaagc tccgcttgca cagggaggtc    1380

atggccagat gctattattg attatttgtt atttccgtgc tataatcctt ataggagttc    1440

tatattatta tgcattcgca taagatttta cagagcattc cttcaacata acacagcata 1500
aggaaataca caatggctga taaattcgac acactctacg aagctcttgt agctctcggt 1560
acggaaggtg cggctaaagc tggatacttc gcggaaggag gagaaggagg cgtagaggct 1620
ttcactgtga acgggaagtc tcctgttaat ggtgatatcg aagtaggtac tgtacggaaa 1680
gtaaacggag ttgaagctaa cgcggctggt gaggttgttg tcccaactgt cgtcactgtc 1740
aacggcgttg aagcggacgg aagtggtaac gttgcagtgg gtactgtccg cactgtgaat 1800
ggtgcttctc ctgacgcaca aggtgctgta actgtagcta tcccggacat cacccctgtc 1860
acggtaaaca atatcgcggt gggcactgca accctcacca tggggacttc tcagaaggat 1920
aagctgaata tccttcggac cacagacgaa gtggttgtca cccttccgcc taacctaggt 1980
accgacacca agccatacac ggccatgatc gtgaacgctg acggcaacat gaaggtggtc 2040
ggtggcgatg gtgtagagat cgtctccttc gctgacgtgg ccgtcaaagg ggttaccctt 2100
ggcttgctga atacagggac aacttgggta gtcatcggaa ctagataaca cataagacta 2160
agccccgggc agcgaaagct gaatcccggg gctttttatt gtcatcagtt tattgtcatc 2220
agtttattgt catcagtcga gtaggagacg ctctaggtct tctcgggttc cccagtccat 2280
catcagactg caatactccc tgttaatctg atccatgtac tgtaacgctc gttctttgct 2340
ctgggcagga ctgaatccgt tgtggcggag aattttattc agattctcag cgtcccagaa 2400
gtccctactc caagcctccc aagctactga ttgcagactt tttatgcaac cgatccgagc 2460
tggataccac caattgtccg agcacagctc gttgtttgga ttgctattca ttggtattgt 2520
cctcggtatt gtcctcggta tcctcatctg tgtagtagag cgttgccatg atcccttgtc 2580
gagccagagc tttacggaga tcatcaatcg ccacagtctc tgagccgatg gtcaccatct 2640
tgcactccgc tttgagagtc attacgcact tggtgtggaa actgggctta tacagggcat 2700
acaaatccgg ctgccaccta ctcagcaggc ccaccccata ggtgacataa tggtcctcgg 2760
gagcctcccg gatgtacagg caccggcgat cagcgtctgg ctcgtccatg gagatggggt 2820
tctcaatcca ctccttgatc ttaccaagga cacgttctcc taccttcaga tgctcctcct 2880
gacagtaccc aaggaatgcg tcaaactgtt cagcggtgaa cccacttaca tcgatctctt 2940
taatcttcat tctcttcctc catatccgca atgatctcct tcagagcctg aagagtgtaa 3000
gtgccattta gctctgtagt agggccagtg tcacactcat gaagcgtgta cgctgtcact 3060
ttcttctcag tgacgaaatg gaagtccacc cttcggaagt cgtagatcga tttcgatatc 3120
tcgatagcct ctgcgagttc tgcctcatac cacaggaatg tcattgaccg ttcattggct 3180
ccgcgagtgt agtcgcatag gtcttccccg acccatagca gcacttgagc cttgtcatac 3240
acgaagctgt ttccactccc aaactcgatg ccataagcct tcaggtgctg ttgtactgcg 3300
atgttctctt caggggtctt cacgatagtg taaaacttca ccaaacgctc tttcaattgt 3360
attcctcctg tctgtgtttt atgagagtat agagtgttct cagtaccctg tcaagctctc 3420
tgtgatagcg tggtttaggc gctcctctgc ccgcttcaga attgtatgcc actcgtctcc 3480

ctcttggatt cctacatact tcttcatcat cagcgcacga tcaacaagcc ccgctctgta 3540
gttctgtcgc tcttttctac ggtccagcac tcttgagata tggtcctttt tctttgccaa 3600
cgtgctgata gctttccttg ccataagagt tgcatactcg cgctcctcgg cggtcatcca 3660
ttcctgatac agcagtccta gacgagtatc atgttgtatg agtggaaggt ctttccatat 3720
gatccctgct cgtccagtat cagagacggc tttccattcc agcgtagttc cggtcttaca 3780
gtctgtgatc tttacattct ccgagttaga ttgagactgg aaagcgtgcc tccagtctcg 3840
gtatacaatc caccgcttcc agtcgaagaa tgtctcacag atggctttaa caggctcccc 3900
gattttaccc tctagttctt tcaggcggtg ctcctgctca gcactcatat atttgatagg 3960
acgggaggca ggatcgtaac actccttggg aacctcccag cccttttgtt ccagagcgac 4020
ggactctcca ctcttatctt ccttgtaaaa caaccaagcg aacagcttct taaacatatt 4080
cagtcctcct ctcctgtgac ggctacccta gtcagtctcc acactttccg agggagccta 4140
acgaggaata gcatagtcat aaatggtact gcgataggcc agaatattcc agccaacacc 4200
gcccgggcct ctctcttacg cctctctgta ctttctcccc tccacccaag gaggggtcgt 4260
agagattccc atacgaacat tcctattaca gcgtagaaca ataggcagac cactccgacg 4320
actatgcata cgagaatgag aagccctact tgttccatgg tgcgatcctc ttaagtagtt 4380
tacggctgtt ccagatagac agagcccact taggtaacaa caccactttc aaaacgctgt 4440
atgttgcgta gagtagcaga aagctagcat acactgtgta tgtaataggc catagcagtc 4500
ccagtacaat ggcaaaagcc tcgataccct ctctgtgatc tttagtcgca ctagctggga 4560
agttatcttt gaaatactcc gtgataccca tgtaggtcca catgaacaca attccccacg 4620
cgagcgcgag gacgaatagc attgggatta tctccattac tcacctcctt tgcgaattaa 4680
atcgaaataa gcccacacgt agaagttgca ccacgctcgg cccatgtacc atccgtccca 4740
gtaatcctcc cagtccttgt ccggcggatt attcgcaaat atagccacaa tatcgggatc 4800
atatcttttg tctagattgt tcattaccga gttgagaggc cctattccaa aagtttcatc 4860
tagccatccc ttaaatcgcc ccacgtcaga aatggtgacg gaggagttat tgtctagggc 4920
cagtctctcc agaatgtagc acatgtatgg ctcctcttcg ttctcgaaga atgcccgcat 4980
gccctccttg attagaggtg caatagaatt agagttcatt gttttcctcc tttgagagcc 5040
ccactagggc tctaaatttt tggttcgacc tgcggtcagc acatcacaac ccagttgtac 5100
aggtcagcct cctcgtaagc cgatgggtag cgattgtact ccaagaagaa cgcatggtca 5160
atagctgcgt atttggttgc gaagctgttg atgtacgtta taaacccgtt gtcgccccgg 5220
ctaaacaggg cgtattcctc agtaccatcc ttgaacgtat ctaacgcgat aatatacttg 5280
tcgaagatca tagcatttcc tcccggctga tgtagtagtt gtattcatcc tcttggacga 5340
ggaatgctcg atagaatccc ttctcgaaat ctcctttgac cagtaccaga tttccatcca 5400
ccttgcaggt gtaaatgtac atggtgtttg ctactcggat aaccggatca tcacccgggc 5460
gaacatccag agcagccaac atagcttcca ttggcatgct tgtcaagtga gcaaccacgg 5520
cggagcagta ttctgcgtcc cgtcccgagg tatcctctgc ataagcagga gcgaccccgc 5580

atgaatgcag ggcaagtgta aacgcaacgg ctaccaattt cttgaacata ctaaccctcc 5640
tcttgatctg tctggggaac atcctgccta gggtagacga atgtctctgg atatttcatg 5700
catcttagct catccttgca gaatccgtca atcagttttt ggtgtgcgaa ttcggctgct 5760
ctgtagtacc gattagcctg tctagcctca acccgcagga ggtggcttat cgtgtgagca 5820
ttgatattac taaggcttgc aaaaattgtc aagagcttct ttgcagagag ggggattgac 5880
ctttcgtcag gtctgtacca atcaagtctg gccacccctt ggagaacatc atcgacccat 5940
agagggtagt ctccgtctag ctcgaccatg acctcgtaca tttcaggctg aacagcgttg 6000
aaaaagaaag agggctttcg ccctctgcta gtatcgagag gaggcacaag gcccctctca 6060
accttacgaa tcaaccgatc agcgttagac cctatccgag ccttcagctc ctcgatctct 6120
tcacatgtca acatacggag ggcacacctt ggattttgcc tgagtgaaca cctcagcaca 6180
cgtaaggttt gcaggaccat accacttgaa catgttgttg atatcgtagc ggatcagttg 6240
tctaaacttc tggccgtcac acatatcaac gtcaaacgag gcgataaccc acctaatagc 6300
ctcttcctgt tcgtttgatt ctcgccagcg gcactccaag tctgcgtggc tgggctccaa 6360
gacataaatg tcttcggcat acgtgtcttg gtagtttttc agacgaagta ttctggctct 6420
aatccactgc atgcatctgc ggttggggtc ggtgatatca caaacgaccg gaacctctgg 6480
aacctcaact gtatacatat aacacctcta tttattttac aagaatgcca tgattctgtc 6540
tctatctgca tggcggtaca gattgaagca aggcacacct ctagaggacg ccagttgcca 6600
agcggttcta gtcccgcctt ctggaattcc gttgtggtct gtcttgctcc agcacaccaa 6660
gaacttgcta ggtgtgctga gattggttcc caacacttgg aaaacattcc gagtgtgggc 6720
gcgtcttgcg aatgggctca gaacaggctc tccatcattt ctgacagagt tccaagctgg 6780
gtgtatccgg ctagcaatac tcccggcttc ctcccactgc tccggccact cttcttctgg 6840
aaggattgta gctcctagag tctcagttat cacatactga ttgaacccgg gctccggcag 6900
atagatttct gctgtagttc ctcctctgtt attggagata tagtctagcc atccagcctc 6960
gaacgccgag tccgctccct tcgctcctcc tgaccgaaga ataaaacctc gttccgctaa 7020
tttacgacct agctccctga gaatgcccca ctccttgtta ggcgtatctc tacttccgac 7080
tcctgtgtaa tacttcataa gggcaccatt ctagataaat cttctggtgt cacccattgg 7140
tgcctatatt ctccgttttt cgggttagaa tactccaata gaacagggta gtactcgtct 7200
ccgtagtcgg tatctataac ctcgcagtcc caccctacag ggatagcaca agaaccttcc 7260
ttggcagtca tcctagttcc aatagacagc ctattggtct caatagatag cctgcctaga 7320
ttatgactgc atttgtgttt taacgcttca attaccttaa cttgtaggtc gagaagctct 7380
tcgattgtaa agccacttac atcgattgtc atattactcc ctcccttcta gctttttgaa 7440
ctctttgtct agctttgcta agtgcttgtc cagtttatcc atcttcttat cttgatggag 7500
ctggaacgcc tccgagcccg gggccaacca agtcccctta tacttaattg caccccggat 7560
catatctcct cctgcttaca attcgtaaac gtatacctga aactggccga gcgtctcctc 7620

gataagaggc tcgacattgc tccagtcctc tccggctaaa ccgcatccaa tcttcggcag 7680
acctacacac aactccgagg taggaatcgc ccgagcgtca gcctctgcct caaggaacag 7740
agacatagtg tacaaggctt ggcccagtgc gtcaagctga gtgtagactc gaccctcttc 7800
tcggccatag ttgtactgtc cgtacagatt ataaatcgat ccgtactcgt tcattgtgaa 7860
ggtcagggag ccgagtttac ctcggtcccc tttcaccgtt gcttggtctg cctcgtaggc 7920
ctccgggaat tgctcccgga tttgcttagc aatgccgctg cccatggtgt tgaagcaatt 7980
agcctgatgt gcaatggcgt gcaactcttt gtttttgaac gcagcgataa gatcgccctt 8040
tcggtattta atcatttgaa cctctcaaca cttgtgaatc tgacttcaga gcctctcgct 8100
ccgtgagcaa tgtctgcgta catcgagtta gtggcagcaa acggggacca atactccttc 8160
tcccagattt tgaacttgaa cattttgtgc cctttatttt ctgggcgacc ttgttcgcag 8220
aaggttgtaa agtaatattt agctaacacc tttatcctcc ttatactccg ttcttaatcc 8280
gataacggat agatgccaag tcattgttct tcaggtcttc ggtgtcccat ttcccgtctt 8340
tgaagaacgt ccgcaaggaa ccagtagcct cctcctcttc agatacatta tctttcaact 8400
ggaggaatcg cccattttgc aggtctcggt acactttcag gaggccttta gccgacttct 8460
tcttgctgtc agttttcggg tctttgaaga ttgcaatgcc ttcaccgttc acttccgtgt 8520
aggtggcctt cactgcgaac ccgaaggtgt ctcgggtatt gcactgatag gtgtaactcc 8580
caactccgaa gaccacgttc aaggatgcga aacctttctc cttgagccgc tccaagattt 8640
tggctgcccg acgtgtggtg atcgaatccc catagatcaa accgatatga gaatccagca 8700
tcttataacc agcttcgttt acagtaccac cgaaggtttc ccaaagaact tggatagcac 8760
ctttcacctg agcctcgcta acttcctcta gtttatagcc aactacatac tcgtactggt 8820
cccactcggt tggaatgacc ttgaaatatt tatcttcgaa ctctagaaca aaagcgtcat 8880
ttccgttgac cgcatccatg atatcccagt gctcaagaga ttcaacaact tccacatcgc 8940
ccagtccgca gataacctct acaggatcgc cagaatcggg acgaacaacc agcttgccgt 9000
cacgttccat gatctgatcc ttgatggacg gcagaatgtc agtaagcacg ccccagaagt 9060
cgaagctgtc tgccacgtaa gacagaatac cactcgggaa cttacccatc agttcaacaa 9120
ggaatcgacg ttctgcttcc tcacggccac ctttaccttc tccatccttc atttctttct 9180
caatgcggag gatgttacta gtagctacag cgtgttcagt cgcaggaacg gagcaagcta 9240
ccaaaccagt cgcgttatag tagtcctcag cataaatagc tgctgccaat gtgtccgaac 9300
ctacgaacga ggtcaagtga ccaacaccac tacgggctgc gtcttcgatc ccgctcatgc 9360
cccgggcact gaagtcgtgg aactgaacag acagcaggaa gtcgtcatag catccagtgt 9420
gattcgcgta gtatcggccc atagctgcgt attctgcggc gatagtggcg ttagttgcgg 9480
tcttccaagt catagcagaa atcagtgttt cgtggtagtt caccagccag taaaactcag 9540
gcagggtgtt cttgatggtg agaacaggca cacccatcgg cacacggacg ccttcggtaa 9600
gagattttac ctccagaggc agataaccga gcttgtggag ggctcgaagc tgctctacag 9660
cttccatctc acggccaaag taaccagtca ggaaccgggc ataccggcca actgcgttct 9720

cttcgtcagc cttgaagaag ccttcccaag actcgacgat ctccatccaa gcagcccgcc 9780
cgccgaccca cttaagagtg ccgtcgtaga agtcggtggc gtgagtctta cgataaattt 9840
tgtcggaacg aggagtcagg ttgctgtaga cgagctgagt tccttccgag tacatcgagc 9900
cgtgcccaat cttatagcca tcggtggaca ggtgtggcag aattttcata ttatttcctc 9960
ctgttgtgtt gaatgggccc agattatatc aatctgggcc ttatgtcaat actcaatttc 10020
ttctactggt agtgtcagta cagggtagac acaagggagc cgcagcaaag ggtctttttt 10080
tttgctatgg aagtcttccc agaacttctc atgcagcacc ttgtaccgag cgtccagttc 10140
ttccttggtg tcgaactcct ctaccttgac aaccctcttc tctggagccg agcgcttata 10200
ctccacaatc catatactca agctcatagc ttctcagccc tagagtattt gacaggaaca 10260
accattagct ccgggtggtt ctcttgaggg agtgtatcag atgtatatat gcacccgaag 10320
tcgtccagta gacaatcaac acccttgctg aagattccgt gagtgacgaa cagatcaggc 10380
agcacttcac tgtaccctct cagagcttta gctacgctac ggaatgtagc gcctccgtcg 10440
cagatgtcat ctagaacaag cagcgggagt tcaggattga tatccgaagg attcagaatt 10500
tcaaagcctt cgattttgcc agtcttaagg tctcgcttct tattgcaacg gatgatgact 10560
ccggctccag tttcctttgc aaactgctca gtccttttag tagctccgat atctggggct 10620
acaagctggt actgttccca atccgaccga tccaacagtc caccgaatac agatgcctga 10680
gacacaacct ctacattgtt aatcaagtgc agagtagtct cactgtgagg gtccacaacg 10740
ataacacgat catacccagc cgagttgatc agattggcga acactttcag gctaaatgcg 10800
tccccgggat tgcatcgacg gtcttgtcgg gcatacggga catagcccaa aagaagagtc 10860
atatgcggtc gacccatcgc agctcgaata gcctcagtga cattgagaag tgccaagata 10920
tcgtcgctgt tctgaatctg agctttaacg ttaacaaagg accctgcact ggcatcgatt 10980
ccacttacat tgacgcccac ctcacctccc gggaatgtcc agacctttgc ctccttcata 11040
ggaagtgcaa aacccggttc ctcaatagta gcaaggcaac ctgttacgta gctaatcata 11100
tatcctcctt tacagcttca cagagaagcg attccgcatt gcttccatgg cactctcagg 11160
cacatcgtgt accgatttat tgccatgacg gttctctaca atcagagaga taacagtgta 11220
tccaaactct tcggccatgt caatgtaggg tttgatctcc cgttcccggg tgccggtgtt 11280
ggacacaatc actgtgtcaa accctacaac ttccattgcc cttctggctc gatccatgca 11340
atccctgtgc gcctttgcca cacgctcagg tctccacaca tactcccctt cttccatcag 11400
gaagtggtct gcttcgcaga ttatagcgtt gcttcctgct tcccaagctt tatcattcag 11460
aattctggca agattggact tcccggctcc cgggagtcct cgaatgatgt acatgatctt 11520
ttcctgcata tcctctcctt tagattcgag ccatatcgaa gtaggtctcg atcaaaagcc 11580
gacgctgtgc gtcagtcatt tctgtaaacc gagtctcatc tacctgattt accaagaagt 11640
ctaccagagg gtagaactct tcgtcaagcg tctgcttgaa tccagccggg tcttggtaca 11700
tcgcattgac cttcttgctg cccatccgca tgataaactt cttaccgagg tagtgagtgg 11760

acttgcactt caccgtccct acgctggtcc gcaccatata accctctttc tcacaggagc 11820
tgaggaggcc cttagcatgt gcccagctac actcaatgtg atctgggcgg agaattttaa 11880
ccgagcttcc agccgacata ccaagtgcgt atgcatccag aatatgctca ggctgctgct 11940
gaccagtgaa gatgtcccga acaccgatca gataagcacc ttcttcttcc tccacaatgt 12000
gagggtcttc cggggagcag atttcaaagt tgtacgtgaa ccctgcaatg tagtcatctg 12060
cgttctcaga cacatacttc cgaccgagtg caacatagtc actagtcaac gaccctgtcg 12120
tgctgaagat tagcgagccg ttgtgcttag ttacagagaa catgaagcca ttcactttct 12180
caatcaggcg aacacggaat ttaggatgga ggaaacgacc cgctccattc tcccctaggt 12240
tgaacacctt ctcgaaggga cgaacaatac aatcgccgga atgttggtca aacacatgtc 12300
cccgcgcttc aagaagaagc gggtgcttgt tccacaagtt gtcgtagaat accttacgag 12360
cgtatttaat aaccgacata ccgttgaaat gcttaaccgt cactagcttt ttgtcgatca 12420
ggtctttgta catctcaata tggagtttac cttccatcct attcctcctt attctacttc 12480
tacaatcggg cagaatttag acttctttgc ctttaaatat gctaccagaa tcccatcttt 12540
gctagtcgat ttactcgaca taagacgcgc cagtcggtag aagacccata ctccgtaaaa 12600
gatcgcgatg ataccaatcc atacaaaccc cattacagcc agagtgctac ctagaacata 12660
cccagtgagc gggcctagaa tcagcgagta caggacagag cctacaacgg cagctagaga 12720
tacaaccgtt gccgcaagcc acacaacaag gagcactgtc tgccagaaat aggagcagag 12780
agttgtaatc ttccacccgc ttgtaatatt caagtagact ggggtctcta atgcacgatg 12840
caggcgcata tgccacgaat tcttattaat cttcatgctt gctcctccgg gaaaatgtta 12900
aagtttaaca cgttgtgctc ttcaagctcc atggaggcta ccatcttgta acctccgagc 12960
ttgtgaatga tctccagtat actctcaata gagaatttgt caacatgctg tttcatcaga 13020
tttgagattc ggctctggtg agtaccgcac cgctcagcta cttcccgctg ggtcagtcca 13080
gactgagtga tgtagtgtgc agcaacaacc attacagact tcttgaaatc cttgaccgga 13140
tcgttcatgt taatcctcct tattgctcag tgtcactttg ccattgaata ctcggaattg 13200
agtgatctca aactgatccg tccatcgggc gtcctcgaaa gccactcctt caaaagtgcc 13260
atcttgtttt acatctgaag tcatgaggac gataaaagga actccgtcgt cgtggatcag 13320
ttcaaccaga gtgttagctc taggtgtaac atcgtgctcg ctacgtgaaa tcaatacatt 13380
aaccatatta tcctcgctgc ccgtcgtggt ggtctactac cacaaattct tgattccgca 13440
tgtattctct cacttcgtcg taagagaata cctgatgatc tgggtgattg tcaataccta 13500
catcaagcct ctttcctttg gaaggcaagc tgccgtgagt gtggccgtgt agatgccaag 13560
caccatgatg agcagtattc cacgtctcga aagggtagtg gcacatcacg atcttcactc 13620
cgtcaattcg aatctccttg tactcacgaa tgtcttctac gtgagagaga ttgctatcag 13680
cgatcaactt ccacagacga gagtcgcaat ggtttccctt caggaaggtg atacggccat 13740
tcaactgttt aatgatctcc agtacagagt gcagcttgtt cctgccggcg aatgcgaagt 13800
cgcccaagtg atacacttcg tcaaaaagtc ctacccggga gttccaccga gaaataagct 13860

catcagtttg ctcctcgaaa gtccaaggcc tgttgcagta ttcgatcaca ttcttatgga 13920
agaagtggag atcggaagag aaaaatacag acattatgcc tccttttcaa attcataaat 13980
cttgttgatc ctgcagatca gtcgaatagc agcgcctccc agcagatgct ctcgatccgg 14040
tttcatatgg gccattgcct tgaactctga cagcatatct cggcgttcat agtggaagtc 14100
tactacccag taggtgtctg cattaatctt accctctcgg tacaggtgcc cttcaacacc 14160
aagccagtgg tctactgcgt ctttcttcag taactctttc ggagaccact tgtctacacc 14220
ccgcaacttc tcgaagaaag agtaaggacg agaatagttg ttgatggctt cctctacagc 14280
cgactcagcc tgcaatttaa tgaactccca tcttgcaaga acttctttaa tctcctcggt 14340
ggtggctggg gtgtagctgt actgctcttt atattcattc cacattatac tgttctccta 14400
cgtagccttt gaatttgtcg gaacgggttg tcttcttcag tgccttgttt tccagaaccg 14460
atgcttccct ttcgagcatg ctcagttggt tgctatactt cttgatccgt ttcgcattgc 14520
cactcatcac agaatttgta agattggcaa tagtgtcgca gattttaacc tcttttgcaa 14580
ccggatgatt acggcaaccc tcgatatact tccgatagct ttcagctcgg tttttgtcaa 14640
gaatgaccag agcctctacg atatgcggca tcacgcccat agactctaag tcctcctctg 14700
tcaggtcggt atcctcaaga acgtcatgga ggaagcaaac cgctagaagg gtgtccatgt 14760
acggaaagtc cgagaaggcc tgattcgggt ccatatcctt cggagcatta cgtgctacgc 14820
agattgtatg cacttggttc agatggtagg catacggata gttggtctct ccataccgct 14880
gaccttcatg ggccgagagt gcgatctcga tagcaatacc gagctgagtt ttcatgttaa 14940
tctcctttgt agaatgtgtg gttgcctatc ttgatggtac gcttgtactt ccgagcccac 15000
cgagggagcc tgccattatt ggctctcctt acctttctct cgttcaggta gtggtccgct 15060
cctcccgttg gatcaacagt atacccatac agagcgttgt atgcaacatg ttttgctgtc 15120
tcccacttgt cgggctcctt gacgccctgc cgcatgctac catcgcaggt gtacgaatat 15180
tggcatttga agtataacac tccagctacg gtgctgggcc acctatcgtg ctgcatcctg 15240
ttgatgacgg tatgagctac agcaatctga ccgatctccg gttctccccg ggcctcgaag 15300
tatacagctt cagccaacaa cctgcaaggc tttctgtact cacacaaggt gcgcttgctt 15360
tctatgattt gaccgggact ggcttggaac tctactacag gttctgtgcc gtatttgaga 15420
cgagagtatt ccatatctag gaacatccca ataaaagcta gaaggatcga caaaacgaga 15480
gcttctaagg tttccataac acctccttag acaaagaagc ccgccttgtg ggcgggctgt 15540
atatcactcg gtaatccgag agtggtagct agttgtatac ggcccttgaa ccgtgaacga 15600
cttctggaag caacggttca tgagcatatg acctagctct tcagctaggc tttgcaaatg 15660
agacgcttcc gcagggaacc gagggtagtt tataaaaccg atcacagctc cttgctcttc 15720
tcctccagtg tatatgaacg ttgtcggatc aaccgtcacg caaaggccct tctccatgca 15780
gaactcccta caggtctgtt tcactacgga gatatcgcca ccgatgaaca gttttacact 15840
aaaagtgtct gcatatttta catccgcatc ccgggagaag tacactttaa taccccaagt 15900

tgtaacggcg tgcgtaatcc gcgttgggga actcgaccat catcgactga atatgaatgt 15960
tgcctttacg atccttctgg attttggcat ttttcacaat cttactagcc tgcgcctttg 16020
ccataagctc tggacttaca accagatcga tgaaccccgc gcttcgatac ccacacccgg 16080
ctggggccca gaagccatgc tgggtcatcg tcgagccggg aacgaggtac aggttgtgca 16140
gacgcacagc agtgttgaaa ctcagcttgc tgatgtgctt cgcttcccga gtttgggttt 16200
caaaacaccc agtcgtgtag ttgaatttgt aagtgagagc atccatgaac tgaatgacgc 16260
ttcgaatctg gatagcagaa gtatccaaga gctggaactc ctcagccgcg tcgattgcag 16320
tggtcacgtt gacagactta gcgttgaaca ttttatatcc tccttattct tcagggacaa 16380
atacaccgcc gattacccgg ccagcgcgaa ttagtttacc agttgggaac tgagcacgca 16440
aggtcttttt gaaacgactt tgaatttgca cagtcagcca cttaccccgg tagttctcaa 16500
aattagtgat ctggtcttta cgggcttcaa agtcgccata cgtcacctca agcatctctt 16560
gaggtacagg gtcgaacacc accagaacag cgtttccatt cttgtcaacc acgtagttca 16620
cgatctgcat ttccatatcg aagaactctt tgtacttctg gagaccgctg gagcgcttac 16680
cagattcgta caagccctct gggctgcgcc acatgatacc ctcataaccg ttctgacggt 16740
acacgtcgtg aagacgtttg aggtcttcga aagtggagca gaatacgtac tcaacgaatc 16800
gaattttacc agccccgtca agagtcgcag acttgaactg gaagtctacc agacgctcct 16860
tgaatgtcaa gccttctttc acgatatcga atacgtgaaa ttccagtcgc tgccgcagtc 16920
cgtaaataaa cgatgtgcta tctacttcct taaggagccg atcatactcc tcctctccgt 16980
cttcaatagg ataggcgtcc agagctttct cggccttcat ccacttgtcg taagcatcct 17040
cccgcttaac cgcagagttg atatcttcca acacctcccc atggacgtac agttcgccgt 17100
cccacacatc accctcttgc atgatctctt cgagttcttt gatgatgtgc ggtacgttgt 17160
aaggcttgcc catacgagac gctagcgtca ccttgccgcc gaccttctta gccatgcatc 17220
gcaggccgtc tagtttaggg gagccatacc cctctttgta tttagcggga tcaagcggtt 17280
ttgcgagcat agccataaca gggagatcgt tgagttcttc cttggttgca cgataaccct 17340
tgtccaactg cttgctcacc ttggactgag cctcagacac ggcctgctcc tctacagagc 17400
gtttctgctt gcccgcacgg atgttggtaa cctgagaggt catggctcca tgctcttttc 17460
cgtatttcac gacgatattg aagctaccat cctcctcccc gatcacctct acagtccact 17520
ccttgaaccc gcccttcttg tcgagtccgt acagtcgttg aaaataactc attgatcctc 17580
ctcgtatttg atgacaagaa catcattctc ccgtaggtgc ctaaggcctt cgtcgagccg 17640
atattcgtgc ttatagtaca ctactttgat cccagcttca accaccgaga tagcacaatt 17700
tttacagcaa gagtgagtga cgaacagggt agccccttgg gcactctcag ggctgcgtat 17760
aagggccctc agtgcgtttt cttcggcatg cctcacctct gccttggtta ctcccgtttc 17820
gtcctccagc ggcccgttta ggtgctctgg aagggcatta tacccgcaac tgatgatacg 17880
gttacgtttt actagaactg caccaacctt ggaacgagtc gagtcgctgc acttagcgaa 17940
ggcctcagcg gcctccatgt aagctttaat atgcttaacc ttcacaccta gcctccgccg 18000

acgaggacag caatctcctc gtactcttca gtggatgcga actccatgta atagtcgtcc 18060
gtatctgaca ggtaaacaac gataggcgga taataactcc atacagggtc tccgtgttcg 18120
atttcagcta ccctcacatt gccatacact gtgcgaagag tctgtagagc ttcgatcaca 18180
tcgtcaatac gcattacagt tggttcagtc atacctgcct cctcagtttt ctactttaca 18240
aacggcatcg ccgcgtcgaa ggaacacttg cactgacggc tttccgctcc acttgtccca 18300
cgcttcagct ttaacactga cagtcttagg attgatggca accacctcgc ctaccagcat 18360
aaccattcca gttccgccta ggcagaatgc tactgcgtcg ccgatctcga tatcccttcc 18420
tgctacgtcc ttcatttgta ctctccatct gcacccttga ttaaaataac gaagtccgag 18480
gggtcatcac tgccgtaggc ctttgcccga aactcctgct cgtatttatt caccgcctcc 18540
ggatcacttt taacctcaag ctcttggtag aggtcttcca taaaccaacc caagtccgtg 18600
gtcgcagccg aagccgagag gtgaccaaga agtcctacca gcatattgat ctcgtcgctc 18660
tccaactcca acttgtgacc gttacgtttt actactttca tatttcctcc tatcgcttct 18720
gtccgcgacg agtggctacg ccgtaagctg gcttgctccc ctcgcccgtg ttgtgtttct 18780
tctgcactcg gctggcattg acggtgtaac gtcctttgcc atactcttcg tctatccacg 18840
cttgagcctt agctcgctga gacgtgtgga caaagatgta gtagccagat gcatccttga 18900
tgaaccaagt ggccgggggc tggaagtcca gacgtgaaaa ctcttcatag tcaatcacgg 18960
tcaccttctg cttagccact gtatactgtc ccactaccga ctcttttgca atgattttat 19020
caatcatctg agttgaaaga tcagggttct tcgctgtcaa tttctacctc ctcccctagg 19080
ccatcatctg caaagaaact gtcaaggttg agccagtggt attgagacac tttgtaacca 19140
ccgctcttca gaaggctacg agcggcctcc tctactttgt cctctgtcag cctccagtcc 19200
gcccctagga gtacttcagc taactgctct acactcggag ggttcccaat ccacagaccc 19260
attctattgt caggctcgta atcttcttcc catacaagtt cccacactag gttgctcatt 19320
gatacacctc cttcttacgg atagaataca taacccactc gtcttttcct gtcttcccgc 19380
acggaatgtc tacttcacac tctgcattta caatcaggcc tgctacgatg caatcaagaa 19440
gctcctcgtc tggctctggt agtcccgggt aggacgatac aaccctgcgg atagtctccg 19500
ctttggtatt gcccagtacc ccaagcatgt ggaggtcgaa ctctccactg tagtcgtccc 19560
agagttgtcc aaatatcacc catacactat tcatacctag taatcctcct tcttcttgtc 19620
cagcaactta aggcatcgtt tgcaggtgat atgattgcgg tagcgggaga agttcttctc 19680
ctcgaagtcg ccatcggtgc cacagaagac gtcatatggt tcttcactgt ccgcttctgt 19740
gccgccgtcc cactcgtcgt agtgaactgt tcgcgtcgcc attcacttca cctcgattcc 19800
ggcttgctgg agggcttctt ttgccttctc tatggcgtcg ttgaacgcgt gacattcatg 19860
cttggttacg tacatgctgt atgagcgctt tgcaggcaac tccaccatca gagccgcgcg 19920
gctggcttgc caagcgtcaa atgcgactgc gcgctttgat ttaagaactt cggcggacct 19980
ccaccacgct tcaaactcgt ctctcatact ccgcaatcct cctgaatctc ttttagcagc 20040

tcggaaggca taaccgacag cgccagttcc tcaagagcgt ggatgtaatc ctcgacatat 20100
ttccattctt ttctgatctt ctgaaggtac acgccttctt tgctgttgtc aataacctga 20160
ccttcaagct cctgcatcag ttcgaacatt ttgtatatcc tcctctagcc gcaaaaatgg 20220
ccggcgacta ctgtcccggc ctgatgatac tactctgaaa tttcttccat tgcaaggcgt 20280
ttcttttcaa agaaatcgct gagcttgtct tctagctctt ctccgccaat gtaaagctct 20340
tcccctgcct tgacacgctc cagctccttc tcagtcaaga agccactgta gacgtgatct 20400
acccatttat cacaccacgc cgagctgaac gctgcatttc gcaggctatt cctgaaatca 20460
gcagatacaa cgccaaaaga aggcgcgtgg atcatgaaat atgtgaactg gtcgatctca 20520
acggagtcac aggctagcgc gatagcagtc atggcagacg cacactcacc ttcaagacgt 20580
gcatgcactg gagcttgaca ctctttgatc gctcggatta tagcgttggc ggtctctacc 20640
tgtccaccgc atccattgat ggtaatgtac acttcatcat tctgacctgc attttcaagg 20700
gtccaaagct cttctgtgag ttcttcgagg ctcttgatta cgtatggcag aaggatggag 20760
taacgattgc taacactttg tgtacatgaa acaggcataa tacctccttt acaaaaatgg 20820
gcccgaaagg gcccttatta ctgaatcact cctcgctttc tttctggcca gagcccagca 20880
ttgcaaagaa tggaaggagg attgtgctca gctctccgct gacagcttca ccaacttctg 20940
caccagcctg agctacactc aggaactctt cagtgcgtac aaacttgtca accttaacga 21000
gggttttaaa gtctactcgt tccaagaacg cagcgccggc tgcttccgcc agaggttcga 21060
ggtttacgac ttccatagcg cgatccagag cttcctgatt gatctctagg ccgttctgtg 21120
cggcaacctg catcagggta gacttcacag tctggattgc gtagtctttg aaaaactcac 21180
gttgtgcttt gttaaacttc attcactttt ctcctttgta gtacgacgac ggcgggtttt 21240
tggtttatct tccgattcag tctcttggac ttcctctaca ggaacttcct tagctacttc 21300
tgtagccttt tcctcagtct gcttctctgc accttctcgg accatcttga cagacttgag 21360
agcgaaattc atgttagcga agtgcgggtc ttccacatca attcggaagc cttggtcaaa 21420
ggcgttctgc atgcccatca gaagactgaa aacgtcccat tcttgcacaa tgacttcatt 21480
gccgtcaagc tctttttcga tttcctcaga aacgccctct tgacggaaca tctgtacttg 21540
acagagggtg ccgtacacag ggtagccttc gatggagcgg acgactcgga aaccttcctt 21600
catgtgggcg tgaatcgagc gaagccagac gatagggttg tattctacga tattgataac 21660
ttgcatattc tttttactcc ttgcgggtta gtgttttaca ataataccac gtttctcagg 21720
aatgtcaacc cattttgttc tggtagaggc ttgacataga ggaaatctgt ggtattatca 21780
atattcagcg tgggagagat atgtctgaaa gcactcaccg gcccacgcct agcgcctatg 21840
aagaggaatc cacaagggca acctgaactc gaatgctgtt aaagggtgga gacgacgcga 21900
aagcggaatg acagcaatcc gatgcagcct acatgctgcc cctcaccgaa ctcatctgca 21960
tgagtaagag gaattggata gagccaaggc acgtcttagg cagattaaat tctgtatggc 22020
gagccttggg acgactctat ccttccctag ccgaatctat ctgcatgggg tgttctcctg 22080
atacacatac acagaatacc caccacagat aatccatcaa tcaacccata aggaatttct 22140

atgagatgta agtgctgtga tagcgaactc tcgtttgaga ctccagtcat caacaaacat 22200
actggtaagg aggacgatct ctgctcctct tgccgtaggt ttgctctcaa tcctacactg 22260
gatcatgagt attttggtgg ttggtatcca agcgaaggag ttaccgctcc tctaccgacc 22320
ggggactgac attgtatata gagtgcgcgt actctgacaa tacatataaa taatagatta 22380
cggtgcgaat aattctttaa gggcttgaca acaggagatt tcgtgctata atcggagtat 22440
atggggctta caaagcttca gaggatttaa tatgactcaa gaaagaaaca agggcggtcg 22500
ccctacgaat gccgagctag aagcccgggg tatcattcaa acagacctaa aagcaggact 22560
cagacttcta aagaagagct ttgcagagaa cattaggttc ctacaagagc aatctgacaa 22620
tcctgaggtt tctctacaaa tgagaatcaa gctcaagaaa gagctttctg atatgtttgt 22680
cgcttactac aaagctgaca ttgcactcaa aaaagagctt gcaaaaggaa cagaatcagg 22740
agataatgag aacactgaag aggacaaggc tcccggagta gttctcgcgt tttgattaat 22800
tgccgcttcg ttcaatggta ggacgccaga ctttgaatct ggagatgatg gttcgatccc 22860
atcagtggct gccaaatgta gtaaaagaac attcacccga caaggaatgt gtctcgctca 22920
gaggacagcg agtaataagt gtcctccaga attaagggtc ggtgagcgta attggcaacc 22980
tactggattc caaatcctcg tattctctgt tcgaatcgga gtcggcccgc caaattatat 23040
ggaacttgaa tccttctggg accaagttcg cagccggaga gctaggctat gctgctagcg 23100
accgatgttc tttagagact cggtattctc tccaattggt gttgacagga gagtaaggct 23160
cctgtatagt gacacaaatt gctcgtatag tgtaatggat gcacaacggt cttctaagcc 23220
gtaaggtcta ggttcgagtc ctagtacgag cgccaaattt aagatggacg gaagctaaag 23280
tggataggca tctggctttt aaccagacta tagtgagttc gagtctcacc cgtccaacca 23340
aatttagtga gtaaatcaga taggttctga ggctccctgc taaggagtac gtctgctaac 23400
gcggatgtgg atcgagacca ctgctcactg ccaaaatcac tgccaaaata agtgatttta 23460
agtgttgaca aactgaattt ttcatgtttt aatacactta tcagatgaag aaacaattag 23520
cgagtagccc tgagtaggtc gggaactagt ctagaaaact aggccactgc gatgagcggt 23580
gagggttcaa ctcctttact cgctgccaaa ttaaggccct gtggtggaat tggtatacac 23640
atcagtctta gaaactgacg ccgagaggat tgagggttca agtccctccg gggccaccaa 23700
attaagaatc tcttttgcta tttggttagg gttcgagtcc ctacccggct actatggccg 23760
gtctatggcg gccaaatagt gagagagcta ggggacatga cgaaggtagc gctgctttcc 23820
gatctccccg gcgctataaa agttatcatg aaggggttga caaacaagaa attcatgata 23880
atataggtac atagagtaac aaacaatgta gccgggtagc tcaattggta gagcacccga 23940
ccgataatcg ggtggttgaa ggttcaagtc cttctctggc taccaaaatt taaaggccca 24000
ttagctcagt ctggactaga gcaagcccct gtctagggca aggtcgtcgg ttcgaatccg 24060
acatgggtcg ccaacaaggt cgttgtagag atagatggct actatcatcc ggctgtaacc 24120
cggacgccat aggctagagg ttcaattcct tccttcgacc accaaatagc gagagcgtta 24180

agactggctg gcgctgtcac tcggctcata accgaacaca agtggttcga ttccacctct 24240
cgctaccaaa ttgcactccg aggaacccta agtggtgggt tcagtgtcgc tacctatctc 24300
ggtaagggga agcgcctaag ctggcacggg taattgagta gctgtgataa gctactggca 24360
gccagtataa atcgtccggc tcgggcccgt tatagctcct agatttgaat tagatgctgt 24420
aagccatagg aggtgatcca gtatctcccg gaggggcccg ggttaacaac ctcatgaatt 24480
tagcccgcgt tggccgagag gtttaggcgg cggattgcaa atccgtctca catcggttca 24540
aatccgatac gcggctccaa agtttattgg gatgtagctc agtggtagag cagggagctg 24600
ttaactctca ggtcgcaggt tcgagccctg ccgtcccagc caatttacaa aagagactct 24660
tcgctttaga agcagagtcg aacgcccgtt acgggcctta ttcatctcct cgtagctcag 24720
tctggttaga gtgtctgatt tggaatcaga aggtcgaagg ttcaaatcct tccggggtga 24780
ccaattaaag cgggtatagc tcagttggta gagcgtctgc cttccaagca gttcgtcgtc 24840
ggttcaagtc cgtctatccg ctccaaacaa tgcatttgaa gctaactagg tagaagcgct 24900
gggttgaaat tccagaggac ttggatcgtt accaagcaga tgcaccaaat aaagcagtta 24960
tagattaatt ggttaaatcg ccagactttc aatctggtgt tccgggttcg atccccggta 25020
actgctccaa tcaagtccaa gccggaacat tcctccgtat gtgctttcag ccggcttggc 25080
attattcgtg tggagattgt gtagcccgga tgctgccttt cggttgtgac ccgattgttg 25140
gggttcgagt ccccgcctcc aaccaatgtc gtgcaatggt atatcgcgtt gacgctttta 25200
ggctctctgg atcgtaagcc agtaaagcga gaatagcggg ctctcgcagc agagcgatag 25260
gtggttcgat tccacaacat gacactcttt cctcctcgcc tgtgctgaac ggtttcctcc 25320
tcctttcccg tctcacaggc tctattccct gccctttaag catttatggt gatgcaccgg 25380
ctttgtaacc cggcgaattc tgttcaagtc aggaatgggg caccaattca ctatcaaaga 25440
acccgcaagc tcccgatagg gttcacactc tataaggttg tagcatggaa aacacgcagg 25500
ttcctgataa aaaactcgga ccaatcgcgc tagcgcttaa agaggccaga gagaatttcg 25560
atctcagccc tcatgttatt ggtccaaaat ctttcaaaca gaaagagttc acagacagcg 25620
atgcaaagat caccgtattc ggtggtgctg ctggtgctgg taagtcttat ctcggtgtaa 25680
tggacttcat gaaatatatc catgatccta acttccgagg tgtaatgact cgtcgtacca 25740
ctcctcagat taaaggcccg ggcggtctgc ttgaaaaagc aacagaccta ttcaagctcg 25800
ttgaccctaa agtcaagtgg aaagacaaag acggcaagtt tgtgttctct tctggtgcag 25860
ttattttcct tcgacacttc gaacaagaat ctgacaaaga ctccttccaa ggttgggagg 25920
ttagtaagtt cctagtcgac gaaggtcagc agttcacaga aggcatggtg acatacctta 25980
tctctcgtat gcgtaacccg aaatgctcgg ttgttccgca catgaagatt acatgtaacc 26040
ctgattacaa ttcgttcctt cgtaaatgga ttgaatggtg gctagaccct gacacaggaa 26100
tcccgatccc tgagcgctcc ggcgtaactc gctggtttgt acgcaaaggc ggtaagatgt 26160
tctggggaga caccaaggaa gagtgcattg cccttcatgg taatcccagt ctagcgcctg 26220
acgatgaaga tcaggtgaag cctatcagct tcaaattcat cgcggctaac tgtttcgata 26280

acccaatctt gatgctcaac gaccctgagt atgtagctaa ccttgaagcc cttcctaggg 26340
tagaacgtga aaagctccta gacggtagct ggcacgctag agaagaggga gctggatact 26400
tcacaaggga ctggtgccaa atcgttcccc atccgcctct acgggtgctt aaaagggtac 26460
gtgcatggga catcagcggt agcattgaat ctgaaactaa cagaaacccc gactggacag 26520
ccggcgtgct aatgagccgt acaaaagaag gcttgttcac tgtcgaggat gttgtcagag 26580
agcgtaggtt gttcggaggt gttttcgacc tcatcctaga aacagccaag caagacggtc 26640
aagacgttga aattcaaatc ccatgtgacc cgggtgctgc tggtaaagct tatgctgccc 26700
agctaattag agacctagcg gatcatggat tccacgccag accgaagaca actaacaaat 26760
cgaaagttac tcgattcgcc ccgttcgtat ctgtagctga aactaaatca gtgcaagtcg 26820
ttgcgggaga ttggaacgaa gattacttcg atgaacttga gcgattcgac ggaagtaagg 26880
ctatcaaaga tgaccaagtt gacgctacat cagatgcatt taatgctcta tcccttggca 26940
gcttgattat ccctgatttc ctaccgcctg atatgactca atccaacaaa ttcaaactaa 27000
attaatagga ggcggcgatg gccgagactg aaaagacagc accgggtatc ccgcgacttc 27060
gcctaggcga gattggtagt acaggcctta aacaagtcaa tggtactatt ctggaagaac 27120
gccgaccaga acttcggttc cctagggcat gcagaacatt ccaaatgatg gcagaagacc 27180
ctaccatcaa atcagctctt gatctcttcg aaatgatgat gagcagggtt gactgggaag 27240
ttgatctagg agttgacccc gacgaggcta tgaaggcccg tggtaaattc ctgaaagagt 27300
gcatgcatga catggagcac agttggtact ccttcatcaa agaggttact agcttctaca 27360
cctacggatt ctccgtacat gaaatggtgt ttaagcgacg tgaaggatac cctgtatcga 27420
agtacaatga caacaaattc ggtattaaaa agttgccaat ccgctctcag tctactatca 27480
ccaaatggtt gtactctgaa gacggtagaa acttcctagg gtgtgagcaa agtcttgcca 27540
atgttgtcaa tggcgaccgt tacgtcaacc taggtaataa ggacggaact gtagagattc 27600
cagctaagaa gatgcttctt ttccgggtcg atgctaaacg tgacagtcct gaaggtaaca 27660
gccctctccg tgcgtgctat aacgcttgga gatatcgagt agagattgaa gagcaagagt 27720
ccgttggcgt aacacgcgat atgaacggta tgcctacgct ctatctgcct cctcgatata 27780
tgtccgaaga tgctacagaa gctgagagtg cagtctacga atactacaag cgagtaattc 27840
gtaacattca aatgaatgaa caatctggac ttatccttcc tcaagcgttt gacccagaaa 27900
gccgacaacc tctgttcaaa ttcgaactga cctcttcgca gggcagtaag atgtatgata 27960
cagatgccat cattcgtcgc tgggacaaca aaattctgca agccttgttt gccgatatgc 28020
tgaagatggg tcaggaccaa gttggctctt actctctagc tggtgccaag actaacatca 28080
tggctatggc gattgaatcg cggctccgcg aaatcaaaga cgtactagat aataaactca 28140
ttcctacgct gttcgcactg aacggtgatt atagtccaga cttgccgaag ctgcaatacg 28200
gtgagctaga tgaaatcgat ctagaagagt tctcgaaagg gattcagaga atcggttctg 28260
tcggtggtct tgaacgtgac cgtgaggttt acaacaagat tcgcaaagct ctcaagatta 28320

agcctcgacc agatgacgaa cctgttgatg tggataacat catgggaggt cagtctcaag 28380
caggtaaagc aggactaggt aatggtgcat caacatccgc agccggcaga gacaatgctg 28440
ctgcaaataa cgcataagga gacagaatgc tgactcttga aagtgttatt gaagctctca 28500
gcaatgcttt cggggctccc gctaaggcgg agcaagcccc ggctgtagag gtcacaaagt 28560
ccctagataa tgagaagagg atggctttgt ttgtcgttct tgagccccaa gagggcgact 28620
ccacgaccga cctccacgca gacacatact ccgaagagga agtagagaaa gcttgcatca 28680
acttcaatac tcattgcaac gtagctaaca tcttccataa gattgaaaca caggaagctg 28740
agattgtcca gtccttcatc gctccatccg cattcacaac tgacgacgga agagagatca 28800
agaaaggtac atggcttcag tggtggcatt tcccagaggg ctctgaagta gccgatacac 28860
tgtgggaagg cgttaaatct ggcgaattca caggcgtctc tatcggtgcc agagctacag 28920
tcgaggatat cgaatgaccg taaaagcaaa acgtaaactg accgatatca agtttgaaca 28980
cgaaggtgct cacgtcgctc tggtcagtaa gcttcaaggt ggtgcagcca acgggcacac 29040
tactctaatc acaaaagcta ccaatggcgt aactgaagag cttatcgata aagctgctga 29100
agttacagtc acaatgcaat tccctgaatt tcttcgtaag ttcttcggga tgtattatga 29160
cgatgccgaa gttctctctg ttgccatggg atatggcaga acggagtatc cagaactaga 29220
tcacaaagac tggatcgacc agcgggtcga gtctatgtcc attcttaagt cggtttataa 29280
atcggctgac ctgaatcaag ctctctccga gcttacccct gagcagaccc tttcccttaa 29340
acaggatcag gaactgcttg aaaaggctct ggaggtagtc tccgagcaat ccaatgaaaa 29400
tccatccaag gagattacca acatggaaac tatcctgaaa tcggcccacc tagaagctct 29460
agctgacgct gttgcagttg aaaaagctgc tggtgccgct gctgttgcag aagttcagaa 29520
gcaactagac gccgctaatg tagagcttac agctcttcgt gaaattgtca aggctgctga 29580
ggcagagaaa accgctgcta tcaccaaagc ccgtaaagag caactggctg aagtcctccc 29640
ggctgaccaa gtagaagata ctttcaagtc tctggaagtt ctggacgatc aagcttttgc 29700
acaaacagta ggaacgctcc gcattcttaa agctgctgct gaccaaaatg acatgcttgt 29760
cgagaaaggt gtatcgggcg ctggggaaga agacgactct gttgtcgccg gtgttgccgc 29820
tatcctgaaa gctcgccacg gctccaagta atatctagcc aatctagagg aattccaaat 29880
ggctcaaaac attatcgcaa aagatcatca gcgtcttagc aactggctga aagaagaaca 29940
aatgggccac cgtggcctgt tctacactcg cgaaactctg cctgtcgctg acattgatgt 30000
taaaacaact ggttctgtcc tagacagcac tggcaagctg gtaactaagg ctactatcgc 30060
cgacgctacc tacatcctaa tgaccgacct gcacgactat gccaacgctc agatgagcca 30120
cgctgtcgtc cttgctcgcg gttttgccaa gatcggctcc aaggctgtta tcttcggtgc 30180
tgacgttgac gacgctgata aagctaccgt ctttgaagct ttcaaagcca agaacatctt 30240
cgccgttgac cagatcgaag gcgcattcga gaacgttacc ttcgcgtaag cgggggtcgt 30300
tccagtaaat aaacactagg agtaacttta aaaatggcta acacccgttc ctacctaaac 30360
gatggtcaat tctacattgc cgaccagact gaaaacctgc ttatcatccc gaacacttgg 30420

accctcgttg agaacatggg cgtcttcaca agtgaaggtg tgactcagaa cactgtccag 30480
ttcgaagaga tcgaaactcg ctacggtctg gttaaggatg ccatccgtgg aactcgtcac 30540
caagttgcta gcgaccaacg tcgtcaactg cgtgccttcg cgattccgca cttcaaccaa 30600
gacgactaca tcactccaga agacattcaa ggcaagcgcg ctttcggtgc tgaccgtgaa 30660
gaaactctga acgaggttcg tgctcgtaag ctggaaacca tccgtcgcaa ctgggccaac 30720
actgctgaag ttgcttccgt tagcgctatc gtaactggta agagctacgc cccagccgga 30780
accatcgaat acgactggta cgacctgatg ggtaaaactc gtaaggttgt cggcttcgac 30840
ctgaccaacc ctaccgctga cgttatgggc aagaccgaag aaatcttcgt ccatatgcaa 30900
gacaacagcc aagacggtct gatccgtggc gacttcgttg ccctgtgctc tcctgagttc 30960
ttcactgccc tgattaacca tccgagcatc aaagagttct acaaagctta ccaagctagt 31020
cctcaatact ggcgtgaacg tctaaccgct cgcggtctgg accttcgctt ccgtgagttc 31080
tacttcggaa acattcactt catcgaatac cgtggcgttg acccgtacgg caaccgtctg 31140
atcccggctg gcgatgctta cttcatccca accgacagcg gtgacctgtt cgctcgttac 31200
ttcggcccgg gcagcacttt cgatgaccta ggaactctgg gtaaagaact gtacgctacc 31260
gagcgtatgg ctgaagaccg tcgttcgatc ctgatcgaaa ccgagtccaa cttcatccac 31320
gttctgcgtc gtccacagat gatcgttcgc ggcactgtga atgcgtaaag acttcaggga 31380
ggcttcggcc tccctttcgt cgtaggaggt accaaaatgg cttacactgg tgatccagca 31440
aacaacccta tcgaccgtct acgagagatt gtaggggacg tgtgggagcc tccgatgctt 31500
tccgacgaaa cctatcaatg ggtgttggac aagaacgaag gcaacgaaag acgagccgca 31560
cttgaactta tgcgtatgat gctcttccgc ctgacaaggg gaatgagaga acgcacaggt 31620
gatatcgaag tatatggtgc tgagtatttc aacaactact tgaaagctct tcagctcatt 31680
ctcaaagacc cgaatattgc aatttctttg gccgtgccat atgctggcgg tatctccaaa 31740
tcggatatgc tggctaatga cctagacccc gacaacgtaa cacgggaatt ctacatcggt 31800
tttgcaagac gggagaagct ttacaaccag tgcaaccccg gtcctcaaga ccttagcatg 31860
gggtgtgatt atcttggcaa gcttcagttt taaaacagat cgccgtcgcc tcacatcctt 31920
gattaagagg gtggaggctc tagacggaac aacggtagaa gttggtttct tccccgagga 31980
ccgatatggt tctgaaaatg gaaacctacc agttgcacaa gttgcagctt acaacgaatt 32040
cggtactact cgcaacccaa cccggccatt catggctcca acctttgaag aattcacatc 32100
tcaattccac tacgcaagac taatgaagtc cacgttcgag aacgtcctac gcgatggaag 32160
gcagacgaat actcttctga agaaattggg taaaatggtt gcagagcaga tgcaagttaa 32220
cattgacgac tatccgggct ctaacagtcc ggcatgggca gcttacaaag gatttaacga 32280
tcctctattc catactggaa agatgcttga gtctgtcaag ttccaaatcc atcggaggca 32340
ataatgctct atcctacctt ctccatgacc aatttcgtca agttagactt gattagaaga 32400
ggacagcccg gagacgacgg ttttggaaga cctactcctc cagttgagac agtagtaaca 32460

atcacagcta acgttcaacc tatcgagaaa tcgacagaca ctcgtatcct tccagaagcc 32520
gacagaagta aagcttgctt caaggtgtat tccagaggcg aagagattcg acagctaaag 32580
gaaggcccgg gagggtggag tgctgatagg tttatctggg aaggtgagct gtacgaggtg 32640
atgaaagtca tcaactattc gatgggaatc ctaaaccatt acaaagcgat ttgtatgaga 32700
gtagagagga atagcacagc atgatccaca cagagctaga aaactccctc tatcttatga 32760
tcaaagagtt gttcccggat tggcgtgtaa ttcaagctta cacgaataat caggaaccac 32820
aaacccctta tctggctatc gacattaaga ggctggatga actgggaagg gaaaatgtct 32880
ccaatatgag cgatcccatt agtccttcgc atggaactat tcaagttcaa caggacttcg 32940
cagccaaggt cgtattcgaa ctgattggca agtatggaga gacagcaagt gtctctgaca 33000
tggcaatggc gattactcgg gcaatgcgta ctccagcagg acacgctgct caaagaaagt 33060
tcaatctatc ccttttcaaa ctccctagca caagacgagt tcctatgcta agagagacag 33120
atatgtacat gttctatcaa gtgacatgtg aatttggatt ctctgtgata gagaccacaa 33180
ctcaagagtt tgctgctggg gctgatattc acggcgttta ttatgacgct ggtcgcccgg 33240
gtcacattat tgagtcccac atcgacataa acttcgaaca ctaataggag acaggaatga 33300
ctgttcttac tgatgtaatt gacattcaga tcagccgtga gactgctgct gtcgcacaga 33360
caaacttcaa cgttcctctg ttcatcgctt ctcacacaaa cttctcggaa cgtgccagag 33420
tctacaacag cctgaaaggt gtcgctgaag acttcggaga atccgatcca acatatcttg 33480
ccgctgttcg ctacttcggt caagctctca agcctcgcag cctagttatt ggacgcagac 33540
aagttccttc tgccaccgtt tctgtcagtg ttgttcagga aggacagagc tacgttctga 33600
ccgtcaatgg cctccctgtc agctatgtta gtcagccagc cgacaccgct acactgattg 33660
caactggcct aaaagctgca tatgatgtta cccctgttgt tggcgttacc gtaactgaca 33720
acgaagacgg aactctgact gtcgcttcga acggagactg gagcctgaag gtttcttcta 33780
acctgactat ggctgctgct ccttctactg aaggttggcc ggctgctatc acagcagttc 33840
aaggagaaaa cgacgaatgg tatgctctga gcattgacag ccacgccgat gatgatatca 33900
tggcagtagc tactcatatc gagggcacca agaaagtgtt tatcggagcc actgctcagg 33960
ctaacaccaa gacttccgca gagaatgaca ttgcttctcg tctggttgct gccgggttcc 34020
aacgtacggc tctaatttac catccaaacg ctgacgctca attccctgag tgtgcttggg 34080
tagggtacca actgcaagag caaccgggta gcaacacatg gacccataaa gctctggccg 34140
ctgtagacgc ctaccgtctg actccaaccg agtctaccaa cctgaagaac aagaacgtta 34200
ccacgttcga gcgtgttggt ggtgtcaacc gtacattcgg tggggctatg gccggtggtg 34260
aatggattga cgtaatgatc ttcgtagatt ggctggaagc ccgcatgacc gagcgtctgt 34320
ggttccgcat ggccaactcc aagaaaatcc cttacgacgc tgttggtgca acaattctgg 34380
aatcggaaat cagagcccag cttaacgaag gaattcgtgt tggcggccta gctgaagctc 34440
cggctcctaa agtcttcgtc ccagacgttc tctctatgtc tccgaacatg agagcccaac 34500
gtatcttcga aggcatcgag tttgaagccc ggctggccgg tgccattcac ttcgtacaca 34560

ttcgtggaac tgtgaccgtc taaagacggt ccttcccata aggagactca taaatggctg 34620
ttcaacgtct agctacattc tctccagccg atgttaccat tgtcatcact caccctgcaa 34680
ctggtgagag catggttctc ggcggattca gcgaagacag tatcgttaac attgaaagaa 34740
acgctgacac ttacgtgatg tataccggag ccgacaacac atcgactcgc gtctacaacg 34800
cctctaagtc tgctactcta actgttagcc ttcaacaaac ttcgccttcg aacgacttcc 34860
tgactgctct ttacaactac gacgacgccc gtcgttcgtc tgagggattg ttcacaatcc 34920
acgttaaaga caactctggt cgttccgact acttcagtga tgacgcctat gtcggtgttg 34980
ttccgggttc taacttctcg aacagcatgc agacacgcga ttgggttatt catgcacaca 35040
acctgcaaac tctaatcggc ggtaacgcca agctgagccc gggtgatgcg gatactcttc 35100
gtaacctagg tgtcacacta gaccagcgct ggctgtaata tagcgcttat gccacatcga 35160
cggggagctg ccttaacggg tggctccctt tgttgtttct ggaggataga aattgattac 35220
cacctactcg ccgcgtgacg tggttgtaac tctcgctggt atccattccg tgacagggta 35280
cgcagaggga gagtttatca gaattgtaaa agatattaag cctttcatca agcacggatc 35340
gatggacgga gaaatcgccc gggtctacaa caaggaccaa ggctggcgag tagagttgac 35400
aatcatgcag tcctcgccaa ctaacgacat tctctccatg ctatacaacg tggacattgc 35460
aacccgaatg ggaaaattcc cactcatgat taaggacaca aaaggctcca ctagcttcct 35520
cgctctcact gcgtggattg aagatttgcc acgggtgtct ttctctggac aactggagac 35580
ccgcacttgg attctagggt gctctgaggt ggctatgaac atcggcggaa acgtagacca 35640
aagtctcgtc gagcaagcga ttctgcttgg cagctcgctc cttcctgccc tgactcaatt 35700
tggaggattc taatatggcc aatacggtat tgacatatag cccagcggat gttaaaattg 35760
tcctttgtgg ctatgctcta actggcgttg tttcgtttga aatgacgtgg ctgtccaagc 35820
cttacacgct ggtgcgagga atcagagggc accataccag agtcttcaac aaagacctag 35880
cagctcagct acgactggag cttctacaga cctcggtatc taacgacgct ttcttctctc 35940
tggtagagca agacagaagg actcaatcgt ctcgtattac cctgtccgtg aaggatacaa 36000
ggggtaacac tatgatgagc acagacaacg cctacgtcaa cggataccca aacataacgt 36060
tcactgacgg aattcagaac cgggtctgga ctatcgatgt tctagactgg acagatggta 36120
ctgtcggtgg aaaccagcaa gtaggcttcg atgtatttgg aacagtccaa ggagccctaa 36180
gctacctacg ataacagaga taaggagtac acatgttcgg aaatcaacgt actgtcatcc 36240
acaatgggac agaatacact atcaacctgc ttcctgcaac acgcgggatc agtgttctta 36300
agcaactgac cagcctgctc ggtccgtctc ttgcaaaata ccaagctgac catgactttg 36360
caggagccat gaaggaactg ttcgaaaaca tcgacaaggt tcctgtagaa aaccttatcc 36420
agaacctgat gcttaccgtg ttcaaaggtt ccatggctat taacttcgac aatgagttcg 36480
ccggcaagta cgatcttctg tacgctctaa ccaaggacgt tatcgagttc aacttcggct 36540
cggttttttc tctaatcggt ttcgcagagg caccgacagc ataaaggctg gggatgggca 36600

atccgagact tcagtcacta ctgaccgagt tcagagtgat cggagggtta tggaggccca 36660
ccagaagttc tcgcagcatt gggagattta tgtggccacc actcaccctc tcaagcttac 36720
aacgtatgaa gcactgaata cagtgcttac aacagaagac ctatataata ttctagagat 36780
cattgacgtt cacgatacga tcactcaggt tgcaaaagag cacgcagaga aggataagaa 36840
atgataacag aaaagattgc ccagctaaca ggggaactta aattcacagt cgactctcgt 36900
cctctcactg ctttcgataa gaaacttgca ggggttgaat cgagactaag agagttctct 36960
aagctaacaa acaagaagtt tggtgtcaag cttacgctag ataccaaggc tctaagagaa 37020
gagctggcta aagcagcaac gcaacgagtc gtactgaaaa acatcgcagt cgactctgcg 37080
gctgtaaggc ttatctctga gaaacttcaa gagcgcctaa atgctacgcc tatcagactg 37140
aagtctgttc gtcttgacct cagcggtatc cgagaccaga aaaactttgt caagactgct 37200
ctaggccaga ccaaagtaga cctacctgtc gagctaggac ttgctcaggc tactcgcact 37260
ctccatgaat ggaagaagag aactgagagc cggtttaaga ttcacatcaa cgccgatatt 37320
agccgctcta aattgctcca caatgcacga aacactctga gggatgtaca gggtaggctg 37380
aatggacttt cggttgctac gcctcaaatt aggctgtctg tggaccgtgc ccacctacgc 37440
agagagattc aagacgttct tgagcaaatc agacgcgaag ttagaatccg cgtagaccta 37500
gactctacag tccgaggccc taccggtaca agaggtggcg ctgggcatgt gaggcaaggt 37560
gtcggaatgg gaatcggagc tgaaatggca ggctggggtc gaggattcat tcccgggctc 37620
ggtggtgcat tcgccatcat gcagcttaac cgtgcaaacc aagaacttca aggtcagcgg 37680
ctagcgatgc aagctgtcgg aggcggagta caaggtggcc aagaactgca agctacccta 37740
cgtgatatct ctcaacgcct aggtctcgac gacagagcac tcggcagctc ctatgttaag 37800
atgatggctg ctggacaggc atcggatttc agcaaggaac aagttgacag catcttccag 37860
tctatggctg aatatggtcg agtgatgggc cttgacggcg aagcgatgaa aggctctttc 37920
cgagcggttg agcagatgat gggtaaaggg cagattatgt ccgaagaatt gaaaggtcag 37980
ttggccgaaa gattccccgc tgctgtagct ctgatggcca agtctcaaga catgactatc 38040
gcggaactga tgaaaacgat ggaagacggg aagcttaaaa gtgatgccct gattccattt 38100
gccagaactc tcgctgaaga agcccgtaaa ggtggcgctt tggatgctgc tatgcaaggc 38160
accgcagctc agcaaggtcg attccagttc ggatggaacc gcactatcga agcattcgcc 38220
gctggcggat tcgatagagg tatgagtgac ttcttcaaga tcgcctctga cggcatgaaa 38280
gaagcgcttc ctctggtgac tgcacttggt ggcgggttcg aagctcttat gagacctgtc 38340
aacgctctgg tcggtatcgg aggggagctt ggttcccagt gggagaacat tgccaagcag 38400
ttcaacatga ccggcacagg gctgactctt accacggctc aaaccctagc cttgctgacc 38460
cctatgggca ggttgatctc tgctatcagc tggggcgctc tagttctcga agacttcatc 38520
gtcttcctag aaggcgggga ctctgtattc ggagacttca taaacaacaa cgtcgaagct 38580
gctgaagctt tcgagaagat ggctctagcg tctggtgaac taaaaaccaa tctagacgga 38640
atcttcgctg ttatcccggg tctagctgat gccctgaagg gcctagagct taacgaaatg 38700

ctggttagca ctatgcggga actagcagct attatggagt tcttcaacgg cattgtagaa 38760
cgttttgcta ttgctgacaa gtataaaaat gctaagattg cagaggcagg cggtgaccga 38820
agcacaatca tgtcgaacat cgatacaatg tatgctatgg tcaacccgga ggcagccaag 38880
gacgaagccg ctttcattag cgacagactt ctagctcaga acctagacag cattgcttct 38940
gagactcata cacgtgcaaa acaatccttg acaccagatc agtttgggta tatgatgcaa 39000
cgtggccaag taagaagtga aatggaaggt gctctacaga agagcgctat cgatattagc 39060
ttcaatctga atgtgtctgg aatcgatgca cagggtaacg tcatgacgac tgaagcgcaa 39120
gagcgcgtac gagagatcgt aagcaacgtg ctggaagaag aaatctccag agcttcggca 39180
agttataagg aatcccaatg acaattgcta tcaagagaga taacggagac ctgatttggt 39240
tcgatgctgt cactgagttc ggaagacaat accgtggttc tgttagttcc agccctattg 39300
aaacgggagg gaagatcaca gaccacgtta caactgaaaa cccggtgttc acgctcactg 39360
ctatcgtatc cgatgcagac ttcaacctga acaggcctgt tatcaacgac aacgaggctc 39420
aaacttacaa gatcaacaat aaagagttcg tgaatactca gccggtcagt gtgccggctg 39480
ttatcacaac ttctagattt gatccgagcc gaattttccc ggaagttatt actcagttta 39540
ttcctcctga gattccgtct gctactgtcc tccctcaaaa gggtgataaa gtcgcttacg 39600
atattgagcg ccaactgatc gacatgcagc gtaacgctga ggtcttcagc ttgctggact 39660
ttagagacgg tatcatctgg gatcaaattg agaggtgcat ctttactgac ctgtcgttca 39720
gcgagaatgc tgaaacggga cagagtattc agcctagaat gactatcgag gccgtgacat 39780
tcacagacac tcgctacatt gaagttaggg tgaacaaggg aagaaagaca gccaagaagg 39840
aaaagagaga caccaaagac ggtgatacag gggctagcaa cgctactgga caagactctc 39900
ccacattcaa gcgtagccag atgaaagagg ctcaaattag aactcaagtt gcgaggtgat 39960
taatgagcac gacttacatc gacacacttc ctttgtacca agataggaag tatagatacg 40020
cggtagctct ggagggcatt tcccgtgtcc tccaattcta ctggaacagc cgctctaggc 40080
agtggcacat ggacatattc gacgaggagc ttaatccaat cctaacaggg ttggcattgg 40140
ttccacaata cccgataatt gcagattacg ctatgcagca catcgggttt aatgggtact 40200
tcttgctcat gcctgtcaac ttagaacagg tgcagtacaa gcacgatgca agcgacattg 40260
tacctcagtt ttttgaactt ctgtatgtta atgttgattt ggaggaagat gcagagtgag 40320
acaatacgac agagtatata aattgacgct cggtaatact gagtctggtc agggagtgga 40380
gattacaaac cttcatcctg atgggagtgt taacagagag ggacttcagt tcaggttcga 40440
tatctctaaa agctccgata acaaaaagag cggcaactcg gctactgttg agatatacaa 40500
tctgtcgatt gcaactctaa accttcttga aaccgaatac ctcacttgca gactagaagt 40560
ggggtataaa gaagctggaa cttcagttgt tctagatggt aatgttgtcg aaacatccac 40620
aagaaagagt ggaaacgact acgtgactca gcttattctt ggtgagggtt acactgctct 40680
aactgagact aagcttaagg gcacagtctc tccgggtaag actgtaaaag acgttatcga 40740

agagattcgt ctacagatgc cgggagtcga tagaggagcc tacacaggcc tcaactgcaa 40800
taaccccatc atgtatggct accagctacg aggcctcgct aaagatgccc tgaatagcgt 40860
gtgcgaggct aacaatatcg agtggaacat aagcggcaac gtgttaaacg ttacagatgt 40920
gaacggccct acaacgaaga gcgttcagat ggcccctctg gttagcaaag agactggcct 40980
aattgatatc ccattctacg ccagtgccag cggcacgtct cagaagaaag acaagcgtcg 41040
cagaagaggg gtacagttca agtgcctcct gaacccagag ctgacccccg gcgttattgt 41100
cagggtggag tcggaaaggc tctctggcac gtttagaatc aacaacgtcc gtatttcagg 41160
cggatataga gacaacgaat ggtacaccga atgttggtgt gctgatctta accaagagga 41220
tatagaccaa tgaggagaac aggcctacag gagcttctaa acctccactc gtccacagaa 41280
ggctctaagc agtacacagc catcccttgc gtcgtcctgc gcgttctgga cgattttaga 41340
cgcctctctg tcgatgttcg tcccgttgtc aacgatctgt acaaggacgg aacatcggaa 41400
gaacaaccgg agattctgtc tgttcctgtt ataatgcccg ggacggctaa cactcttatc 41460
agcttcccat tgaacgtagg agataccgtc ctgtgcgttt tctctcaaag gactatcgac 41520
gttttcaaag ggagtgcaac cggtcagccg catacaccga acgatcttcg taagttcaac 41580
atggcggacg ccatcgctat tccggggctg ttcacattcc cccggtcgat gaatgacccg 41640
tctagacaca gttggccaca cgatacaaaa gacctgacaa tcgctcacaa cctcatgaca 41700
gggcaggaag cagaggtcag aatcaaagcc aatggagaca ttctaatcaa tagtcgcaag 41760
actataagca tcaatgctca gaatgtcaac gttaagtgtg cctctctgac ggtagatgca 41820
gcccatacaa catggaatgg caatattgcc catactggca attacgttca gacaggcggc 41880
acctcaacat tcaatggtat tccgttccat acgcacaaac acggcggtgt tatgccgggt 41940
ggcggtgtta ctgctgtacc acaggcataa ggagaaatca tggacctatt tgtcaacccg 42000
gacactcacg atctagtttt tataaacggg cagtgtccgg taactcagag gatggtggac 42060
attgttgctc aacgcctcaa gatcaaacta tatacgttcc tcggtgaatg gttcttggat 42120
gaccgcatcg gtctcccata cttcgagaga attctaggta aatcgagaag tcttccagca 42180
gttgacgcta tcttccaatc ggagattatg cgagacccgg gagttctcga aatcactagc 42240
tggaaatcgg gtattgaccc ccatacaaga gaatactcca tggagtttac agttagaaca 42300
actgacaaca ccgagtctct tccaatcaca ttccaaatgg taggagttta acatggctgg 42360
aattacggct gaaggtctaa cgatcaaaga cttggaccaa atcctgaccg actacaggaa 42420
cgtagcgtct caggtattcg cagacctagt ttccacagga gacgaggttg atacatcggg 42480
taactcggca cttggccgtc ttatcggtgt agttgctcca tcggacgagg caatctggga 42540
agtaatccag atggtttaca actcgttcaa cccctcggct gccactggtg tagctctgga 42600
caatctggta tccttctctg cgattagcag atatccggct cgccctacca gagcacaggt 42660
catcctagag ggtaacattg atacagttat caactcgcct ccggctaaga tgatctccag 42720
ttcgacaggg cgagttttcc acttgcttca aggtgtcatc cttagcccga agggctgctc 42780
tggtgtggga atcttccctc agaccgtggg gaataacctg acctatgaaa tcaagatgta 42840

tgttgacgat attaacacta catcgatcaa atacacaagc cccgcttccg ggactgtgac 42900
ctctgatagt attcttgctg ggctggccgc tgatgtagct gctaatcacg gaaatacctt 42960
gacaagctac gagcaaaatg gtatactttt catagtacca atcgatccgt tcaataccaa 43020
gaccttcgaa gaggtgagta acctcagtat tcagaaggtc agaaaacttg gtatcgcggt 43080
agacgacgta attggacctg tccctcagca ggctctagct atcgacacca tttctattcc 43140
catcgcagga tgggacagtg tagtcaaccc tgtgccggcc atcactggac gcatgaggga 43200
gaccgatgac gagcttcgcg agaggttccg aaactctaag tttgtccaag caacaaacat 43260
cctagagtcc cttatcgacg gactgctgaa cgttgaagga gtagaagatg tgcgtattat 43320
cgagaacgat acggacaatc cagaccccat tcacggcgtt cccgggcact ctttcatgcc 43380
aatcgtgcta ggcggtattc ctaccgaagt tgcacaatcg atttggctaa acaagccttt 43440
cgggatcggt tctgtaggag ataccgaagt tcaggttgtt gacagtctag ggtataccca 43500
cagggtgaat taccaacgtc ctactgaggt tccaatcgag atcaaagtca gcgttacgaa 43560
cacaggcgcg atgccagaga atatcgaagc tattcttcgc ccacgtattg tcgcatacgg 43620
gaccgagaac tacaagattg gggatgatgt aatttactcc cgattctacg ctcctatcat 43680
ggaggttccg ggattccaag tgaacagcct gacaatcgcg agaaagggcc aacctcaagg 43740
tatggcgaat atcgagattg ggtttaaaga agttgctaca ttcgctcagg cggatatcac 43800
agtgacccca gtataaggag ttatatcatg gctgtcaacc aatttgacag agaagattat 43860
ctggaggtgg cccgggagcg ggtcactgaa cagttcaaag agaagcctat cttcgaccgc 43920
ttcctacagg tgctgctatc tggcaagcta gatatccaga atgcactaga agacctccag 43980
actctacggt ctctggacac agccactggg aagcaactag atatcatcgg ggatattgta 44040
ggccgtccac gcggcctagt attccaagag attttcgaat acttcggatt tgccggtaca 44100
cagcgtgctg gctctttcgg aagcctttcc gaccccacag tcggaagtcc ttggtactct 44160
attggagctt ctactggtaa tgccagagag cctagcgatg aagagtaccg gatgatcctg 44220
aaagccaaga tcatcaagaa tagaacaaac tcaactccag agcaagttat cgaagcttac 44280
aaatttgtgt ttggggttcc agaagtattt ctagaagagt atgcgcccgc tgccgtacgt 44340
atcggtattg ggaagatttt gactaacgta gagcgtagtc ttctcttcga ccttggtggt 44400
gctggagctt tgcttcctaa aaccatcggg gtaaattaca cttacactga attccaagct 44460
ggccgggtat ttgctacaga aggcttcccc ggaggacaag gcgttggaga cctaaatgac 44520
cccactgttg gtggtattct gaccaaccta gtgacataag gagttataaa tggctgatat 44580
cggtcaacta cctattgaaa atatttggtc cacaggcggg gatatggtag ccccgacccc 44640
agcccagcaa caaggcggat ggggaattca atcggttcct cgtcaatggt ggaactggaa 44700
atggaacctg cacgatacca acctagctta tctgctacaa aagggtatcc cggagtggac 44760
tagtactcaa gagtacattg caaacaaatc gttctgcacc cgaggagggt ttgtttacaa 44820
agctgtacgt acacacacag gaagcgatcc agcccaagtt agtgcaaact gggcgagagc 44880

tttcgcagat tatacgacag ctagctctgc tcttggaagc ttgaccccaa gagagggtgg 44940
aatcccattc ttcctgagcg ctacaggtgc tggagtattt gactctacag cttatggccg 45000
tgacatgctg aacgtagcca acgcggcagg agctaggaac tacatcgcgg cccaagaaag 45060
ttctatcatt ctgtctaacc tagctgctgt aacgcgagct gctaactctg taccgtactt 45120
caatacggac acgtccatgg caacattcaa catcacagca tatggccgtg gtcttgtgaa 45180
tgctgctaac gcggaaaacg caagaagctt cctaggtcta gctaactctg ctatcatcac 45240
ggccgatccg gcgaacaggg ctcataccct cgtctaccgc gatgctgctg gaaacttcaa 45300
cgcaggtgtg attacggcta cgctttctgg taacgcgact acagctaata agcttagaac 45360
tcccgtaact attaacggtg ttccattcga cggaagttcg aatattgttc tccccggtct 45420
tgacacaagc tatgctggaa cagtagctag gctccacgtt aatggtgcca acatgactgg 45480
aacggacaaa acgactcaaa ttgctctgag aaacaaatcg gacaacgatt ggatcagtct 45540
tgcagtagtc gatgacaaca tccttcagtt tgttttcaga agtgcaacaa acccaatcgt 45600
gcagatcggt agtgaagttg tcctgcacac tggtaaccaa ttctcccttg gacctactct 45660
gagcgatgca cgcagccgac taggccttga taggctgtct caagggtctt ctgatacagt 45720
tgtgttctcc agtgttctga acaacggacc atatctaact gttcagccgt ctagcatcgg 45780
aggtttcgac ggtgctgcca ataactggat gttccgtttt gatgcgaatg gtaacctgac 45840
tcatggaact gttccggttg caaggattac agggctagcc aactctgcta caattcctgc 45900
cgtaaccact gctcaggcta actctcttgt ccagagagac ggaaatggaa gtttcagcgc 45960
tattggaatt aacgcctacg gagccatcat tgggtacggc tctaacatct acagccgtgc 46020
ttccgcatct ggaggcaacg ctcatatcgg attccaaagg gcagatggta ctgagctagg 46080
actcatctgg ggtattcctt ccagcggagc tatgagcttc cgcacatcgg gtggaacaac 46140
tggtatgacc ctgacagggc aagatttgct tgtcgccgga agagttaatg ctgcaacctt 46200
aaacgcctct ggtaacgtca acgctacagg gaacgttaac gctggttctg ctactctgaa 46260
cacagcaggt aacatcacag gggccgccta cggagcgtat ggttctctta ccaactgggt 46320
agattctgta tatgcgaaga agggagagat tcctaacgat atcgctagag caggcgcagc 46380
ttgggacgca gttggacagt acatcctagc tggcgatcaa tctggagggt ctggtggacc 46440
ggggacaact agagctggaa gccagttgaa gccttattcg actattagct acacagcagg 46500
ggcactacca gcagcaggca cttacagatg tatgggagcg tttgcaggtg gtggtaacca 46560
gattaccctg tggcagagaa tttcttaata gagggcttcg gccctccttt tagaggagat 46620
tatagtggaa ttcagaaatg ttgtaaaata caaaatgggg ttcgaatgcg aggtaaatca 46680
tcctcagtgg ggatggattc cgttctacgc ttgtgattac gacgatagag ccaaactggt 46740
atatgacgcc atcgcccgtg ctgtcgataa cggagaaatc cccgaaccaa aggacgttac 46800
agatagagac ctcgttgtag atgcacgtct gtggagagat aatgagctaa cccgggctga 46860
tcgtcaagta gaaattgcta tcgacgatga agacgaagta aaagagaagg cttggcgtaa 46920
gtaccgctct gcactacgta agtggcctga gcataaaaac ttcccagccg agaagagtaa 46980

accaaaagct ccgaaggagt gaggtaagat aatatgccaa acattatgaa gcctacggga 47040
atcaacgcta tctggtctga aaatggccag aaggttgacc cgggcgctgt gaaagtaggg 47100
ctgggatggg ttacggaact tcctccatac cagactgcaa acttcattga gtacaaacaa 47160
gacctgttta acgctcacgt caaccagcat ggtatccccg aatgggatag cgtgacagag 47220
tatcagggta acctgagcta cactcaaggc gctaacggga tcatttataa gtgccttaga 47280
acccattccg acaagattcc aaccgaccct ctcaacatca cagcaggata ctggcgggtg 47340
gcgttcgagg atgcagggga ggcggccaag gttcaagcta accttgatcg gcacgtgaca 47400
aactacaaca ccctctctgg gattggtaac gtagttattg caagacagaa cctgagtgtg 47460
tatagtaagg ccgaaggcga tgcacgttat gccatgcgac acggcaacgg gtctaacgtc 47520
ttcagcgtcg ctcccgcaac acagccaact cacgcgatcc cgctgagcca gctatcgact 47580
ctcgttcctc ctgcgaccga gactgtagcc ggggttatgg cagttgctac gactatcgag 47640
acagaagctg gagtcaatga taccaaggct gtgagtccgc tcaaggccgc acaggtgtat 47700
ctgaagaaga aagacaacct ttccggtctg tctaacgtca ctgccgcaag agctaatcta 47760
ggactgtcgg atacagcgac tatgccttct tctacattcc tgaaggcagg tagcaatctg 47820
gcagatgtgc ctaacaaggc gctggcaaga tcgaatcttg gaattacaag tagtgctacc 47880
cagccggaaa cctatttctt gaggagcgct cagaacctag cagatgtacc taacaaggca 47940
caagctagag tcaacctagg cctcactggg atggctacaa cagaccctgc ggcggttatg 48000
atgaaggcag acaaccttgc tggattggct aacactgcta ccgctcggtc caacctaggg 48060
ctaggcacag cttctactag aaatacagga gacttcctgt cgtctgggga taacctgtcc 48120
gacctcacga acgtccaagc agctagaaac aacctagggt tgaagggagc ggctacgctc 48180
gatgtatggg gtcttcctgc gaatacaact gctatggact tccagtctaa tcagtcggat 48240
atttccagag gatgggctcg actaccaaac ggccttctgc tacaatgggg aactggtccc 48300
ggtctgtctg atgacacaag gacgaatatc caactccctg ttccagcacg aatcttgaac 48360
gttcaagtga ccgtaatggg gacgtttaac aactccatcg gcccgggcgc tttcatgacg 48420
gatatgtgga gcaacaccgg gtttagagtt agctgcaact ggggcaactg gtcttacccg 48480
ttcaactggt ttgctatcac ttccaccctg taattcagga gtatcaaaaa tggctctaac 48540
tgagcaagac ttccaatcgg ctgccgatga tctcggagtc gatgttgcca gtgtaaaggc 48600
cgtcactaaa gtagagagtc gtgggagcgg ctttctactt tctggcgtcc ctaagattct 48660
attcgaaagg cactggatgt tcaagcttct caaaaggaag ctaggtcgtg accctgaaat 48720
aaacgacgtt tgcaacccta aagctggagg atacctcggc ggacaagcgg agcacgaacg 48780
tctagataaa gcagtcaaaa tggatagaga ctgcgcactt caaagtgcct cttggggcct 48840
attccagatt atgggattcc attgggaggc actaggttat gcgagtgttc aggcatttgt 48900
caatgcccag tacgctagcg agggatcgca actaaacact tttgtgcgct tcatcaagac 48960
caacccggca attcacaaag ctttaaagtc taaggactgg gcagaattcg caagaaggta 49020

taacgggccg gattacaaga aaaacaacta cgatgttaag ctagcagaag cctatcaatc 49080
cttcaagtaa atctaggaag gttcctaatg aagaagctat tcaaaagctg gaagttctgg 49140
gctgctgtag gctctgcaac ttttatggct ctggaagcca tggttcatct ttgggagcct 49200
atccttcctc ccggcgtgtt cgccgcagta gccacactgg tagctgttgg tgtgagaatt 49260
tcggcagtgt ttgtaactgc tggtaaagtg gaggaagcga tttcggaaac catggaacat 49320
gacaccatag aagatggctc aatagaaaat gatactaact aaactcaaaa actacgccct 49380
cgtagtagct gccgcaatgg cagcagggtt tggatatctg tcgttctctt tgtacggcga 49440
cttagctgtt gcgaagaatg atatccaatc tcttcaagct gcgtctcgac agatggaaga 49500
gcagttccag aaagagcgag cagggtgtga gatcaccact gggagactag aggcacatat 49560
agcccaaatg aagagccgac aggagcttct acaaggccat ctgtgtgagc ttgagtctct 49620
acccaaccca accccaaggg agaccaaaga aaatgccaca gagacgatct ctgagccttc 49680
tactggggat agtctggacc ctgctcttac aagcttgctc gacagggcct tctgttccgc 49740
agtaccaaac gatccttatt gtgcccccgg caagcctgac cgaggaggtt aaggtatacc 49800
gagtggagca agatacagtc aaggggctcg ctagagctta cattctaaat actaacagtg 49860
ctgccatgtg caatagtcaa ctacagacga taaagctatg ggcagaagag cagagggctc 49920
tgtatggaaa gtaaagacgg caaagaaggc ctaatcaagg tagctacaca gggagttatc 49980
tctctgctaa ttgctatcct aatggcttgg tctggattcc taagttccag tctttctaag 50040
caggaagaac gtatctttgc gatgcaaaga gatatggtat ccgaggcaaa acttaacgca 50100
ctagagtcga gattcaatag ccgacttgaa agtgaaatta gtaacgtaag aagagaacaa 50160
caagaaacca accggtattt gattttgatt atgaatgccg tcaagggtcg ggatacagca 50220
actcaataac tacaacccgt taacccaact gggagacaat ataatgacaa ggaaacaaga 50280
ctggccgcta gtttcagcag tcggggcttt gtttatctcc ctccttgtct gtatggctat 50340
tctggctttc acagcgatga ctcccgaggc tcggtttgac tctgtacgcc agacctacga 50400
gattcgaatt agcgaatctg agcggcggta tgaactcaaa ctgaacagac tacaggatca 50460
gataaactcg gcctcgtaca cacaggatcg gcaatcccgt ttgcaatatg aagaactcga 50520
ctcgcttaaa aggaagatta gcgaacttga gcggagaata aaagaaaaac aagaataatt 50580
aagccccggg agctttcgct cccggggctc tttgttactg ccgggttact tcccacactt 50640
cgtcaaacag cttgtcaatt ttcgagataa tcttgtttac ctttttctta cgacgctcat 50700
tatcttctgg atcggaatgg ataagctcaa tgtcggcagc gttaccccag tccagctccc 50760
cgtaatggaa cagcaccttg acgttcaggc aggtcgctga tctgtcaacg cctgcgacaa 50820
ccccggtgga gcgcgagttg tgttgctcga atgtgggtct tggaatgtcc atgttcaggt 50880
agttaacctg tacaacctga cccttcttaa cgtcctctag tttcatttca ctacctcctc 50940
aatctagtct cattaacgta ttgaactgat acctcaaaga attgaaagcc tgtgggccat 51000
cgtcctccgc tccttcatgg atatgatgag cgatcaagtg tatcagtcct ctatctccta 51060
gcaatacaga gctaggaatc tcttttatcc aagccacaaa gcaacagaag tgtagaactt 51120

ccggcatgga agccttgaag ctggaatcat ccagcttcga taggcgttca tacgtagctt 51180
taagatcggg atagcttaac tgatatgtag catcttcgtc aatatgctgg tcccctctaa 51240
ctacatactg caagctcata ttacaccttc tttccgcctt ctgcaagacg tgcctctcgc 51300
ttatggtcgg aacgggtcat attgtactcg atcttctcga tgatcgcacc cgcaacatcc 51360
aaaccccgaa cgttcgcgta gtccatgata cgaatgatag cgtcagctag ctctacctcc 51420
tccatcttcc gctctggcaa gtggttgtcc attaaatcct tacggacgcc ttcagccgct 51480
tcggcgattt cagtggtaac gagcatcagc atgtgcagag cgtcacgttt aatcggctcg 51540
cccgtgacca gatcggtgtt ccacttgcgg ttcacaacct ccagaccagt catcattttg 51600
ttaaaaccgc taacaaactc gaagacggac tctcgttcga atagttcacc gaacgttggc 51660
tcttgatctt caacaatcgc agtgtggaaa ggccattcca tccaagtgtc gtggtatttt 51720
ttgataagct cttgttcatc aatgtacgag aagaagtccc tctctccatc aatctcatta 51780
acaacttcag ctccaccttc ccaatcgcgc ttatcaataa gactcggatc gattctcagg 51840
gaagtgctgc caacttcttc ccatgatctc cagcctacta tgccatcgcc gaagtacaac 51900
agaatgggca gattgaatcc gcgttcttcc tgatcctgag cctccatagc tataacaaac 51960
gccggtactt tttctccacc tacaccattg gccaagacga aagaacccaa gtgggtggtc 52020
tcccggcaag ctgggcggtg agcttcctcg atatgatctt taactttact cattaacttc 52080
accctcctcg cagaacattt caaaataaca atcggcacaa gtcagcatgc cgtcgtcgct 52140
tacagcttcc ttgagaggga agctgtttcc acaacactca cacacgaatc tgtcatcgat 52200
atctccgcag ccccaataag ccatactcac tcctccttca gaaggtactc gttgctgatc 52260
actttgaagc tgaagtctcg gctcatgctc ttgaatacca agccctctcg gtacttgccg 52320
ttcagagctg aatctccact ggccatctta atcacagact ccatttcagc agggacacct 52380
acattccaca ggtacagcgg aacagactta accctttcgc cttctgtgaa gttgagctgc 52440
gataggattt cctctcgctc ttcgggcagt gcgtagcggc tctgcgagat attgtagata 52500
tcatagacgt agtattctgg cttgtcaaca ccttcgaagt taccttgaat gttgggagca 52560
accaactcgc cttgcagtgc gatattcaag cccagcttac ggagacgatt gccaaggtca 52620
agctgatcgg ccatttcagt aaaatggttg ctatctttct tgatcacacg attccaaccc 52680
atgcctttga acagattttt gaccgcagtg aagaagccaa tcttctcgtc ctcaagacgg 52740
aacccaacat tgcgagaggc aacgcctact tcgccgtctt tatgccagat ggtgagagac 52800
gatccgtcta gtttaaaggt cacttcaaac ttctcaccct tcacgtaggc attctcgtag 52860
gccattttga tattctgcac gcgggcctga tccgttttcg ggatgaagtt ggggaaccct 52920
agcttagagg tgccttttcg gtcaccctta gcctttgctc caccgtcaat cgctgcctgt 52980
tccgaaggct tgacatactt cagaataccc atttggtcgg tgaggtcttg attctcttta 53040
agattaggaa ggccggcggc atcaacaggc acgatcagac cttggctaag ctccttacgc 53100
agcttcatag ttttaatccg agagtactcc ttgccgtcct tctctaccat attacgacca 53160

cgcaggaact cgaataccgg attatccatg ggcaccatgg agtccacttc gatgtagata 53220
gccagatcgc cagccttata ctcgccacgc ttaattacgc actgccagcc tccgacgaga 53280
gccagatcga ggctgtctgc gttagggtgg gtcttcacat catcgatacg cactactcgt 53340
gccagtacac gttcttggga attgctcata tgttatcctc cttgtttgaa aattggtata 53400
ttacttgtta aaccagccga ggtcaatagc tcgctctatg gccagtagca ggttctcagc 53460
atctagaagg tcccggatag cgatccaact cccactgtct cccggtagac cgtctgtgat 53520
atataccgga atgtcactat actcactctt tttaagcccg caagcacagg ctacattgtc 53580
tccgaagaca atctcgctga cttcccttgc ctcgtctcga atatcgatct tcacacctac 53640
cctcctatgt ttgaaagtcg ttgtattctg ccattctctc ttctcagtgt caatagcctt 53700
gacaagaaaa taatccgtga tagagtaggc tctctgctgg agagaaatgt ccacgtcact 53760
caccggccag cagtatagcg tatgaagagg aaagctgcaa gcgcaacctg agtcgaatct 53820
gagaacaggg cagcgacgac ccgcgagggg aaagctcaga gcctataagg agcccatgct 53880
ccccgctcac cgactcatct gcatatacat atatgtatat ctgcatgaga gcgagaggta 53940
gaccaaaggc atgtcttagg cggagattcc gtatggcaag ccttgggaca ggtctgcctc 54000
cactaccgat ctatctgcat aaagggggta caatgtattc agcaactgtt gtgaacagac 54060
actacgagaa atttgatgtg tatataggaa gggggagcaa gtggggtaac cccttctccc 54120
acaaggaagg caccagagcc ctctacaaag tagatacagt ggaacaagca attgagtgct 54180
tccgaagtca tctttggcgt aacataatgg aaggattaat cacagttgat gaccttctgg 54240
agcttgacgg gaagagattg gggtgttatt gtaagccccg tccttgccac ggcgatgttc 54300
tggtgagggc aatcgaatgg gccaaacaac acagaggagt gtataaatga aacatgttat 54360
ttatcgtgaa gaccttatcg acctgctacg cgaccgaatg aagctccaag cgctggaggc 54420
aggtggggtt gacaattggg actggtatga tgcatcactc atcaacggcg ggtattacga 54480
tgatgacgag cgtgaacttg aggtcgaagc tcttgcagat tgcttcccaa ccgtaggtgc 54540
gtaaccgtgg aggagtcgaa agctaccttc aaagaccctt accctaggtt cttcgacgct 54600
tctcgtaggt atggcgaaac caacgactgc atggtggtga gctttgcagt cgtatggaac 54660
accacctatg aaaaggcaca ccagcacctg aaagtgaagt gcaagcgcgg ttaccggcaa 54720
ggggctactc agaagcgctt cagctacgca gacacttggt gccctaagac ccggatgctc 54780
taccgggagg atgctaagga taagactctt aggcagtttg ctgaggaaaa ccctgaaggt 54840
cggttctgga tcacggtaaa aggtcacgct attgcaatca tcgacggagt gttgtacgat 54900
cacactaatg acaaaagacg caccatctgg cactgctggc aagtgtggcc cggggagtca 54960
cctgagaaga tcgagaggaa gcctgatcca atctcacctc ctgtgagaaa ggccccggac 55020
tggtatgtct aaacacaaac atctgaagta caaagaggag gatttatgga aattatttcg 55080
agagggacgc tccctcaaga ggatttgttt acggctaagt gccaccattg cctaagcatt 55140
ctccgagcta agagatcgga acttgagtat gtttcgttct cattctgtcg ccccggggag 55200
actgggtatt atttcgcaga gtgccctgtc tgtcacgaag aggtgtctgc ttgggttgcg 55260

gctcctgtca ttcctgtggc accggccacc acggagtata tcacaaagga gcgtaaataa 55320
tatggaccag ttagcaggat tcatgggcga acctttcgta atagcattcg attatgacga 55380
gacgatcagt ctctaccctc tggccttcca agtcgtcatg gataccttca ggggctttgg 55440
aggcgctttc aatgtgatcg tagtcactgc acgccaccct gacaacagcg gagagttaga 55500
gtggctacgg gattacgggt tcccgatcta tttcacggca cagaaggcaa agcgtcaatt 55560
catggaaaat cttggaataa aagtctctgt atgggttgac gacaacccgt tcagcgtgga 55620
taatgacctt cctccgtgga cagcggagca atacgacgcc taccttgagg caaagaagca 55680
acgagctatc aacagaggga gtaaagcatg agcgagatta tgaaggttga agtgggagat 55740
cgggtggtcc ttaaggccta ttgcgataca tacactggat gtgcaaagcg atgggcgcag 55800
gcgcatggtc gtttgcatga actgctagct acggggtttg tagaagacag ttgggtccac 55860
accgacgaac tggccggagg cttgaaaggc accgttgtga tgactgaact gggactgaag 55920
gaagagtgct gtgcagtacg tctagacgcc tacactgacc acagggctgt tgtagttgtt 55980
gacgcttctg atctttacat cgcatgtaag gcagcaaaag ctcccgtaag caccctagac 56040
gaggccttcg gttgcgacca cgactacctt atggaatctc cggctaacgc tcagcgactg 56100
aatgagtccg tcgagcagct acgaactggc caagtcgtag aagtggacgt gcctgtgagt 56160
gtaaatcaag acgaaacccc gcctatcgta gtgtatgtag tagagaagat ttacgatagg 56220
aaaggtaacg tacacctcat tctacagtat gaggtagcag gggcgctaat caggacggtg 56280
gtggaacagg gagaactcat ttacttcttg gatgaaatgc aaggtatgta cttgaccaag 56340
attcaactgg aggtgaacaa tggagttgta tgaccagtgg cgtaagtata tgagcgttgt 56400
aatcttagaa gaagaagata atgagcgtca gtgcatgcgg cttattgagg cagttggcga 56460
actcgatccc tatatccaag atttcctaga ttctttctct attaaaactg aaggaggtgt 56520
gtaagcatga gtcagagtaa gcgtgatgag attgtagttg aagcacgtgg gctagagagc 56580
tacttcattt cccactggaa cggctgggat gtgatggaca ctgcttgtct cggattttat 56640
atcaaacctg agtatgttct tgacccggat attcgagagc ttgagcctag cttcttggag 56700
cttgatttcg agcgttgccg tttccgattc tggggaaatg aagacgaagg cgtggacggc 56760
aaactcatgg agggcaagtt gcagattatg accaactcct tcgagtacgt cttcttcaag 56820
gaggtttgag tggctagaca gacttacgag tctgcatcag atagggcggt cgaggaggcc 56880
gtccgttctg agcttgaagg gcggtggaag tgttctcttc ataagctacc tagagcccac 56940
tatatggatt gggcagcctg ccggaatgga aaggttgtta gctttgtgga gcttaaggct 57000
cggaataatg aaatgcagaa atatcccgac ttctttgtgt cccagatgaa gtggctccat 57060
gggatcgaaa tgcaaagggt gtttgacatt cccgcattcc ttgtggtaaa gtggacggac 57120
tatacaggat atgtcaaaat gacagccaat ggcgtcaact acatgagcat gggtggcagg 57180
attgaccgtg gagactggca agaccaagaa gttatggtcc atatcccgct gaaagacttc 57240
acacgtttat aggaggacaa atgaaacgac tctgctaccg gtgtatgcac ttcggggagc 57300

ccaccatgca ccgccataag acgggcattt ggtataaggg ttgtagtaaa tgtggatcag 57360
gagtattcta tggctaagaa gaaccctgtg tttaagtttc tgaacacagt caacaaaccc 57420
tcggtagaga gggataaaac caaatacagg cggaagccta agcataaaca gaaggagcga 57480
ttaaatgaag aagggtgaca aagtgaagat caatgatgac gctgatacca tcttccgtgg 57540
gcgagttgcc accatcgtct cggtagacga agagggttgt gttatcgacg tgggcaacta 57600
cgaagacgag agcggagagc ttatcgagta ccttcacctg ttcttcgagc gctctgagtt 57660
gtccaagcag gcgtaggagg gtataatgaa cggacccgag tgcagatggt gcggagagag 57720
aactgaagct gagttcgtgg acgttgggct tggatggatg cagcaagtca ctggaggcat 57780
gtgtcatcgg tgtggagggt acgagaatgg ttactaccag aacgacggaa ggctcaccga 57840
ggtcgagttt gccacctact ggagcggtcc gttcgaagat catgcggatt tctcaccatt 57900
caaccacatg agagaaggcg actcgccatg agagccacag tacgaaacca caatctgcta 57960
tacatctact tccacacgtt ctcggaaatg aatgagtggt tgacagagaa cgctgtagat 58020
cagtggacag tcactactct gggctccggc tatcaagtgg ctgttcctat ggaccgtgca 58080
atgcgggacc aatacgcctc ggagaggggt tgacacgaac acaaggtgat gctaaactcc 58140
gtggttgtct gaagtaagga ggttttatgt ccaaaatgac cgtggcccaa attgcggcta 58200
tgcacggtat cgatctgtat tctgaccaca agactgcttg tccccaatgc cggaagaaag 58260
gcgctgacaa cagcggagac aacctaaagg tctatggtac tgatcgtaat accggggaac 58320
acaaaggtgc ctattgttgg gcgtgtgggt acaccttgct ctctgaagag caggaaagag 58380
acgcccgggg agaagacgaa gaggagtgga atttcgtggg aactgaattt aacgcagaga 58440
tcaacgctca aattaaagag caatgctcgt atgatccgcg tggttatcgt ggcttgtcta 58500
aagagacttg ccagtatttc ggagtgctac atggtttcga cccggagact ggcgagattg 58560
caactcagct ataccctact actactaatt acgaactcac tggttacaaa cagcggatca 58620
tgccgaagaa cttctcagag cctatcggcg agaccggtaa ggattgcgag ctttttggtc 58680
agttccggtt caagaatagt aacagcaaga aggtgcttat cgtaggcggt gaggttgacc 58740
agctatcggc attccagatg cttaaggaat atgacgaccg acgagcaggc ggtaagagtg 58800
attatgaacc gactccagtg gtcagtccta ctatcggtga gagcggggcg tggaaacagc 58860
tacaggctca gtacgagtgg ctagaccgat tcgaacaaat catcctctgt ttcgacagtg 58920
atgatgcagg acgagaggca gcagacaagg cagccgataa gcttccgaag ggcaaggtgt 58980
tcatgatgga aatgaaccgt aaagacccta acgagtattt gaaagctggc cgtcagtcgg 59040
agtttgtaag cgcgttctat cgagcccgta catggacccc cagcggtatt gtagggtctg 59100
atgcactaga tgcccgcatg tatgaagctg tacaagtgga gaagattcct ctgccaccgt 59160
tcgctaagaa gctccagaag gctatggcag gaggcatccc gttgggccgg attgtgaata 59220
tcggcgctgc atcgggaatg ggtaaatcta ctgtagtgga cgagttcact ctgttttgga 59280
tttataactc gcctcatatg ccgggaactg tgactctaga atcggactgc ggtgagtatg 59340
caatcaagct gctgagtcgt aaggtaggtg agaaagcaga cctgtatgag actgtagaag 59400

agaaagtggc gttccttgat cgtcaccgag acgctcgtta tgatctgtgg cacaaggaag 59460
acggaagccc gcgcttctac ctgattgacg accgagacgg aagcctttcg gctctcaagg 59520
ctcaaattga gcaactgatt attcagttcg agtgccgggt gattattcta gacccgcttc 59580
aggatattct ggatggattg agcaatgaag agcagtcggt tttcatgcgt tggcagaaag 59640
gtatgactaa aagccaccga gtgacgttca tcaacatcaa ccacgtccgt aagagtggtg 59700
gcggtcagaa ggctaactca gcaggtgcgg aactgtacga agaagatttc caagggtctt 59760
cgtccatctt caaatctggt gcagctaaca ttctgatgtg gcggaacaag gacgctgaga 59820
acgagttcga aaagaacctg atcaacgtga agctttctaa atgccgatgg acaggccgga 59880
caggaatgat gggcaactgg tactatgacg ttgagactca cacaatctgg gacttggacg 59940
actaccttga ccgctaccct gacaagcgtc gagagtatga agcttggatg gctcagaagg 60000
aagaggaagg acgcgacacg gaatactaag gttgacaaag gggtgatgct tatgtatcat 60060
ccctttcgtt gtttacagga ggaaaggaaa tgcaacagaa gtttgaaaca atcgagcaat 60120
acctcgaatg gatgggaaat aacaatttca ccaacatgac ttgggatatc gaggccacag 60180
gcctgctgga tgaaacgtct atcgattaca ctgccagccc ctacaagatt aagcctagct 60240
acaaacacca ctgtatcgta gtagaagagc acggcggtaa cattctggcg ttctacgatg 60300
ggccgaccta cgtctttgac gggcgagagt acactgagaa actgccgaag gcttcttcca 60360
tgctgcacac cgaagaagtt gtgactctca aggactatac tcctgtcgag tatattcacc 60420
tgccgctgaa agagttcaag aactatgtgg ccaagcgtcg gatcaagcgc gtagtggctc 60480
acaacatgat caacttcgac cttatgtcga tgaagctcgt agaagatatg gacttcacta 60540
tcggtgttga gcacagggat ggcggtctaa ctacttggtc tgctgatact tggatgggca 60600
agcaagtggt attcgacgat accctcgtta tctccaagac ttgcaaccct gacctattcg 60660
gcggccacag tctggataag ctgtctcgtc gtgcgggtgg tgacaccaag attgacttcc 60720
gtaagcattt ggcaccggat gttcgattcc tcgacttcgc agccgatatg ctttactact 60780
gtatctatga cgtaaaggcg aacaccgctg tatggcattg gttgcaggac cacgaagatt 60840
accaactgtc tgaccgcgac aactacatca agaactggtt gtctccgatt cagttggaga 60900
aaaaggttgc agagatcatc acctatcagg agcaccgagg gtttgttctg aacgtggacc 60960
tctgcgaaaa ggcagtgaag gatttggatg ctaagatgga agagcgtcga gcccgggtag 61020
agcctgtgct tcctcctcgt cctgcaacca agaagttcat ggctgactac actccgcctg 61080
ttcgacagtt caagaagaac ggggagatca ccacttacct acagaagttc attgaaaaga 61140
tcggcgctca attgaaagag ggcgacgaca gaaccatgat ctttgagggt aaggagtaca 61200
agctgcctct cccggcagac gtaccgctga agacggaaat gcctgctaag atcggggaca 61260
caactcacat caagcaatgg cttatgggtg agtttggttg gattccatca gagtggaaag 61320
agactgatct gactgtggat cagaagaaga tcaagcttcc gatggaaaac atcgttgcta 61380
agatcgatcg ctggctggat gtaacgtata gcacagcgta caaggaagag cggctggacg 61440

ggttcgggga ttacatgaag atcacccctc gtagcacccg cgcgtatgta cgtcagaggt 61500
tgattgatcg tgcaagcaag gggggctgta aggttctcac taaccctagc ttcacaaagg 61560
gccaagagaa agaaatgtgt ccagacctcg aacgcattgc agaacagttt ccgttcgcga 61620
aggatgtgat tgagtacctg acctttaagc accggcgaag cgcaatcctc ggtggcggca 61680
tggactggga cgaaatggag gatggagagg agccggaaaa aggttacctt gcaagcattc 61740
gtcaggacgg caggattccc acgcctgctg acacttgcgg ggctgctact agccggatga 61800
agcaccggaa ggttgctaac gttcctcgga tcacctcgtt gtacggggct gagcttcgag 61860
agttgtttgg ggttggagat gggttcttcc aaattggata cgacttcgat tccttggaag 61920
cacgtaaaga gtcggcctac tgcgatcagt acgatccgac aaaggaatat tgcaaatctc 61980
ttatgatgga gaagccattt gacgttcaca caatgatggc taagcgtatt tctgagatca 62040
ttggtcgcca atttaaccga tctcctgcga aaaacgtaaa atacgggtgt acgtacggtg 62100
cccaagctgg taaagtggca aaaaccattg gtgccgattt gcaaacgggt caactggtat 62160
tcgatgcgtt ctgggatgca gcattcccat tgaaagctct gaaggacgac ctcaccaagc 62220
aatgggaagc caatggcaag aagtatatca ttggagtaga cggtcgccgg gttcctaccc 62280
gttcggctca cgctatcctg aacagcttgt tccagagtgg cggcgttatc tgtgctaagc 62340
gggctatggt gattcatgat gtactactgg agaaagaagg tctgaaggtt gactttttca 62400
aagaagactg gaagaacaag ccggaattct gccaacaaat gatcgcgtac cacgacgaag 62460
cgcagttgga agtaaccgcc aagtcggtga gcttcaagat gttcccttgg tccctagtag 62520
gcggcaagcc ggaaggggag aaggagcgag aggcagccga gaaggagttg aaagctctag 62580
ctcaggctta caaggacgaa caattggcta gcactggcaa ggtgtggagt gatatcgcac 62640
actacagcaa aggtgaaggc ggggtgtttg tagcttactg ccgcgctgga gaacttgcca 62700
cgaaggccgt gagagctgcg ggagagttct actctactcc tctggtgcct atcgagctga 62760
ccgctggcta catcgttggt cgatcttgga aggattgcca ctaaaactgg cattaagcga 62820
ggaaaggggc ttgacaggcc cctccgcctc ctatacaatg gccacatatt cacaacatac 62880
aaggagaaac acaatgactc tggctttcat tatctatctg gtaatgaccg tattcccagc 62940
cttgtcgtcg gctggtagct ttgccctgat cgtcaccatc atcgcatggg ttgcaggagg 63000
gttcgtttac ttggcatcca ttggtggccg agagaattcg gcagttaaag ccacactcaa 63060
aaatcaccta cggcccttga cgttcaagct gtttctgccc atcattgtaa tcggccatct 63120
ggtgccttcg tctgagacgg catggtggat ggtcggcggg tacgctgcac agagcctcta 63180
tcaagcagac gctactcagc aggttctcag cgaatctact gaactgctcg aagctctggt 63240
taagcgggct aagaaagaaa ctctgaaggc tgtcgaaggc agcagcgagt gatacggtaa 63300
tcacagaata ggaggtatgt atgaacacgg ctcgactcta cgtctacggc aaatcgtcta 63360
tggtagggat cactgttgac aacaaggcca atcgagagta ctatacctct aaaggcttcg 63420
aggtatcgga gacaccgtta gcgcataaga gtgttgctaa caaatacaaa cagaaaggag 63480
gtcaataata tgtctagtat tatcccgggt actatcgtca agctcactca aaatggatgt 63540

gacgtcaccc ttggctggcc gaaggaagcc cgatacgtag gcgcatatgc cgttgcagta 63600
gatggacgcg atatcggact cccggaaggt gaagtccggt tctgctctct tcactggtcc 63660
atcgaccgag acgttaatga tggactcgga gactaccaaa cgatgctggc ggattcaggt 63720
cgctacgaaa ttgtagcagt tccgggcaag aagtactacg ttaagaacgt tgccaagcac 63780
agcgcacaga tcggccatta cttggttcct tcggagccag ccttgtacgt aggtttcgag 63840
ccgtgctggc tgaagagcat gcacgaaggg gagagcccgt tctgcatgac ttttaaagga 63900
cgcgttgaca ttggcggtga aacctctgac aagccgcagt tccagaccct ctaccctgag 63960
ctggtactgg aagagtttaa agaagaggcg gagggcaagg ttgaagctcc ggttgacgaa 64020
gagaagaaag aaaaggttgc aaagcctgct gtaatgggtt atgctgttct cgatgcagac 64080
gggagtcctc ttaaattctg cctcactcgc gaagaagcgc gagccaccaa gcgctggtgg 64140
ggtggcaagg ctgagggagt ctcgattgta aaagtaatca aaggagagga ggtgcgttaa 64200
cgcgcttctc ctcttgaaga atccgggaat gtccggtata atcgtcagtc aagtggctga 64260
cacactcgcc cagtctaggg tatttagata ggagaaataa aatggctttc aaactgaacg 64320
taactgctaa cactcaatcc ggcaacggcg gcgtccgaaa ggaagtcgat tgggatgctc 64380
tgaacaacca cgtagttgaa gctgctggca ctgcctccaa ggctcgctcg atcccgggct 64440
acatctcggg catcatcgac ctcggtgaac aagagcagaa cgatgcagag gttgtgttca 64500
ctggcactac cgaagatgaa gttcaaatca tcaaggagtt cccgaacact tactttaagg 64560
atggcattga caagcagact cgtaaacctg cacgcctgaa gtgctggccg caacgtgata 64620
tgcaacaaat cgcaatcacc gtggacttcc ctcagatcga agtagacaag agcctgtacc 64680
tgaccggtaa cagcaacaag tctccgctcc gtgttctgta caatggtgat ttcactctgc 64740
cgggtcagaa gactaagatt gttgggcgtc cgttcaccat caaggaagtg aagcacccga 64800
atggcaaatg ggcgttcgcc aagaactcca tgttgcacaa acttgctgtg gctactggcg 64860
tcatcaacga agacgaactc ttcaccaagg accgcgtagg cgagcttctg ggtaaagtgg 64920
ctcagttcca agttcgtgtg tggatgaagc cgggcaaaaa cgacatgaaa ttcttcactg 64980
aggatatcag tctggtcggt atggttcctg aaggtgttcc ggttcccgag ctggacgaga 65040
agtacatcta cggtgtcaac ctgtatggcg agaacgacga agaagcagtc aagcaactgc 65100
gtgctaacgt catcaacacc atcaagcgag ccaagaacta cgaaggttcg gatatcaagg 65160
cgatgatcga agccgttgaa gctgcacgcg gtggtaaccg ctccagtggc gacagcgaag 65220
agcaagctga agataagccg cagacccaag ctgcacctaa agtggagccg gttcaaacca 65280
acgtagatga tgacattccg ttctaaaggg cggtggtaag acaaggggct tcggcccctt 65340
cctttcaaaa tttcaaagga ggcaacacat gcaaatcaat tttggtccca tcttccttct 65400
cgcagcaatc gtcctgtgcg ttctgaaatc tgccggagtt ctcgctatct cttggactgt 65460
ggcactcatg cccatctgga ttcctcttgt aattgtattg tcgattctcg tactgtttct 65520
tgtaattgta ggtctgattt acgcattcgg tgatcgtagt aaactgaact ttagctttaa 65580

aaagcacttc cggaataagt gaggactaat gatgagtagt ttggttattg ttcgagtacc 65640
tgagtacatt ctaaaaggcc ccacagtaaa caaccagttc agcggtgacc tgtgggagcg 65700
aaatctcttc aaagatttga agaacgctgc ggagtgtatc attaaaggag agccggctta 65760
tgttgctctt cctaacaagg taaaccctga cactctacaa acagagtggg acattcaggt 65820
tgtacagttc taacccaagg ggagcttcgg ctcccttttt cattggagat aaaaatgagt 65880
gaaaaggaag aggatatcgt cctcatcgac ggcgacctta tcgcctttaa gtgtgcaagt 65940
gtcaacgaga cccgctccat cattgtcaga aacaagctaa caggagaaga ggaaacttgg 66000
aagaaccgca cgacatggcg agagaacaac aaagacgaag agggattcaa cgaggaaaac 66060
ttcgaaattg aggaccatca agaccccaag cacgtatcct atggtatctc tgttgttaag 66120
accatgatcg accgaatctg ccgccaagct gggtgcaagc agttcaagat tctcctatca 66180
ggccctgata acttccgtga cgccatccca ctcccgaaag aatacgagat taccaagggt 66240
aaaaagacct tcactcgtgg cggcaggtac aagggtaagc gtacagggca gattaagcct 66300
cttcagctag gtcagctaag gcagtatatg atcgaagcgt atgacaccat catccatccc 66360
ggagaagcgg acgacttgat ggccgaaatg atgtacaaga acggtgtaag ctactccaga 66420
ggggagacta ctcagcgcgt gatcggggct accatcgata aagacgcaga cggcactcta 66480
ggatggcttt gtaactacga acgtgagcct gttcaagtca agttcatttc aggcctagga 66540
tcgctctaca gggactctaa gggcaaggtg agaggcgagg gacgtaagtt cttctacttc 66600
cagttgctgt tcggcgaccc tgttgactgc tacagacctg ccgacctgtg cgtagggaaa 66660
gactttggag aagtggcagc gtttaacatc attaatccct gcacaacgga caaggagtgc 66720
tggcaagcaa tctacgacac ctacaaatcg tggtaccctg agcctgtcac ttacacagca 66780
tgggacgaca cagagcacac taaagatgca atcgatatca tgcaaatgta ttgtgactgc 66840
gcccacatgc aacgttgggc gggggatcgg gtagactgta gggctgtttt ggctaaactc 66900
ggggtagact taggaggtgc agaatgagtg taaggcctcg cccttgggaa gacttcccgg 66960
gcatctggaa gaccgaagcc gcgttcttga gttgggtcag aggtggcatt cgccgctatc 67020
tctggtctaa gaacccggtc aaacttgagt ttgctaaatc ccgaaggatc aagatagcca 67080
acaccaatga aaagagcaag ctcaggaacc ccactgtatg gggctatgtg tgcgagcagt 67140
gcgggaagga gactcctcaa gctaacgttg agattgacca caagacgggg gagttctccc 67200
tgaagcgagt agaggatatc caagcgttcg tggaaggtgt ggtgtttgtc cgcataggag 67260
atttggctgt cttgtgcaaa ccgtgccatg agatcaagac ctatgcagag cgttacggac 67320
tcacccttga agaggcgggt atcgtaaagc aagctattgc aatccagaag cagaaaaggt 67380
atgataaggc cttcttgcag gaggtgggaa tcaaacccgc atccaacgca gagcttagaa 67440
aagaacagat cattgaatac cttaagtcta agaaggaggg tggatgaacg aggatcgggt 67500
ttacattgta gtaagagacg aggcaccaga gtacatggtg ccgactctgg ttgcacatag 67560
catcctaggg gcagacatgg caatggttct cctcggacag gaccgggccg ttccatcgac 67620
ggaccttccg tcccgtccgt atatgcatgc agcggcaaaa accgccatta agtgggtgga 67680

gtggaaacag tggagcttcc gtaagatcgt tgtacgagtt tctgacaaag agtacgataa 67740
gatcaagcaa atccctttcg tatatgaggg gtatgagaga accatctgca atggcgaagg 67800
ctcctgccta gtagtacctc ctctgagcaa agaggagacc ccgaatgttc ttaaatttgc 67860
aaaattatgg aggcctctgt gagagagaag tttttgagaa ttatgcggaa gctgcccatc 67920
cgggtaaggg cttcccttgt tatggcttgg attgctctgt tcttctggct cccggttgca 67980
actgcgctaa tcgttggaat tctagagttc ttcgataatg caaaagaaga gttaagaaac 68040
ggatactctg ttttgatcga agctgccaag ggagcttacc agtgcattaa aacgggagag 68100
gagttggagt catgaccaat ttagctacgt atgttatacg ccacaaaaag accaaagagc 68160
tttggaccac tcctaaagga aagcggcaat ggaccacgaa aggaggggca gcccgagctt 68220
ggagaaacgc atatgagagt actacactag gagcgtacta tgctaaaaga gatatggaac 68280
ggtggcctga cctgaacatt cctttgatag taggggccgc tcccgcacgt ctggccggct 68340
gggaagcagc atcttttaaa aaccaatcag ctttcgagat tgttgatatt gctgaatccc 68400
tgaagcacga aagccagttg gtgctcgatc tactgaaaga tgttattggg ttcgaagtgc 68460
cagaccacat cggagacgga tatgtccgac tcaccattaa aacgtccaca tacgacgata 68520
tcatcaaact actgaaggag gcaaaatgag catgactgac ccccgagcgt atttcatcaa 68580
acaggagctg gaggaagcaa ctaagcttct agaaagagcc ctcaaccttc tagataacgt 68640
ccattgctac gatacagatg tatacgagga cattgcagca ttcctctacg gtcaagagtg 68700
cgaggaggaa taaggtgcag cgcgatatta gtaaaactat agcagaactt cgggacttga 68760
tcgaagctgt ctctatgctc gacgaggcct tggttatgat gcagaaaaac ggactatatg 68820
atacggactt gtacaaacgt atcgatgcat tttgtattta tcaaccgggc gagggcaagt 68880
gggaggaagt agaatgagca gcaagaaaca ctttgtaatc tctgatacac aatgcaagcc 68940
gggtatcagt ctggattaca tggaagccat cggtaaatat atcgtagcca aacgtccaga 69000
tgttgtgatc cacattggag atcactttga cctagccagc ctgtcgtcct acgataaggg 69060
taaacgctcc tctgagggcc gtaggtactc tgaagacgtg caagtcggac gtgagggtct 69120
ggaccgactg ttcaagcctc tgtgggagct acaggaacgt caacgggcta atcggaagaa 69180
agtgtaccaa cctcggttga tctttacact gggcaaccac gaagagcggg ctgatcgagc 69240
agcgaacgat aaccctgagt tgtatggcac cgtggggagt gaggagcttg ggattcagga 69300
ctacggtttt gaggttgtcc cgttcctcaa gcctatcgaa gtggatggca tctactacgt 69360
ccactacctg caaaaccact tcactggcaa gccactaggc ggtacagcga acaacatgct 69420
caaggctgcc ggtcattcgt ttgtcatggg ccaccgtcag cttctggact gggcgatcca 69480
tcctacaatt gatggcaaac aaaggctcgg cattgtgact ggagcgtgct atcctcacga 69540
cgaggcttac aaaggctatc aaggcaacaa tcacttccgt ggctgcgtga tgattcacga 69600
agtgaaagat ggtttcggcc ttccaatgcc agtgagtcta gactttatga tggagaaagg 69660
aggcttcaaa tgacagttta taagttctgg tgtgaatggg atatcggtat caatgagtgc 69720

ctgtggcgag actactacca gatggaagaa gatgtagtta aagccttgtc agactgcggg 69780
attgatgata ctattgagga actggaggac gcaggcctgc tgagcttcga ctctgttaaa 69840
gttatcggct aattaggtaa taggagggca tatggctgag tttttccttg gggcggccct 69900
tgcaggcttg ttctttatgt ttagtatcac tgcgagcttt accgccgaag atattgagaa 69960
agtgacttca acatgcgaga ataacgaagg cgtcaagtat ttcggagttg acttcttcgg 70020
agacggtgct gtacactgca acagcggcgc tattttcaaa atcgaccgca aatctaaata 70080
atcgaaaagg agatataaaa tgactgattt caacatcacc cctgagcaaa tggaagaagc 70140
aatggcaggt atgaacccgg agcagcgggc tctggtagag aaagccttga ccgaccgtaa 70200
atctctggaa gattccatcg tgaccgagga cggtgaactg gactatggca tgcttgcacg 70260
acacatccgc accagcttgg gtgatctgcc tctggtaatg ggtggccctg acttcctgaa 70320
gctgatgatc ttggttgctg cgatgccgga gaccaaagac gatctcctga ctctgcacat 70380
gaacctgtcc ttcattctgg actgcattcg tgaatcgatt agcgacgagg agtaacaagt 70440
ggccgaagag attgtaaaag aagacctatc tgtcgacgat ctcatggttg actcagcagg 70500
gctagatgcc ctgctggcct tcctagagag tcagtttaaa ggtccgaagg gtgtactgaa 70560
gggatatgtc actatgagag tgatgtgcgc taagatcgag agtcttgacg ggtttcaaga 70620
gttgaaagat atggaggtga actttgagta acgatatcat cgacgttcat gctgggcaac 70680
cgatttctct ggtgcaacgg tcaaagaatc ctatcaatat tcaatattgg gacgccgata 70740
ccatccctta cggcacagtc ttggttgtta ccgaggaaag gggtgatata cgaaagggag 70800
aattggtcgt aaggctggct agtagtgcag atgatgagca ctctgttaag gtatgtagat 70860
tctctgattt cttcctccta atggagaggg gctacaactc agctagcctt agcgacgtta 70920
gtcaagcgta tgacgttctg ttcgaagacc ctccggcgac tacctttgtc aactgggatc 70980
gggctcagcc tttgacaaac agtgagcgct actacggaga ctttctgatt ggcggcgacc 71040
ctaaagggat gccactattc aacatggctg ttcttaaaaa tgttctgtcg ccttacactg 71100
ccgacgatga ggaactttca gctatgtctg ggaatcccga gacagtagag cacaaagaag 71160
ttgacgaact aagtgagagg gacgaggctt tagccgaggt cgacagactc aatgaagagc 71220
ttgttgcact gggtaagaag gttgtgttcg ccaacctaga agattaccta ggcaaagaaa 71280
actatgccac attggaagct atggctaacc acaaggaggc tgtaggtagc ggaggctcct 71340
ctagttacta cactgtcaag gtgaacaacc ccactacgaa ggctaacatg ccgtattttg 71400
cagagtgcaa cgatatcatc gaagcgctgg atatgcgatt cggtcaaggc aacatgttca 71460
aagcgttgtg gcgggctgcg gcagcacaga acctaggcaa gctgaaggcc ggtaacgatg 71520
ttatccggga ctacgagaaa gtcatcttct tcgctcagtt ggagatcgat aagattctca 71580
gagaaagggg ttgacattct ccccgaggct tgcgataata ggctccaagt tgaaacgaag 71640
gaggtatgta gatatgatcc agaaagtaaa acagaagctt atgatggtgc atcctatcct 71700
cgctagcgag atcgtctggt gcctcacctt cgcaatgctt caataagata agggagcttc 71760
ggctcccttt attgtatcaa agatttaata aggagagttt atgcaaattt ctgcaaaagt 71820

gattcaacac agttcccgga tcggcctagg tacgaagaat cacagattca tcactacatt 71880
cgaactggag tatcctcggt ttattcacag tgaacttatg actcaccgac tttttagtcg 71940
aaatgctgca agtagtcgtg ccattcccgt tgcccatatg atccgacagg tggaagagaa 72000
tcctgcgatg cctgttcact ggggaatgaa tcaaccgggt atgcaagctg agttcgaact 72060
gagtgagtgt ttgcagcgta gctctcagta cctgtggaag aaggctgcaa agagtgcggc 72120
tcggatcgct agcgcactaa ataaaatggg actccacaag caggtggtta accgtctcct 72180
tgagccgttc cagatgatga agaccgttgt tacagccacg gaactagata acttcttcta 72240
cctgcgttgc cataaagacg cacaaccgga gatcaaggtt ctcgctgaga aaatgtatga 72300
ggctcttatt aaagcgggcc cggcagaggc tctatatgac ggagagtggc atgttcctta 72360
tgtgactcgg ttccgcatag gggatggtaa gctgcgttac gcagattgca acggtcaaga 72420
actcagtgtg gaagatgcta tcaaggtgtc cgctagctgc tgtgctcagg tcagctacag 72480
gaagaacgat gagactctgg agaaagctct tgtaatctac aatcgattgg tagatactaa 72540
gcccgttcac gctagcccgt ttgaacacca agcaacaccg atgctccgca tctcggacaa 72600
tcaaaccgga tgggaagacg gtgtgacgca cttggaccaa tatggcgacc tctggtctgg 72660
taacttccga ggtttcatcc aacatcgtca actaattaaa gggcacgtat gtaatgagta 72720
ctacggccaa gaagaggaag gagtttaaaa atggcagaat ataacctgag tctggaagac 72780
ctcatgctgg tagatggttt caaagaggca ttccagagta acaatgagaa ggtagtgcga 72840
gagcatctat ggactaacgg catggatgtt aaaaactact cgtatgaaat ggttttctgt 72900
caacatcgca cactgattgg ccgggtcgta gagggtctac gattctctgg ctttgagcgg 72960
acagataaag agtggctgag cctcggttgc gcctctctgg aagctcatat tgcagcgtgt 73020
gatgatagta atctacggtt cactcttcgt aagatgcgac cagagggatc gacagaggca 73080
acgttccaca attaatgcag taggagtaga tatggaagtt acaaaggctc tcgcagatga 73140
ggccgatatt ttcaataaga ttacacaaat caggactcca acggagtcct atgcccggaa 73200
atacccggtc attgtcgaac tagcaaacca gcaactagaa gagaagttgt ggttctctag 73260
cgaaatgaag gtagagctgg ataagttgaa cctgaagttc aagttggaac ctcatcagct 73320
tcacgctgta aagactgtcc ttcaactgtt cctcaaatac gaactgatcg taggcgagga 73380
gttttggatg ggtaaagtag tccgcacgtt ccctcgccca gaggttaaac tagcggcttc 73440
gatcctgtgc atgatggaat tggcagtcca cgctgaattc tacaaccaga tcaacgtagt 73500
gctggggatg gatacagatg ccgactacgt ggcgtacaag gatgacccgg agcttatggg 73560
acgtatggag tggcttgagt ctgtgctagg cgacgaagac gatgttcttt cggtaattat 73620
cttcagccta acagagaccg cccttctgtt cagttccttc gctatcttga agagtttcca 73680
gtgcaacggg tacaacgata tccctgtgat cgcacgcggg gctaaccaga gcgctgtgga 73740
tgaggacttg catggtgtag tttcggctga gatcatcaac caatactacg cagagattgg 73800
gcgaccactg tctgaagata ctcggcgtgt agagaagatt cgacaagcga ttgatcacgt 73860

gtacgcgcat gaatgtcgta ttgttgatat ggcgatcccg ggcggagaac tgaacggaga 73920
atcggctgag aactacaaag cgtttgttcg ctatcgtctg aatgtctggt gccgtcgtct 73980
gggtctagag gatcacttcg agaatgacga tactccaatc aaggattggt ttgaaatgaa 74040
cacttacgct tataaaatga tcgacttctt cactccggga atgggcatgg aatacgaact 74100
agggtgggat gaagaggaac tggcaaaagc ttgggaagag gagctgaaat atgagtaaga 74160
gagacacgtt caactattcc gaagctagga aaaacagcca agcggcaggt gaaacgccgt 74220
tgtggtatac tacggctggc taccagttgt tcatgaagaa atattcgaat gagggcgaat 74280
ctgttcgctc tcgttttgag actgtagcca gagctatggc tcagcacgcc cccacggttt 74340
atcctgagtg gtgggatcag gacgagtaca ccagaggcaa gaactgggaa caagcgttct 74400
tcgacgtgat gtgggacggt tttgtaagcc catccactcc gcttctgtcg aatggaggtc 74460
tccgcaagaa gggcactacg gtatcttgtg ctggcgggct gatggacaac aacctctatg 74520
accgttacaa tgtaatgaca gagattgccg tgctaacgaa gcacagccat ggaacaagct 74580
tcagcctgac caattggcca tccgaaggcg atgccattcg tggtggagag tcgcagggtg 74640
ttatgcctgt cattcgtgac gttatcaacg tgatggaaga ggtcgcacag ggttctcgtc 74700
gtggtagctg tgcctacagc attaaccctc gccatgggga cttctggaac gttatcgatc 74760
acctgtacaa gcgtacagaa tcgaacaacg tcggatggtt gctggacgac aactggtgca 74820
aggctatggc cgagaaagac cctgaaacgc tgaaacgatg gaagcggatg atgttcgtta 74880
agctggcaag aggcaagggc tacttcacgt tcattgacaa gatgaaccgg caccttgcag 74940
agccgttcaa gcgagcaggg ctgaaggtag aggcatccaa cctttgccaa gaaactgtgc 75000
ttccagctaa tgactggtac acgttcagtt gcgtaattct gaactataac ctcgaactct 75060
accgttcttg gcctgaacac cttgtgttca tcgggcaggt tatgtcggat tgcaacatca 75120
gcgagtacct cgcaaccatg gacgaagtta gtattcaaga ccgaagggct ctggagaaga 75180
tttaccggtt caccaaggac ttccgggcac ttggtagcgg cgttctaggc ttccacactc 75240
tgctccagcg ggaacgattc ccggttggaa gtatggatgc gatgttcctg aacaacgcca 75300
tcttcaaagg gatgaaggaa caagcggaag cttgtaacag ttggctggct gcggttctag 75360
gagagccgga ggggtgccgt ggactaggga agcgtaatgc cactacgatg atgatgccgc 75420
caactaaatc gacggcagaa ctcatggctg gggcttctga gggcatcgga ctggacgttg 75480
ctatgtgctt caccaagcag agcgctggag gtgaattctt ccgtgtcaac aaggtgttgc 75540
tggagattat tcaagagcga ggcctcgatt gggaagagtg tgcaaggcag atcaacgaac 75600
gcaagggaag tgttcagcac gtagactggc taaccgatca tgaaaaggcg gtattccgta 75660
ctgctttcga agtgagaatg gaagactacc tacggctgtg ctcccagcgg cagaagtaca 75720
tcgaccaagg gcagagtatc aacttgtact tcacgtcgaa cgactcgccg gcttacattt 75780
cgtacatcca tcgtctggcc atggaagacc cgaacatcct gtcgctatac tacatctata 75840
gtatgcgggg agctggggat atctctagaa cagaagaatg tgaaatgtgc atgtaaaacc 75900
atcggcccgg ggcttgacag ctcccgggcc tttgtgtatt ctgagcccat caaacagacg 75960

gagggtcgaa atgaacaagt ttactgaggt ctacttctct tggagtgcag caagttctcg 76020
cgcaagctat cttatagata agggatggac tgctacgctc cggcggtcct ttgtaggatc 76080
ggagcctacg tgggtagtgg agttcacacg atgaacaacg aactgagaga agagtttaac 76140
cggtggtggg cccgagagga gcaggaagag cttcgcaaat cttgtgctaa aggctgggca 76200
gagtatatct ggaggtctag tcgtgaggcc ctgaaggtgg agttgccgaa tcctgaatac 76260
tttggcgatt actggtctgg ggattgggcc tacaacgtag aggacaccag agacgcgctc 76320
ctacaatctg gaatcgaggt gatctaatgg aaaaatattc taaaaagcga ctgatgcaaa 76380
ctagtgagcc ttacggctcg gcaacttacg cgctggatgt agcagccggt ctacaaagaa 76440
ctagattttg tgaatttgcc tgtgtaacgg aggataatcg agtaattagc gtgtgggagt 76500
atagtgacgt cgagccagac aaacctgtgc atcccggtca gcaaagggtg ctcgtaccta 76560
actactaaaa aggggaaagt ataatggaac agactattga caaaatccgg tatatcgttc 76620
tcgacaacga acagcggcat gttctgaccc tatgtcagtt agccgttcat aatctcctcc 76680
gtgggattca gtggacgaga gataagcatg gtcagattca catccatgat gttacaggcg 76740
tggaactcgg tgtgcgtcaa gggattatcg ctgaagtgtt cctgtacccg gggtatacta 76800
tcctagagga aactgaggaa tgaacggggc ccagaaattt cacagcccgg acggtcctgc 76860
cttcaatcaa ggagagctgt gggagagctg cgaagggcat gtggtcgaaa ttaaaggaac 76920
tgagcgctac ggggctgaca agtgggacgt taacgtttac tatgtctacg taggtaagtc 76980
gcctaatgtg cctgctgttt tagcgagccg acaggagctt ctagcccggg agttctcaaa 77040
agacgcttgg aatttccaag ttaggtatcg gcaccaagct gacaaggaga tttgatatgg 77100
atatgttcga caagtacatg caagtgatgt atgaccgaga cttgaacgct aagaaggtcg 77160
aaaagctgga ggcgaaactg ctggcggcgg tagggttact tcaggaagcg cgtggtttcg 77220
tggactgtga caatcctctc aacgagagcg ctcaaatcaa gcttcttgag gaaatcgacg 77280
aattcctctc atccccagta gctcagcaca gcgtgccgga ggggtggatg ctggtgccgg 77340
ttgagccgct tctcaacatg atgagcgaca aggaccacga caccagaatc acggctgagc 77400
gccaactgct ttccatactc ggcgccgcac ccggcaaggg ggactgaaga ctatgcgacc 77460
taatgttgtg gatacactgg gaatcaacat gtgctatcac tgtggaggca agctgtatgt 77520
tcgcggagtt tgcaccatct actgccctga ctgcgagaag gcagagaaag ataaaccgtt 77580
cgtcaggatg cagcttctac aggatatcgc agacagtaag aggcttgaca cagaataata 77640
ttctttgtaa gataagccca tcacagacaa ggaggagccg agaatggcca ctacagagaa 77700
aattattgag tacgtcgagg tgtgcatata tcgcttgcag gaggacgtga tggcctattc 77760
taagggcttc agggacagag ccttgtctaa tcggacaggc tacattcacg gcctgattac 77820
catggctgaa atgaatgagc aagtgagtat cgaagactgc gacaagctcc gagctaatct 77880
gcgggacgct tctgaagcag tgatgaacac attggacttt taaagtcaac taggagggag 77940
taggatggat atgaaaagcg ttgttcaagc tctggttatc actatcgcat tggcactctc 78000

agcgataggt tttgtaactg ggattcataa attggtcagc aagcccgtca agaaagacat 78060
tatcgtgtct accgacgatg gcaccatcaa ccttggtcgc tctgtgacga gcgagaaact 78120
actgtacagc atgtgtatca aggtggaaga taacaaacct gcatgtgaat tcaccaccaa 78180
ggacaaggta gtacatgaag tgcgacaggt gatgcgcccg ggcgagtcta cggcacgcga 78240
tattaaggtt gcagtgaacg cagcagtgac gtatactgga gagaatggtc agaaggctac 78300
ttcactgatt ccgcctaaag gcgagttcct tccggctgga actccgtcag aggtgcatat 78360
ccagacgatt gaccgcttgt ttgatagtgt gtataatgac tacaagcaaa acttcagcaa 78420
gatgtgagga ggactcatga aagcacagcc taaagaagta gaaactctat ccctcgataa 78480
gcgacccaag gcaggtgatg ttgttcgact gcggtctgac ggtaaaacct acgaggtaaa 78540
tgctgtatat cccacggcag taggctggga agcgattctc cgcaacatgt acgacaagac 78600
taagaccgtc actgttagca tggagtggtg aaatgtccgc gactaacaag tgcgtcaact 78660
tcctccctgt cactccggac gggcgagtga tcgtccggtt cggtaaaaag aggttaggat 78720
ggctcggtaa agagggtaaa aagtggtatt attaccctta tggatgcgaa ggcaggatca 78780
aatccgaagg cttcaaaact ctgatcgata ttaaggcgtg gattaaaaac cccgactcgg 78840
tttggcccgg gcccctagct tgagattgac taataagccg acgaatggta caatgggatg 78900
taggaaattc cctactcagt ataaggaaac tagcaatgac tagccgctgg aaagaggccg 78960
tgtatcgcaa gtactcggta cgtcgtctgc ggcaggaact cgaagagttc aaagaagcta 79020
gacgctttgc accaaatcaa cgcatccgtg catggtttga tgctcatatc gaatttctag 79080
aacgccactt ggctattcga gagtccggcc aagcctaccc agacgacctt ctgtaattaa 79140
gtgttgtagg tagagattca aatcatggtt agtggcaagg acgccacact ttcagcccgc 79200
ccacaaggcg ggcttctttt tgcctgaaat ttgtggttga caaggttggc cggaatatgt 79260
aaacttgcgg catccaacaa aggagggaag aaaatgcaat tcaatatatc gaagcaacta 79320
tgggtagcta aaagcgagcg gaagggcgac tataaaggga cgcttttggc atacatgtct 79380
caagtggact tcaataaaga tggtagcttg gctgccagct tcaagaaaaa gcagcagact 79440
ggtatgagct gggcaggtaa gaatgccgag acttgcttca tagacaacat ccctatctcg 79500
gggcatttca tcggggacag tgttgaacgc tgggtcactg agaacaaata tttccgtgtg 79560
acggacccta ggggctttgt tctagaggtt cctaccggga atatctcgac cttgctctac 79620
aactgcacag taatcaaggg agttattcaa gagccttgcg tgtgggttcg gatgggctct 79680
cagcacgttc tactgccgga aggctcctct ctctacaaag aagctgtaag tttcgtagag 79740
cgaaaagagg cggccaaaac tgttaaagat atggttgcag gggatcgatg cactcttctt 79800
agctacggga cagaggagag actagaggca gtctacctcg gacaggtgaa gatcacttgg 79860
cacctcctag ctaaaaaggg ccggtactct agccgagggt ggggtggggg tcggtggggt 79920
taccaccatc tagagaggga actagataga aaagaattca ctgagaaaga ttcgaagtgg 79980
gttagtgtgt tcgctaagcc ggtaggaaag cggtggagta tccattttat cctgtctcca 80040
aaggcaaccg acattcaatc tggaaatgta ccggatgaac ttcaaaacct cgacctagtg 80100

aatggtgtaa aatgggagta caacgagaaa gacgaccgcc catcttccta ttactctgta 80160
ccggataggg tagttaagtg tatcagtgtg gatgatcccc tgtgggataa gctgcacagc 80220
tcttcggagg tgcggtggac ccgcgaatgg accgtggtcg gagcagagtg gcgaaaataa 80280
gccaccatta ttttagaatt gcccggcgag agccgggctt tttcgtggca ggggttattt 80340
tcaaattatt tttagatttc tgggagtttc ggccaatatg ggcaatatga aatttctcag 80400
gaaaaggctc agaatgctcg tataagagcc gttcgagtgt gggcaaggtg agctactact 80460
tagccgtaga aggacagtgt gacgcttaat aggagggtta gaatgaagat tgacaacggt 80520
ggcgaatatg cgtattggct ggcggatatc aaagagtttg tttctcagat ggatgccaca 80580
gagatcgaga agagggcttg tatagcatac ttaattagct ggtatcagcg aagagtactg 80640
cacagaatta aacgggagga atacgaggcg atctaagcta gggaccggat ggatttttag 80700
ggactggata gattttcaca gactgcaaaa ttgagaactg ggcagatttt taggaactgg 80760
acagtttttc acagaagggg atttttctac agaggaattt cccctgaaat ttagccggat 80820
ttctcagaag ggcggatcgt tagcaggcta tctaattggt tagcaagcta cctttaatcg 80880
ttagcacact aactaaaaat tcctaggacg gacctcagtt ggcacgaacg ttgcaagccc 80940
gcaccagttt cccgggcagg cgtaggcctc gactatccgt cgtgaaggcg atatgtagtt 81000
ggggactcgc accccgcgtt taagatgtgc ccattctact atgacaaacc cctttgtcaa 81060
cataattttt atggcacagt ctttgcaatt gcaattagcg tgccaaccta aatctcaggc 81120
tccctccgtg tttgcctccg gtgctcggcc tcctgcctgc gttgcccgcc tgcctgtcat 81180
tatacacaag caagaaaaga aggcaagcga aaatatagag aaaaatctac ataaaatagg 81240
ttgacaggct gtctctatat aggcacaatg agccaatatt caacggaacg gtactctaca 81300
atgttgcaca atgacgcccg ggaattggcc ctacaggaaa tggacaagtg gggcctcctc 81360
aaacaaggct ggaaattcag attcaatcgt aagctgaagg ctacactagg tcggtgccat 81420
tatactaaaa agctcataga gctaggctct gattatgtag ctaacaacgg cttgcctgct 81480
gttttggata ctatccggca cgaaatagcc cacgccaaag ctggacgagg gcagggccac 81540
ggcgcggtat ggaaagagtg gtgcgaattg atcggggcca aaccgaacca atacgccagc 81600
actaaggaaa aggtagtcgt ctatccgtgg cgactggcca tccatcacgg aggcggccag 81660
cttgaactac tcaaccatta cggtttcaga cggaccagca tggcgagaaa acaggtacgc 81720
gggcgtcctg agacgctagg caggttagtt tgggtgaaga acgaaaataa ttgagaaaaa 81780
gatgttgaca gcttgggcca ttcgcgtaga atggccctca agcgaaggga aacaccctga 81840
gcacccacgc aggaagcaga tatgaaagct ctttgtaatc atcgggcact ctacgcggct 81900
tacaatcgta gcgagcttga tcgtattgcg gtagatttga accgatactt ccctatcgag 81960
aaagatgatg aatgggaaga cggcgcatac atgtatcgtc gccgcaccta tctggtgaag 82020
cattcgaagg gtacagcccg ttggcacagc ctttactgca atggaaccgt ccaaggcgtc 82080
ggggttatct ttgacgtgaa gtcggacaca cgctctttcc gcacagcata aggagattca 82140

gaaatgcaag cttggaacgt ataccagaat ggccagttga tcgatactgt tttcttcgat 82200
atcatgtgtg ctgccgaaga ggtgcggaaa tcgctggtca accatgacgg gtatccgtct 82260
gatatcactg tggaggaggc cgagtgatgg atagcctttc tgttaaaatc gttctgtcgg 82320
aactgagtta caaggccttg attcaagcct tcgccttgct tggccgaacg ctcgacctag 82380
acgaggggtt ggacgatata cttaatctta ttgcggatga gatcgctttc cgcgacggcc 82440
tgccgccttt cgcagacgac tgcgataaaa cagaatggat gctccgtcga tgggaatcgc 82500
tcccggtggt atcgtacaat atcgggcagg acggaccgat tgtcgctcgg tctatcgaag 82560
taaaaacggc atgaggggag aactgatgac cgtcagagta ggagatatcg tggctatccg 82620
gggtgacatt cggaaaaagc tcgacggaac gtgccaatac acgcccgaac aattggtagg 82680
tggtaagttc ctcaagcata ccggcacggg agaaatatgg actggccgag tgagtaagat 82740
acaaggtgat atggccaagg tgggaggggc ttggcgcggg atcgaattct atgtgctcat 82800
tagtgaaata aacgcttgac aacctgaccc actagctgca aaatagcacc cacagacaac 82860
gaagacgccg acaggcgagg agaaagcaga tgcaaatgtt tttcaaaacc cgtacccaag 82920
cccgtgcaat ggccaccaat ggccgtaaag tggtagacaa cggcgctcaa gctgaaaagc 82980
gctgggctgt caagctgaac aaaaaggagg ctaagtaatg gctaagtctg ttagcgtaga 83040
ccgcaagcga tccattctcg acgaagcgaa ggatggcagt ttcttcagtg tggagtttat 83100
caaggcggac ggcaccgttc ggaaaatgac gtgtaaacgt cagatcaagt cggcgtatgc 83160
taacggccag tttgctaccc gcaagcccac cagcgccggc aagccgcacc tgtataccgc 83220
cgccgaagtg ctgaacatgg aagagggtaa atccgcattc cgcactatca accttgagac 83280
cctgacccgt gccaaggtaa atggaatcga atataaattc gattaaaagc ttgacagcct 83340
aggccattcc tgtagaatgg ccatcaagca agacaaactt aaacagaaag gagattaaaa 83400
tggcacgttc tatcaaggct tccgacttcg attccgctta ccagttcgag gccgccaaac 83460
gttccgaacg taaagaatcc cgcaaatcgc gtgacaatcg caaagctagc cgaggccgcg 83520
ctttcgactt ccgcgacgat atgtaaggct tgtgcctagc cggtgggccg attcgccggc 83580
aatcctaagc ccattcgtcg agtggtttta ggattctcaa ccccagcgga gataccaaaa 83640
atggttcgtc aatgcgtacc ctgtcccgat gctcgccatg ctttttgcct tgcatggttg 83700
ttcgccatgc cggagttcgc cggtctgccc gctcaaaagg acataaaact ctttaagact 83760
tcgggacagt gggcggtatg ttatggctag acgacagcgg ctcccgtcgt gggtgcagga 83820
ccagactgag gatgatactc cgctacggga caagatagac ccctacctta gcaagcttgg 83880
cgaagtcgat aagctagagg gccaattgcg acgggtcaag gatgcaacgg acccggatga 83940
atacaacgaa atggcaaggg ttctgaaagc tcgacgccgg aaaataatgc ggcagagggc 84000
ccgggcctac ggtctaccag aggaagagcc taccaacgtc caaggctacc acgtcccggc 84060
agaagattac cgcagggtga tgaggctgaa atgggtcgag cctcggatgt tctgggcagt 84120
cctatctttc gtcattttcg tatggttcat ctcaaaaaac agttgacacg cccactgatc 84180
actgtagaat gggcggcacg agaggggata agcccctaga ctgaaaggaa caacgatatg 84240

aaactgttca cctgtgcgtt ctccattgaa aactggcaat ccatcgaaaa agctggcgga 84300
gtggtctata ccgccggctg cctctggctt tcctactatg cgaaagaaaa gattgtctaa 84360
ggcttgacaa gtctcgttat ttaaaacaga atacatccga tcaccactca caaggcgaac 84420
caaaatgaat ttccatctta accccgtctc ctccaattct aaaactggtc cgatccctgt 84480
cagcacgtct cacaaagaca cttgtccgga cgcttgcccg ctcaaggcta aaggctgcta 84540
tgcctcttac ggtccggtta atctccattg gaaaaagctg agcaatggtg aacgtggcgt 84600
agagtggaaa gagttccttc aacaggtgcg atctatctcc cgtggcgact tgtggcgtca 84660
taatcaagct ggtgatttgg taggtcagga tgacgttatc gacggcgttg cgctaatgga 84720
tttggtgaaa gccaacaaag gacgtcgcgg cttcacctac acacattacc cgatgaacaa 84780
tttcatgaac cgccagcacg tattgtcggc caatcgtagc ggcttcacta tcaacctgtc 84840
gggcaacaat gtagcgcacg ccgatgaact ggccgacctg aacatcgccc ctgtagtgac 84900
tattctcccg atggacgcgg agaatgtaag cttcacgccg aaaggacgta aagtggtagc 84960
ttgtccggct gagaaaagcg acaaggtgac ttgtaagtcg tgcggcttgt gccaagtggc 85020
cgaccgggag tatattatcg gcttccgtgc ccacggcaca gcaaagaaaa ctgtcgatct 85080
cattgctaag ggttaacaca atggacgaat ctgtttacgc accattcaat cagtgtaaga 85140
tgatgggcct agaacgaacc gtgcggagcc tagcggctcc aaacggatgc aaggtagtct 85200
tgtatcagga tcacaagaac aagcatagaa tcgtcatata cgatgcccta gggcagggaa 85260
tgtccggggt catggtgcaa gcggacgggg aaattcatta caaggaaact cgcaaggcta 85320
gccaagggca gaatctgaca aggcagctac aagctcagct aaccgcatgg ggagtgaagt 85380
ggttcccctc tcagtatcag acagcggcgg gcgcggcctg ctacaaagga ggcgagtgat 85440
gcggcagctt aacaaatccc agcttaaggc cttggggcct aagatcgacc tagccttaga 85500
gctagcttct actggccgtg gtggtctaat gaatggccac ggctctacct gctacattgc 85560
aaacaaagcc gggcataaca ttctgcgggt tagctaccat aactcccgat tcgttgcata 85620
tggtgctgag agtgtccggg tgactgggaa tgtcattgca gcatgggtgg aatggaaggg 85680
cattaaccgg gctgcaatca acgctctgag tgagtacggg tatcatccta acctgagcat 85740
agggcacaac gatgctctta agactctgcg tgatatcgag cgttaccagt gacggcctct 85800
tactcgacag agaaacgggg ccagaggtgg gccatcatcc gtactgagcc cggttacaga 85860
ggtgaccggg tgaccgtgct aggattctgt atcaatcaaa cgcacgctct taaaacggta 85920
gccaaattcc gggcttggca agcgctaggc tggccctaca cactgaaaga cgacagaaaa 85980
taatgcttga cagcctaggc cattcgcgta gaatggccat caagcaagac aaaccgccca 86040
ttaggagact caaaatgaaa gctgtagctc agttcaaaag tgaagactac gcaagcggtg 86100
atagccgtac tgctactgtc tatgaggatg tttatggctg ttacatcgtg agcgttttcg 86160
gaactagcct gagcgagcct gtcagctacc attggggtcc gttcgagtat aaagaatctg 86220
caatcgatgc cgctaagtct gctatctaac tgaaaaaagg gccacagaat gaaaatctcc 86280

gctatcctga aaaataacct gaagttttac aagaacaatt cgttcatgtg cggtctggtg 86340
accgactacg gcggtatttc gtgctgcgag tcaatgaaag ccaaggagtt tattcagggt 86400
ttgttagggc gctatgccac cttgcacgct aagcttatga aagaggtgcc gggttacgaa 86460
agagccgtat atgatgatga gctagagagt gaatactcct tctttgtatc ggatagggcc 86520
tatcagatgc gttgcttgtg gtgggaaacc gtcattgcac agctagagga acaagggaaa 86580
tgactcaagc taactggagc aaaggcgtcc gggttgtcgg cggaaagcgg gacggtgaga 86640
tcacactatt caccgatgcc cacttcagcg aatctatggg cctctgcggt ggtgatcccg 86700
atttgatcga ggctctgccg atgcctgctg cactccgcct gctggtgaaa tggaacaaac 86760
tgtgccgcca aatgaagctt gactatcggt acgaactact gtaatctgca atctccatca 86820
aaacaggaat gctccaatgt cgatcttagc cttaactctg accctttgct ctgccgttca 86880
atgtgatgat tacattatcg atcactcgct cccgggacaa gccgcagagt gtaatgctcg 86940
gctggtagag gaggccgagg agtggggcga cgcatgggtg gcgaccaatg ccgacgcccg 87000
actgacccgc taccttagcc ggttcaatat tcaagttgac cctcgctttg ttttcgacta 87060
cgattttacc tgccaactca tcgcagagga tgaactacca tgaacgcgcc ccatctttcc 87120
ctcaagcagt accgtcaacg tgccgctgtc atcgtccgcc gagtgaaata ctatgaaagg 87180
cgctggctcc ggaccgtaga gtttttctca ggtgctgccg ctcagaacat cgcagaacgc 87240
tttgcctacg ctgggcatgc tgaagagcaa atgaccaagc tcaaggctga gtttgcagcc 87300
ttcccggtgg aggtgaggct agatgctcta ggttggatca attggaatac tcacaatgac 87360
ggcctgctag aggagtggat tcatcaggaa accgaccgag ccatcatgat cgaatatgac 87420
gaaaccacgt ggcgtcctgt agcttgctgg gaaaccactg tcgatgctct tcccaagctg 87480
gcaatgctgt aaaatatcgc ccgttaggct gaaataatgc ttgacgggcg tttttgtacc 87540
tgtagaatag gcctcaagaa aggaaaacag ccgaaaggct caatctgaaa cccggagatc 87600
gataaaatgc tgctagttca tggaaccact gcaaaaggcc tgaaagctat catgaaccgt 87660
cgaggcaagg agggattggc cgcaccttgg gacgtttcgg accatgacgg aatgtcctac 87720
ttttacaacg taggcaagat ggagtggcac gacgaagagg agtatgctaa ggcagaatgc 87780
atcgttcgtt gcctagaaca ggcccaattg cagcaggcta tgatggccga aggaggtaaa 87840
atctatgctc tggtggttga cattcccgaa gagtttcagg attgcgtaga ggatgattat 87900
tcgtgcccta acgctagcca ccaagctagc tgttttccag aggataattt tgatccggca 87960
tggattgtag aagtgtatga ggcagatgtg agtgtatggg ccatcccttt catcgtagcc 88020
ggagtctcag gtaattctat gttccaatcc caccaactac ctgacaaact ggtggcgata 88080
gcggagtcgg tggcgcggtc cagtgacata gacacatacg aagctttcga gatcgaccat 88140
tcacaggtag agctatctat tctcgaaaat ttcaaagata acgcttgaca gtctagccaa 88200
ctagcgtaga atgcctagcc aaggggtgca taagcgcccc acctcgaaag gatacagaaa 88260
atggccatcg ttgatcgaac cgcaggtaat gaaagcccga atcactgcta catcgttact 88320
ggcagcattg gaccagagtt tgagccgacc ctgttcacta cccatgcgct ggcaaagaag 88380

tatgcccagc agatagtgaa atacggtaaa gcaaacgacg ttttcccgcc ttttacgcct 88440
gttatcgaga cccggcacaa ggcagatatg aatcggttca ctgttcacaa caaaatcccc 88500
gaataggaga cgtatctatg aaactttctg aaattttcca aaaaggcctg aactatgcaa 88560
ttgagtacgg aatgccgtgg tatatgtgca tccgtctccg ggagattcat ttgttcccct 88620
cgcccgttga aatggcagtt aactcggtca tgatgtctgc cggtatctca tcaaccccag 88680
agggatgcga attcgccggc gttgatgact atttcgagga aagcggccta gcagccgcat 88740
acaaaatgaa caatcaggaa atgtccgaat acgggcgctc atcctacgca atgtgggtgc 88800
aattttggtt cttcgtaatc tttgacttag cccgaaaagg gcagtagaat gcattacaag 88860
cgtagaacag ccagcaaata atgtgatcta gagggacggt catgggtgac aaaactgtag 88920
tttatcgggt ggagtgcagg aacggcgagg gaccgtatgt taactatggt atggagattg 88980
atacgtcctc cactcccgag cggcccgtac cggagcatga cgggatacct gatgtccgcc 89040
attttgagta tttcgccttc acgtctccta aacaactaga tgactggttc ggggactact 89100
acagcaatct gaccgagcac tccagcgcct caatcaaaat gtatgaggtg gacaggcagc 89160
atgtaaggac tggtatccgg cagtgtgtat tccgcaagga tgcggcggtt tgcgtcaagg 89220
aagtaaccat tgcccaatgg gtgggcctac tcaaggctgg catgatcgct gcggttgtca 89280
cacaagctct ggccccgatt ccttcggagc aactacccct agacctaggg gacgatccgg 89340
cctgtgaggc ttcgaaagaa attttgaaaa tcttcggttt gtagggttga cagcttgggc 89400
cgttcgcgta gaatggccct caagcaagac aaaccgccgg caggcaaaac cacaaacccg 89460
gagattcacc atgaacaagc caatgaacac tttcgcatac gcccactacg tcgcccgcat 89520
ggacaaggcc tctggcacta gcctgcccta cgccaccctg ttccgcatcg ccctgcgtaa 89580
cgcctaccgc aagccgggtg tccgccagta cgattgtgca gcactcgtcg aggatatcaa 89640
ggcacgtgat ctggtccaaa agcgcatgcg ggctgaaaag gctgtagtga tggcagcagt 89700
aatgcgtgca atccgtactg gcttcaccgt gagcgtgaag gatggcagcg atggcgagtg 89760
ggtagtgaag aagtctaccg atatcaagga aatctctgac agcctgcaat cgaccgatga 89820
ggatatcatc cgcttccgca agggtgacgg cccggacaac atcgtcggct ctatgtgggc 89880
aatctacggt aacagcgctg gcgaagtgat cgccgactgg accgaaaacg ccaccatggc 89940
catgattatg gctcccgcag aacgcaagat ggaaaaatac gcggaattgg gtatctaatg 90000
cttgacgggg cccgaaaagg gccccataat gcacccatac cagcaaacaa catcaggact 90060
gacccgatga gccagatcaa aatccaagag tacatcatcg ccgagatcaa ggttaaccat 90120
tgtgctcacc tgcgcaaggg ccagaccatc caagcccgcg atgatcgtag cagtgaaaag 90180
tgggttgcat tcatggtgga gcgcgggtat agtgcctctg aagccgctat cctgttcaaa 90240
gacgcccatg atgtggcaat gctcgaactg gcagcctgaa gaaaatccta ctaggagtac 90300
tgacaaatga aacgccaata tactgattcc taccagtaca aagcaattga cggcatgatt 90360
tctgtcctcg gcggtaagaa aattcaagta ccgccgtcct tgaaagcctc tcgaaaccgc 90420

ctgctgaaaa agatggtaaa atacgtcagc cggtcaggat ggaaaccgta accgacagac 90480
tggcatagcc ccggaatgcc cggggccacc ccgtccataa taacccctgt aggagataat 90540
atgagcgagc gtttgatgaa actgaaagag gagtattccc gtcttgaacg agagtggggc 90600
ggattggctc ggtacgagtt ggaaatgctc cgcatcgaaa tcgaaaccga acaaaagatt 90660
agcgaaacta accaatatct ttccatattc ggatgcggca atgccttcag tggccgaaag 90720
tatctctaac ccttcaacag gagatagaaa aaatgaatat tctgtttatc cataaagact 90780
ccgcctacac tgttcaatcc atccgtgaat tcctgcaagc ggcagtagat aacatccggg 90840
catcgggtct cattgtaaac gaatatagcg gcgacggttt gggccctaac gctccagact 90900
accgaatagc tatcgtcacc ccggacgagt tctaccgcac tttctatatt caggtggagc 90960
tggccacgct gtatcagagt gatcctcggc tggtgtttac agacgctgac agcccggctt 91020
atcatctaac accggcggag cggagcgaga tttgcgatag ttacatggac gcttggcagc 91080
atatccgtct gaaagatatg acagcaatgg ccagcttgtc ggattggtca acgcatcttg 91140
catcgccgag tttgcttatc gatgttgacc cgcagcccat catgcgtcat cagagccaga 91200
caattcacta accccatagg aggcctaaag atgcgcctga agacactatt cgaagcccgc 91260
tgctgccgta tcaggtttgt caccagtcga gggctcgctg tcaattctcc cgccgtggga 91320
tcaatagaac aagcccagca atgggccgcg catatgtgta atcctaatgc taccggtatc 91380
cgcctatcct tcttcgacga agaggagggc tgcgaagtga ttatctgtcg gctcaattac 91440
gaaggagaaa tcgtcaaatg aagagcgagt atgcaaagag catcgccgag gagtttcatt 91500
tgacccgccg ccccggggcg gtctccctgc gtgcagcgct acgctatcct gatagccgag 91560
tcgtgaaggt ggcatcgctc tacgcctgtg ctgcctatgg ccgcccttac aaggactgta 91620
ttggtgccta caaggctgcc aagcggcggg agaaggcaat gcgaaacccc gccacgtgga 91680
agctcagccg ggaagctaag cgggccgtac agtggctaaa agctgccatg aacaaacgca 91740
aataaggcag aaatacctgt tgacgtgaga ctaaagggtt cctatactgt gcctatcgag 91800
acgcacaagg cgtctcctaa gctagaagga ctagcaaaat ggcaaaggca aatctgaagt 91860
tctcccgcat tggcaatgac ggctacgact acgcccgcag ggaagacggg gtttggtttg 91920
tcagagaagg gtactacggg cggtacggct gggctaaaac caaatggact caagttcaac 91980
ccggggatgg catgactata gtcgatggct gcctgaaggc tcacgaagct ggaagctcca 92040
gcacatacat cggattcggg aatctggtgg tattctccga cgggaagggg ctccgtttac 92100
cctaaacagg aactcaaata aagttgttga cagcttaggc cattgctgta gaatggccat 92160
caagcaaagg gaatcaccct gatcaactga ataggtaaaa gacgatgcaa gcaacctacc 92220
aagccctgaa aactctgcgc gatgcttgtg aagctgtcaa ggatgaaaaa ggcaccatca 92280
acggaaacaa attgaatgca ctgcgtaaca aggctgtcaa ggaaatgcaa gctggtggtg 92340
aaacctactc tgacgccatt agcatggctc acgacttgat taagaagtat cgcaagtaac 92400
cgtaaccccg gttcgccggg tcatcacaga gcatgaccga ggagacaaca tgaataactc 92460
gcctgtagtt gtatgtctta agtgcggagc cgtaaatggc ggacagagca aacacaatgt 92520

tgtagtagct ctggggaaga agtggtgcgg tcattgtggg tccaatctcg caacccttcg 92580
tcgctaacca ttcactgaag cctaatcgag gaaagatcat gaacgccact ctggcacgcc 92640
tacagaacga acgctgtgca accctccgcc tgatgctcct gccggagcat cgtgggtatc 92700
agttctctgg ccatccttgg gccgagcgca tccgcaatct ccacgatgaa gctaagtgca 92760
tccgcagcca tcactaaacc gcactaatcc attaaagctc taacagcccg ccttgtgcgg 92820
gctttgttct atctgcgaaa gcgacagctt tcaagtgtaa ttaacatgta atgttgtaac 92880
actctgtaga aggtagcacc agcatgacaa gatagaccac ttgtaacgtt gacgttacat 92940
gtataacagc atgacagcga aagctgtaca gatagcattc tgtccagttc acccctatca 93000
aataacccca caataaccca ccttaaacct accggtttta agcggcctgt gaggccgtat 93060
atgaagcgat agcttcgcta tgttagtctg caccgtcaag gcgaaagcct tatggcatga 93120
ggcttgtagg aggttttaag gccatatgcg attagcatag gatgttggtt gttttgagta 93180
gatttttggt ataacccctt tccgcgctac cactttgccg attgtgttaa gcgacagctt 93240
acatccgttc tcattttgca atgagaattg cgtacagttc atcatgcgct tcagaagtga 93300
cttcactttg catgcggatg catgctatag tataccttgt gatagctccg ctatcccgtt 93360
ttgcatggca cacgaattgc ataagcaatg acaagggt 93398

<210> 2
<211> 82124
<212> DNA
<213> Unknown

<220>
<223> Complete nucleic sequence of PAK-P2 phage

<400> 2
   atccgtaaca agccccgggt gaccggggcc tctcatagga aaagaccatg aaaaaacgtt 60
   caaatctgtt gttcgaagtg tggagccatc aacggcggta acagcaggca caattgggtt 120
   atcgcattag gcaagaagtg gtgcggtcat tgtgggtcca atctcacaac ccttcgtcgc 180
   taaccattca ctgagaccta atcgaggaga gatcatgaac gccactctgg cacgcctaca 240
   gaacgaacgc tgtgcaaccc tccgcctgat gctgctgccg gaacatcgtg gataccagtt 300
   cactggccat ccttgggccg agcgaatccg caatctccac gatgaagctc ggtgcatccg 360
   cagccatcac agctaacccc attcccagcc cggcacagcc gggctttctc tcataggagg 420
   caagtgaaca atggttaact atctgatcct ctttttcgtc atagcaatgg tctattgcgt 480
   gataggcgct ttagtctctg gaatgctact aatgctaaaa gaggctagct ctgaatttga 540
   atggcctacc cgcaaagaga aagatgttat ctacgtccta ggtgcaatat ggcctatctc 600
   gttaccattt ctcgcagtag gcattgtcct aggcataccg gcgatgggaa tcgcaaaggc 660
   agtgaaacgc ctaatcaaaa acaactacta agcccactaa aaccaacaaa gctctaacag 720
   cccgccttgt gcgggctttg ttctatctgc gaaagcggag ctttcaagtg taattaacat 780
   gtaatgttgt aacaccctgt agagggtaac accagcatga caagatagac cacttgtaac 840
   gttaacgtta catgtataac agcatgacag cggaagctgt atgttcacat tctgtataga 900
   taaccctatc aaataacccc acaataacct acctaaaacc taccggttta aagcggcctg 960
   ataggccgta gatgaagcga tagcttcgct atgttagtct gcaccgtcaa ggcgaaagcc 1020
   ttacagcatg aggcttgtag gaggttttag aggcatatgc gattagcata ggatgttggt 1080
   tgttttgggt agattttggg tataacccct ttccgcgcta ccattgtccc ggtgatgtta 1140
   acgtttcccc gattgtgtta agcgacagct tacatccgtt ctcattttgc aatgagaatt 1200
   gcaactgatc tcccatgcgc ttcaaaagtg acttcacttt gcatgcggat gcatgctata 1260
   gtataccctg tgatagctcc gctatcccgt tttgcatggc acacgaattg cataagcatg 1320
   acaagggtaa ccctcgaaag cggagctttc aaagtgtagt ctctacctct attatatttg 1380
   cgaccggaag tagtgacact gaaaaccttg actctagccc agtaaatacg ttaaactaaa 1440
   gggttgatag ctgccgctat cgagtaacca acattcacac aaggaggcac aatgacaagc 1500
   aacaatcatg ccaagattca catccattat gctaagcaaa tctattacgc catgcgtaaa 1560
   cgtgccatcg caagcgatga gatatgcaag gaatgggcca agcccaagac aggctttgaa 1620
   gcgttctatg attgggtggt gaatgtgtca gggtacgccg taggctaccg cctagacagg 1680
   aatattctct tcccgggaaa caagctctat gctccgcata catgcgagtt cacacctaca 1740
   tacatgcata ctctattcac caactgcgac aagtcatgca ctaaccgtgc caacaatgat 1800
   ctacctctag gcgtccgcct agcaaggttt gaccgatgga gactgcctgt atacgtatca 1860
   cagtgtagaa cgctagggaa gcaaaccaca ctcggatcgt tcgacgatcc aatggcagcg 1920
   catgtggcat ggcaacgtgc caagatggaa tccatccttg aatgtatcga tctctatcag 1980
   atggaagacg ttcacagtgt aaacgttgtt caagcgttgc ttgaaagggt aaaccaactt 2040
   ggcaaggata ttgcacaggg gaaagagacc acattgttgc agtagatcga cggggccatg 2100
   gtcaacccct cttagagtgt cctgtcgggg tgcgagtaat ccgatagcga agctatcaaa 2160
   tgtatatgca tattccatgc caacatgtgg aaagatttta cccggcaggg cgggagcccg 2220
   ttggcattag ttttgcatag cttagctgtc gctaagaagg aaagaccacg gccacccatt 2280
   attttggatt tactaagggt taggatggcc cagtgggttc gtggttaagt agctcggaaa 2340
   taaatccata actggcagaa ggcgaaccag cagagggcag gcccgctccc tccattattt 2400
   tcccagcagt agaggagaca cactctacaa acgtatcact ctcttcggcg gggggtttag 2460
   gattctaata tacctttagc ggctaaatgc catcattgct tatttcctat tttcgtgcta 2520
   taatccttat aggatgtgct tattcttcat tatagctgga taagcgatgg ttgtatagat 2580
   tcaccacaaa tagaaggaat atagaatgga agataatatt caagctccgc ttgcagctag 2640
   tgcagatggc aaattcgcaa ctgtatacga agctattgtc gcactcggag tagaaggggc 2700
   tgccgctgtg tacagggtag gtgtagggaa tagattgaca acagctagac cataagcgtg 2760
   gtaaagagaa gccccgggca gcgaaagctg atcccggggc ttttttattg ccttgagttt 2820
   atgtatgaag agattccagt tgtctctgct cttccatata ctccctagtg gcgtagttca 2880
   tcatgaggtt gcaatactct tcgtttatct ggtccatgta ttgcaacact ttctccatgc 2940
   tctggcacgg actgaatccg ttatgacgga gaatcttgtt tagattttca cttccccaga 3000
   agtctctggc ccaagcctcc aaagctactg cgtagaggct tttgatgcac ccgatagggg 3060
   ctggatacca atattgctca gacattagac cctcccgaac gttacgatca cgccttgctt 3120
   ggctagggtt gcttgtagct catccagtga gaggggctta ccgcccacga tgacatactt 3180
   atctgcctct cctagagtca tcacacactt agtctggaag gtaggcctat acaaggcgta 3240
   catatcgggt tgccacttgc ttcgcaggcc tgttccgtag ctaatgaacc ggtcttcgga 3300
   atgatcccgg atgtacaggc accggggact atcgtctggc ccatctacgg agatagggtg 3360
   ttccatccac tccctgatct tatcaacgac gccctctcct accttcaatt gagcgtactg 3420
   acagtactca aggaacgcat caaactgttc ggcagtgaaa ttgctgacat cgatctcttt 3480
   agttttcatt ctcttcctcc atgtcagcaa tgatttcctt caacgcttga agcgtgtagg 3540
   tgccatttag ctctgtagta ggaccagtgt cacactcatg aagtgtgtac gcggtcactt 3600
   tcttctcagt gacgaaatgg aagtctaccc ttcggaagtc gtaggtcgat ttcgagtatc 3660
   caatggcctc tttgagttct tcctcgtacc acaggaatgt cattggactt ccgtcgcttt 3720
   tacgagtgta gtccagcaga ttatctccaa cccacagtag cacttgagcc ttgtcgtaca 3780
   tgaagctgtt tccgctccca aactctatac cataagcctt aaggtgctgt tgtactgcga 3840
   tgttctcttc aggggtcttc acgatagtgt aaaacttcac caaacgctct ttcaatttca 3900
   ttcctcctgt ctgtgtttta tgggagtgta aaacgttccc agtccattgt caagctctct 3960
   gtggggatag agcgttccag acgctctcta gcctctttta gaatgatatc ccactcatcg 4020
   ccttcttgga ctcctacata cttctccatc atcaacgcac ggtcaatcag accagccttg 4080
   tagttatccc ggctacgctt gcgggctacc agcttgtcga tgtagtcctt cttatctgcc 4140
   aaagcggcga tagccttcct agccataagg gcagcgtatt ctttttcctc cgaagtcatc 4200
   cacggcatat tggtcagtac gaggtcagcg gcgtccctga ccaaggggat atctctccat 4260
   aggaatccta ccggtctcag gtctgagtct gcggtagctc tccactgtag cactgttccg 4320
   gtcttagaat ctgtaatatg caccttctca tcgatacggt gggccatcat cgaaaatctc 4380
   cagttgcggt ctacgatcca tcgcttccag tcgaagaatg tagcacaaat agccttaacc 4440
   gggtctccga ccttagcatc cagttccttc agacgtttaa tctgcttagc gtttatatac 4500
   tttacaggcc gggaggctgg atcatagcac tcctctacct gaaactcctc agcccgagac 4560
   tccaacactt taggctcatc gttactttct tcctctcgga accatgcgaa cagcttctta 4620
   aacatattca gtcctcctct cctgtgatag ctaccctagt cagtctccac acttttctag 4680
   ggagactaac gaggaatagc atagccataa acggtacggc gataggccag aatattccag 4740
   ctaacaccgc tcgggcctct ttcttacgcc tctccgtact ttctcccctc cacccaagaa 4800
   ggggtcgtag agattcccat acgaacatcc ctatcacaga gtagaacaat aggcagacca 4860
   ctccgacgac tatgcatact agaattagaa gccctacttg ttccatggtg cgatcctctt 4920
   aagtagttta cggctgttcc agatagacag agcccactta ggtaacaaca ccactttcaa 4980
   aacgctgtat gttgcgtaga gtagcagaaa gctagcatac actgtgaatg taataggcca 5040
   tagcagtccc agtacaatgg caaaagcctc gataccctct ctgtgatctt tagtcgcact 5100
   agctgggaag ttatctttga aatactccgt gatacccatg taggtccaca tgaacacaat 5160
   tccccacgcg agcgcgagga cgaatagcat tgggattatc tccattactc ccctcctttt 5220
   cgaatcaagt cgaagtaggc ccacacgtag aagttgcacc acgctctgcc tatggcccag 5280
   ccttcccaat agtcttccca gtccttgttc ggagggttat tctcgaacat ggataacacg 5340
   catgaatcag ctacttcgtc caagaggtgc attaccgaat taagcggccc gattccaaag 5400
   gtcttatcta gccacccctt aaatcgtccc acgtctgaga cggtgacgct ggagtcactg 5460
   cacagtgcca atctctccag aatgtagcac atgtaaggct cttcttcgtt ctctaagaat 5520
   gcccgcatgc cctccttgat tagcggtgca atagaattag agttcattgt tttcctcctt 5580
   tcgaatcagg tcgaaataag accagacata aaagttcacc cagtttagat cagtatctag 5640
   caggtaatta cgatgaagaa gagagataac tgaatcccac gacggaatcc catgctcctc 5700
   tagtagccac tctctgaatg accgatactc tccgtgggag atttgccctt cttctgctag 5760
   gtcgcttaga atataacaca tatacgtgtc ccggtcgttt accaagtaat cttccagtcc 5820
   catctttatc aactctgcaa ctgatctcat tgtttcctcc tctgagagcc ccactagggc 5880
   tcgaaatttt tggttcgacc tccggtcaac acatcacaac ccagttgtac aggtcagcct 5940
   cctcataagc tgatgggtag cgattgtact ccaataagaa cgcgtggtca atagctgcgt 6000
   atttggttgc gaagctgccg aagtacgttg taaatccgtt atcgcctcgg ctaaacagga 6060
   cgtattcttc agtgccgtcc ttgaacgtat ctagcgagat aatatacttg tcgaagatca 6120
   cagcatttcc tcccggctga tgtagtagtt gtagtcgtcc tcttggacga ggaacgcacg 6180
   gtagaagcct ttctcaaagt ctcctttcac tagcaccaga ttgccttcca ccttgcaggt 6240
   gtagatgtac atggtgttcg ctactcggat aacaggatca tccccgggac gaacatctag 6300
   agcagagagc atagcttcca tgggcatgct tgtcaagtgt gccactacag cggagcagta 6360
   ttctgcatca cggccagaag tatcttctgc ataagcggga gcgaccccgc atgaatgcag 6420
   ggcaagtgta aacgcaatag ctacgagttt cttgaacata ctaaccctcc tcttgatctg 6480
   tctggggaac atccggtcta gggtagacga atgtctctgg atacttcata catcttagct 6540
   catctttgca aaatccgtca atcagtttct catgtgcgaa ttccgcagcc ttgtagtagc 6600
   gattagcctg tcttgcctcc actcgaagga ggtggcttat cgtgtgagcg ttgatattac 6660
   taagacttgc aaaaattgtc aagagcttct ttgcagaaag aggaatcgac ctttcgtcgg 6720
   gtctgtacca atccagccgg gcaactcctt ggagaacgtc atccacccac agagggtagt 6780
   ctccgtctag ctcgaccatg acttcgtaca tttcaggctg aacagcgttg aaaaagaaag 6840
   agggctttcg ccctctgctg gtatctagag gagggacaag ccccctctcc accttgcgaa 6900
   tcaaccgatc agcgttagac cctatacggg cttttagctc ttcgatttct tcacatgtca 6960
   acatacggag ggcacacctt agatttctcg atagtgaata cctcagcgca tgtaaggttt 7020
   gcaggtccgt accatttgaa catgttgttg atatcgtacc gaatcagttg cctgaacttc 7080
   tgaccatcac acatgtctgc gtcgaaccat gcaatagccc atctaacgtg ctcttcgggg 7140
   tcgttctctt ctcgccatct gcactccaga tcaacgtggc tgggctctag aacgtaaatg 7200
   tcttcgaacc cagtgctctg gttgttctta agtcggatga ttcgagcgcg aatccattgc 7260
   atgcacctgc ggtttgggtc tgtgatatcg cagactaccg gaacctctgg aacctcaact 7320
   gtatacatga ttgtacctct atttgtttta caagaacgcc atgattctat ctctatctgc 7380
   atggcggtac agattgaagc aaggtacgcc tctggaggac gccagttgcc aagcggtcct 7440
   agtcccgcct tctggaatcc cattgtggtc tgtcttgctc cagcacacca agaacttgct 7500
   aggcgtgttg agatcgattc ccagtacttg gaaaacgttc cgagtgtggg ctcgtcttgc 7560
   gaacgggctt agaacgggtt ccccgtcatt tctgacagaa ttccaagctg ggtggatttg 7620
   actggcaatg cttctagctt cttcccattc atctggcaac tcttcctctg gcaggatggt 7680
   agctccaagc gtctccgtta tctggtactg attgaagccg ggagacggaa gatagatttc 7740
   tgccatagtc cctcccttgt tattggatat ataatccagc cagcccgatt cgaacgctga 7800
   gtccgctccc ttcgctcctc ctgaccgaag aataaaacct tgttctgcca gtttacggcc 7860
   tacctctcgg aggatacccc attctctgtt aggagtgtcc ctgctcccga ctcctgtata 7920
   atatctcatc gtctcaccat ttgtcccagt tctccgtatg taatccagtg gtgacgataa 7980
   tccccttctt ggttggtgta ctccactaac accggataga agttgtcttc gtggtctatg 8040
   cttaccacct tgcattcaca tcctctggga atagtgtatc catcactgcc catggttaag 8100
   agcattcccc ggtacacgtc ttttatctct agctcacctt ctccagactc ttcaaacttg 8160
   agtctcaatt tctcgattat ttgagcttgc aaatagagaa gttcgtacgc tgttaagttg 8220
   tccagatcaa tagacatgtc actccctccc ttctagtttc ttgaatgcct tgtccggctt 8280
   ggctagatgc ttatccaact tgtctagctt tttatcctga taaagctgga acgcctccga 8340
   gcccggggcc aaccaagtcc ccttatactt aattgcaccc cggatcatat ctcctcctat 8400
   tacagttcgt aaacgtatac ctgaaactgg ccgagtgtct cctcgataag aggctcgaca 8460
   ttgctccagt cctctccggc taaaccgcat ccaatcttcg gcagacctac acacaactcc 8520
   gaggtaggaa tcgcccgagc gtcagcctct gcctcaagga acagagacat agtgtacagg 8580
   gcttggccca gtgcgtcaag ctgagtgtag actcgtccct cttctcggcc atagttgtac 8640
   tgcccgtaca gattgtaaat cgatccatac tcgttaattg tgaaggtgag ggagccgagt 8700
   ttgcctcgat cccctttcac cgttgcttgg tctgcctcgt aggcctccgg gaattgctcc 8760
   cggatttgct tagcaatgcc gctgcccatg gtgttgaagc aattagcctg atgtgcaata 8820
   gcgtgcaact ctttgttttt gaacgctgcg ataagatcgc cctttcggta tttaatcatt 8880
   tgaacctctc tacacttgtg aacctgactt cagcgcctct cgctccgtga gcaatgtccg 8940
   agtacatcga gttcatggcc gcaaacggag accaatactc cttctcccag attctgaact 9000
   tgaacaattt gtgccctttg ttttctgggc gtccttgctc gcagaaggtt gtaaaatagt 9060
   atttagccac cacctctcct ccttatacgc cgttcttaat ccggtagcgg atcgactcta 9120
   gatcattatt cttcaggtct tcggtgttcc attttccgtc tttgaagaac gtcctcaagt 9180
   gcccagtctc ttcctcttcc ttcgttacat tgtctttcag tcggaggaac cgtccgttct 9240
   cttggtctcg gtacactttc aagagacctt tagccgactt cttcttgctg tcagttttcg 9300
   ggtctttgaa gatcgcgata ccttcaccat tcacttcggt gtaggtggct ttcactgcaa 9360
   atccgaaagt atcccgggtg ttgcactgat aggtgtagct accaactccg aacactacgt 9420
   tcagggaagc aaaacctttc tccttcaagc gctccaagat tttagctgct cggcgagttg 9480
   taatggaatc cccatagatc aatccgatat gagaatcgag catcttataa cctgcctcgt 9540
   tcacagtacc tccgaaggtt tcccacagaa cttggatggc tccttttacc tgtgcctcgc 9600
   tcacttcttc gagtttatag ccaactacat actcgtattg atcccactca gtgggaataa 9660
   ccttgaagta cttatcttca aagttcagaa caaaggcgtc gtttccatta actgcatcca 9720
   tgatatccca gtgttcgaga gattcaacaa cttcaatatc gcctagtcca cagataacct 9780
   ctacagggtc accagaatca gggcgaacaa ccagcttgcc atcccgctcc atgatctgat 9840
   ccttgatgga cggcagaatg tcagtaagca cgccccagaa gtcgaagctg tctgccacgt 9900
   acgacagaat accactcggg aacttaccca tcagttcggc aaggaaccga cgttctgctt 9960
   cctcacggcc accttcacct tccccatcat tcatttcttt ctcaatacga aggatgttgc 10020
   tagtagctac agcgtgttca gtcgcaggta cggagcaagc taccagacca gtcgcgctat 10080
   agtagtcctc ggcgtagata gccgctgcca aggtgtccga gcctacgaac gaggtcaagt 10140
   gaccaacacc acttcgggcc gcgtcttcga tcccactcat gccccgggca ctgaaatcgt 10200
   ggaactgaac ggacagcagg aagtcgtcat agcatccagt gtgattcgcg tagtatcggc 10260
   ccatagctgc gtattctgcg gcaatggtag cgttggttgc agtcttccaa gtcatagccg 10320
   aaatcagagt ctcatggtag ttaaccagcc aatagaactc cggcagggtg ttcttgatag 10380
   tgagcaccgg tacgcccata ggaacgctaa cgccctcggt caaagacttg acctccagag 10440
   gcagatagcc gagcttgtgt agggcccgaa gctgctccac agcctccatt tcccggccaa 10500
   aatagccagt caggaatcgg gcataccggc ccaccgcgtt ctcttcttcc gccgcgaaga 10560
   agccttccca agactctacg atctccatcc aagctgcgcg cccgccgacc cacttaagag 10620
   tgccgtcata gaagtcggtg gcatgcgtct tccgataaat cttatcggag cgaggggtca 10680
   ggttactata gacaagctga gtcccttccg agtacatgga accgtgtcca atcttataac 10740
   catcggtgga aagatgtggc agaattttca tgttatttcc tcctgttgtg ttgaataggc 10800
   ccagactaca tcaatctggg cctcctgtca atactcgact tcttctcccg gcagcgtcag 10860
   cacagggtag atgcatggaa ggcgcagcaa tggttgcttc ttgctgtgga agtcttccca 10920
   aaacttatcg tgcatcgtct tgtaccgagc gtccagttcc tccttggtgt cgaactcctc 10980
   caccttgaca accctcttct ctggagccga acgcttatac tctacaatcc agatactcaa 11040
   gctcacaggc gttccgcctt agagtattca accggaatta cagttagcgc ttcgtgctct 11100
   ttttgaggga gcgtgtccga ggtgtagatg catccgaagg aatccagcaa gcagtcgata 11160
   cccttactga agattccgtg agtgacgaac aggtcaggga gcaccttact gtgctctcgc 11220
   agggccttag atacgcttcg gaatgtagcg cctccatcgc aaatgtcgtc gatcactaga 11280
   agcgggagtt ccgggtcaat gtccgaagga ttcaggattt caaagccttc aatttttccg 11340
   gtctgaagat cacgcttctt attgcaacga attacagcgg ctgctccggc cttctttgca 11400
   aaatcctcgg tccgctttgt tgctccgata tcaggggcta cgagttggta ctgctcccac 11460
   tctgagcggt tcagcaggcc accgaagacc gaagattgac ggacaacttc gacgttgtta 11520
   atcaggtgca gagttgcgtc gctgtgaggg tccacaacga taactcggtc atatccggca 11580
   gagttcagca ggtttgcaaa cactttcaaa ctgaaggcgt cgcccggatt gcaccgacgg 11640
   tcttggcgag catacgggac ataacccagc agaagagtca tgtgcggatt aatcatttct 11700
   gctctgatcg cctcggtgac gttgagaaga gccatgatat catcactatt ctgaatctga 11760
   gctttaacgt ttacgaacgc atggctttcg atgccgccca cattaacgcc cacttcgccg 11820
   cccgggaacg tccacacttt tgcctcttgc cacggacggc tcataacacc ttccatttca 11880
   tgaagacaac ctacagaata actaatcata tatcctcctc acagtttcac agagaagcga 11940
   tttcgcattg cttccatggt gctctcaggg acgtcgtgta cagacttatt cccatggcga 12000
   ttctctacaa tcagagaaat aacagtgtag ccaaactctt cggccatgtc aatataaggc 12060
   ttgatctctc gttcccgggt actagtgttt gatacaataa ccgcatctag tcctatctcc 12120
   atagaccttc gagccttgtc catgcacttt ttatgggatt cagctacccg ctcagctttc 12180
   cacacatact tgccgaactc catttggtaa tcgtccgctt cgcagataga cgcctgccgg 12240
   cctgactccc aagccttgtt atacagaatc tcggcaagag aggacttccc ggaacccggg 12300
   agtcctcgaa tgatgtacag aatcttctcc ttcacatatc ctcctttaga ttcgagccat 12360
   gtcgaaatag gtctcgatca gaagacggcg ctgtgcgtca gtcatttctg agaaccgagc 12420
   ctcatccact tcatttacca agaagtctac cagaggatag aactcttcgt caagcgtctg 12480
   cttgaatcca gccgggtctt ggtacatcgc attgaccttc ttactaccca tcctcatgat 12540
   aaacttcttg ccgaggtagt gagttgactt gcacttaaca gtacctatgc cagtcctcac 12600
   catgtaaccc tctttctcgc aggaactcag caaatccttg gcgtgctccc aactacactc 12660
   gacatgctct gggcggagaa tctttacaga acggtgatcc gttacaccta gagcgtgagc 12720
   atcaaggata tgctccgcaa gctgatcgcc agtaaagata tctcgcatac caatcaaata 12780
   cgccccttct tcctcttcga cgatatgcgg gtcttccgag gagcacacct cgaagttgta 12840
   tgtaaatcca gcaatataat catctgggtt attcgacaca tacttctgac caagggcaac 12900
   gtaatcgctg gtcaatgacc ctgtagtact gaagataagg gagccattgt gcttagtcac 12960
   ggagaacatg aatccattca ctttctcgat cagccgcaca cggaatttag gatggaggaa 13020
   actcccagca ccattctctc ccagattaaa caccttctcg aagggacgta caatacaatc 13080
   gccggagtac tggtcaaata catgaccccg tgcctccaga aggagcggat gctcattcca 13140
   caggttttcg tagaacacct tccgggcata cttgataatc gacatgccgt tgaagtgctt 13200
   aacagtcacc agcttattag cgattaagtc tttatacatt gcaatgttga gattaccttc 13260
   catcctactt ctccttattt caattcgaca atcgggcaga atttagcctt ctttgccttt 13320
   agatacgata ccagaatccc atccttgctg gacgatttac tcgacataaa gcgagagaat 13380
   cggtagaaaa cccacacgcc ataaaagacg gcgatgatcc caatccatac aaaccccatg 13440
   acagccagag tcccacctag aacatatccg gtaagtggac cgagagccag agaatacaat 13500
   acagagccta caacagcagc gagcgctaca accgttgacg ccagccacac gacgaggaat 13560
   actgtctgcc agaaataaga gcagagggtt acaatttgcc aaccgcttga gatagccgaa 13620
   tagaccgggg tctcgaacgt acgatggagc cacatatgcc acgaattctt atcaatcttc 13680
   atgcttgttc ctccgggaag atgttgaagt taagtacgtt atgctcttca agttccatgg 13740
   aggctaccat cttataacct ccgagcttgt gaacgatctc cagtatactc tcaatagaga 13800
   atttgtcaac atgctgcttc atcagattgg agattcggct ctgatgggtt ccacaccgct 13860
   ctgccacttc ccgttgagtc agcccagact gagtgatgta gtgtgcagca acaaccatta 13920
   cagttttctt gaaatccttg accggatcat tcatgttagt cctccttatt gctcagcgtt 13980
   actttgccat cgaatactcg gaattgagcg atctcaaact gatccgtcca gcgggcgtcc 14040
   tcgaaagcca ctccttcaaa agtcccgtct tgtttaacat cagaagtcat gaggacgata 14100
   aaaggaactc cgtcgtcatg gatcagttcc accagagtgt tagctttagg tttaacatcc 14160
   tccttgtcac gcgaaatcaa cacattgacc attctttcct ccttttattt atggtggtct 14220
   acaaccacaa actcttgctc tcgcatatat tccctcactt cgtcgtaaga gaatacctga 14280
   tgatccgggt gattgtcaat acctacatca agtcgcttac ccttggaagg cagactaccg 14340
   tgagtgtggc cgtgcaggtg ccaagcgccg tggtgggcag tattccacgt ctcgaaagga 14400
   tagtgacaca tgacgatctt aactccgtcg atcctcactt ccttgtagtc tcggatatct 14460
   tcgacgtgcg ataggttgct gtcttcaatc agcttccaca gtcggctatc acagtgatta 14520
   cccttgagga aagtgatccg accattcaac tgtctaatga tatccagtac agcttgcagc 14580
   ttgttcttac ctgcgaaagc gaaatcacct aagtgataca cttcatcccg aagacggacc 14640
   cgagagttcc accgagagat aagctcctcg gtctgctcct cgaaagtcca cggcctgttg 14700
   caatactcga tcacattctt atggaagaag tgtagatcgg aagagaaaaa tacagacatt 14760
   atgcctccat ttcaaattcg taaattttgt taatcctgcg gatgagtcgc atagcggccc 14820
   ctcccagcag atgttcacgc tccggctcca tactagccat agccttgaac tctgataaca 14880
   tatctcgacg ctcgtagtgg aaatctacta cccagtaatg gtccgcacta atcttaccct 14940
   ctcggtacag atgcccttct acgcccagcc agtgatctac tgcgtctttc ttcagaagct 15000
   ccttcggaga ccacttatct acacctcgca gcttctcgaa gaaagagtaa ggacgagaat 15060
   agctgatgat agcttcctct acagccgatt cagcctgcaa ttggactaac tcccatcttg 15120
   caaggacttc tttaatctcc ccggtggtga ctggggtgta gctgtactgc tctttatatt 15180
   cattccacac tgtactgttc tcctacatag cctttgaatt tgtcggaacg agttgtcttc 15240
   ttccgtgcct tgttttccag aactgatgct tccctttcga gcatgctcag ttggttgcta 15300
   tacttcttga tccgcttcga gttaccactc atcacagaat ttgtaagatt tgcgatagta 15360
   tcgcagattt tcacttctct tgcaaccgga tgattacggc aagcttcgat atacttacgg 15420
   tagctctcag cacggttctt atcgagaatg accagagcct ctacgatgtg aggcatcacg 15480
   cccatggact ccaagtcttc ctctgtcaac tctgtatcct ccagaacgtc gtgcaggaag 15540
   cacacagcga gaagagtgtc catgtacgga aggtcggaga aagcctgacc cgggtccata 15600
   tctttaggag cgttgcgagc cacgcagact gtatgcacct gattcagatg gtaagcatac 15660
   gggaagttgg tctctccgta ccgctgacct tcgtgagccg agagtgcgat ctcaatagcg 15720
   ataccaagct gagtgttcat attagtctcc tttgtagaat gtgtggttgc ctatcttgat 15780
   tgtgcgcttg tatttcctag cccaccgagg aagcttgcca ttattagccc gtctcacctt 15840
   cctctcgttg aggtagtgat cggctcctct cgttggatcg acagtatacc catatagggc 15900
   gttgtatgca acatgtttcg ctgcctccca cttgtcaagc tccttgacac cttgccgcat 15960
   actcccatcg caggtgtagg aatactggca cttgaagtag agcactccag ctactgtgct 16020
   cggccacctg tcgtgcttca ttctgttcac tacggtgtgt gctacagcaa tctgaccgat 16080
   ctccggttct ccccgggcct cgaagtatac agcttcagcc aacaacctgc aaggctttct 16140
   gtactcacac aaggtgcgct tgctttctat gatttgaccg ggactggctt ggaactctac 16200
   tacaggttct gtgccgtatt tgagacgaga gtattccata tctaggaaca tcccaataaa 16260
   agctagaagg atcgacaaaa cgagagcttc taaggtttcc ataacacctc cttagacaaa 16320
   gaagcccgcc ttgtgggcgg gctttatatc acttggttat tataagccca agttgtaacg 16380
   gcgtgcgtaa tccgcgttgg ggaactcaac catcatggac tggacatgaa cgttaccttt 16440
   acgatccttt tgaatcttag caactttcac gatcttgcgg gcctgtgcct tagccataag 16500
   ctcagggctt acaaccagat cgatgaaccc tgcactccga tacccacagc cggcaggggc 16560
   ccagaaacca gccatcgtct gcgtcgagat aggaacgagg tacaggttat gcagacgcac 16620
   agcggtgttg aaactcagct tactgatgtg ctttacttcc cgagtttggg tttcaaaaca 16680
   cccagtcgtg tagttgaatt tgtaagtgag agcatccatg aactgaatga cacttcgaat 16740
   ctggatagca gaagtatcca agagctggaa ctcttcagca gcgtctattg cagtggtcac 16800
   attgacagac ttagcgttga acattttata tcctccttat tcttcaggga caaatacacc 16860
   gccgattacc cggccagcgc gaattagttt accagttggg aactgagcac gcaaggtctt 16920
   tttgaaacga ctttgaattt gcacagtcag ccatttacct cggtagttct cgaagtttgc 16980
   aatctggtct ttccgagcat caaagtcgcc atatgtcacc tcaagacgct cttctggcac 17040
   aggatcaaat actaccaata ccgcatttcc gttcttgtct tcaacgtagc cgacgatttg 17100
   catttccatg tcgaagaact ctttgtactt ctgaagacca ctggaacgtt tacctgattc 17160
   gtacagtcct tcaggactcc gccacatgat gccttcgtag cctttctgcc gatacacatc 17220
   atgaagacgt ttcagttctt caaaggtgga gcagaatacg tactcaacaa aacgaatact 17280
   ccccagatca ctgtgtagtg cagactggaa ctggaagtct accagacgct ccttaaatgt 17340
   caagtcatct tttacgatat cgaagacatg gaactccatc cgcttacgaa gaccgtaaat 17400
   aaaggacgtg ctgtccactt ccttcaggag acgatcatac tcctcctctc cgttctctag 17460
   agcgtaggcg tcaagagcct tctcagcctt catccactta tcgtaggcat cctctcgctt 17520
   gaccgccgag ttgatatcct ccagcacctc accgtggacg tacagttcgc cgtcccacac 17580
   atcaccctct tgcatgatct cttcgagttc tttgatgatg tgcggtacat tgtaaggttt 17640
   acccatgcga gacgctagcg taactacgcc attggctttc ttagccatgc aacgcaggcc 17700
   gtctagttta ggagagccat acccctcttt gtatttagct ggatcaagcg gttttgcgag 17760
   catagccata acagggagat cgttgagttc ttccttggtt gcacgatagc ccttgtccaa 17820
   ctgcttgctc accttggact gagcctcaga cacggcctgc tcctctacag agcgtttctg 17880
   cttaccaact cggatgttgg taacctgaga ggtcatggct ccatgctctt tgccgtattt 17940
   cacgacgata ttgaagctac cgtcttcctc tccgaccacc tctacagtcc actccttgaa 18000
   cccgcctttc ttgtcgagtc catacagtcg ttgaaaataa ctcattgatc ctcctcgtat 18060
   ttgatgacaa gaacatcatt ctcccgtagg tgcctaagac cttcgtcgag ccgatattcg 18120
   tgcttatagt acactacttt gatcccagct tcaaccaccg agatagcaca atttttacag 18180
   caagagtgag tgacgaacag ggtagcccct tgggcactct cagggctgcg tataagagcc 18240
   ctcagtgcgt tttcttcggc atgcctcacc tctgccttgg ttactcccgt ttcgtcctcc 18300
   agcggcccgt ttaggtgctc tggaagggca ttatacccgc aactgatgat acggttacgt 18360
   tttactagaa ctgcaccaac cttggaacga gtagagtcgc tgcacttagc gaaggcctca 18420
   gcggcctcca tgtaagcttt aatatgcttg accttcacga ctagcctcca attagaaccg 18480
   cgatctcctc gtagtacccc tcttcagggc acgcgaattc catgtagtac tcgtcagtgt 18540
   ctgtcaaata gacaacgcta ggcgggtaat agctccaagt cgggtctccg tgttcaatct 18600
   ccgctacctt caagttgcca tacacagtgc gaagagtctg tagagcctcg atcacatcat 18660
   cgatacgcat tacagtgggt tcagtcatat ctcctcctta gttttctact ttacaaacgg 18720
   catcgccacg tcgaaggaac acttgcactg atggctttcc gctccacttg tcccacgctt 18780
   cagctttaac actgacagtc ttaggattga ttgcaaccac ctcgcctacc agcataacca 18840
   ttccagttcc gcctaggcag aatgctactg cgtcgccgat ctcgatatcc cttcctgcta 18900
   cgtccttcat ttgtactctc catctgcacc cttgattaaa ataacgaagt ccgaggggtc 18960
   atcactgccg taggcctttg cccgaaactc ctgctcgtat ttattcaccg cctccggatc 19020
   acttttaacc tcaagctctt ggtagaggtc ttccataaac caacccaagt ccgtggtcgc 19080
   agccgaagcc gagaggtgac caagaagtcc taccagcata ttgatctcgt cgctctccaa 19140
   ctccaacttg tgaccgttac gttttactac tttcatattt cctcctatcg cttctgtccg 19200
   cttacgtcaa agtacgattt ccattcttct ccgaatagtt ctctaccctt atcatcgtag 19260
   actttatgag cctcttctgc tgttttatag caaccgaggt aaattctctt accttcatac 19320
   ctgatccggg caacgtacct gttgtttcta aaaaagatac cgggataccc gtttgtatta 19380
   cgggtagacg cgggtttatt ctgctgctgc tccgagtccg tcgcccaccg gcaattagac 19440
   ttgcaatacc cttttgtcga gtcctttcta tcgagagtct tttctcgggg tcgttctccc 19500
   atgtcctcat agaacccctc gaatgtcttc cacttaggat cgatggtaac atccttataa 19560
   tatttgtaac tcttgtgctt tgggttcgta catctgtcga tcattgtcct ccaagaattg 19620
   tatgtcggag ttccgtacat tccatgggag gatagtagtt tagactgcat gcacccgcaa 19680
   ctcttattat ctcctacctt gagtgctcca gttctaattt tcaaaattgc cccgcagatg 19740
   cattcgcata gccaaagaac ctctcttgaa ttactcctcg ctccagagtc tccgactacc 19800
   ctgagatatg tgaatgtctg ccctgtcagg tctttaactg ccatcctacc tccttacttg 19860
   acgagtggcc acaccgtaag ctggtttgct accctcgccc gtgttgtgtt tcttctgcac 19920
   tcggctggca ttgacggtgt aacgcccttt gccatactct tcatctatcc acgcttgggc 19980
   cttagctcgc tgagacgtgt ggacaaagat gtagtagcca gatgcatcct tgataaacca 20040
   agtggccggg ggctggaagt ccagacgtga aaactcttca tagtcgatca cagtcacctt 20100
   ctgcttagcc actgtatact gccccactac cgactctttt gcaatgattt tatcaatcat 20160
   ctgagttgaa agatcagagt tcttcgaggt caatttctac ctcctttcct agtccagtat 20220
   ctgcaaagaa gctatcgaga ctcagagcgt ggtattgaga cactttgtaa ccaccgcact 20280
   tcagtaaact gttaactgcc tcctctacct tgtcatcctt tagcctccaa tccgcccctc 20340
   ttagcacacc ctctagttgt tctttgcttg gagggtttcc gatccaaatg cctatcctgt 20400
   ggtctggctc gtaatcttct tcccacacca gttcccatac taggttgctc attgatacac 20460
   ctccttcttg cggatagaat agattaccca ctcgtctttt cctgtcttcc cgcacggaat 20520
   gtctatttcg cactctgcgt ttacaatcag gtctgctaca atgcagtcaa gaagttcctc 20580
   gtctggttct ggtaagcccg ggtaggacga tataaccctg cggatagtct ccgctttggt 20640
   attgcccagt accccgagca tgtggaggtc gaactctcca ctgtagtcgt cccagagttg 20700
   tccaaatatc acccatacac tattcatacc tagtaatcct ccttcagctt gttggtgtcc 20760
   attaggcagc ctccttcttc ttgtccagca acttcaggca tcgtttgcag gtgatatgat 20820
   tgcggtagcg ggagaagttc ttctcctcga agtcgccatc ggtgccacag aagacgtcat 20880
   atggttcttc actatccgct tctgtgccgc cgtcccactc gtcgtagtga actgttcgcg 20940
   tcgccattca cttcacctcg attccggctt gctggagggc ttctttcacg gcatcaagca 21000
   ctgcgttgta gctgccgatt atcaggttgc cgtagatgtt tcttgtgggc tccattttct 21060
   ccggcaactc caccctcaga gccgcgcggc tggcttgcca tatccgccag tcatgatgtg 21120
   gtgtgtcgtg gatgtattga tgcggcggat gctcatatcg atcaagggtg aagccatggt 21180
   gcctatcctt aatttgggcc gcgtagttac gcacgaacca cgcttcaaac tcgtctctca 21240
   tactccgcaa tcctcctgaa tctcttttag cagctcggaa ggcataaccg acagcgccag 21300
   ttcctccaga gcatggatgt aatcctcaac atatttccac tcctttctga tcttctgaag 21360
   gtacacgcct tctttactgt tgtcaatagc ccaacccttc agttcctgca ttagttcgaa 21420
   catcttgtat atcctccttt ggccgcaaaa atggccggcg actaatgtcc cggcctgatg 21480
   atactactct gaaatttctt ccattgcaag gcgtttcttt tcaaagaagt cactgagctt 21540
   atcttctagc tcttcgccac caatgtaaag ctcttctcct gccttgacac gctctagctc 21600
   cttctcagtc aagaagtctc tgtagacgtg atctacccat ttatcgcacc acgctgagct 21660
   aaacgctgca ttcctcaggc tattcctgaa atcagcagat acaacgccaa aagaaggcgc 21720
   gtggatcatg aaatatgtga actggtcgat ctcaacggag tcgcaggcta gcgctatggc 21780
   agtcatggca gacgcacact cgccttcaag acgtgcatgc actggagctt gacactcttt 21840
   gatcgctcgg attatagcgt tggcggtctc tacctgtcca ccgcatccgt taatggtgat 21900
   gtacacttca tcattctgac ctgcattttc aagggtccaa agctcctctg tgagttcttc 21960
   gaggctcttg attacgtacg gcagaaggat ggagtaacga ttgctaacac tttgtgtaca 22020
   tgaaacaggc ataatacctc ctttacaaaa atgggcccga aagggccctt attactgaat 22080
   cactcctcgc tttctttctg gccagagccc agcattgcaa agaatggaag gaggattgtg 22140
   ctcagctctc cgctgacagc ttcaccaact tctgcaccag cctgagctac actcaggaat 22200
   tcttcagtgc gtacaaactt gtcaaccttg acgagggttt taaagtctac tcgttccaag 22260
   aacgcagtcc cggctgcttc cgccagaggt tcgaggttta cgacttccat agcgcgatcc 22320
   agagcttcct gattgatctc taggccgttc tgggctgcaa cctgcatcag ggtagacttc 22380
   acagtctgga ttgcgtagtc tttgaaaaac tcacgttgtg ctttgttaaa cttcattcac 22440
   ttttctcctt tgtagtacga cgacggcggg tttttggttt atcttccgat tcagtctctt 22500
   ggacttcctc tacaggagct tccttagcta cttctgtaga cttttcctca gtctgcttct 22560
   ctgcgccttc tcggaccatc ttgacagact tgagagcgaa attcatattg gcgaaatgag 22620
   ggtcttcgag atcaatgcgg aaaccttgat cgaaagcatt ttgcatgccc atcagaaggc 22680
   tgaaaacgtc ccattcttgc acaatgacct cattgccgtc aagctctttt tcaatctcag 22740
   ggctgacgcc ttcctgccgg aacatctgca cttgacagag ggtaccgtac acaggatagc 22800
   cttcaatcga ccgaacgaca cggaaacctt ccttcatgtg ggcgtgaatc gagcgaagcc 22860
   agacgatagg gttgtattct acgatattga taacttgcat attcttttta ctccttgcgg 22920
   gttagtgttt tacaataata ccacgtttct caggaatgtc aacccatttt gttctggtaa 22980
   aggcttgaca tagaggaaat ctgtggtatt atcaatattc agcgtgggag agatatgtct 23040
   gaaagcactc accggcccac gcctagcgcc tatgaagagg aatccacaag ggcaacctga 23100
   actcgaatgc tgttaaaggg tggagacgac gcgaaagcgg aatgacagca atccgatgca 23160
   gcctacatgc tgcccctcac cgaactcatc tgcatgagta agaggaattg gatagagcca 23220
   aggcacgtct taggcagatt aaattctgta tggcgagcct tgggacgact ctatccttcc 23280
   ctagccgaat ctatctgcat ggggtgttct cctgatacac atacaccaga tacccaccac 23340
   agataatcca tcaatcaacc cataaggaat ttctatgaga tgtaagtgct gtgatagcga 23400
   actctcgttt gagactccag tcatcaataa acatactggt aaggaggacg atctctgctc 23460
   ctcttgccgt aggtttgctc tcaatcctac actggatcat gagtattttg gtggttggta 23520
   tccaagcgaa ggggttaccg ctcctctgcc gaccggggac tgacattgta tatagagtgc 23580
   gcgtactctg acaatacata taaataatag attacggtgc gaataattct ttaagggctt 23640
   gacaacagga gatttcgtgc tataatcgga gtatatgggg cttacaaagc ttcagaggat 23700
   ttaatatgac tcaagaaaga aacaagggcg gtcgccctac gaatgccgag ctagaagccc 23760
   ggggtatcat tcaaacagac ctaaaagcag gactcagact tctaaagaag agctttgcag 23820
   agaacattag gttcctacaa gagcaatctg acaatcctga ggtttctcta caaatgagaa 23880
   tcaagctcaa gaaagagctt tctgatatgt tcgtcgctta ctacaaagct gacattgcac 23940
   tcaaaaaaga gcttgcaaaa ggaacagaat caggagataa tgagaacact gaagaggaca 24000
   agactcccgg agtagttctc gcgttttgat taattgccgc ttcgttcaat ggtaggacgc 24060
   cagactttga atctggagat gatggttcga tcccatcagc ggctgccaaa tgtagtaaaa 24120
   gaacattcac ccgacaagga atgtgtctcg ctcagaggac agcgagtaat aagtgtcctc 24180
   cagaattgag ggtcggtgag cgtaactggc aacctactgg attccaaatc ctcgtattct 24240
   ctgttcgaat cggagtcggc ccgccaaaca ctggagattg aatccttctg ggaccagtct 24300
   cgcagccgga gagctaggct atgctgctag cgcccgatgt tctttagaga ctcgggattc 24360
   tctccaaatt agtattgaca ggatgatttc actcctgtat agtgacacaa ataggtgtgt 24420
   accgtagcgg tcttctaaac cgtagagcgt aatggagtca atgctggttc gaatccagtc 24480
   acacctgcca aatgagagga aataatgaag aactgtccaa gatgttcgac tttgaaacct 24540
   ttgtcggaat tctctgtagt aaaaacagga ccgagaaaag gcaaagctaa ctcgtattgc 24600
   aaacgatgca tgacaaacca atcactggaa agacagagag catttaaagc tctttgcgtc 24660
   gagtacaagg gagggaaatg cctcgattgt ggtattgtag acgggcagtg gatttatgat 24720
   ttccaccata gagacccttc cactaaagac tttacgattg gcagagtaaa gagcctaaag 24780
   tggagtgagt ctataaaaga tgaactggat aagtgcgatt tgctatgcgc aaattgccac 24840
   aggcgtcgcc atgcgatgga cggaagctaa agtggatcag catctggctt ttaaccagac 24900
   ttatagtgag ttcgagtctc acccgttcaa ccaattttag tgagtaagtc agataggttc 24960
   tgaggctccc tgctaaggag tacgtctgct agcgcggatg tggatcgaga ccactgctca 25020
   ctgccaaaaa taagtgattt taagtgttga caaactgaat ttttcatgtt ttaatacact 25080
   tatcagatca agaaatactt agcgagtagc cctgagtagg tcgggaacta gtctagaaaa 25140
   ctaggccact gcgatgagcg gtgagggttc aactccttta ctcgctgcca aattaaggcc 25200
   ctgtggtgga attggtatac acatcagtct tagaaactga cgccgagagg attgagggtt 25260
   caagtccctc cggggccacc aaattaagaa tctcttttgc tatttggtta gggttcgagt 25320
   ccctacccgg cgactatggc cggtctatgg cggccaaata gtgagagagc taggggacat 25380
   gacgaaggta gcgctgcttt ccgatctccc cggcgctata aaagttatca tgaaggggtt 25440
   gacaaacaag aaattcatga taatataggt acatagagta acaaacaatg tagccgggta 25500
   gctcaattgg tagagcaccc gaccgataat cgggtggttg aaggttcaag tccttctctg 25560
   gctaccagaa tttaaaggcc cattagctca gtctggacta gagcaagccc ctgtctaggg 25620
   caaggtcgtc ggttcgaatc cgacatgggt cgccaacaag gtcgttgtag agatagatgg 25680
   ctactatcat ccggctgtaa cccggacgcc ataggctaga ggttcaattc cttccttcga 25740
   ccaccaaata gcgagagcgt taagactggc tggcgctgtc actcggctca taaccgaata 25800
   caagtggttc gattccacct ctcgcttcca aattgcactc cgaggaaccc taagtggtgg 25860
   gttcagtgtc gctacctatc tcggtaaggg gaagcgccta agctggcacg ggtaattgag 25920
   tagctgtgat aagctactgg cagccagtat aaatcgtccg gctcgggccc gttatagctc 25980
   ctagatttga attagatgct gtaagccata ggaggtgatc cagtatctcc cggaggggcc 26040
   cgggttaaca acctcatgaa tttagcccgc gttggccgag aggtttaggc ggcggattgc 26100
   aaatccgtct cacatcggtt caaatccgat acgcggctcc aaggtttata ttgggatgta 26160
   gctcagttgg tagagcaggg agctgttaac tctcaggtcg caggttcgaa ccctgccgtc 26220
   ccagccaatt tccaaaagag actcttcgct ttagaagcag agtcgaacgc ccgatacggg 26280
   ccttattatc tcctcgtagc tcagtctggt tagagtgtcg gatttggaat ccgaaggtcg 26340
   aaggttcaaa tccttccggg gtgaccaatt taaagcgggt ttcgtatagt ctggttatta 26400
   ttcttggctt ccacccaaga gactagggtt cgaatcccta agcccgcacc aaaagtcgta 26460
   gacctcagta acccggagta acgcccggga gaagatgtga gttctacgta tatgcgcttg 26520
   aagctaacta ggtagaagca ccgggttgaa attccggagg acttggatcg ttaccaagcg 26580
   ggcgcaccaa acaacgcagt tatagattaa atggataaat cgccagactt tcaatctggt 26640
   gttccgggtt cgatccccgg taactgctcc aatcaagtcc aagccggaac attcctccgt 26700
   atgtgctttc agccggcttg gcattattcg tgtggagatt gtgtagcccg gatgctgcct 26760
   ttcggttgtg acccgattgt tggggttcga gtccccgcct ccaaccaatg tcgtgcaatg 26820
   gtatatcgcg ttgacgcttt taggctctct ggatcgtaag ccagtaaagc gagaatagcg 26880
   ggctctcgca gcagagcgat aggtggttcg attccacaac atgacactct ttcctcctcg 26940
   cctgtgctga acggtttcct cctcctttcc cgtctcacag gctctattcc ctgcccttta 27000
   agcatttatg gtgatgcacc ggctttgtaa cccggcgaat tctgttcaag tcaggaatgg 27060
   ggcaccaatt cactatcaaa gaacccgcaa gctcccgata gggttcacac tctataaggt 27120
   tgtagcatgg aaaacactaa ggttcctgat aaaaaactcg gaccaatcgc gctagcgctt 27180
   aaagaggcca gagagaattt cgatctcagc cctcatgtta ttggtccaaa atctttcaaa 27240
   cagaaagagt tcacagacag cgatgcaaag atcactgtat tcggtggtgc tgctggtgct 27300
   ggtaagtctt atctcggtgt aatggacttc atgaaatata tccatgatcc taacttccga 27360
   ggtgtaatga ctcgtcgtac tactcctcag attaaaggcc cgggcggtct gcttgaaaaa 27420
   gcaacagacc tattcaagct cgttgaccct aaagtcaagt ggaaagacaa agacggcaag 27480
   tttgtgttct cttctggtgc agttattttc cttcgacact tcgaacaaga atctgacaaa 27540
   gactccttcc aaggttggga ggttagtaag ttcctagtcg acgaaggtca gcagttcaca 27600
   gaaggcatgg tgacatacct tatctctcgt atgcgtaacc cgaaatgctc ggttgttccg 27660
   cacatgaaga ttacatgtaa ccctgattac aattcgttcc ttcgtaaatg gattgaatgg 27720
   tggctagacc ccgatacagg catccctatc cccgagcgct ctggcgtaac ccgctggttc 27780
   gttcgtaaag gcggaaagat gttctggggc gacaccaagg aagagtgcat tgcccttcat 27840
   ggtaatccca gtctagcgcc tgacgatgaa gatcaggtga agcctatcag cttcaaattc 27900
   atcgcggcta actgtttcga taacccaatc ttgatgctca acgaccctga gtatgtagct 27960
   aaccttgaag cccttcctag ggtagaacgt gaaaagctcc tagacggtag ctggcacgct 28020
   agagaagagg gagctggata cttcacaagg gattggtgtc aaatcgttcc ccatccgcct 28080
   ctacgggtgc ttaaaagggt acgtgcatgg gacatcagcg gtagcatcga atccgaaacc 28140
   aacagaaacc ccgactggac agccggcgtg ctaatgagcc gtacaaaaga aggcttgttc 28200
   actgtcgagg atgttgtcag agagcgtagg ttgttcggag gtgttttcga cctcatccta 28260
   gagacagcca agcaagacgg tcaagacgtt gaaattcaaa tcccatgtga cccgggtgct 28320
   gctggtaaag cttatgctgc ccagctaatt agagacctag cggatcatgg attccacgcc 28380
   agaccgaaga caactaacaa atcgaaagtt actcgattcg ccccgttcgt atctgtagct 28440
   gaaactaaat cagtgcaagt cgttgcggga gattggaacg aagattactt cgatgaactt 28500
   gagcgattcg acggaagtaa ggctatcaaa gatgaccaag ttgacgctac atcagatgca 28560
   tttaatgctc tatcccttgg cagcttgatt atccctgatt tcctaccgcc tgatatgact 28620
   caatccaaca aattcaaact aaattaatag gaggcggcga tggccgagac tgaaaaaaca 28680
   gcaccgggta tcccgcgact tcgcctaggc gagattggta gtacaggcct taaacaagtc 28740
   aatggtacta ttctggaaga acgccgacca gaacttcggt tccctagggc atgcagaaca 28800
   ttccaaatga tggcggaaga ccctaccatc aaatcagcac tcgatctctt cgaaatgatg 28860
   atgagcaggg ttgactggga agttgatcta ggagttgacc ccgacgaggc tatgaaggcc 28920
   cgtggtaaat tcctgaaaga gtgcatgcat gacatggaac acagttggta ctccttcatc 28980
   aaagaggtca ctagcttcta cacctacgga ttctccgtac atgaaatggt gtttaagcga 29040
   cgtgaaggat accctgtatc gaagtacaat gacaacaaat tcggtattaa aaagttgcca 29100
   atccgctctc agtctactat caccaaatgg ttgtactctg aagacggtag aaacttccta 29160
   gggtgtgagc aaagtcttgc caatgttgtc aatggcgacc gttacgtcaa cctaggtaat 29220
   aaggacggaa ctgtagagat tccagctaag aagatgcttc ttttccgggt cgatgctaaa 29280
   cgtgacagtc ctgaaggtaa cagccctctc cgtgcgtgct ataacgcttg gagatatcga 29340
   gtagagattg aagagcaaga gtccgttggc gtaacacgcg atatgaacgg tatgcctacg 29400
   ctctatctgc ctcctcgata tatgtccgaa gatgctacag aagctgagag ggcagtctac 29460
   gaatactaca agcgagtaat tcgtaacatt caaatgaatg aacaatctgg acttatcctt 29520
   cctcaagcgt ttgacccaga aagccgacaa cctctgttca aattcgaact gacctcttcg 29580
   cagggcagta agatgtatga tacagatgcc atcattcgtc gctgggacaa caaaattctg 29640
   caagccttgt ttgccgatat gctgaagatg ggtcaagacc aagttggttc ttactctctg 29700
   gctggtgcca agactaacat catggctatg gcgattgaat cgcggctccg cgaaatcaaa 29760
   gacgtactag ataacaaact cattcctacg ctgttcgcac tgaacggtga ttatagtcca 29820
   gacttgccga agctgcaata cggtgagcta gatgaaatcg atcttgaaga gttctcgaaa 29880
   gggattcaga gaatcggttc tgtcggtggt cttgaacgtg accgtgaggt ttacaacaag 29940
   attcgcaaag ctctcaagat taagcctcga ccagacgacg agcctgttga tgtggataat 30000
   atcatgggtg gtcagtctca agcaggtaaa gctggagtag gtaatggtgc atcaacatcc 30060
   gcagccggta gagacaacgc cgctgcaaat aacgcataag gagacagaat gctgactctt 30120
   gaaagtgtta ttgaagctct cagcaatgct ttcggggctc ccgccaaagc ggaagaagcc 30180
   ccggctgtag aggtcacaaa gtccctagac aatgaaaaga ggatggcttt gtttgtcgtt 30240
   cttgagcccc aagaaggcga ctccacgact gacctacacg cagacaccta ctccgaagag 30300
   gaagtagaga aagcttgcat caacttcaat actcattgca acgtagctaa catcttccac 30360
   aagattgaaa cacaggaagc tgagattgtc cagtccttca tcgctccgtc cgcattcaca 30420
   actgacgacg gaagagagat caagaaaggt acatggctcc agtggtggca cttcccagaa 30480
   ggctctgaag tggccgatac actctgggaa ggtgttaaat ctggtgaatt cacaggcgtc 30540
   tctatcggtg ccagagctac agtcgaggat atcgaatgac cgtaaaagca aaacgtaaac 30600
   tgaccgatat caagtttgaa cacgaaggtg ctcatgtcgc tctggtcagt aagctccaag 30660
   gtggtgcagc caacgggcac actactctaa tcacaaaagc taccaatggc gtaactgaag 30720
   agcttatcga taaagctgct gaagttacag tcacaatgca gttccctgag ttccttcgta 30780
   agttcttcgg gatgtattat gaagatgccg aagttctctc tgttgccatg ggatatggca 30840
   gaacggagta tccagaacta gatcacaaag actggatcga ccagcgggtc gagtctatgt 30900
   ccattcttaa gtcggtttat aaatcggctg acctgaatca agctctctcc gagcttaccc 30960
   ctgagcagac cctttccctt aaacaggatc aggaactgct tgaaaaggct ctggaggtag 31020
   tctccgagca atccaatgaa aatccatcca aggagattac caacatggaa actatcctga 31080
   aatcggccca cctagaagct ctagctgacg ctattgcagt tgaaaaagct gctggtgtcg 31140
   ctgctgttgc agaagttcag aagcaactag acgccgctaa tgtagagctt acagctcttc 31200
   gtgaaattgt caaggctgct gaggcagaga aaaccgctgc tatcgctaaa gcccgtaaag 31260
   agcaactggc tgaagtcctc ccggctgacc aagtagaaga tactttcaag tctctggaag 31320
   ttctggacga tcaagctttt gcacaaacag taggaacgct ccgcattctt aaagctgctg 31380
   ctgaccaaaa tgacatgctt gtcgagaaag gtgtatcggg cgctggggaa gaagacgact 31440
   ctgttgtcgc cggtgttgcc gctatcctga aagctcgcca cggctccaag taatatctag 31500
   ccaatctaga ggaattccaa atggctcaaa acattatcgc aaaagatcat cagcgtctta 31560
   gcaactggct gaaagaagaa caaatgggcc accgtggcct gttctacact cgcgaaactc 31620
   tgcctgtcgc tgacattgat gttaaaacaa ctggttctgt cctagacagc actggcaagc 31680
   tggtaactaa ggctactatc gccgacgcta cctacatcct aattaccgac ctgcacgact 31740
   atgccaacgc tcagatgagc cacgctgtcg tccttgctcg cggttttgcc aagatcggct 31800
   ccaaggctgt tatcttcggt gctgacgttg acgacgctga taaagctacc gtctttgaag 31860
   ctttcaaagc caagaacatc ttcgccgttg accagatcga aggcgcattc gagaacgtta 31920
   ccttcgcgta agcgagggtc gttccagtaa ataaatacta ggagtaactt taaaaatggc 31980
   taacacccgt tcctacctaa acgatggtca gttctacatt gccgaccaga ctgaaaacct 32040
   gcttatcatc ccgaacactt ggaccctcgt cgagaacatg ggcgtcttca caagtgaagg 32100
   tgtgactcag aacactgtcc agttcgaaga gatcgaaact cgctacggtc tggttaagga 32160
   tgccatccgt ggaactcgtc accaagttgc tagcgaccaa cgtcgtcaac tgcgtgcctt 32220
   cgcgattccg cacttcaacc aagacgacta catcactcca gaagatattc aaggtaagcg 32280
   cgctttcggt gctgaccgcg aagaaactct gaacgaggtt cgtgctcgta agctggaaac 32340
   catccgtcgc aactgggcta acaccgctga agttgcttcc gttagcgcta tcgtaactgg 32400
   taagagctac gccccagccg gaaccatcga gtacgactgg tacgacctga tgggtaaaac 32460
   tcgtaaggtt gtcggcttcg acctgaccaa ccctaccgct gacgttatgg gcaagaccga 32520
   agaaatcttc gtccatatgc aagacaacag ccaagacggt ctgatccgtg gtgacttcgt 32580
   tgccctgtgc tctcctgagt tcttcactgc cctgattaac catccgagca tcaaagagtt 32640
   ctacaaagct taccaagcta gtcctcaata ctggcgtgaa cgtctaaccg ctcgcggtct 32700
   ggaccttcgc ttccgtgagt tctacttcgg aaacattcac ttcatcgaat accgtggcgt 32760
   tgacccgtac ggcaaccgtc tgatcccggc tggcgatgct tacttcatcc caaccgacag 32820
   cggtgacctg ttcgctcgtt acttcggccc gggcagcact ttcgatgacc taggaactct 32880
   gggtaaagaa ctgtacgcta ccgagcgtat ggctgaagac cgtcgttcga tcctgatcga 32940
   aaccgagtcc aacttcatcc acgttctgcg tcgtccacag atgatcgttc gcggcactgt 33000
   gaatgcgtaa agacttcagg gaggcttcgg cctccctttc gtcgtaggag ggtaccaaaa 33060
   tggcttacac tggtgatcca gcaaacaacc ctatcgaccg tctacgagag attgtagggg 33120
   acgtgtggga gcctccgatg ctttccgacg aaacctatca atgggtgttg gacaagaacg 33180
   aaggcaacga aagacgagcc gcacttgaac ttatgcgtat gatgctcttc cgcctgacaa 33240
   ggggaatgag agaacgcaca ggtgatatcg aagtatatgg tgctgagtat ttcaacaact 33300
   acttgaaagc tcttcagctc attctcaaag acccgaatat tgcaatttct ttggccgtgc 33360
   catatgctgg cggtatctcc aaatcggata tgctggctaa tgacctagac cccgacaacg 33420
   taacacggga attctacatc ggttttgcaa gacgggagaa gctttacaac cagtgcaacc 33480
   ccggtcctca agaccttagc atggggtgtg attatcttgg caagcttcag ttttaaaaca 33540
   gatcgccgtc gcctcacatc cttgattaag agggtggagg ctctagacgg aacaacggta 33600
   gaagttggtt tcttccccga ggaccgatat ggttctgaaa atggaaacct accagttgca 33660
   caagttgcag cttacaacga attcggtact actcgcaacc caacccggcc attcatggct 33720
   ccaacctttg aagaattcac atctcaattc cactacgcaa gactaatgaa gtccacgttc 33780
   gagaacgtcc tacgcgatgg aaggcagacg aatactcttc tgaagaaatt gggtaaaatg 33840
   gttgcagagc agatgcaagt taacattgac gactatccgg gctctaacag tccagcatgg 33900
   gcagcttaca aaggatttaa cgatcctcta ttccatactg gaaaaatgct tgagtctgtc 33960
   aagttccaaa tccatcggag gcaataatgc tctatcctac cttctccatg accaacttcg 34020
   tcaagctaga tttgattaga agagggcagc ccggagacga cggttttgga agacctaccc 34080
   ctccagttga gacagtagta acaatcacag ctaacgttca acctatcgaa aaatcgacag 34140
   acactcgtat ccttccagag gccgacagaa gcaaggcttg cttcaaggtg tactccagag 34200
   gcgaagagat tcgccagtta aaggaaggcc cgggagggtg gagtgccgat agattcatgt 34260
   gggaagggga gttgtatgaa gttatgaaag tcatcaacta ttcgatggga atcctaaacc 34320
   attacaaagc aatttgtatg agagtagaga ggaatagcac agcatgatcc acacagagct 34380
   agaaaactcc ctctatctta tggtcaaaga gttgttcccc gactggcgtg taattcaagc 34440
   ttacacgaat aatcaggaac cgcaaacccc ttatctggct atcgacatta agaggctgga 34500
   tgaactggga agggaaaatg tctccaatat gagcgatccc attagtcctt cgcatggaac 34560
   tattcaagtt caacaggact tcgcagccaa agtcgtattc gaactgattg gcaagtatgg 34620
   agagacagca agtgtctctg acatggcaat ggcgattact cgggcaatgc gtactccagc 34680
   aggacacgcc gcccaaagaa agtttaatct atcccttttc aaactcccta gcacaagacg 34740
   agttcctatg ctaagagaaa cagatatgta catgttctat caagttacat gtgaatttgg 34800
   attctctgtg atagaaacca caactcaaga gtttgctgct ggggctgata ttcacggcgt 34860
   ttattacgac gctggtcgcc cgggtcacat tattgagtcc cacatcgaca taaacttcga 34920
   acactaatag gagacaggaa tgactgttct tactgatgta attgacattc agatcagccg 34980
   tgagactgct gctgtcgcac agacaaactt caacgttcct ctgttcatcg cttctcacac 35040
   aaacttctcg gaacgtgcca gagtctacaa cagcctgaaa ggtgtcgctg aagacttcgg 35100
   agaatccgat ccaacatatc ttgccgctgt tcgctacttc ggtcaagctc tcaagcctcg 35160
   cagtctagtt atcggacgca gacaagttcc ttctgctacc gtctctgtca gtgttgttca 35220
   ggaaggacaa agctacgtcc ttaccgttaa cggactacct gtcagctatg ttagccagca 35280
   agacgacact gctactctga ttgccacagg cctaaaggca gcttatgacg taactccggt 35340
   agccggcgta accgtcacag acaacgagga cggaactcta accgtcgctt ctaacgaaga 35400
   ctggagcctg aaagtttctt ctaacctgac aatggccgct gctccttcta ctgaaggctg 35460
   gccggcaact atcactgctg ttcagggaga aaacgacgaa tggtatgcac tgagcattga 35520
   cagccacgct gacgatgata tcatggcagt agctactcat atcgaaggga ccaagaaagt 35580
   gtttgtcggg gctactgctc aggctaatac caaaacttcc gcagacaacg acattgcttc 35640
   tcgtctagtt gctgccgggt tccaacgtac ggctctaatt taccatccaa atgctgacgc 35700
   tcaattccct gagtgcgctt gggtcggata ccaactgcaa gagcaaccgg gtagcaacac 35760
   atggacccat aaagctctgg ccgctgtaga cgcctaccgt ctgactccaa ccgagtctac 35820
   caacctgaag aacaagaacg ttaccacgtt cgagcgtgtt ggtggtgtca accgtacatt 35880
   cggtggagct atggccggtg gtgaatggat tgacgtaatg atcttcgtag attggctgga 35940
   agcccgcatg accgagcgtc tgtggttccg catggctaac tccaagaaaa tcccttacga 36000
   cgctgttggt gcaacaattc tggaatcgga aatcagagcc caacttaacg aaggaattcg 36060
   tgttggcggc ctagctgaag ctccggctcc taaagtcttc gtcccagacg ttctctctat 36120
   gtctccgaac atgagagccc aacgtatctt cgaaggcatc gagtttgaag cccgtctagc 36180
   aggtgccatt cacttcgtac acattcgtgg aactgtgacc gtctaaagac ggtccttccc 36240
   ataaggagac tcataaatgg ctgttcaacg tctagctaca ttctctccag ccgatgttac 36300
   cattgtcatc actcaccctg caactggtga gagcatggtt ctcggcggat tcagcgaaga 36360
   cagtatcgtt aacattgaaa gaaacgctga cacttacgtg atgtataccg gagccgacaa 36420
   cacatcgact cgcgtctaca acgcctctaa gtctgctacc ctcactgtta gccttcaaca 36480
   aacttcgcct tcgaacgact tcctgactgc tctttacaac tacgacgacg cccgtcgttc 36540
   gtctgaggga ttgttcacaa tccacgttaa agacaactct ggtcgttccg actacttcag 36600
   tgatgacgcc tatgtcggtg ttgttccggg ttctaacttc tcgaacagca tgcagacacg 36660
   cgattgggtt attcatgcac acaacctgca aactctaatc ggcggtaacg ccaagctgag 36720
   cccgggtgat gcggatactc ttcgtaacct aggtgtcaca ctagaccagc gctggctgta 36780
   atatagcgct tatgccacat cgacggggag ctgccttaac gggtggctcc ctttgttgtt 36840
   tctggaggat agaaattgat taccacctac tcgccgcgtg acgtggttgt aactctcgct 36900
   ggtatccatt ccgtgacagg gtacgcagag ggagagttta tcagaattgt aaaagatatt 36960
   aagcctttca tcaagcacgg ttcgatggat ggtgaaatcg caagggtcta caacaaagac 37020
   caaggctggc gagtagagtt gacaatcatg caatcctcgc cgaccaatga tatcctttcg 37080
   atgctttaca acgtggacat tgcaacccga atgggaaaat tcccactgat gattaaggac 37140
   acgaagggtt ctacaagctt cctagctctg acagcatggg tggaagacct acctagggta 37200
   tctttctctg gacaactgga gacccgcact tggattctag ggtgctctga ggtcgccatg 37260
   aacattggcg gaaacgtcga tcaaagtttg gtcgagcaag ctatcctgct gggaagctct 37320
   cttctgccag cccttacgca attcggaggc ttctaatggc taacacggta ttgacatata 37380
   gcccatctga tgttaaaatt gtcctttgtg ggtatgctct cactggtgta gtttctttcg 37440
   aaatgtcttg gctttcgaga ccctacacaa tggttcgagg aatcagaggg caccacaccc 37500
   gggtcttcaa tagagacctt tctgcgcaga ttagaatcga ggttcttcag acttcggtgt 37560
   ctaatgatgc attcttctcg ctagtagagc aagaccgaag aacacaatcc gcccgaatca 37620
   ccttgtctgt caaggatacc cacggtagca ccatgatgag cacagacaac gcttatgtca 37680
   atggctaccc tagcataaca ttcaccgatg gaatcgagaa cagggtctgg actattgatg 37740
   ttctggactg gaccgatggc acggtaggcg gaaaccagca agtcggcttc gacgtgtttg 37800
   gaactgttca aggcgctctt agctacctca gataaataag gagtatttat gttcggaaac 37860
   caacgtactg tcatccacaa tgggacagaa tacactatca accttctgcc agcaactcgc 37920
   ggtatcagcg tactgaagca gctaaccagc ttgctgggtc catcccttgc caagtatcaa 37980
   gctgaccacg acttctctgg agccatgaaa gaactgttcg ataacatcga caaggttcct 38040
   gttgagaatt tgatccagaa tctgatgctg acagttttca aggttcgatg ggcaatcaat 38100
   ttcgacaccg aattcgctgg caagtatgac ctgctgtacg cactcacaaa agacgtgatc 38160
   gagttcaact tcggctcggt tttttctcta atcggtttcg cagaggcacc gacggtataa 38220
   agaccgggga tgggcagtcc gagacttcag tcactactga tcgagttcag agtgatcgga 38280
   gggttaggga ggcccaccag aagttctcgc agcattggga gatttatgtg gctatcactc 38340
   accctctcaa gctaacgacg tatggggaac tgagtacagt cctcactaca gaagatttgt 38400
   acaacatcct tgaggttata gacgttcacg atactatcac tcaagtggca aaagaacacg 38460
   cagaggctga taagaaatga ttacagaaag gattgcccag ctaactgggg aacttaaatt 38520
   tacagtcgat tcccggcctc tcactgcttt tgacaagaag ctcgctgggg tcgaagcccg 38580
   gctaagagaa ttctccaagc tgacgaacaa gcggtttgga gttaagctaa ctctagatac 38640
   aaaaactctg agagaggagc ttgccaaggc agccactcaa agggtagtct tgaagaacat 38700
   cgcggtagat gctgccgctg ttcgtcttat ctctgagaag cttcaagaaa gactgaatgc 38760
   aacgccgatc aggttgaagt ctgttaggct agaccttagc ggtatccgag atcagaaaaa 38820
   cttcgtaaag acggctctcg gccagaccaa ggtcgatcta ccggttgaac tgggcttggc 38880
   tcaggctagc cgtacgctgt atgaatggaa gaagcgcaca gagagtcgtt ttaaaattca 38940
   tctgaatgca gacattagcc gctctaaatt gctccagaat gcacgaaaca cactaaggga 39000
   tgtgcagggt aggctaaacg gtcttgctgt cgctacgcct caaattaggc tgtctgtaga 39060
   ccgtgcccat cttcgtagag agattcaaga tgttcttgag caaatcagac gcgaagtgag 39120
   aatccgcatt gacctagagt cttccatccg aggaggtggt gcaggtggat cgagaggtac 39180
   tgccggccat atccgtcaag gtatgggaat ggggattggt agcgagctag ccgggtgggg 39240
   acgcgggttt attcccggcc ttggtggtgc gttcgctatt atgcagctta accgtgctaa 39300
   ccaagaactg caaggtcaac gtctggccat gcaagctgtc ggtggtggag tacagggtgg 39360
   tcaagagcta caagccaccc tccgcgatat ctctcagcgt ctcggtcttg acgatagagc 39420
   aataggtagc tcctatgtta agatgatggc tgccggacaa gcatccaact tcgacaaaac 39480
   acaggttgat ggcatcttcc aatccatggc tgaatatgga cgagtgatgg gtctggatgg 39540
   cgaggccatg aaaggctcct tccgtgccgt agaacagatg atgggtaaag gacaaatcat 39600
   gtccgaagaa ttgaaaggtc agttggccga acgattccct gcggctgtag ccctgatggc 39660
   taagtctcag gatatgtcta tcgctgagct aatgaaaacc atggagaaag gggagcttaa 39720
   gagtgatgct ctgatcccat tcgccagaac tctagcggaa gaagctcgta aaggtggcgc 39780
   tctggacgcg gctatgcaag gtacagccgc tcagcagggc cgattccagt tcggatggaa 39840
   ccgcactata gaggccttcg cggctggagg attcgaccga ggaatgagcg acttcttcaa 39900
   gattgctgcc caaggcatgc aagacgccct accacttgtt accgctctag gtggtggatt 39960
   cgaagcgctt atgcgtccag tcaacgctct cgtcggaatt ggcggggaac taggttcgca 40020
   atgggagaaa attgccaatc agttcaactt gactggaact ggactgacgc tgacgactgc 40080
   tcaaactcta gcacttctaa cccctatggg ccgactggtt tctgccatta gctggggtgc 40140
   gttggcactg gaagacttca ttgtcttctt ggaaggcggt gattcggtgt tcggggactt 40200
   cttgaacaac aacgtacaag ctgccgagac attcgaaaag ctagccagtg aatccagtga 40260
   actgaagaac aatctcgacg gtatcttctc tgttgtcccg gggctagctg aagcgctcaa 40320
   aggtctagag ttcaatgaaa tgctggtcag cacgatgaga gaacttgctg ccatcatgga 40380
   gttcttcaac agtgttgtag agagattcgc aatagccggc aagtacaggg acgctaagat 40440
   tgctgaagcc ggaggagaca gaagtactat aatgtcgaac atcgacacga tgtacgctat 40500
   gttcaatccg gaagatgcaa aagacaaggc caccttcatc ggtgacaggc tagtagctca 40560
   gaacattgac gcgatccaga cagagactca tacccgtgca acccagtcct tgactccaga 40620
   ccagtttggg tatatgatgc aacgtggtca agttagaaac gaaatggagg gtgccctcaa 40680
   gaagagtgcc attgacatta gtttcaacct caatgtttct ggtatcgatg ctcagggtaa 40740
   cgtcatgaca actgaagcgc aagagcgagt acgagagatc gttagtaacg ttcttgagga 40800
   agaaatctcc agagcttcgg caagttataa ggaatcccaa tgacaattgc tatcaaaaga 40860
   gagaacggag acctaatctg gttcgacgct gttacagagt tcggaagaca gtaccgtggc 40920
   tctgtgagtt ccaacccaat cgaaacggga ggaaaaatca cagaccacat tactacagaa 40980
   aacccagtct tcactcttac agccgtcgta tcagacgcag acttcaactt gaacaggccc 41040
   gttatcaacg acaacgaggc ccagacatac aagatcaaca acaaagagtt cgtgaatacg 41100
   cagccggtaa ccatcccggc tgttatctca acctctagac tagaccctag cagaatcttt 41160
   ccagaagtta tcactcagtt tattcctcct gagattcctt ctgcaactgt cctccctcaa 41220
   aagggtgata aagtcgctta tgatattgag cgccaactga tcgacatgca gcgtaatgct 41280
   gaggtcttca gtttgctgga cttccgagac ggtatcatct gggatcaaat cgaaagatgc 41340
   atcttcaccg acttgtcttt cactgaaaac gccgagacag gtagcagcct tcaacctcgt 41400
   atgacaatcg aggctgtgac attcacagat actcgttatg tcgaggtgag ggttaacaag 41460
   ggtagaaaga ccgctaagaa agaaaagaga gacaccaaag aaggtgatac cggggctagc 41520
   aatgctacca gccaagactc tcctacattc aagcgtagtc aaatgaaaga ggctcagatt 41580
   agaactcagg ttgcgaggta aactaaatga gcacgactta tatcgacaca cttcctttgt 41640
   accaagatag gaagtataga tacgcggtag ctctggaggg catttcccgt gtcctccaat 41700
   tttactggaa tagccgctct aggcagtggc acatggacat attcgacgag gagcttaatc 41760
   caatcctaac agggttggca ttgattccac aatacccgat aattgcagat tacgcgatgc 41820
   agcacatcgg gtttaatggg tacttcttgc tcatgcctgt caacctagaa caggtgcagt 41880
   acaagcacga tgcaagcgac attgtacctc agtttttgga acttctgtat gttaatgttg 41940
   atttggagga agatgcagag tgagacaata cgacagagta tataaattga cgctcggtaa 42000
   tactgagtct ggtcagggag tagagattac aaaccttcat cctgacggga gtgttaacag 42060
   agaaggactt cagttcaggt ttgatatctc taaaagctcc gataacaaaa agagcggcaa 42120
   ctcggctact gttgagatat acaatctgtc gattgcaact ctaaaccttc ttgaaaccga 42180
   ataccttact tgcagactag aagtggggta taaagaagct ggaacttccg ttgttctaga 42240
   tggtaatgtt gtcgaaacat ccacaagaaa gagtggaaac gactacgtga ctcagcttat 42300
   tcttggtgag ggttacactg ctctaactga gactaagctg aagggcacag tatctccggg 42360
   caaaactgta aaggatgtta tcgaagagat tcgtcttcaa atgccgggag tggacagagg 42420
   agcttataca ggcctcaatt gcaataaccc gatcatgtac ggctatcaac tccgtggtct 42480
   ggccaaagac gctctaaaca gcgtgtgtga agccaacaat attgaatgga acatcagtgg 42540
   aaacgttcta aacgtcacag acgttaacgg accaacaacg aagagtgttc aaatggctcc 42600
   tctcgtaaac agagagaccg gtctgatcga tatcccattc tacgccagtg ccagcggaac 42660
   ggcacagaag aaagataagc ggagacggag aggcgttcaa ttcaaatgcc ttctcaatcc 42720
   tgagctaact cccggggttc ttgtccgggt agagtcggat agattgtcag gaactttcag 42780
   aatcaacaac gtccgaatct cgggcggata cagagacaac gaatggtaca ctgaatgttg 42840
   gtgttccgat cttaaccaag aggatattga ccaatgagga gaacaggcct acaagagctt 42900
   ctgaacctgc actcgtccac ggaaggctcg aaacagtata ctgccatacc ttgtgtggta 42960
   ttgcgcgttc tggacgattt tagacgcctt tctgtcgatg ttagaccagt ggtcaatgac 43020
   ctgtacaaag acggcacatc agaagagcaa ccagagattc tatctgtccc tgtcattatg 43080
   ccgggaacag ctaacaccct tatcagcttc ccattgaatg tcggggatac ggtgttgtgt 43140
   gtattctctc aaagaactat ggacgtgttt aaaggctctg ccactggaca gcctcacact 43200
   ccaaacgatc ttcgtaagtt caacatggcg gatgccattg ccattcccgg gctgttcacc 43260
   ttccctaggt cgatgaacga ccctgcaaga catagttggc cacatgatac caaagacctt 43320
   actattgccc acaacctgat gacgggacaa gaggcggaag ttaggatcaa agccaatgga 43380
   gatattctaa tcaacagccg caagactata agcattaatg ctcaaaatgt caacgtcaag 43440
   tgtgcttcac tcactgtcga tgcagcccat acaacatgga acgggaacat tgcacataca 43500
   ggtaactacg tgcagactgg tggaacttct acattcaacg gtattccatt ccatacccac 43560
   aaacacggtg gtgttatgcc gggcggtggt gttactgctg tcccacaagc ttaaaaggag 43620
   atcatatgga cttgtttgtc aaccccgaca cacatgactt ggtttttata aatggtgaag 43680
   ctccagtcac tcaacgaatg gttgatattg ttgctcagcg actaaagatc aaactgtaca 43740
   cattcctagg tgagtggttc ttggatgata ggatcggcat cccctacttc gagaggattc 43800
   ttggtaagtc taggagcctt cccgctgttg acgcgatatt ccaatctgag attatgagag 43860
   accccggcgt ccttgagatt acatcttggc agtcggggat cgaccctcac acacgtgaat 43920
   actccatgga gttcacagtc agaaccacag acaatacgga atcccttccc atcactttca 43980
   gaatgatagg agtttaatat ggctggaatt acagcagaag gtctaacgat taaagacttg 44040
   gaccaaatcc tgacagacta cagaaacgta gcctctcagg tcttcgccga cctagtttca 44100
   actggagacg aagttgacac gtctggtaac tctgctctag gccgtctgat cggcgtagtt 44160
   gctccatcgg acgaagctat ctgggaagtt atccagatgg tgtataactc cttcaacccg 44220
   gcagccgcaa ctggcgtggc tctggacaac ctagtgtcct tctcggcaat tagccggcat 44280
   gctgcccgtc cgacccgggc tcaggttgtt ctagagggca acatcgacac cgttattaac 44340
   tcgcctcccg ctaagatgat ttccagcagt acaggccgag tgttccacct gctacaaggc 44400
   gtgatcctca ctcctaaagc ttgctctggt gttggaatct tccctcagac agtaggcaac 44460
   gacctgacat acgagcttaa gatgtacgtt gatgacatta acacaacgtc tatcaagtac 44520
   acaagcccta ccactggtac agtaacatcc gagtccatcc tagcaggact ggccgcagac 44580
   gtagctacaa atcacggaaa taccttgaca agctacgaac agaatggtat acttttcata 44640
   gtacctattg acccattcaa caccaagact ttcgaagaag cctccaacct gagtattcag 44700
   aaggttagaa agcttggtat cgctgttgac gacgttattg gccctgtgcc tcagcaagct 44760
   ctagctatcg atacgatctc cattcctatt gccggatggg acagcgttat caaccctgtt 44820
   ccagctatca ctggccgtct gcgcgagacc gacgatgagc ttcgcgagag attccgaaac 44880
   tctaaattcg tacaagccac caacattctg gagtccctaa tcgacggtct gatgaacgta 44940
   gaaggggtag aggatgttag gattattgaa aacgataccg acaacccaga ccccgttcat 45000
   ggagtcccgg gacactcgtt ccttccaatc gttcttggtg gtattcctac agaggttgca 45060
   cagtccatct ggctgaacaa gccattcggg attggttctg tcggagatac agaggttcag 45120
   gtagtagaca gtctaggcta cactcacaga gtcaactacc agcgacctgt cgaggttcca 45180
   atcgagatca agatcagtgt aacaaacaca ggatcgatgc cagagaacat tgaagatatc 45240
   ctacggccaa ggattgtagc gtacgggacc gagaattaca agatcggaga cgacgtaatt 45300
   tattctcgat tctatgctcc aatcatggaa atcccgggct tccaagttaa cagcctgacg 45360
   attgccaaga aaggacagac tcaaggcatg gcgaacatcg agatcgggtt caaagaagtg 45420
   gctacattcg ccgcagcaga tatcaccgta actacggtgt aataatagaa ggagaattcc 45480
   atggctgtca accaatttga cagagaagac tatctggagg tggcccggga acgggtcact 45540
   gaacagttta aagagaagcc aatctttgat cgcttcctgc aagtgctgtt gtctggtaag 45600
   tttgatatcc agaatgcact ggaagacctc cagactctcc ggtctctgga cacagccacc 45660
   gggaagcaac tggacattat cggagacatt gtagggcgac cacgcggact ggtgtaccaa 45720
   gatattttca actattttgg attcgctgga acagagcgtg caggttcttt cggaagcctg 45780
   tctgacccta cggtcggtgc tccttggtac tcggtcggtg ctccaactgg taacgccaga 45840
   gagccgagcg acgaagagta tcgtatgatc cttaaagcaa agatcatcaa gaatagaaca 45900
   aactcgaccc cagagcaagt tatcgaagct tataaattcg tattcggggt tcctgaagta 45960
   ttcctagagg agtacgctcc cgctgctgtc cgtatcggca tcggtaagat tctaacgaac 46020
   gtagagcgta gtcttctatt cgacctaggt ggtgcaggtg cattgcttcc taagactatc 46080
   ggggttaact acacatacac tgagttccaa gctggccggg tatttgctac agaaggcttc 46140
   cccggaggac aaggcgttgg agacctaaac gatcccactg ttggtggaat tctgaccaac 46200
   ctagtgacat aaggagttat aaatggctga ttacagtcaa ctacctattg aaaatatttg 46260
   gtccacaggc ggggatatgg tagccccgac ccctgcgcag cagcaaggcg gatggggtat 46320
   tcagtcggtt cctcgccaat ggtggaactg gaaatggaac cttcacgata ccaacctagc 46380
   gtatctgcta cagaagggca ttccagagtg gaccagcact caagagtata ttgcaaacaa 46440
   atcgttctgc acaaggggtg gtttcgtcta caaggctgtc cgcacccata caggaagtga 46500
   ccctgcacaa gttagtgcca actgggttcg agctttcgca gactttacca catctagctc 46560
   tgcacttgga agcctgacac caagagaagg tggaatccca ttcttcatta gtgctacagg 46620
   agctagcgtg tttgactcta cggcttatgg ccgtggcatg cttaacgtgg ctaacgcagc 46680
   cggagcaaga aactatattt cggctcaaga aagttctgtt gtactgtcta acctgtccac 46740
   tgtgactagg gctgccaaca cagttccgta cttcaatacg gatacctcca tggctacctt 46800
   caacatcaca gcgttcggaa gaggacttgt caacgcggct aatgctgaaa acgcaagagg 46860
   tttcctaggc ctagctaact ctgctatcat cacagccgat ccagctaaca gagcccacac 46920
   tctggtctat agagacgctg ctggtaactt caacgctggt gtgattacgg ccaccctgtc 46980
   tggaaacgca accacagcta acaagctaag ggctccagtt acaatcaacg gtgtagcatt 47040
   cgacggaagc cagaacattg ttcttccggg cctagataca agttatgctg gaacggttgc 47100
   acgcctccac atcaacggag caaacctttc gagtgcagac aagacgactc agctagcatt 47160
   gaggaataag tctgacactg actggattag cctcgctgta gtagatgaca acattctgca 47220
   attcgtgttc agaagcgcta caaacccagt tgtccagatt ggtaacgaag ttatccttca 47280
   cacaggaaac cagttctctc tgggccctac tcttacagac gctaggtctc gtctgggtct 47340
   cgacagactc actcaaggta gctctgatac tcaagtcttc cccagcacct tgaacaatgg 47400
   cccctacttg accgtacaac ctacggctat cggaggattc aacggtagca ctaacggatg 47460
   gctgttccga ttcgacgcta atggtaacat gactcatggt acagttcctg ccgctagaat 47520
   cactggtctg tccaactcgg ctcagattcc agccacaact acggcgcagg ctaactctct 47580
   tgttcagaga gacgcagacg gaggattcag cgctattgcg attaatgcgt atggcactat 47640
   cgtcgggtgc ggctctaaca tcttcagtag agcatcgggc actggcaacg cccacatcgg 47700
   gttccagaga gcgaacggaa cagaactcgg tcttatctgg ggagcccagt ctaacaactc 47760
   gatgaacttc cgggtagctg gtggctccac cgcagtgtcg attaccggtc tggacatgac 47820
   tgttactgga agagtcaatg ctaccacact aaacgcctct ggaaacgtga atgcgacagg 47880
   caacgttaac gccgggagcg ctacgctcaa tacagcaggg aatatcacag gagccgctta 47940
   cggagcctat ggttctctga ccaactgggt tgattcggta tatgccaaga agggagaaat 48000
   tcctaacgat atcgcaagag ccggagccgc ttgggacgct gtaggtcagt atattctggc 48060
   gggagatcaa tctggaggct ctggcggccc gggtacaatt agagccggga accagttgag 48120
   accttactct acgatcagct atacagccgg tagcctgccc gctggctctt acagatgcat 48180
   gggcgcgttc gctgctggcg gaaaccagat cactctctgg cagcgcattt cttaatagag 48240
   ggctacggcc ctctttttag aggagattat agtggaattt agaaatgttg taaagtataa 48300
   aatgggttac gagtgcgaag tgaatcaccc tgattttgga tggattccat acttcgcgtg 48360
   tgactacgat gacactgcga aagtcgttta cgactctatt gttaaagcta tcgacgatgg 48420
   cgtaattcca gagccagaag acgtatcgga cagggatttg gaattcgaag caagggtttg 48480
   gagaaacaat gaactaactc ttgcagatcg tcaagtagag attgctatcg acgatgaaga 48540
   cgaagtaaga gagaaggctt ggcgtaagta tcgttctgca ctacgtaaat ggccagagca 48600
   caagaacttc ccatccgaaa agagcaaacc taaagctcct aaagaataag gcgaggtaat 48660
   atatgccaaa cattatgaag cctacgggta ttaacgctat ctggtctgaa aacggccaga 48720
   aggttgaccc gggtgctgta aaagttgggc ttggatgggt gacagaacta cctccctacc 48780
   aaactgccaa cttcattgaa tacaagcaag acttgttcaa cgcgcacgtt aaccagcacg 48840
   gtattcccga atgggatagc gtcacagagt atcagggtaa cctgagctac actcaaggcg 48900
   ctaacgggat catttataag tgccttagaa cccattccga caagattcca accgaccctc 48960
   tcaacatcac agcaggatac tggcgggtgg cgttcgagga tgcaggggag gcggccaagg 49020
   ttcaagctaa ccttgatcgg cacgtgacaa attacaacac cctctctggg attggtaacg 49080
   tagttattgc aagacagaac ctgagtgtgt atagtaaggc cgaaggcgat gcacgttatg 49140
   ccatgcggca cggcaacggg tctaacgtct tcagcgtcgc tcccgcaaca cagccaactc 49200
   acgcgatccc gctgagccag ctatcgactc tcgttcctcc tgcgaccgag actgtagccg 49260
   gggttatggc agttgctacg actatcgaga cagaagctgg agccaatgat accaaggctg 49320
   tgagtccgct caaggccgca caggtgtatc tgaagaagaa agacaacctt tccggtctgt 49380
   ctaacgtcac tgccgcaaga gctaatctag gactgtcgga tacagcgact atgccttctt 49440
   ctacattcct gaaggcaggt agcaatctgg cagatgtgcc taacaaggcg ctggcaagat 49500
   cgaatcttgg aattacaagt agtgctaccc agccggaaac ctatttcttg aggagcgctc 49560
   agaacctagc agatgtacct aacaaggcac aagctagagt caacctaggc ctcactggga 49620
   tggctacaac agaccctgcg gcggttatga tgaaggcaga caaccttgct ggattggcta 49680
   acactgctac cgctcggtcc aacctagggc taggcacagc ttctactaga aatacaggag 49740
   acttcctgtc gtctggggct aacctgtccg acctcacgaa cgtccaagca gctagaaaca 49800
   acctagggtt gaagggagcg gctacgctcg atgtatgggg tcttcctgcg aatacaactg 49860
   ccatggactt ccagtctagt cagtcggata tttccagagg atgggctcga ctaccaaacg 49920
   gccttctgct acaatgggga actggtcccg gtctgtctga tgacacaagg acgaatatcc 49980
   aactccctgt tccagcaaga atcttgaacg tgcaggtaac cgtaatggga acgttcaaca 50040
   actctatcgg cccgggcgct ttcatgactg acatgtggag caacacaggg ttccgagtta 50100
   gctgtaactg gggtaactgg tcttacccgt tcaactggtt tgctatcact tccaccctgt 50160
   aattcaggag tatcaaaaat ggctctaact gagcaagact tccaatcggc tgccgatgat 50220
   ctcggagtcg atgttgccag tgtaaaggcc gtcactaaag tagagagtcg tgggagcggc 50280
   tttctacttt ctggcgtccc taagattcta ttcgaaaggc actggatgtt caagcttctc 50340
   aaaaggaagc taggtcgtga ccctgaaata aacgacgttt gcaaccctaa agctggagga 50400
   tacctcggcg gacaagcgga gcacgaacgt ctagataaag cagtcaagat ggatagagac 50460
   tgcgcacttc aaagtgcctc ttggggccta ttccagatta tgggattcca ttgggaggca 50520
   ctaggttatg cgagtgttca ggcatttgtc aatgcccagt acgctagcga gggatcgcaa 50580
   ctaaacactt ttgtgcgctt catcaagacc aacccggcaa ttcacaaagc tttaaagtct 50640
   aaggactggg cagaattcgc aagaaggtat aacgggccgg attacaagaa aaacaactac 50700
   gatgttaagc tagcagaagc ctatcaatcc ttcaagtaaa tctaggaagg ttcctaatga 50760
   agaagctatt caaaagctgg aagttctggg ctgctgtagg ctctgcaact tttatggctc 50820
   tggaagccat ggttcatctt tgggagccta tccttcctcc cggcgtgttc gccgcagtag 50880
   ccacactggt agctgttggt gtgagaattt cggcagtgtt tgtaactgct ggtaaagtgg 50940
   aggaagcgat ttcggaaacc atagaagatg gctcaataga aaatgatact aactaaactc 51000
   aaaaactacg ccctcgtagt agctgccgca atggcagcag ggtttggata tctgtcgttc 51060
   tctttgtacg gcgacttagc tgttgcgaag aatgatatcc aatctcttca agctgcgtct 51120
   cgacagatgg aagagcagtt ccagaaagag cgagcagggt gtgagatcac cactgggaga 51180
   ctagaggcgc atatagtcca aatgaagagc cgacaggagc ttctacaagg tcacctgtgc 51240
   gagcttgagt ctctacctaa cccaacccca agggagaccc aagaaaatgc cacagagacg 51300
   atctctgagc cttctactgg ggatagtctg gaccctgctc ttacaagctt gctcgacagg 51360
   gccttctgtt ccgcagtacc aaacgatcct tattgtgccc ccggcaagcc tgaccgagga 51420
   ggttaaggta taccgagtgg agcaagatac agtcaagggg ctcgctagag cttacattct 51480
   aaatactaac agtgctgcca tgtgcaatag tcaactacag acgataaagc tatgggcaga 51540
   agagcagagg gctctgtatg gaaagtaaag acggcaaaga aggcctaatc aaggtagcta 51600
   cacagggagt tatctctctg ctaattgcta tcctaatggc ttggtctgga ttcctaagtt 51660
   ccagtctttc taagcaggaa gaacgtatct ttgcgatgca aagagatatg gtatccgagg 51720
   caaaacttaa cgcactagag tcgagattca atagccgact tgaaagtgaa attagtaacg 51780
   taagaagaga acaacaagaa accaaccggt atttgatttt gattatgaat gccgtcaagg 51840
   gtcgggatac agaaactcaa taactataac ccgttaaccc acactgggag acaatataat 51900
   gacaaggaaa caagactggc cgctagtttc agcagtcggg gctttgttta tctccctcct 51960
   tgtctgtatg gctattctgg ctttcacagc gatgactccc gaggctcggt ttgactctgt 52020
   acgccagacc tacgagattc gaattagcga atctgagcgg cggtatgaac tcaaactgaa 52080
   cagactacag gatcagataa actcggcctc gtacacacag gatcggcaat cccgtttgca 52140
   atatgaagaa ctcgactcgc ttaaaaggaa gattagcgaa cttgagcgga gaataaaaga 52200
   aaaacaagaa taattaagcc ccgggagcga aagcttaccg gggctctttg ttatgggttt 52260
   tcagcggaca actcgatgaa cttcgtcgaa cagttcgtta agcttctgga tgaccttctt 52320
   agccatctta ttgcgagctt tgttagactc tgggctggaa tagatcagct tcatgtcgac 52380
   ggcgttgccc cagtctacat tcccgtcctc gaacaaaact ttaacattca gattcatcga 52440
   gtgcgagtca gtgtcgataa ctactccaat ctgcatgcta ttggactttt cgaaggtcgg 52500
   cctcggaatg tctatgtcca gactaccaac cataacaacc tgccctttct taacgtcgga 52560
   tagttccatt aaaacctcct taatctaact tcattagtgt attgaactga tgcctcaaag 52620
   acttgaaagc ctgcggcccg tcgtcttctg ctccttcatg aatatgatga gcgattaagt 52680
   gtatcaatcc tcggtctcct agtaacaaag aactaggaat ttcttttatc caagccacaa 52740
   agcaacagaa gtgtagaacc tccggcatgg aagccttgaa gctggaatca tccagcttcg 52800
   ataggcgttc atacgtagct ttaagatcgg gatagcttaa ctgatatgta gcatcttcgt 52860
   caatatgctg gtcccctcta actacatact gcaagctcat attacacctt ctttccgcct 52920
   tctgcaaggc gagcttcccg cttgtggtct gcacgggtca tgttgtactc gatcttctcg 52980
   atcatagccc ctgcaacatc cagaccccgt acattcgcat agtccatgat gcgaatgata 53040
   gcgtcggcca gttccacctc ttccatcttc cgctctggca agtgatcgtc catcaaatcc 53100
   ttacggacgc cttcagcagc ttccgcaatt tcagtggtaa cgagcatcag catgtgcaga 53160
   gcatcacgtt gaataggctc gcctgtgaca agatcagtgt tccacttgcg attaacaacc 53220
   tccagaccag tcatcatttt gttaaaaccg ttcacaaact cgaagacgga ttcacgttcg 53280
   aatagctcgc cgaacgtcgg ctcctgatct tcagcaattg cagtactgaa aggccactcc 53340
   atccaagcat cgtagcactt tttgataagc tcttgttcat caatgtacga gaagacaccc 53400
   tgttcctcaa gctgactaat agcttcagcc ccctcgaccc agacgcacgt accaataagc 53460
   cttggatcga ttctcagaga agtcttgtag acttctttcc acgacctcca tcccacccta 53520
   ccattgccaa aatacacaag gagtggcaca tagccttctc gcttttcctg ctctggcgtt 53580
   tctataacta caacaaacgc cggaactttc tccccatctg gacgttcagt tagtacgaaa 53640
   tctcccaagt tgatattctc ccgactagct gggcgaagag cttcctcgat atggtcttta 53700
   actttactca ttaacttctt cctcctcaca gaacatttca aaatagcaat cacagcatac 53760
   cagcataccg taatcactta taatttcttc gaccgggaga atgcttccac agcattcaca 53820
   cccagcccgt tcaacaccat ctacacagac ccaagagtcc atcttcactc ctccttcaga 53880
   agatactcgt tgctaataac cttgaaacta aagtctcggc tcatgctctt gaataccaaa 53940
   ccttctcggt acttaccgtt caaggcagac tctccgctag ccattttaat tacatcttgc 54000
   atgtcttctg gaacgcctac accccaagag tacaccggca catgtttgat tgtctcacct 54060
   cccaacaagt tcagagtatt cagaatggca tgccgagccg ctggaagaag ataacggctc 54120
   tcggaaatgt tgtagatatc atacacatag tattccggtt tgtcaactcc ctcgaagtta 54180
   ccttggatat ttggtgcaac cagttcacct tgaagggcaa tattcagacc gaaataccgg 54240
   agacgttcac caagaccaag ctcttcagcc atagcagtga agtgattgga gtccttctta 54300
   atcacacgct tccagccacg acccttgaaa aagttcttga cagccgcgaa gaatccgatt 54360
   ttctcgtctt ccagacggaa gccaacattt cgagacgcca cgcctacttc tccgtccttg 54420
   tgccagatag tgagagacga gccatccaat ttgaatgtaa cttcaaactt ctcgtccttc 54480
   tgatgagcct gatcgtaggc cattttaata ttctgcacgc gagtctgatc cgtcttcggg 54540
   atgaagttgg ggaatccgag cttggaagtg ccagttcggt cacccttgtt gccgccattg 54600
   atagctgcct gctcagatgg ctttacatac ttcaggatgc ccattcgatc agtaacgtct 54660
   tcgtctactt tcggctctgg actgccggct gcatgaacag gcacgatcaa tccttggcta 54720
   agctctttgc gaagcttcat agtcttgatt cgagaatatt cttttccatc ccgctcaacc 54780
   atattacggc ctcgcaggaa ttcaaagata gggttgtcca tagggaccat ggaatccact 54840
   tcaatgtaga ttgccagatc accagcctga tactcacctc gtttgattac gcactgccag 54900
   ccaccaacaa gcgccagatc aaggctgtca gcgttaggat gggtcttcac atcgtcgata 54960
   cgcaccactc gggctagtac gcggtcttgg gaattgctca tatatttcct cctttatttt 55020
   aaatttacta gattaattgt caaaccaacc gagattaagg gcgtgctcca aggctgcgag 55080
   aagattctca gcgtccatgc ggtcgctaat tgacacgcgg cgccatgtga tgccctcttt 55140
   gtttctagct acaatatagg ccgaggaact atctcttccc tttattaacg agcttgcaaa 55200
   ctcagtcttt cccccgaaca ctatctcgct aactttttgg gacggctctc gaatatcaat 55260
   cttcatcgtg ctctccttgt taaaaattgt tagatttgat agattacttg ttaaaccaac 55320
   cgaggtcaat agctcgttct aaggctcgta gcagattctc agcatctaga agatcacgga 55380
   tcggcatcca actcccacta ttccccggta agccatctgc gatgtacaca ggtacgtcag 55440
   tatactcgct cttttcaagt ctgcaagcac agtctacact atctccgaag acaatctccc 55500
   tgacctctgc cgcctcgttt cgaatatcga tcttcacggt gtttcctcct gttttgttga 55560
   aagtcgttgt attctggacg gattcctctg ctgtgtcaat agccttgaca agaaaataat 55620
   ccgtggtaaa gtgggtgacc tgctggagag aaatgtccac gtcactcacc ggccagcagt 55680
   atagcgtatg aagaggaaag ctgcaagcgc aacctgagtc gaatctgaga acagggcagc 55740
   gacgacccga aaggggaaag ctcagagcct ataaggagcc catgctcccc gctcaccgac 55800
   tcatctgcat aatgagagcg agaggcagac cagaggcatg tcttaggcgg aaaaatccgt 55860
   atggcaagcc tagggacagg tctgcctcca ctaccgatct atctgcatac aagagcttga 55920
   caaagctctg caatccgtgt agagtattct caaatcaatc acagaggagg tataaatgaa 55980
   acatgttatt taccgagaag acctaatcga cctgttacgc gaccgtatga agctccaagc 56040
   tctggaggct ggtggcgttg acaactggga ctggtatgag gcctcgctaa tcgactgcgg 56100
   atacttcgat gacgatggcc gggagctaga ggtcgaagct cttgcagatt gcttcccaac 56160
   cgtaggtgcg taaccgtgga ggagtcgaaa gctaccttca aagaccctta ccctaggttc 56220
   ttcgacgctt ctcgtaggta tggcgaaacc aacgactgca tggtggtgag ctttgcagtc 56280
   gtatggaaca ccacctatga aaaggcacac cagcacctga aagtgaagtg caagcgcggt 56340
   taccggcaag ggctactcag aagcgcttcc agctacgcag acacttggtg ccctaagacc 56400
   cggatgctct accgggagga tgctaaggat aagactctta ggcagtttgc tgaggaaaac 56460
   cctgaaggtc ggttctggat cgcggtaaag ggtcacgcta ttgcaatcat cgacggagtg 56520
   ttgtacgatc acactaatga caagagacgt accatctggc actgctggca agtgtggccc 56580
   ggagaggcac ctgaaaaaat cgagaggaag cctgatccaa tctcacctcc tgtgagaaag 56640
   gccccggact ggtatgtcta aacacaaaca tctgaagtac aaagaggagg atttatggaa 56700
   attatttcga gagggacact ccctcaagag gctttgttta cggctaagtg ccaccattgc 56760
   ctaagcattc tccgagctaa gagatcggaa cttgagtatg tttcgttctc attctgtcgc 56820
   cccggggaga ctgggtatta tttcgcagag tgccctgtct gtcacgaaga ggtgtctgct 56880
   tgggttgcgg ctcctgtcat tcctgtggca ccggccacca cggagtatat cacaaaggag 56940
   cgtaaataat atggaccagt tagcaggatt catgggcgaa cctttcgtaa ttgcgttcga 57000
   ctacgacgag acgatcagtc tctaccctct tgccttccga atcgtcatgg ataccttcag 57060
   gggctttgga ggcgctttca atgtgatcgt agtcactgca cgccaccctg acaacagcgg 57120
   agagttagag tggctacggg actacgggtt cccgatctat ttcacggcac agaaggcaaa 57180
   gcgaccattc atggaaaatc ttggaataaa agtctctgta tgggttgacg acaacccgtt 57240
   cagcgtggat aatgaccttc ctccgtggac agtggagcaa tacgatgcct acatggaggc 57300
   aaagaagcaa cgagctatca acagagggag taaagcatga gcgagattat gaaggttgaa 57360
   gtgggagatc gggtggtcct taaggcctat tgcgatacat acactggatg tgcaaagcga 57420
   tgggcgcagg cgcatggacg tttggatgaa ctgctggcta cgggattcgt agaagatagc 57480
   tgggtacatt gtgatgacct cgcaggaggc ttgaaaggta ctgtcgtgat gactgaacta 57540
   gaagaatgtt gtgcagtacg tctagacgcc tacaccgatc acagggctgt agtagttgtc 57600
   gactcgtctg acctgtacat cgcatgcaag gcagcaaaag ctcccgtaag caccctagac 57660
   gaggccttcg gttgtgacca cgactacctt atggactctc cggctaacgc tcagcgactg 57720
   aatgagtctg tcgagcagct acggactggt caagtcgtag aagtggacgt gcctatgagt 57780
   gtaaagcaag acgaaactcc acctgtcgta gtgcatgtag tagagaagat ttacgatagg 57840
   aaaggtaacg tacacctcat tctacagtat gaggtagcag gggcgctaat caggacggtg 57900
   gttgaacagg gagaactcat ttacttcttg gatgaaatgc aaggtatgta cttgaccaag 57960
   attcaattgg aggtgaacaa tggagttgta tgaccagtgg cgtaagtata tgagcgtcgt 58020
   aatcttagaa gaagaagata atgagcgtca gtgcatgcgg cttattgagg cagttggcga 58080
   actcgatccc tatatccaag atttcctaga ttctttctct attaaaactg aaggaggtgt 58140
   gtaagcatga gtcagagtaa gcgcgatgag attgtagttg aagcacgtgg gctagagagc 58200
   tacttcattt cccactggaa cggctgggat gtgctggaca ctgcttgtct cggattttat 58260
   atcaaacctg agtatgttct tgacccggat attcgagagc ttgagcctag cttcttggag 58320
   cttgatttcg agcgttgccg tttccgattc tggggaaatg aagacgaagg cgtggacggc 58380
   aaactcatgg agggcaagtt gcagattatg accaactcct tcgagtacgt cttcttcaag 58440
   gaggtttgag tggctagaca gacttacgag tctgcatcag atagggcggt cgaggaggcc 58500
   gtccgttctg agcttgaagg gcggtggaag tgttctcttc ataagctacc tagagcccac 58560
   tatatggatt gggcagcctg ccggaatgga aaggttgtta gctttgtgga gcttaaggct 58620
   cggaataatg aaatgcagaa atatcccgac ttctttgtgt cccagatgaa gtggctccat 58680
   gggatcgaaa tgcaaagggt gtttgacatt cccgcattcc ttgtggtaaa gtggacggac 58740
   tatacaggat atgtcaaaat gacagccaat ggcgtcaact acatgagcat gggtggcagg 58800
   attgaccgtg gagactggca agaccaagga gttatggtcc atatcccgct gaaagacttc 58860
   acacgtttat aggaggacaa atgaaacgac tctgctaccg ttgtctgcat cttggagagc 58920
   ctgtcatgct ccgtcataag actggcatct ggtataaggg ttgcagtaaa tgtggatcag 58980
   gagtattcta tggctaagaa gaacccggtg ttcaagtttc taaacacagt caacaggccc 59040
   tcggtagaga gggataaaac caaatacagg cggaagccta agcataaaca gaaggagcga 59100
   ttaaatgaag aagggtgata aagtgaggat caatgacgac gctgatacca tcttccgggg 59160
   ccgtgttgcc actatcatcg cagtagacga agagggttgc gttattgatg tgggcagcta 59220
   cgaagacgaa agcggagagc ttatcgagta ccttcacctg ttcttcgagc gctctgagtt 59280
   gtccaagcag gcgtaggagg gtataatgaa tggacccgag tgcagatggt gtggagagaa 59340
   aaccgaagct gagtttgtag acgttgggtt tggatggatg cagcaagtca ctggaggcat 59400
   gtgtcaccag tgtggagggt atgagaacgg tcactaccag aacgacggaa ggctctccga 59460
   ggtcgagttt gccacctact ggagtggtcc gttcgaagat tatgcggatt tctcgccatt 59520
   caatcatatg agagaaggcg actcgccatg aaggctacac tgagaaacca caatctgcta 59580
   tacatctact tccacacgtt ctcggaaatg aatgagtggt tgacagagaa cgctgtagat 59640
   caatggacag tcacgactct gggctccggc tatcaggtgg ctgttcctat ggaccgtgca 59700
   atgcgggacc aatacgtctc ggagaggggt tgacacgaac acaaggtgat gctaaactcc 59760
   gtggttgtct gaagtaagga ggttttatgt ccaaaatgac cgtggcccaa attgcggcta 59820
   tgcacggtat cgatctgtat tctgaccaca agaccgcttg tccccaatgc cggaagaaag 59880
   gcgctgacaa cagcggagac aacctaaagg tctatggtac tgatcgtaat accggggaac 59940
   acaaaggtgc ctattgttgg gcgtgtgggt acaccttgct ctctgaagag caggaaagag 60000
   acgcccgggg agaagacgaa gaggagtgga atttcgtggg aactgaattt aacgcagaga 60060
   tcaatgctca aattaaagag caatgctcgt atgatccgcg tggttatcgt ggcctgtcca 60120
   aggaaacttg ccagtatttc ggagtgctac atggtttcga cccggagact ggcgagattg 60180
   caactcagct atatcctact actactaatt atgaactcac tggctacaaa cagcggatca 60240
   tgccgaagaa cttctcagag cctatcggcg agaccgggaa ggattgcgag ctttttggtc 60300
   agttcaggtt caagaatagc aacagcaaga aggtgcttat tgtaggcggt gaggttgacc 60360
   agctatcagc attccagatg ctcaaggagt atgatgaccg gcgagcaggc ggtaagagtg 60420
   attatgaacc gactccagtg gtcagcccta ctatcggtga gagtggggca tggaagcagc 60480
   tacaggctca gtatgagtgg ctagaccggt tcgagcaaat cattctctgt ttcgacagcg 60540
   atgacgcagg acgagaggca gcagacaagg cagccgataa gcttccgaag ggcaaggtgt 60600
   tcatgatgga aatgaaccgt aaagacccta acgagtattt gaaagctggg cgtcaatcgg 60660
   agtttgtcag tgcattctat cgagcccgta catggacccc tagcggtatt gtcgggtctg 60720
   atgcactcga cgctcgaatg tatgaagctg tgcaggttga gaagattcct ctgccaccgt 60780
   tcgctaagaa gctccagaag gctatggcag gaggcatccc gttgggccgg attgtgaata 60840
   tcggcgctgc atcgggcatg ggtaaatcta ctgtagtgga tgagttcact ctgttttgga 60900
   tttataactc gcctcatatg ccgggtactg tgactctgga atcggactgc ggggaatacg 60960
   cgatcaagct gctgagtcgg aaggtaggtg agaaggcaga cttgtacgag accgttgaag 61020
   agaaagtggc gttccttgat cggcaccgtg acgctcggta cgacttgtgg cacaaggaag 61080
   acggaagccc acgcttctat ctgatcgatg accgagacgg aagcctagca gccctcaagg 61140
   ctcaaatcga gcaactgatt attcagttcg agtgccgcgt gattattctc gacccgcttc 61200
   aggacattct ggatggtttg agcaatgaag agcaatcggt gttcatgcgc tggcagaagg 61260
   gtatgaccaa gagccaccgt gtgacgttca tcaacatcaa ccacgttcgt aagagtggtg 61320
   gtggtcagaa ggctaactcg gcaggtgcgg aactgtacga agaagatttc caagggtctt 61380
   cgtccatctt caaatctggc gcagctaaca ttctgatgtg gcggaacaag gacgcggaga 61440
   acgagttcga aaagaacctg attaacgtga agctctctaa gtgccgatgg actggccgca 61500
   ctggcatgat gggcaactgg tactacgacg tcgagaccca tacaatctgg gacttggacg 61560
   attatctgga ccgataccct gacaagcgcc gtgagtatga ggcttggatg gctcagaagg 61620
   aagaggaagg ccgagacacg gaatactaag gttgacagag gggtgatgct tatgtatcat 61680
   ccctttcgtc gtttacagga ggaaaggaaa tgcaacaacg atttgaaaca atcgagcaat 61740
   acctcgaatg gatgggaaat aacaatgtca ccaacatgac ttgggatatc gagtccacgg 61800
   gtctgctgga tgaaacgtcc atcgactaca ctgccagccc ctacaagatc aagccaagct 61860
   acagtcatca ctgtgtcgta gtagaagagc acggcggtaa cattctggcg ttctacgacg 61920
   gcccgactta cgtcttcgac ggtcgagagt acacggagaa gctgccgaaa gcatcttcca 61980
   tgctccacac tgaagaagtt gtgactctca aggactacac tccggtagag tatattcacc 62040
   tgccgctgaa agagttcaag aactatgtgc tcaagcgcaa gatcaagcgc gtagtggctc 62100
   acaacatgat taacttcgac cttatgtcga tgaagcttgt ggaagatatg gacttcacta 62160
   tcggcgtaga gcacagggat ggcggtctga ctacgtggtc tgctgacact tggatgggca 62220
   agcaagtggt attcgacgat accctcgtta tctccaagac ttgtaaccct gacctgttcg 62280
   gcggtcacag tctggataag ctgtctcgtc gtgcaggtgg tgacaccaag attgacttcc 62340
   gtaagcattt ggcaccggat gttcgattcc tcgacttcgc agccgatatg ctctactact 62400
   gtatctatga cgtaaaggcg aacaccgctg tatggcattg gttgcaggac cacgaagatt 62460
   accaactgtc cgaccgtgac aactacatca agaattggct gtccccgatt cagttggaga 62520
   agaaggttgc agagatcatc acctatcagg agcaccgagg gtttgttctg aatgttgatc 62580
   tctgtgaaaa agctgtgaaa gacttggacg ccaagatgga agagcgtcgg gcccgggtgg 62640
   aacctgtgct tcctcctcga ccggcaacca agaaattcat ggcagactac actccacctg 62700
   ttcgacagtt caagaagaac ggggagatca ccacttacct acagaagttc attgaaaaga 62760
   tcggcgctca attgaaagag ggcgacgaca gaaccatgat ctttgagggt aaggagtaca 62820
   agctgcctct cccggcagac gtaccgctga agacggaaat gcctgctaag atcggggaca 62880
   caactcacat caagcaatgg cttatgggtg agtttggttg gattccatca gagtggaaag 62940
   agactgatct gactgtggat cagaagaaga tcaagcttcc gatggaaaac atcgttgcta 63000
   agatcgatcg ctggctggat gtaacgtata gcacagcgta caaggaagag cggctggacg 63060
   ggttcgggga ttacatgaag atcacccctc gtagcacccg cgcgtatgta cgtcagaggt 63120
   tgattgatcg tgcaagcaag gggggctgta aggttctcac taaccctagc ttcacaaagg 63180
   gccaagagaa agaaatgtgt ccagacctcg aacgcattgc agaacagttt ccgttcgcga 63240
   aggatgtgat tgagtacctg acctttaagc accggcgaag cgcaatcctc ggtggcggca 63300
   tggactggga cgaaatggag gatggagagg agccggaaaa aggttacctt gcaagcattc 63360
   gtcaggacgg caggattccc acgcctgctg acacttgcgg ggctgctact agccggatga 63420
   agcaccggaa ggttgctaac gttcctcgga tcacctcgtt gtacggggct gagcttcgag 63480
   agttgtttgg ggttggagat gggttcttcc aaattggata cgacttcgat tccttggaag 63540
   cacgtaaaga gtcggcctac tgcgatcagt acgatccgac aaaggaatac tgtaagtcac 63600
   tcatgatgga gaagccattc gacgttcaca cgatgatggc taagcgtatt tccgagatta 63660
   tcggtcgtca atttaaccgg tctcctgcga aaaacgtaaa atacgggtgt acgtacggtg 63720
   ctcaagctgg taaagtggca aaaaccattg gtgccgattt gcaaacgggt caactggtat 63780
   tcgatgcgtt ctgggatgca gcattcccat tgaaagcact gaaggatgac ctaaccaagc 63840
   agtgggaagc caacggtaag aagtatattg ttggagtgga cggtcgccgg gttcctaccc 63900
   gttcggctca cgctatcctg aacagcttgt tccagagtgg cggtgttatc tgtgctaaac 63960
   gtgctatggt gattcatgac caactgctgg agaaagaagg tctgaaggtt gactttttca 64020
   aagaagactg gaagaacaag ccggaattct gccaacaaat gatcgcgtac cacgacgaag 64080
   cgcagttgga agttacagct aagtcggtta gcttcaagat gttcccttgg tcccttgtag 64140
   gcggtaagcc ggaaggtgag aaggagctag aggcagccga gaaggagttg aaagctcttg 64200
   ctcaggctta caaagatgag caactggcga ccacaggcaa ggtgtggagt gatatcgcac 64260
   actacagcaa aggcgaaggt ggagtgtttg tagcctactg ccgcgctgga gaacttgcca 64320
   ctaaggctgt cggtgctgcc ggggagttct actctacgcc gttggtgccc attgaactga 64380
   ccgccggcta catcgttggt cgctcttgga aggactgcca ctaatctggc actaagcgag 64440
   gaaaggggct tgacaggccc ctccgcctcc tatacaatgg ccacatattc acgacacaca 64500
   gaggagaaaa actcaatgac tctagctttt gttatctatc tcgttatgac cgtattccca 64560
   gccttgtatt cggcagggag ttttaccttg atcgttacta tcatcgcgtg ggttgcagga 64620
   gggtttgttt acttggcatc gattggtggc cgagaaaatg aggcagttaa agctacactc 64680
   aaaaatcacc tccggccctt gacattcaag ctgtttctac ctatcattgt aatcggccat 64740
   ctagtcccct cgtctgaaac ggcatggtgg atggttggcg ggtacgctgc acagagcctc 64800
   tatcaagcgg acgctactca gcaggttctc agcgaatcta ctgaactgct cgaagctctg 64860
   gttaagcggg ctaagaaaga aactctgaag gctgttgaag gcagcagcga gtgatacggt 64920
   aatcacagaa taggaggtat gtatgaacac ggctcgactc tacgtctacg gcaaatcgtc 64980
   catggtagga atcactgttg acaacaaggc caatcgggag tactatacct ctaaaggctt 65040
   cgaggtatcg gagacaccgt tagcccacaa aagcgtggct aacaaataca aacagaaagg 65100
   aggtaaataa tatgtctggt gttattcccg ggactatcgt caaactcact caagtgggat 65160
   gtgacgtcac cgttggctgg ccaaaggaag cccgatatgt aggcgcatac gcagtcgcag 65220
   tagatggacg cgatatcggt ctcccggaag gggaaatccg gttctgctcc ctccattggt 65280
   ccattgaccg agacgtcaat gatggacttg gagactacca atcgatgctc gctgacaacg 65340
   accgcttcga aattgtagcg atcccgggta agaagtacta tgttaagagc gctgccgagc 65400
   gcccggactt gatctcccac tacttggtta cttcggaacc agctttgtac gagggtttcg 65460
   aaccgtgctg gctaaagagc atgctcgaag gagagagccc attctgcatg ctctttaaag 65520
   gacgagttaa catcggcggt gatatttctg acgatccgaa gctccagact ctctaccctg 65580
   aattggtact tgaggagttc aaagaagagg cagaggaggc tattgaagct ccggtcaacg 65640
   aagagaagaa agaaaaggtt gcaaagcccg ctgtaatggg ttatgctgtt ctcgatgtag 65700
   acggattccc gcttaagttc accattactc gcgaagaggc acggtccact aagcgttggt 65760
   ggggtggcaa ggcggaaggc gtctcgattg tcaaactgat taaaggagag gaggtgcgct 65820
   aatgcgctaa tcctcttgca gaatccggga atgtccggta tgatcgtcgg tcaagtggct 65880
   gacacactcg cccagtctag ggtacttaaa taggagaaat aaaatggctt ttaaactgaa 65940
   cgtaactacc aacactcaat ccggcaacgg cggcatccgc aaggaagtcg attgggatgc 66000
   tctgaacaac cacgtcgtgg aagctgctgg cactgccgct aaagcacgtt cgatcccggg 66060
   ttatatctcc ggcatcatcg acctcggcga gcaagagcag aacgatgcag aggttgtgtt 66120
   cactggcact gccgaagatg aaatccaaat catcaaggaa tatccgaaca cttacttcaa 66180
   ggatggactg gacaagcaga ctcgcaagcc tgtacgtctg aagtgctggc cgcagcgcga 66240
   tatgcaacaa atcgcgatca ctgtggactt ccctcagatc gaggtagaca agagcctgta 66300
   cctgaccggt aacagcaaca agtctccgct tcgtgtcctg tacaacggcg actttactct 66360
   gccgggtcag aagactaaga ttgttggccg tccgttcacc atcaaggaag tgaagcaccc 66420
   gaacggcaaa tgggcgttcg ctaagaactc catgctgcac aaactggctg tagctactgg 66480
   cgtcatcaac gaagacgaac tcttcaccaa ggaccgcgta ggtgagcttc tgggtaaagt 66540
   ggctcagttc caagttcgag tgtggatgaa gccgggcaaa aacgacatga aattcctcac 66600
   cgaggatatc agtctggtcg gtatggtgcc tgaaggtgtt ccggtccctg aactggatga 66660
   gaagtacatc tacggcgtga acctgtatgg tgagaacgac gaagaggcag ttaagcaact 66720
   gcgtgccaac gtcatcaaca ccatcaagcg agccaagaac tacgaaggtt cggatatcaa 66780
   ggcgatgatc gaagctgttg aagctgcacg cggcggtaac cgctccagcg gtgacagcga 66840
   ggaacaagct gaagctaaag agcagactaa agctgctcct aaagttgagc cggttcagac 66900
   caacgtcgat gacgacattc cgttctaagg ttacgcggaa gtagaggcaa tagaggggct 66960
   tcggcccctc cctttcaaac ttcaaaggag gtaacacatg caaatcaatc tcggtcctat 67020
   ttttcttctg gctgccatca ttctgtgcgt actgaagtct gccggcgtac tcgctatctc 67080
   ttggactctg gcgctcatgc ccatctggat tcctttcgta atcatgctgg ttgttcttgt 67140
   aggttttggc gtggtagctg gtgtgcttta tgccattggg agccgcagta aggttaagta 67200
   cagttttaaa aacaacctcc gaaagaagtg aggaccgatt atgagcaccc tagttattgt 67260
   tcgagtacct gcgtacattc taaaaggccc tacagtaaac aactccttct gcggggaggt 67320
   ctgggaacga gagcttttca gagacttgaa gagtgctgcg gggagtatcc tcaaaggaga 67380
   gccggcttat gttactcttc ctaacaatgt aaaccctgac actcgacaga cagagtggga 67440
   cattcaggtt gtacagttct aacccaaggg gagcttcggc tccctttttc attggagata 67500
   aaaatgagtg aaaaggaaga ggatattgtc ctcattgacg gcgacctgat cgcctttaag 67560
   tgtgcaagtg tcaacgagac tcgctcaatc atcgttaaaa acaaattgac cggagaagaa 67620
   gagacttgga agaatcggac gacttggcga gagaataaca aggacagcga aggattcgac 67680
   gaagacaact tcgaaattga ggaccatcaa gaccccaagc acgtatccta tggtatctct 67740
   gttgttaaga ccatgatcga ccgaatctgc cgccaagctg ggtgcaagca gttcaagatt 67800
   ctcctatctg gtcctgacaa cttccgtgat tccattcccc tcccgaaaga gtacgagatt 67860
   accaagggta aaaagacctt cactcgtggc ggcaggtaca agggtaagcg tacagggcag 67920
   attaagcctc ttcagctagg tcagctaagg cagtatatga tcgaagcgta tgacaccatc 67980
   atccaccccg gcgaggcgga tgacttgatg gcggagatga tgtataagaa cggcgtaagc 68040
   tactccagag gggagaccac tcagcgcgtg atcggagcta ccatcgataa agacgcagac 68100
   ggcactctgg gatggctttg caactacgaa cgtgagcctg ttcaagtcaa gttcatttcg 68160
   ggcctaggat cgctctacag ggactctaag ggtaaggtga gaggcgaggg acgtaagttc 68220
   ttctacttcc agcttctatt cggagaccct gttgactgct accgacctgc tgacctgtgt 68280
   atcgggaagg acttcgggga agtggcagcg tataacatta tcaatccctg cgcaacggac 68340
   aaggagtgct ggcaagcgat ctacgacacc tacaaagcgt ggtatcctga gcctgtcacc 68400
   tacacagctt gggatggcac cgagcacacg aaagacgctg tagagatcat gcaaatgtac 68460
   tgcgactgtg cccacatgca acgctgggcg ggagacagag ttgactgtag agccgttctg 68520
   tctaagatgg gagtagagct aggaggtgtt gaatgagcgt aaggcctcgc ccttgggaag 68580
   acttcccggg catttggaag accgaagccg cgttcttgag ttgggtcaga ggtggcattc 68640
   gccgctacct ctggtctaag aacccagtca agcttgagtt tgctaaatcc cgaaggatca 68700
   agatagccaa caccaatgaa aagagcaagc tcaggaatcc cactgtatgg ggttacgtat 68760
   gcgagcagtg tggcaaggag acccctcagt ctaacgtcga gatcgaccac aagacaggag 68820
   aattctccct gaagcgagta gaggatatcc aatcgttcgt ggaaggtgtt gtgtttgtcc 68880
   gaatggagga cttagctgtc ctgtgcaaac cgtgccatga gatcaagacg tacgctgagc 68940
   gttacggact caccctcgaa gaggcagata tcgtaaagca ggctattgca atccagaagc 69000
   agaaggggta tgataaggtc ttcttgcagg aggtgggaat caagcctgca tccaacgctg 69060
   aactcagaaa agagcagatc atagattacc ttaaggctaa gaaggagggc ggatgaacga 69120
   ggaccgggta tacattgcag tgagagacga cgcaccagaa tatatggtgc cgactcttgt 69180
   cgcccacagt atcctagggg ctgatcgtaa tgctcagcag agatacagag cgccgcgatt 69240
   ccctcctcta gatgagcagg tagaagcaga ggatatgttc acccgcaagt ggatcgcatg 69300
   gctggaagaa agcttccgca aagtcgttgt acgcgtctct gacaaagagt acaacaaaat 69360
   caagagaatc ccgttcgtat atgagggata tgagaaaacc atttgcaacg gagagggttc 69420
   ttgtctagta gtgcctcctc ttagcaagga ggaaaccccg aatgttctta aatttgcaaa 69480
   actatggagg cctatgtgag agaggagttt attaaaatca tgcgaaagct gccaatcaga 69540
   gtacgagctt ctatcgtcct gatttggatt gctctgttct tttggctgcc ggttgtaaca 69600
   gcgctcgtcg ccggagttat agacttcttc tataccgcaa aggatcagtt ggaagaggga 69660
   tactcggtac tcatttccgc tgctaaagga gccgttaaat gtcttcgtac aggagaggag 69720
   ctatgacgag tcttgctact tatgttatcc gccataagaa aactaaggag atttggacta 69780
   ctcctaaagg aaagaggcaa tgggctacga aaggggctgc ggccctagct tggagaaacg 69840
   cttatgaggg aactacactg ggcgtatacg ttgctaaaag ggacatggag cgatggtccg 69900
   acttgaacat tcctatgata gtggagaatg ggtgctaccg tttggctagc cgagaggccg 69960
   cagctttcaa gaatcaatct gctttcgaga ttatcgatat agctgaatct ctgaaaaatg 70020
   agaatcaatt ggtactcgac ttgctaagag acgttatcgg atttgaagta ccggatcaca 70080
   cagggcacgg gtatgtcctg ttgacagtca gagaattcac gtataacgcg atcattaaac 70140
   tactgaagga gacgaaatga gcatgacaga cccgggagca tacttcctca agcaagagct 70200
   agaggaagcc acaaaactcc tagaaagagc ttataatctc cttgataacg tccactgcta 70260
   tgacacggct gtgtatgagg atattggagc atttctgtac ggacaagact gcgaggaggt 70320
   agagtaaggt gagagttgat cttaagggag ctacggagca actaaaggac ttgcttgtag 70380
   ccgttgctct gctagatgaa gtccttgtgt ggatggagag cgaagacctg tgtgatgcag 70440
   atatatacaa acgcatcgac gcatttttgt actaccagcc gggagaaggc gaacgggagg 70500
   aggtagaatg agcagtaaga aacactttgt aatttctgac acacaatgca agccgggtat 70560
   caatctggac tacatggaag ctatcggtaa atacatcgta gccaaacgcc cggatgttgt 70620
   tatccacatt ggtgaccact tcgacctagc cagcctgtcg tcttacgata agggtaaacg 70680
   ctcctctgag gggcgcaggt acgccgaaga cgttcaagtc ggacgagagg gtctggatcg 70740
   actatttaag cctctatggg agctacagga gcgtcaacgg gctaatcgga agaaggtgta 70800
   ccaacctcgg ttgatcttta cactgggtaa ccacgaagag cgagcagacc gggctgctaa 70860
   tgacaaccct gagttgtatg gcaccgtagg cagcgatgag cttggtattc aggactacgg 70920
   ttttgaggtt gtcccgttcc tcaagcctat cgaggtagac ggaatctact acgtccacta 70980
   ccttcaaaac cacttcactg gtaagcctct gggtggtaca gcgaacaaca tgctcaaggc 71040
   tgctggtcat tcgtttgtca tgggccaccg tcagcttctg gactgggcga tccatcctac 71100
   aatcgacgga aaacaaagac ttggcattgt gactggagcg tgctatcctc acgatgaagc 71160
   ctacaaaggc tatcaggcaa caaccacttt ccgtggctgt gttatgattc acgaagtgaa 71220
   ggatggtttt ggtctaccta tgccagtaag tctggacttc atgatggaga aaggaggttt 71280
   caaatgacgg tttataaatt ctggtgcgaa tgggatatcg gtattaatga atgcctgtgg 71340
   cgagactact accagatgga agaggatgta gctaaagcct tgtcagactg cgggattgag 71400
   gatactattg aggaactaga gggtgcaggc ctgcttggct tcgactctgt taaagtgatt 71460
   ggttaatagg agggcatatg gctgagtttt tccttggggc ggcccttgca ggcttgttct 71520
   ttatgtttag tatcactgcg agctttaccg ccgaagatat tgagaaagtg acttcaacat 71580
   gcgagaataa cgaaggcgtc aagtatttcg gagttgactt cttcggagac ggtgctgtac 71640
   actgcaacag cggcgctatt ttcaaaatcg accgcaaatc taaataatcg aaaaggagat 71700
   ataaaatgac tgatttcaac atcacccctg agcaaatgga agaagcaatg gcaggtatga 71760
   acccggagca gcgggctctg gtagagaaag ccttgaccga ccgtaaatct ctggaagatt 71820
   ccatcgtgac cgaggacggt gaactggact atggcatgct tgcacgacac atccgcacca 71880
   gcttgggtga tctgcctctg gtaatgggtg gccctgactt cctgaagctg atgatcttgg 71940
   ttgctgcgat gccggagacc aaagacgatc tcctgactct gcacatgaac ctgtccttca 72000
   ttctggactg cattcgtgaa tcgattagcg acgaggagta acaagtggcc gaagagattg 72060
   taaaagaaga cctatctggc gacgatctca tggttgactc agcagggcta gatgccctgc 72120
   tggccttcct agagagtcag tttaaaggtc cgaagggtgt actgaaggga tatgtcacta 72180
   tgagagtgat gtgcgctaag atcgagagtc ttgacgggtt tcaagagttg aaagatatgg 72240
   aggtgaactt tgagtaacga tatcatcgac gttcatgctg ggcaaccgat ttctctggtg 72300
   caacggtcaa agaatcctat caatattcaa tattgggacg ccgataccat cccttacgac 72360
   acagtcttgg ttgttaccga ggaaaggggt gatatacgaa agggagaatt ggtcgtaagg 72420
   ctggctagta gtgcagatga tgagcactct gttaaggtat gtagattctc tgatttcttc 72480
   ctcctaatgg agaggggcta caactcagct agccttagcg acgttagtca agcgtatgac 72540
   gttctgttcg aagaccctcc ggcgactacc tttgtcaact gggatcgggc tcagcctttg 72600
   acaaacagtg agcgctacta cggagacttt ctgattggcg gcgaccctaa agggatgcca 72660
   ctattcaaca tggctgttct taaaaatgtt ctgtcgcctt acactgccga cgatgaggaa 72720
   ctttcagcta tgaccgggaa tcccgagaca gtagagcaca aagaagttga cgaactaagt 72780
   gagagggacg aggctttagc cgaggtcgac agactcaatg aagagcttgt tgcactgggt 72840
   aagaaggttg tgttcgccaa cctagaagat tacctaggca aagaaaacta tgccacattg 72900
   gaagctatgg ctaaccacaa ggaggctgta ggtagcggag gctcctctag ttactacact 72960
   gtcaaggtga acaaccccac tacgaaggct aacatgccgt attttgcaga gtgcaacgat 73020
   atcatcgaag cgctggatat gcgattcggt caaggcaaca tgttcaaagc gttgtggcgg 73080
   gctgcggcag cacagaacct aggcaagctg aaggccggta acgatgttat ccgggactac 73140
   gagaaagtca tcttcttcgc tcagttggag atcgataaga ttctcagaga aaggggttga 73200
   cattccgccc gaggcttgcg ataataggct ccaagttgaa acgaaggagg tgtgtagata 73260
   tgatccagaa agtaaaacag aagctcatga tggtgcatcc tatcctcgct agcgagatcg 73320
   tctggtgcct caccttcgca atgcttcagt aaaacaaggg agcttcggct ccctttattg 73380
   tatcaaagat ttaataagga gagtttatgc aaatttctgc aaaagtgatt caacacagtt 73440
   cccggatcgg cctaggtacg aagaatcaca gattcatcac tacattcgaa ctggagtatc 73500
   ctcggtttat tcacagtgaa cttatgactc accgactttt tagtcgaaat gctgcaagta 73560
   gtcgtgccat tcccgttgcc catatgatcc gacaggtgga agagaatcct gcgatgcctg 73620
   ttcactgggg aatgaatcaa ccgggtatgc aagctgagtt cgaactgagt gagtgtttgc 73680
   agcgtagctc tcagtacctg tggaagaagg ctgcaaagag tgcggctcgg atcgctagcg 73740
   cactaaataa aatgggactc cacaagcagg tggttaaccg tctccttgag ccgttccaga 73800
   tgatgaagac cgttgttaca gccacggaac tagataactt cttctacctg cgttgccata 73860
   aagacgcaca accggagatc aaggttctcg ctgagaaaat gtatgaggct cttattaaag 73920
   cgggcccggc agaggctcta tatgacggag agtggcatgt tccttatgtg actcggttcc 73980
   gcatagggga tggtaagctg cgttacgcag attgtaacgg gcaagaactg agcgtagagg 74040
   atgctattaa ggtatctgct agctgctgtg ctcaggttag ctataggaag aacgacgaga 74100
   ctctggagaa agctcttgta atctacaacc gattggtaga tactaagccc gttcacgcta 74160
   gcccgttcga acaccaagca acaccgatgc tccgcatctc ggacaatcaa actggatggg 74220
   aagacggtgt gacgcacttg gaccaatatg gtgacctctg gtctggtaac ttccgaggtt 74280
   tcatccaaca tcgtcaacta attaaagggc acgtatgtaa tgagtactac ggccaagaag 74340
   aggaaggagt ttaaaaatgg cagaatataa cctgagtctg gaagacctca tgctggtcga 74400
   tggcttcaaa gaggcgttcc agagtaataa tgaaaaagta gtgcgggagc atctgtggac 74460
   taacggcatg gatgttaaaa actactcgta tgaaatggtt ttctgtcagc atcgcaccct 74520
   gattggccga gtcgtagagg gtctacgatt ctctggcttt gagcgtaccg acaaggagtg 74580
   gttgagcctc ggctgtgcct cgctggaagc tcatatcgca gcgtgtgatg atagcaatct 74640
   acggttcact cttcgcaaaa tgcgtccaga gggatcgaca gaggcaacgt ttcacaatta 74700
   atgcagtagc agtagaaata gggagtgggt atggaagtta caaaggcgct cgtagacgag 74760
   gccgatattt tcaataagat cacacaaatc aggactccaa cggagtccta tgcccggaaa 74820
   tatccggtca ttgtcgaact agcaaaccag caactagaag agaagttgtg gttctctagc 74880
   gaaatgaagg tagagctgga taagttgaac ctgaagttta agttggaacc tcatcagctt 74940
   cacgctgtaa agactgtcct tcaactgttc ctcaaatacg aactgatcgt aggtgaggag 75000
   ttttggatgg gtaaagtagt ccgaacgttc cctcgcccag aggttaaact agcggcttcg 75060
   atcctgtgca tgatggaatt ggcagtccac gctgaattct acaaccagat caacgtagtg 75120
   ctggggatgg atacagatgc ggactatgta gcttacaagg acgatcctga gcttatgggt 75180
   cgaatggatt ggctagaatc tgttctaggt gatgaagacg atgttctttc ggtcattatc 75240
   ttcagcctca cagagactgc cctcctgttc agttctttcg ccatcttgaa gagcttccag 75300
   tgcaatgggt acaacgatat ccctgtaatc gcacgcggag ctaaccagag cgctgtggac 75360
   gaggacttgc acggtgtagt ttcggctgag atcatcaacc aatactacgc tgagattggg 75420
   cggccactat ctgaagacac ccgtcgtgtc gagaagattc gacaggcaat tgaccatgta 75480
   tatgctcacg aatgtcgtat cgtagacatg gctatcccgg gcggagaact taacggggag 75540
   tcggctgaaa actataaagc gtttgttcgg tatcgtttga acgtatggtg ccgtcgtctg 75600
   ggactggaag atcatttcga gaacgatgac actccgatca aagattggtt cgaaatgaac 75660
   acctacgctt acaagatgat tgacttcttc actcccggtc tcggtatgga gtatgaactg 75720
   gggtgggacg aagaagaaat ggcaaaagct tgggaagagg agctaaaata tgagtaagag 75780
   agacacattc aactattccg aagctaggaa aaatagccaa gcagccggcg agactcctct 75840
   atggtatacg acggctggct accagttgtt catgaagaag tattcgaacg agggcgagtc 75900
   tgtacgctct cgcttccaag ctgtagctcg cgctatggct caacacgccc ctatggtata 75960
   cccggagtgg tggaatcaag acgagtacac taaaggtaag aactgggagc aggtgttctt 76020
   cgacgttatg tgggatggct ttgttagccc atctactccg cttctgtcga atggaggcct 76080
   ccgtaaaaag ggcactacgg tatcttgtgc tggcggtctg atggacaaca acctctatga 76140
   ccgttacaat gtaatgacag agatcgcagt gctaacgaag cacagccatg gcacaagctt 76200
   tagcctgacc aactggccat cagaaggaga cgctatccga ggcggtgagt ctcaaggtgt 76260
   gatgcctgtc attcgggacg ttatcaacgt catggaagag gtagcacagg gttctaggcg 76320
   tggtagctgt gcatacagca tcaacccccg ccatggagac ttctggaacg ttatcgatca 76380
   cctgtataag cgcacagaat cgaacaacgt cgggtggctt ctggacgacc agtggtgcaa 76440
   ggatatggcc gagaaagacc cggagacctt gaagcgatgg aagcgaatga tgttcgtcaa 76500
   gctggctaga ggcaaaggct acttcacgtt catcgataag atgaatcgac accttgcaga 76560
   gccgtttaaa cgggcgggtc tacgctgtga agcgtccaac ctttgtcaag agactgttct 76620
   cccggcaaac gactggtata cttttagctg cgttatcctg aactacaacc tcgaacttta 76680
   ccgttcttgg cctaagcacc ttgtgttcat tgggcaggtt atgtctgatt gcaacatcag 76740
   tgaataccta gcaaccatgg atgaggtcag cgttcaggac cgccgagcac tggagaagat 76800
   ttaccgattc accaaggact tcagggcgct tggtagcggt gttctagggt tccacaccct 76860
   gctacaacgg gagcgattcc cggtcggtag tatggatgct atgttcctga acaacgccat 76920
   cttcaagggt atgaaggagc aagctgaggc ttgcaacagt tggctagccg aggttctagg 76980
   agagccagcg ggtgccgtgg gtctagggaa gcgtaacgcc actacaatga tgatgccgcc 77040
   aactaaatcg acggctgaac tcatggcggg ggcttctgag ggtatcgggc ttgacgtggc 77100
   tatgtgcttc acaaagcaga gcgctggagg tgaattcttc cgtgtcaaca aggtgcttct 77160
   ggagatcatc caagagcgcg gactggattg ggaagagtgc gctagacaga tcaacgaacg 77220
   taaggggagt gttcagcacg tagactggtt gactgaccac gaaaaggctg tgttccgtac 77280
   tgcctttgag gtgagaatgg aagactacct gcgtctgtgc tctcagcggc agaagtacat 77340
   cgaccaaggt cagagcatca acctgtactt cacgtcgaac gactcgccgg cttacatttc 77400
   gtacatccat cgtctcgcca tggaagaccc gaacatccta tctctgtact acatctacag 77460
   tatgagagga gcgggagata tctccagaac agaagaatgt gaaatgtgca tgtaaaatca 77520
   ttggcccggg gcttgacagc ttccgggcct ttgtgtattc tgagcacatc aaaacaagga 77580
   gggactaaaa tgaacaaatt ctctgaagtg tactacactt ggagcgcagc gtgctctcgt 77640
   acaaatatct tgatagatat gggatggact gctacaatcc gtcgagactt cactggcggg 77700
   atcaaccctg tatgggttgt ggagttcacg cgatgaacag caaactccgc aaagagttcg 77760
   ataaatggtg gggacaggac gagcaagaag aacttcgtaa atcctgtgcc aaaggatggg 77820
   cggaatatat ctggatggct agtcgaagaa aagtgagtgt tgagcttccc gcgccagaag 77880
   gcggatttat cgacatccaa aagtacacaa tcaatcgctg caaagaagct atccgcgaaa 77940
   caggactgga ggtgttttaa tgaaagaata ttttaaaaag cgactaatgc agaccagtga 78000
   accgttcgat tcagcgacgc aagctatgga cgtagcgatt gcgtggggag gggctaagta 78060
   ctgcgaattc acttgcgtaa ctgcggataa tcgcgtcatc agcatctggg agtacaatga 78120
   cgtagagcct gataaaccgg tattcaatgg acagcaacga atcttagttc caacttttga 78180
   ggaggtagta taatggaaca aactattgat aaatctcggt atattgtgct tgataacgaa 78240
   ggtcggcacg ttctgacctt gtgcgagaag gccgcctata acctgcttaa aggcgttcag 78300
   tggacaaagg acaagcatgg tcaaatccat gtaaatggtg tcacaggcgt tgaactcgga 78360
   gttcgtcaag ggattatcgc tgaagtattc ctgtatccgg gctacatcat cctagaagag 78420
   gtagactaat ggatatgttc gataaataca tgcaagttat gtatgaccgg gacgctttct 78480
   caaggcgagt cggtgagtta gaaaaagagg tagccgaact caaggctaag gtggctatcg 78540
   ccaagtgtga tatgcgtgtc gtgaaaaccc tactggaaga tatccaatgg gtgtatcaag 78600
   acgaagctat ctatactcta ccgggctacg tttgcccttg ttgcggggaa tctgcaaact 78660
   atgggcattt ccaaggcagc ttcgataaga gcccctgcct actagcttgt gtactgagcc 78720
   ggagtgaagc atatgaagcc taatgttgta gactcgctag gcattaacat gtgctacgcc 78780
   tgcggaggta aactgtacgt ccgaggagta tgcactatct actgccctga ttgcgagaag 78840
   gcaacgaaag ataaaccgtt cgtcaggatg cagcttctac aggatatcga agacagcaag 78900
   agggttgaca acgaatagaa gtgctagtaa gatggccaca tcagacagag gaggagccat 78960
   ggctacaaca gagaagatca tcgagtatgt ctcggcctgt atcgaacgct tgcaagagga 79020
   cgtaactggg tactcggaga acttccgtga cagggcgtta tctaatcgca cagggtatat 79080
   tcacgggctt atcactatgg ctgaaatgaa tgagcaggtg agtatcgagg actgtgacaa 79140
   gctccgtgca aagcttcgtg aagctgctga ggctgtgttg aacgtactgg attccaaaat 79200
   taactaggag gtgttctgat gaatatgaaa gagattgtgc aggctttgat tgtcgttgtc 79260
   gctctgactc tcgcagcaat aggttttgta gtcgggatta ctgcgttggc caacaagagc 79320
   gcgcccgccg aggatattgt cgtgtctact gaagacggca ccatcaatct cggtcgctcc 79380
   gtgaagagcg agaagctgct gtatagcatg tgcatcaagg tagaagaccg caaacctgct 79440
   tgtgaattca ccaccaagga ccgtgttctt agcgaagtga aacaggttat gagcccgggc 79500
   gaggctacgg cccgcgatat taagattgca gtaaacgcgg ctgtgacgta tactggtgag 79560
   aacgggcagg aagcaacctc tctgattacc ccgaaaggta agtatctccc ggctggcacc 79620
   ccgtctgaag tacatattca ggctattgcg aatctgttcg acagcgtgta caatgactac 79680
   aaacaaaact tcagcaagat gtgaggagga ctcatgaaag cacagcctaa agaagtagaa 79740
   accctgtccc tcgacaagcg ccccaaggca ggtgatgttg ttcgactgcg atctgacggt 79800
   aaaacctacg aggtaaatgc tgtatatccc acggcagtgg gctgggaagc gattctccgc 79860
   aacatgtacg acaagactaa gaccgtcact gtcagcatgg agtggtgaaa tggcaagaac 79920
   cggcaagtgc gtaaacttcc tacctgtcac gccggacggg cgagtaatcg tccgattcgg 79980
   caagaaaagg ctaggctggc ttggtaaaca gggtaagaag tggcactact acccttatgg 80040
   atgcgaaggc aagattaaat cagaggggtt taaaacactc gctgaaatca aggcttggat 80100
   tcgagacccc gatgctgtct ggcccggtcc aaagcctgag attgactaat tagccgacga 80160
   atggtacaat ggtatgtagg aaattcccta ctcaatataa ggaaactagc aatgactagc 80220
   cgctggaaag aggccgtgta tcgtaagtac tcggtgcgtc gtctgcggca ggaactcgaa 80280
   gagttcaaag aagctagacg ctttgcacca aaccaacgca tccgtgcatg gtttgatgct 80340
   catatcgaat ttctagaacg ccacttggct attcgagagt ccggccaagc ctacccagac 80400
   gacctttggt aattaggtaa ttaggtgtgt tataggtaga gattcaaatc atggttagtg 80460
   gcaaggacgc cacactttca gcccgcccac aaggcgggct tctttttgcc tgaaatttgt 80520
   ggttgacaag gttggccgga atatgtaaac ttgcggcatc caacaaagga gggaagaaaa 80580
   tgcaattcaa tatatcgaag caactatggg tagctaaaag cgagcggaag ggcgactata 80640
   aagggacgct tttggcatac atgtctcaag tggacttcaa taaagatggt agcttggctg 80700
   ccagcttcaa gaaaaagcag cagactggta tgagctgggc aggtaagaat gccgagactt 80760
   gcttcataga caacatccct atctcggggc atttcatcgg ggacagtgtt gaacgctggg 80820
   tcactgagaa caaatatttc cgtgtgacgg accctagggg ctttgttcta gaggttccta 80880
   ccgggaatat ctcgaccttg ctctacaact gcacagtaat caagggagtt attcaagagc 80940
   cttgcgtgtg ggttcggatg ggctctcagc acgttctact gccggaaggc tcctctctct 81000
   acaaagaagc tgtaagtttc gtagagcgaa aagaggcggc caaaaccgtc aaagatatgg 81060
   ttgcagggga tcgatgcact cttcttagct acgggacaga ggagaaacta gaggctgtct 81120
   acctcggaca ggtgaagatc acttggcacc tcctagctaa aaagggccgg tactctagcc 81180
   gagggtgggg tgggggtcgg tggggttacc accatctaga gagggaacta gatagaaaag 81240
   aattcactga gaaagattcg aagtgggtta gtgtgttcgc taagccggta ggaaagcggt 81300
   ggagtatcca ttttatcctg tctccaaagg caaccgacat tcaatctgga agcgtcccgg 81360
   atgaacttca gaatctcgac ctagtgaatg gtgtaaaatg ggagtacaac gagaaagacg 81420
   accgtccgtc ttcctattac tctgtgccgg agagggtagt taagtgtatt agtgtagatg 81480
   atcccctgtg ggataagctg cacagctctt cggaggtgcg gtggacccgg gaatggaccg 81540
   tggtcggagc agagtggcga aaataagcca ccattatttt agaattgccc ggcgagagcc 81600
   gggctttttc gtggcagggg ttattttcaa attattttta gatttctggg agtttcggcc 81660
   aatatgggca atatgaaatt tctcaggaaa aggctcagaa tgctcgtata agagccgtgc 81720
   gagtgtaggt atggtgagct actacttagc cgtagaagga cagtgtaacg cttaatagga 81780
   gggttagaat gaagattgac aacggtagcg cgtatgcgta ttggctagcg gatatgaaag 81840
   agtttgtttc tcagatggat gccacagaga ttgagaagag ggcttgtata gcatacttaa 81900
   ttagctggta tcagcgaaga gtactgcaca gaattaaacg ggaggaatac gaggcgatct 81960
   aagctaggga ccggacagat ttttagggac tggatagatt ttcacaggct gcaaaattag 82020
   gaactgggca gatttttagg aactggacag ttttcacaga agggattttc tacagaggaa 82080
   tttcccctga aattagccgg atttctcaga agggcggatc gtta 82124

<210> 3
<211> 88352
<212> DNA
<213> Unknown

<220>
<223> Complete nucleic sequence of PAK-P3 phage

<400> 3
tacctattca agggaggttt cggtcatgtc aaagattcct actccgacgg tactgcgatg 60

actgcgctgc ttccgttgga gggctccgaa cggatgggat cggtcctgaa agattgcctt 120

gtctgggacc tgtcctcagc ccactcggtg agctataatg gggtttgact ggcaaaaggt 180

tcccttacac ctccgagagg tgttgtggga aaatgaaata caaaacggaa tggtagaaga 240

agtagactgt gcgggggact gcggaaagtc agggtatcca aactacttcg gacactgcaa 300

ggatggtgaa ttctactgcg ggggctctcc ccgctgctgc ccataggagg aatcatgaaa 360

aagatgtcca aaatggaaca actgaagctt catttccaaa ctcccttcaa ccgtgaggac 420

ttggcattcc tcggcgctga agtttgggat gctgctgttc ttctggcttg gtggactgcc 480

cgggtggtca tccttgttgg actgctccct atcagctatc cgctgatccg cctgtacctg 540

aagaaggtag gacgggcatg accaccatcg cttacaagga tgggatcatt gcaaccgatg 600

gaaggatcac ccagggaggg gtaatcaccg acgaggacgc tgaaaaacgg ctagaagtgg 660

atggcattgt tttctggctg tcaggggcgg tatgcgactg gaaagttatc gctgaggctt 720

atgtgaacgg cgacgggatg gaagaccagg agctgaaggt ttccggtctg gttcttgatg 780

gagatggtct gcactttatc tcctgggacg gtggcatcta ctccgaaaag gtgaaccttg 840

aaaggccacg ggctgctggg tcaggcactc agttcgccct gggagccatg atggcagggg 900

ccagcgctga ggaggctgtt gaggcagcaa aggcactgga tatttactca ggtgggacag 960

tgaggtcgta taagctatgg gactagacgt gattgagtta aagctgaaca gcctggaaga 1020

ggcgtttcat ggtctggctt acgtctccta tttggaagtt atgactgacc ctggaaacgg 1080

ttttggaagt gtccaggtaa gtagggtgct gaccgtggag cagtacgact gcctggtagg 1140

caagcttcca aaacgtccaa aagcgtgcca ggtgctggaa cacgagaacc gtcgcaaaag 1200

gctcaacaag agaatcttga atgagatggt ctaccagtgt acgatggaat tccagaaagt 1260
cttgacggac aacgttgagg ctgaagctcg tgaaagagga gagatactat gaaaagccga 1320
ccgatcaccc tcatcctgga gcaagatact gtccggggca tgaaagtctt ctgccagcgg 1380
caatttactg gcgacgaggg ctacctaaag gaagccatgt atagcttcct gaaagagaag 1440
gctgggcagt acgatgattg gacccttcag ggtcatccca gcactatcca gctccggact 1500
gaactggtga cctacctggt taacaacggg gtatgacatg agcactctga ccgtagacct 1560
gtacaccgat catgcagacg gccctcagtt tgagccttcc tggatagttc tgtcgggaaa 1620
tctcaagcga gataccctca agctgttcaa cctggtttcg gacagggctg cacggctggg 1680
ccctgcaagc tatgtacatc tgagccacct ggacagtcag gccaaccgaa acaaagaggt 1740
ccgcacctac ctggacgctc tgcaacaaga tgttctttcc atccaccttc aggagaccca 1800
aatgagcaac aaacatgacc ctttcactgt cagtgtcact tccatacacg gtggtggtgt 1860
gggctggaaa atcaacggtg tgatcacctg tacccgcgag atggacaaag atatggctgt 1920
cctgttcgag gagctggaac ggcgccgcta tctgccccag cctgacacca aagaaacgcc 1980
ggaggaaggt tacccggcaa accaggtact gctctctccc atggaaactc agaacggctt 2040
ccagcgcctt cacatcgccg agatggaacg tcaagccaat accttgcttg tccagggcaa 2100
ggaggctgaa tgaaaaagga aaaagggttc accctgacag aggcgatgat cgtcctggca 2160
atcctgggta ttctggcgtc aatcctggtt gacactaagt cttcctggga tgagtctaag 2220
ttccaggaag cccaagagaa gcagatgctg gaaagaaaag atcatggtgg tacgaggtgg 2280
aatgaatgaa gattgtcgtc aacgttgtat ggaagcggtg gactcccgaa gggaatgagt 2340
atgaaggggc ttcagaggag ttcgaggtct atagctatgc cccggcgtgt gagatcgtct 2400
ccatcgtggt cccaacagta gtacggaagg ttccaggagc ttgtctcatt gatgcagtaa 2460
aagttgaggc aacatttccc gttgaaggcc acgaagaccc tttgagggct aaggatatcg 2520
aggtagccct ggaaagcctc atcaagtggg agcttccgtt ttgacggaag aaaacgcgga 2580
ggatgtaata tctttccctc caggtttgaa acggcaagag agaaggagat gggtgaaaat 2640
ggagcttaat cgtattaagg ccgccgactt gaaagctgag tgcggtattc tggcagggga 2700
agatgaaatt ctgggaactc gctgcctgga cggagggagg tgtcatcaca ggtgtgagac 2760
ttcctgcttc cgtaaagagt gctgcgttcc tttgacagtc tccaggttga aagacgactg 2820
gacagcgcct gatgactatc tgtgcacagg ctctgagagt aacacctagg gtcgaatacc 2880
cgggttgaac gaaaccccgc tgacggaagt tggcggggtt ttcttttgca tgaaagaaag 2940
atgaagggca ggaccggttt gaaaatgcca aacttacaca atagacttcg cagtaggagg 3000
gggggtatgt acaggatcaa aagaagtctt ctcaaaagac gtgaagggga ctacagtttg 3060
tacggaaatg tgggacgagg atcaaagcag ggctgaaaaa gggcaataaa aaaggcagct 3120
aaatagctgc ctttggggtc acttcgatag catcgaggct atgcgatgga gacgttttgc 3180
cttggaatgg ttaacaccct ggcgtaaatc cttgaacagg gcgaccaggt ggtgatacgc 3240
ttttctccgg gaattcagtg tacgtctttc ttgaggatta aagcctggat ggtcgagtac 3300

aaccaactct tcaagcaggg ggctggattt acgaaggttg gagacgtatg ccactacctt 3360
cccccaaacg tctacggaca atttctcatg gacacctttc atcgcccgac taacctttcc 3420
ggaatccgtg atcagggtga cgactgcatt tgcaattatc catatagaac ttcacagcgt 3480
cttcatcggg catagaggct ggagctttct ccatggtctt gtcccgcagg atgcagaggc 3540
atccattttc gaagtattct cgcatatggg attcactttc atcattcagg gcttctatcc 3600
actgaattgc ttcttccgtg tcgttgaata gcttgtagga gccagaagac aggtcaaccc 3660
acttaggacg cttttcatcg acaaccttga cccgaacctt gtctccttga tgttgtagga 3720
ctttaacccc acacagaagg ccagtgtact ttgccaggat cattcctttc tcactcatcg 3780
cgttgcctcc agtttccaaa taaggttagt cgaaccttcg accttccctc tgtgaagaga 3840
gacactccaa cccaggtcag tcagaattcg tgcccggctg gcagccccag accaggagta 3900
gtaagtctca gagaattttt tcatgtaccc tcctatttgt tgagagagat gaagatacga 3960
caaagcccca ggactgtcaa gtcctggggc tggatgtagt cagattttca agtaatccct 4020
ggctccgtga gcatctgcaa tggcgtgtcg taccccagca taagggggtg cttcggttgc 4080
tcacatttag tcaggccgaa gcacatcaca ggcttaccag aacggagcat ccatcgaagc 4140
atctgaggag ggcaccctct aagatgctat tgccttgatg atcttaggcc acagggtgtt 4200
gtcgttactg cgccaggttg gatcatccac tacggcctgt agcgctccac gcagcgaaac 4260
gttctccacc ttgagtctgt cgatctcgtc caacagggag aggacggtct taggatcagt 4320
cgaacaaagg aactctgcat ctgccatcac agatggtccg gatattgcac cgctaactgt 4380
gcagattatc gttccatttc cggagacgat gactctctcg tcagtccctt gatagcgagc 4440
gccaggcgtt gccgcctcag ccaccctccg cagctctgcg tgatcggtca tagccacacc 4500
ccgtcgttga tgcttttcaa tatctttgat gcgatctgct ttcttttctg ttcgatcttg 4560
agctgcattt cgataagggt gtgacgcttt cctttgaaaa tgaatgtcgg aacgagtcca 4620
cgttcatcac gtttccacgt agatttgtat gcgtggaagt cctcaagggc ccgctcaatg 4680
cgctgttcag gtgtttgctt cttggtcatg gcttcacctc gattccggct tgttgaaggg 4740
cttgagtgat tcggtcagtt gcatagcagt aaccggtatc gaagttgtct cggcaggagt 4800
taggccagtc ccgccagcaa tcctcaatac catcgtctgg caactccacc ctcagagccg 4860
agcggctggc tttccaggct ttccacatcg acgtgtagcg cgcagagact ccggcaagag 4920
ccacgtatac gtggacgtta tcggtctgca ccgggaagta atcggtgtcg cgccactcga 4980
tcccctcagg aatcgggaag tggtcttcaa actcttctct catgttagtc atcatcagac 5040
tccttcttac gataacctgc gtcatgcaag gcctctgcta gaacgtaagc gttcccatcc 5100
ccagcttcac ggatgacgtt agccatctcg atgatgtcct tacgacgctc ctcggcggcg 5160
atctgctcag ggttggtatc gggcgcacct cgtgcggatg gcaacctgtg tagccatcg 5220
ttgtgcgaca ccacgatcac gcttccgtca tgtccgacta cacggccgcc gagccagcca 5280
tgcacgctgg acaaccactc tatggcaatc cccacaggag gaatgtcctg accgtcccag 5340

gtctcttgtg gcctcgcttc gaatgtcctc attctctgtt ctgagataga gcgagaggga 5400
ctccacctcc ctacaatctc gtcccgccag aaccaggaat caccctcttt cttcatccaa 5460
cttccgaaga ttcgagggct atctggctcc cagtgcgttg catcctttgg tgccttgctc 5520
cagtcaatgc tcattgtttc atcctcctat tcgagaaatc cgttggtcaa ttcaggcccg 5580
actataactc aggccttagg ttcttgcaat aggggcaaac ccatccatga cggaacctaa 5640
cccatccgag ggaacgagct tcctcctcca ggtgtctgta cgtgttaatc actccttccc 5700
ttgtttcagg agcttctccc attggtcctt cattttcgtg agaccaacac ctgtgcggga 5760
aatagccgtg accttcggac cactcctcgg accggtcctc acacctaact tccatccaga 5820
ttcccattac accacctcct tgccgggagc agtggaatcg aacctggcca gagcggcttc 5880
ggcgcactct ttcgccagac ccaagtcgat gcgcttacca ctggctttga tgttgtgcag 5940
aaggtggcgt aaggcttgtg ccagctcctc agcccgggcc tgggactcgg aggcgagggc 6000
gtcgtagtca tcctcacgga cgcaccggat gttccgcgcc tcatcgcacc agaaccgctt 6060
cacctcactc atggcctacc tccttgccgg gcgcggcggc ggcgagcagc tcggctagct 6120
cgttttcggc gtcccaatcg atttgctgaa acacatcctt tatcccgtcg agtgccgagc 6180
ggcacgcgtt ccagttcgcg tacaccttgt cttcgtcgta gacgttccgt cctcggtacg 6240
catcaacctg ctcggggttg agctgtgcct tgatggcggc gaagtgttcg aggcggatca 6300
ggcggtagcc ttccggcatg ctgtgctgag cctgggcgac cggggcagcg tagagaggga 6360
gagtccaaga cccacaccgt tccttcgacc aagtaaggcc accaggcgag cgctttgctc 6420
gtcccaacaa ctcgtccacc acaagagggt tgatatgtag cagtggctcc tgcttctcca 6480
gctcggccag cttggctttc agctcctgta tctccatttc catgccgcaa gccgcgtggc 6540
gcgcagcgtc acccttggac gcggcgtcgc gtgccattgc cagttctttc tccagctccg 6600
caaccctggc cagggcggcg tcgcgctctt tcccgaacga cttgtagtgc cgagtagccc 6660
atgccaacgc ggtgtcggct gcatgctttt ggcgcagcag tgccccgacg atgcgctcgc 6720
actcggcgac cgtcgtcagc ggttcgcgca gacctcggaa tgcaaccacc tccggctgct 6780
ccgcttctgc ctgctcggcc tgcaccggga agggtttcgc gcagtcaggg cacggtgcgt 6840
agatcggttc cggctcttcc agcatgtgcg attcgacctt tccggggccg atgaacacct 6900
cgccttgatc gtggcaggtc gagcacttcg gggaggggtt cttcagggct tcacgcttat 6960
aagagggcca agtatcaacc tcgtcggcgc actcatccag gtgttggcgg aatttagaac 7020
gcttgggcag ggcggcacgg gccatccagc ctttcttgtt ttccattacg gcggggttgc 7080
tttcgttcaa ctcgtagccc tcttcaacct cgtattcctc attggcccat gccaggaact 7140
cttcctgctc agttaggcaa tcacagggga tggagatagg ctcaccccaa ggttgaacac 7200
ctccagaatc cgcgtgacca gagcctccac acttcgggca agctgcttca acgatcccat 7260
tacggttttc catcattcaa cactcctcag gccagagaga atggtatctc gatcttcaca 7320
ccacagcttc aggacatcct gagggatgct gggacggatt tcactcagca gcttgtatgc 7380
cttgctgagg tcctcttcca gagctttcac cttcttgttc agcgtgcgta tgacctcccc 7440

ggcgtaggtg gtgtctgcta cctcttcttc ctcgcagtat accaccatac ccgtcatgtc 7500
cccaaggtcc aggtggccgg cacactggta gcacaaggtt ggggagtggt aacccacctt 7560
gtcttctagc aggttgatgg cctcttgaac actgttggcc tcaacagata aagagcctgc 7620
cagactggca tcgaacagca cagaagcttc ataagttttc attaaaatct cctcggttaa 7680
aattcaagaa aagacaaggt accggagggg ctacctagtg tcaaccctcc agcacacgtt 7740
aacgtctggc ctgctaagct agtcgtactt ccttccgtgg aatgcgttcc cacatccaaa 7800
gattgacaga tacgagttac gctcactgat tcttctttca gaatcgattt gtatcctgag 7860
catctccagc tcatacctgg ctaggccacc ccattcacgc tcaacacgtg aatactcagc 7920
ttccagtctc cgaagtcgtt cgctcatctg ctcctcccag ggatgtaatt ttaagtcaaa 7980
taccttttcg cttcatgtcg aagacggccc acacatagaa gttgcaccac agatggccaa 8040
ggtcttgtcg ggtaagaagg gtgcctgggt taagctcctc gaaatgaact tcaagcatgg 8100
tccgaacaaa ggagtaatca cggcctttcg tctgtagata agtctccagc cagcagtgga 8160
attcctcctg aacagggtgt tcgtggggta ggagttccat caaatggaaa cacatagcgc 8220
tgagctttcc tgtagaaagc gccttgccca taccctgcac tatggcgtcg ctcagtttaa 8280
ttccctgcat tctcatcccc tccttttgat agtagcgtct taccgcactt cgggcagtag 8340
ttcattccat tttcttgagg accgtcttca atgaaacccc attccaagcc acagtcagaa 8400
aaccagagtc ctccagcctg ggcccaccta caccagacta ccacagacgt gctcttacag 8460
gctttggcaa attccttttg agcttggtaa atgtcactca tgcttcaagc tgccttccat 8520
aagcttaaaa cggctctcca tgtcctgacg gtagctgttt tcccaccacc accacacccg 8580
aagaaattca tcccaacact gggcggggct gaacccgtga gcccggagaa gagggaaaat 8640
gtccacgaaa gtgaaccccg cactccagca gtagcctgca atctctcgaa ccgtggagtg 8700
gtgcaaggac acaccagagc tggagacgag gaatcgaacc ttagcggatg cctcttcagc 8760
gctcatccct tgaccttgga ggtgcaattg gaggtcttgc atgcctagta ccccaccata 8820
caggtgggct tccttttgag taaagcacgt cacatcacgc atatcacctc ctcagataaa 8880
ttgagccagg aaagagagtc ggcgatcatt cttccaaatc tctccctctg tgtcaaaccc 8940
ttcctcactt tcgaaggggt acatgacgcc gaagactttg gacaactcct gataagcttt 9000
ggcgacctct gcctcgctct tccctaacca ggtaaggtag ttctccacgt tccagcagat 9060
tcggaaccga ggttcgaagc aggggaagga ctccttcaaa atggcccagc gggccacggc 9120
tgtgagcgcg tcttccatca tctcactgac ctccacctta ggccctgaaa gcttctccag 9180
ggccagtcgg aggaaccgct gacggagggg gttgtcgtac agttcttcga ctgcatcttc 9240
gtccagaacg aatataccgt cctccgagac acggccttcg aaggggaggt ctacgtccag 9300
accgacacgc atgaactctt cagacaactc cgtctggatc gtccgaccat gtttatcccc 9360
ccacccgtaa tgcaactccc acaggcggag gttggtgcag acacgataac cccggtagaa 9420
ggccgggtgg gtctcacctt cagtccaagg ccccatatcc ttaaacaccc aacgagccag 9480

ctccttcaag gcaacctgag tctgccagga gaacttctcg aagtttatct gttcaatcct 9540
cttgagtcgc atcacagcac ctccgacaga cacttgagga aggccatgcg gagcgggttg 9600
tcgtagtggt tcttgtagat tgtcgcttcg tcgtcgaatg ggaacccctc gtcactgagg 9660
tcaacatcca ggaaggcccc gtaaagagag tacaggcagt cctccagatc acaggccctt 9720
tcaggactca cccctctttc gcagatgagg tgttggtata cgttcccgca tatcctacgg 9780
gaggtgttaa acggggaccc atccgggcag cctgagtggt accaggccac caggtcatgc 9840
agggacggga tcaggtcccg aggcagagct tgcacagcac gtagcagtcg ttcatgggtg 9900
ttcatttggt ggcctcctgt ttgcgatagc ctgcatcata cagcacttcc agggcacggg 9960
caaggtccat gctgctcacc aaagagacca tttcctggat accttgctgg cgttccttct 10020
cggcagccag agtctcttgg gacacagggc taatcagcct tggaaggatg gacgatcccg 10080
tctgcttcct atcattccac acgataggaa gaacggttcc acaaacctcc ttgtgggcga 10140
tcacctccag gtagtcccca agcagccact catcgaaagg cgggctcaca ggagaagaga 10200
tgtccaccag ttggactgtg cagccgacag gagggagtcc ttcaccgtcc cactcttgag 10260
gggaggcctc ttccttcttc tgggggcgtg ggaatctcgg accaggtacc gaaggctggc 10320
gttctgagga atggtagtta tacatcgtac cctgctccgt ccaaaccgtt acgatcttac 10380
cttcttcaac acgcagccag cagttcaact ccccattcag ccccagggag aagaagttgg 10440
cctgacacgg agcctggctc cagatgtagc tgtccagact gctataatcc actttcccac 10500
gtgcccgggg ttctcccgac tcaacctgag gggcggggct ggacgtttcc cgggaggaag 10560
ccacgacagt attatccact gcaccttgat acggagatgg caaaggcttg gcataaacac 10620
tcgcaacatc tttctcgtaa ggggtgggag tacgcccttc ggggagacgg aaaccctgcg 10680
catcagctac ccatatacgg gtaaacagat caccttcctt tctccagaag gttgtaaaga 10740
gagagtccgt ccgaacggag tagtgggtag ccgaagcagg tgctttactc aggttcaggg 10800
tcatgccagt ttcctcctgc aagtttcaat gaagtttaga cgtttggggt tggaatagag 10860
cacatcctgg aggtctttca ctttcccctc gaaagggtac gctcctcctt taagaccctc 10920
gtaacgggtc aggcgctcca tggagtctat tgtaagtatc gtggcatcta ccctatccat 10980
gcccttgtgc tccaggaaag agaccacatt cctgcacaga cgtacccttc gggtaaagtg 11040
agggtgctcg gggaatccct cgtccttcca tttcaccagg gccagcagtg cctcttttac 11100
gtcagatgaa agctcagccc atatatactc acgaatttcc atttaaaacc tcccaacaca 11160
tgtcgatgaa tttcagtcgc ttgggattat cgtacagttc gtactcccag tcaaggtatt 11220
cattctcaaa cgggtaaggc cctcctgaga accccgctgc caggaacaac gctgacgtgt 11280
agggccagta caccacatcc gctcgatccg gataccgata ctccatgaag gctgccacgt 11340
tatcacatag gcggtacgac ttcttgaaca ccttatgccc ctcctggagc ccctcctgct 11400
tccacttccc aagctcgaag agggcttctt gcacagacat gggcataccc ttcagggtgt 11460
caacgaactg ctcagggagc ttcattctgg gcctcctgct ggtcctcttg ctggatcacc 11520
acatggagca ggtccaggaa ctccctgcgg tgagggttat cgtagatgtt gtccgaggta 11580

tccaggcgct ccccttgctc aaacgggaag ctgatgtagt ggtacccggc agcgatgaac 11640
aggttgccca tttcgaagtc atcatagggt ccactatccc agtggttgtt cacgttggtg 11700
cagatacgcc agtagcgggt gaatcggtca ctattcctgc acccggcctt gtaccaggct 11760
acgaactccc tcagcacctc ctggagaccc ttatcagtcc tgtcccaggc ccacttcaag 11820
tcagcaacac gttcagcgta ggttggctca gcagtcatat ccttcctcct tcagcacagc 11880
accgacatcc agcaggaact ggaccctgag agggttctcg tagatggcct ccggatcatc 11940
tgtccgcttc ccttcctcga acgggtacga gtggacgaat gacaggccat agtcccagaa 12000
cacttcgtcc agggcatacg caacacttgt ctcaagaagg atgttggtac agagcctggt 12060
gtacttggaa aaggtgcggt catcaggcat accttccttg taccacttga ccatcctggc 12120
caaccctgcc ttcacctttg ggtggaggag ctttacagcc agtacaaaac gctcgttctt 12180
acacatcatt cttccctcct tccagacgct tcatttcatc gttgtaatac atcttggaca 12240
tcatagtcac catgtccagg aaacgcaacc tgttggggtt ctcgtaatac tctcccttct 12300
catcccgctt accacccagc tcgaagggaa agggatgccc tctggaaaac ccagcccagg 12360
acattaggta ttccagcttc cagttcacca tcgccagggg agtgtagttg ccatgggtaa 12420
gaaggtactt ccccacgttg ttgcagattc gtttttgtct tttaaacacg ggatgctggg 12480
gacatccttc cttagcccag ctagccagct cacacagggc actacagatg tccttgggag 12540
tcttcaggta agccccgtag cagcctacag gcagggagcc ggggtcaaca tcatacataa 12600
acaaaaccat gggtgcctcc tctctggcag cgaatatggg gccattgtga agccccggca 12660
ggggcctgtc aacatttttc aattcagaaa accacccccc gccaaaattt cagatgagca 12720
tggggcaccc ttcctgggca ccagcctggg gaatatatct gggggcatcc tggggggtgt 12780
gactggggag gggcatccgt tttcaaattt cagcgaggcc gctggggtgt ttaacaagac 12840
ccaggggtgg ccatgaggcg ccgtcatgat ctggaaatat ccaggcttcg acgaagcaaa 12900
tgtatctgct gaagctgggg catggggttg aatgtcaggg gtgtggcatg gggagtgggg 12960
caatatgtga gagtgaattg tgtaactcaa gctcactagc tgagcctggc tagcctcgta 13020
gaggcctatc aattcatggg tgagggtaag cctattgtgt aatgcaatcc ttccccgggc 13080
tggccattgt aatggcctac aggccgcatg aaccgggcat tctctattcg gtcgccataa 13140
taaaggggga actcttcccg tgagggtaga gagacgccgg cattagctcg cctcgaagag 13200
gcctactagg gcatgggtta cacaatcgac gtattccagg ggctgagcac ctaggatgca 13260
atggggaggg tcatgggctt cctggctggg gatattctgc tgctgggatg tggaatggtt 13320
acacaattgg gctggctggg gctgtgtggt gatcctggca tgcaatgccg ttacacaatg 13380
ggccatggtc atggccaggc tgttacataa ttgggctggt ggcatgggaa ggcttcgacg 13440
aagccagcta gtttaggagg gcaggaaggg ggcaaatgcc cctgcccgaa tgacaggctg 13500
atgactgtta cacaatgggt cacgacgtga cctagtgatt gttacagaat tcttgacaga 13560
ggggagagac caatggcttg atcatcctac ccggatgctc tgcgcatgat tggtgatatg 13620

actggtgatt atatggcttc gtagaagcct gttggaatgg actcgaagag tccagttgat 13680
aaggcttaga cacggggagg gggcacttgc ccactccctc cccgataggc cccctggggg 13740
cgcgcatacg gccttttcaa ggccttttcc atactagggt actgcctaag ctaaactggc 13800
ttctacgaag cctataggcc ctgttaacat ggccataatt cccctgttat cgtgacggtc 13860
cgtaggaccc tccccagggt cattattatg gcgaccgaat agaaacttgt gaccacctgg 13920
cagagaatct atgacctgag gttagggggt ccagggcagc cactcggcga atggttccct 13980
ttcgacgtag gtaagaacaa acaagctgtc cactccaaac cctaccccta ctaaccatcc 14040
tggctctagc tctatgacgg cattccctac gtcgaaccac tcgaattcaa acataggtcc 14100
tggcactgag gtataggggg atgaatgggg aatgtcccag ggcgttacca gtcatgcaag 14160
gcctcctcag actctaccga cagagcttcg acgctgagta gggctttctc caggtcttcg 14220
aacagtcccc aggcttcaaa agggttgaaa cccccgtaga gccgtctacc gttcaaggac 14280
acactgcaat actctgtagc tagccggttg ttacggatgg gggctgtgat ttccagggta 14340
tgctgaccga tctgaacttt catggttcct cctatgagag aaagcccggc tgtgccgggc 14400
tggtaagggt tagctgtgat ggttgcggac gtagatttcg tagcggtcac gaaggctttg 14460
catgtcggtg tctcctctgg tgatgccctg ctaccggcag ggcggcgggt tataggtcgg 14520
cccagccttg ggttgctgga tcgattgccg gcgcctgctg cttcagcagt tggtcggcgt 14580
acacgcgggt ttcttcccag gtgtccgcat cggcgaagac gtggcgcttg tactcgccag 14640
aagtgacgcc gacgtagcga acaagcgcac cgtagtcgcg gcagatttcc ctgccgtcag 14700
cggccagcat caggttgacg cagcagtaca tggcgccgac ttccagcggg ttgtggtcaa 14760
tgtccagaat ctgttcagtt gccatcgtct tgccctcggt gatgccccgg cgaaccgggg 14820
cgggatggtt acatgccggc ttcggcaaag tgaatggcgg cggcggcggt tacgaagcct 14880
tcagggcgct ggtagcggac ccatctgaca gcgcaccaga gctgaagagc ctcgacccaa 14940
acttgcggat accagttgta gccgtgttcg ttgaactcgc gaaccgctgt aagctttgcc 15000
atcgtcttac cctcagtgaa gccccggtct cccagggctc gattgattag ttatagttac 15060
gtcccagatc gtgagccatg gcccaagcgt cgctacgatt ctcgccagct tccatcagtt 15120
tggccacaag gcgctttttc acagcttgct tgctgttccc gttgatcgag ccgctggcat 15180
tacgcttgca ggctttgaac tcttcgccag cttcgaacag ggcgttgaca atggcttgca 15240
ggttcatcgg ttttctccct tgggggctgt tccgtttcgt tggagacata atgcccccag 15300
ggagaagagg agtcaagctt tttctgtcaa catcgcatca gcccacccat cgataagctc 15360
cacagtctgc gcatgcaaat cgactcggcc ttcggctatc cacttgtcac gcagttcgat 15420
caactgccgc atggtcggag ccttggaatt gtggacctct actcggcgaa tctcttccat 15480
atcggcctcc actgcaaagg cgatgtctac cgagtgaatc aggggttctg ccaggcgctc 15540
ggccaggatt gcgcgggcga ttttggcacg acgggtccaa ccccggcttt cgttgtaggc 15600
cacaaccttc tgaatttccg agatgtcata gttgtcgcgg ttacccatgc actcacgcat 15660
ctggcgctct tcctcggcct gcatggcgtc gatacggacc atcacgtcgg ctgcatagaa 15720

tgcccaagca ttgtcgagga tgtttttgtg attcagagcg atagtgacca ttgtcgtctt 15780
cctgtctagt gcttggggct tatccccttg cttgatggcc attctacagg aatggcctag 15840
gctgtcaaca gccttatttg cgtttgttcc tggcagcttg tagccactgc acggcccgct 15900
tagcttcccg gctgagcttc catgtggcgg ggtttcgcat tgccttctcc cgccgcttgg 15960
cggtcttgta ggcaccaata cagtccttgt aaggacggcc ataggcagca caggcgtagg 16020
atgctgcgag cttcacaatg cgactgtcag ggtggcgccg ggcgctttca aaagtaatcc 16080
ggcgaactcg gcaggaaagg ttatattccg cagcgatgtt ttttccatac ttgctcttca 16140
ttttcttctc ctgtttggcg tcccttggcg attaccgctg gaggattata cctcgataag 16200
cttgatgcca agagcttttg cggtctggcg cgcctcggtc tttgtgtttg cccagggctg 16260
aaccatgtcg cggcccaggg cgtccacgat gcgccatgca ttcactgtcc acgcccgttt 16320
caggttgaaa cgttcttgac tgtccttctt caggtatcca gttttcattg tattgccctc 16380
cagggctgtt gtttggcatg ggtgcattat ggggcccttg ccgggccccg taaagcatta 16440
aatccccatc tcagcgtatt tttccatctt gcgctctgcg ggagccatca ccatagccat 16500
ggtgtcattg tccgaccagt ctgcaatcac ttcgccagcg ctgttgccgt agattgccca 16560
cagggagccg acaacgttct cagggccacg tcctttgcgg aaccggataa catcgtcaca 16620
ggtggattgc aggctgtcgg aaatttcctt gacatcggta gactgacgaa ccacccattc 16680
gccatcgctg ccgtccttca cgctcacggt gaagcccatg cgaattcccc gcattacggc 16740
agccatcact actgcctttt cagcacgggt gcgcttttgg gccagttcgc ggcctttgac 16800
atcctcggcg agggcagtgt aatcgtattg gcggacgcca ggcttgcggt aggcgttacg 16860
aagggcgatg cggaacaggg tggagtaggg caggctcgaa ccagaggcct tgtccatgcg 16920
ggcgacgtag tgggcgtatg cgaaggtgtt catgggaatc tccggggttt gtttgcttga 16980
cgggggtcat tatgccgaag cctgggaccc ccgtcaagat gtttttcaga gaatggagct 17040
acctgtgcca gagcaggaac agcaatcgtc gtgctcccct ccgtccgtgg tgttagggcc 17100
tccccggccc agcccttgac agtctttgca gacgctttcc agtatcaacc tccattctac 17160
aggggagtgc tgggatatga agctgcgggc cgcactgtga tggggttctt tgccagagag 17220
gaacaacgcc catgcggcac ggtcacccga caaacgagga gggcagggga cccccttatc 17280
catgctgtag gcttccgggt tatctatcgc ctgcacttcg taggggcttg attcagagaa 17340
cgtactagcg ataagccagc cctcagccgc tgccgctttt tcaaattctt cggtccaaac 17400
ggtcacatta gacattggta actcctatta aggtcgagtg ttaggcggcc agttcgagca 17460
ttgccacatc atgtgcatct ttgaacagga tagcggcttc agaggcacta tacccacgct 17520
ccaccatgaa tgcaatccac ttttcactgc tacgagcatc gcgggcttgg atggtctggc 17580
ccttgcgcag gtgggcacaa tggttaacct tgacctctgc gataatgtac tcttgaatct 17640
tgacctggct catgggtatg gtctcctgtc tggggagcgt tgtgcttccc tttgatgcgc 17700
attctacagg aagagggggc caggtcaagc cctgtaggaa aaagaaagcc cccgtaatgg 17760

gggcttgtgt tcagtaatgc tctaccagac ttcggatggt ttcgacctct tcctgagaga 17820
agagccggcg aagcttggat gtagtctctt gacacgccat gtaagtgtcc aaggccttcc 17880
ggctatctgt gtcataaccg aagtcgccgc agaactcttc gaaggtggaa ctaccgacat 17940
cgcagtcaag gatgaggctg gacaggatac aggcggcctt gggagctaca ggcttgatgt 18000
gacgtttttc ccacgccacc cgtgccagga tgttcggagc cagacggcgg atgtcaacag 18060
gcatgggaat cgtggccttg cggtgcccga tgccagtacg gaattcagtg tcgagaacgc 18120
cacggcggcc atcgccacgg atgaagctta cactccacag gtcatgcacc cagtcttcaa 18180
ccttgcgctc gccgagaggc acggtggcca gggtaacgtc gattgacttc aggaattcgt 18240
caacagaagt ttcgatctct tgagaggtaa ggttttcagt agtcatgatt tagttccttg 18300
ggtttgcctg tcgggggttt gtcttgcttg agggccattc tacgcgaatg gcctaagctg 18360
tcaagcgatt ttagaagaat tcgtaagtag ttttttctgt ctcgccccag cggacgccat 18420
aaccgcttgc taggtcatcg gcgatgattc cagccttgta ggctatgcag tcttcgtcgt 18480
tactggctat tccccacaaa gagtcgctga tgtccgtttt attcccctcc tcgtcaagca 18540
gggtgacaat aaccccgacc caataccatt catcctggca ccatgcacga agattttcga 18600
aatcggcacg ggcggccttt gcagcttgct ggcgtttgga ttgctcgcca aaaaggttgt 18660
gaggataaga atcccaaccc tcacgaaggg caatccggca ggcctcggag aagtcataga 18720
aacgcttcct ggttccgtta ctgttcaaga ctaactcgcc cgggcgctta tctcgacagg 18780
tccaatcgct cacagggcca tgcccatctt gctcttccca aggggcccca tgcacatcat 18840
ctctcaccac ctccaccttg aaagtgaagt ctttgccacc gatgttcagt tcgatatttt 18900
tcatttgtgt cacctttatg aaagagggcc ccttgccctt tgaagcctag tctacgggaa 18960
gcgggaggga agtcaagctt ttattcaggg attttgtcgt ggacggtgaa ccggtgcata 19020
tcagccttgt ggcgggtctc gataacaggc gtaaagggtg ggaaaacgtc gtgcgctttt 19080
ccgtgcttga tgatttgctt ggcgtattga acagcaagac tcttagacgt gaaaagaagg 19140
ggttcgaact ccggcccaat gctgccagtg acgatatagc agtgattggg actctcgtgg 19200
ccttgggtcc ggtcaatgat gttcattcct gttcaccttc ctgaagcggg aaggctttat 19260
accttccctt tgacacgcat cttacaaacc caaaagggtc ttgtcaactt tatacggtca 19320
cgatgttcgg accgtccgtc gccaggtaga tgccgcgatt agccagttta cgcaggatag 19380
gcgccagcac atccggattg acttcatccc aatgcatcac cttggcggta gagtttccgt 19440
ttagccattg ctccgcgatg tggagatgcg ccctggcatt ggtagtgcag gctgctaccg 19500
gatcatcacc tacaaagaca atccagggag tagcaggctc cacagtcgtc tcctcggcct 19560
ggccaaggtc cataacagcg ccctctgcca gcaggaagct ttccaggggg ttttgatctt 19620
ctcccgcgat caacctggca agaggaatcc ctgccttagt ccagacatat cgggcagtgt 19680
accagttgcc caaagagtcc ttttccacct tgggcaggat ggcagcacgg tagaggtgcg 19740
aatcgtgaat gatgaatttg ggggacactt gcttcattga gggagactcc cgatgtaggt 19800
ggttaccagt ccgtttgtcg aaggatactg tcttcagtca tccgggtcaa cttcagaggg 19860

tggccagtta gacgaagcgc ctccccgaac tctgtcagaa ggctgtcgag gtgtttggcg 19920
gcctggctgc ctgacatttc ataaacttcc tcggcgctca ggtactcgtt catgggccaa 19980
tccccgtccg gatcgttaca atctatccac tcccggtaga aggatgcact acccatagcc 20040
atgcagaaat tcatacagtt ggggtcttcc cggaacactt cccgcgccag gcgttcaacc 20100
tcggcaaatg ccagggaggt tacagcagac agggtgtcac gaattgcctt gtgagttgcc 20160
acagtgttct cctcagaaat aatctttgct caggctgtgc gagccgaact cgacataggg 20220
cagcccttcg aaatactttg ccacgtccgg gccgaagcac tccataccta cacccgttcc 20280
catggcgccc attgccaggt aatgcccgaa attctcggcg gtcgcctcac ggtcataggc 20340
gcgcccacca tgacggctct tgcaagcctt gaacagggca ccgatgatct ctacaggggt 20400
ggctttctga actttttcag aacggtaccc agccttcacg cctgtgacca gttgtgcaat 20460
aagagtgtcc gccgcatgga agggtaccca atccgcgctc caaccctgcg gcaattggtc 20520
gagaatctca ccttgcaagg gctgtccggc gtcttcagct tgatctgcga atgcatgggc 20580
aaagaaggcg cgcgccatgt agaaacgtat ctcagtgaac agggccacag tgttcgggtc 20640
gatgttaggg ttgctaccag tcattttgct ttctccgggt caggttttgt cttgcttgat 20700
ggctattcta tcgggactat cgctggtgtc aagccccggg gattcggctc tgcctgtgtt 20760
tttactgtga agcccggcac agccgggctg gtaaaggaga tttgcgagcg gttatttaag 20820
gacctccatc ccgaaagtga ataccagaaa gtcacggttt cggaagcaaa cgccagtctt 20880
tctcggattc accgtggtgg aacaatcgtg gatgttcccc agcttcaccg ggtcaaaacc 20940
ctcctcagcc caatgcccga gcatatttcc atcacgggca acggttacac gtgcaaacgc 21000
atagcggttt ttgtaggctt ctcgtgcgag ggatttgatc gaacgcatga ttatctccag 21060
atgatgttgt ggaacatttt ccttgtgtat ggcggccatt ctacaggaag ggcctatagt 21120
gtcaacactt tatttcaggc aaacaaaagc cccgcaggag cggggcttgg gttatcgact 21180
gtagtagtcc agggagtcat cgactaatcg acttgcggcg gcctgttgca gggtttccct 21240
tacctcttcc acatcgccag atgcttcctc cagggcttgg aatacttccg tcagaggtag 21300
gtagaactcg gcggacaggg cggccacgtc ttcaatggca aagccatgct tatggcctag 21360
cagggtccag gcagccacat catggaccgc ttcgaacagg aaaaccattg ccaggctggc 21420
gcgtacatcg tcgagaatgt tccaatgcat ttcagttacc ctccacagtg tagtcgtagt 21480
ccactaccat agccggggga gtggagatgt tgaaccgtgc caggtagcgg ccaatgcggg 21540
cattggcgtt aggggccacc caggcatcgc ctaactcttc agattcaacg tacagggtgt 21600
gattgatgat gtcagcatcg ctgtcttgcg cgatcacata atcatcacat gccagggcag 21660
tgcagagagt gagaacgaga ctgatcatta gcaaacctcc cgggctttga tttgaagttc 21720
taacaagctg gcttcttcag caaacgtccg tgccaggtcc agcagcttgt agcggttaat 21780
cgtgtcgctc cacaacgcaa tagcgctgtc agtaacctga acagttgaat cactttcatt 21840
caaccatgca ccggactggt acgcctcgat tcccaggatc atcagaagtt ccaccgcttc 21900

caaagcgtcg gcgggagtaa gggtttgcgc ggggtttgca ttggtattca tgtttcgatc 21960
taccttgtaa gtaaggggtt gtgtttctga gagagatact agggccatcc gttgcgcacg 22020
tcaagccctt tttgatcttt tatttgaaca gccacgcggc gaagagtaac gccaccagtc 22080
ctagcaacag cagtgccccg atgtccacat gttgcgcagg ctggatactg gcatcggcca 22140
aagccttgat agtgtcgtgg ttgtcctcct caggctctgc gccatacttg gattcgaagg 22200
ccttccacaa ggcttgccgg cgctcttcca ggctattggc cagttggtca aaagtggcag 22260
gctctagggt atcttgcttg cgccgtaact cctcgcaatc tcggtccagg gcatttaact 22320
tccgctgaat agggcaagcc cggtctatca cggaaaggac aggcttgcgc ggggtgtctt 22380
cttcatctcc cccagatacc caggtcggaa tggctcttaa acgtctcggc acggaatcac 22440
tccccactct ttaccggttc ggaacagttt cactttaacc cccaccttag ccaggcacag 22500
cgccagggaa aagcaatact ctacgggtcc aggatgacat tcaatgacca tgattaaacc 22560
tcaaatgtgg ggttgcaggg taacatctag agcatctttc gtaaaccctg gagaatgtcc 22620
agggtttagg gaaggcgccc caggcgccag gttagtcctt gctgacccac tggttacgct 22680
tgttctttcg caggttgcga agctcacggc tcgccttgat ggcttcttta cgttcggaac 22740
gggcgccgga tttgtcagta cgcatgatat atctcctcag agtgaaaggt taggctgcga 22800
actcatcgca acgatacaca ttgatcgggc ggccagcttt ccccgcttcc ctctgctgct 22860
ggcgaatggt cgggatttgc acgccttgca gtttccagtg cttgagggcg ttgttcacat 22920
cactaggggt ggccaggtgg tggacgtagg ggatgatatc gtacactgtg aactcctcgc 22980
cgagccgagc tttgatccac tcccggacga tgatccgaac ggtaggggtg tcgggcttga 23040
gcgccttgct ggtggactct accactacct tgacgggagc gttattgaca gcctcttgca 23100
tggcagccat gacgcctggc ttgtcccagc ggcgttggga attcttgaaa cgttgtgcaa 23160
tctcttcgaa cttgaccatg ttgtgttacc ctcagtgcgt tggtgttggg gccagtttat 23220
gagtttcctg gccccttgtc aacccttatt cttcctcttc ttcaaccgct tcccaatcga 23280
tggggtcgat gaagaattcg atacaatcgt cgtcttcgtc tcgcagcgcc tcctccaact 23340
cctcggtcaa ttgagactca tactccccgc atgcgcaacg gaaagctatg gggtccattt 23400
ttagcagcac ttggccggca gagaaagaga tgttggctat cttaacccct tcgtggatgg 23460
aatccaactc ttccacgaac tcctcttcgg acaagtccac ctcgcccagg gtgacatcgt 23520
agtttcggaa tgttgccaca ttctcggcat tccagggaga atctacgccc atatctttga 23580
aagcttgaac agcttccttg atcatgcctt gacgtgccag ttctttaggg tcgagagtgg 23640
tcatgctgat atccttttag gaggaggggg accgtccccc tctgtgatgc ctattctaca 23700
gacacgagat gccgtgtcaa cactttattt caggattcgg gtttcttcga acaacttcac 23760
cgtggccagg cgctgttttt ccaggcgggc agccagtgcg gttaggctgt caccgtcttt 23820
gccatagaca gaggagttca gcatcactac tgtgaactgc gggaagtttt cggactggta 23880
caggcgatcc atggtgattc ggaaagtgac atccttattc agcacgcgat cttcgatagt 23940
gccacctgct gcctcctcca gggcttgctg aacgctgcga ccgttaatgt gcgtactggc 24000

cagcactttg cccttaacga tcattttaac ctcgcccgcc aggcggtccc cgtacacctg 24060
gcccagctcg atagcacctg tagcggtatg gatgatgttc attttgggaa cgtagttgtc 24120
tgccaggtcc ttggcatgcg ccatctccag gctgaccgct tgagctttca gtgcaggagg 24180
gagggcaacg gccttgccga tcatggcgcc agcagccagg gacaggggag tggtcagaac 24240
gaaagccagg acggtttttt gaacagtgtt catcgtgtta tctcctacgg agtggattta 24300
ggcaagctgc ccccggcgtc caggggcaac cctaaaagca ctctgctgtt acttgacgta 24360
tacaggcacc gtattgccag ccttgccaaa ggcgaggtat tcgcagccca gcactcggac 24420
gctgggggag atattcagct tgactcccca gcggcgggaa gcttccgcgc cgttatcggt 24480
tacctggcga ggcaaggaag agcccttcac gaaagcacgg gctgcggtac gggaggagaa 24540
atgaatttgc attgtgtgtt acctctgttg gtgttagtga gtgctattgt gcgggcggtt 24600
agccagtttg tcaagcgttt atttcactca ctggcacata ggattcaacg cctcgccagc 24660
ccccgcctac cttggccata tcaccctgga cccgtgtcac ctggccagtc catatttctc 24720
ccgtgccggc atgcttgagg aatttaccgt ccactagctg ttcaggtaga tactggcacg 24780
atccatcgtt ctttttccgg aggtctcccc ggatggctac gatatcccct accttgatgg 24840
gcatcactgt gcctccttta caacaatgtc agagtggtac ccgtcgtgat tgatcagcgc 24900
tttccgaacc tcttcggtat ccgccatcac gtcgaagaaa actgtatcga tcaattgacc 24960
gttaaggaat acgtcccagg cttgcatctt ccatctcctc aggctgtacg gaatgggtca 25020
tccggtttta cgtcgaagat aacgccgacg ccgtgaacct ggccattacg atagagagtg 25080
tgccacctgg ccacgccttt cgagtgcttc accagatagg tgcggcgacg aaggttagca 25140
tctccgtctt cccattggtt gtcccgctcg atagggaagt accgattcag gtccacagct 25200
acgcggtcat actcgccacg gttataggcc gcgtaaaggt cacgatgatt gcagagagct 25260
ttttgcatgg ttaccttcct acaggttgtt tgccttgctt ggggtcattc tatggcagcc 25320
atggcataag tcaacccctt tttaagaaat tgctctggcc acgccaggta gaggaacgca 25380
tacgcgtagc aatgagcatg ccaggtcggt gatgaaaaat cctggcccaa ggcttgacac 25440
gccaggtccg tcctggcatt ctgtaggcat gcgggaaagc aggccggagg ggccgggcgc 25500
cggagacaaa caccgcccct gcgcgctttt caaggttggc acagctattg catgtgaaag 25560
ggagtttcgg cgggagaggg atagataggg agctacttac cgggggagtg acgtgctaca 25620
gcggggagtt gacgtgctac aagaggccaa ggggtcgggc cacctgggga gggaatcttg 25680
tcagttcttt tgatacctgt ctctgtaaaa aactgtcgag taccttgggc gctcggtgaa 25740
aatctgtcga gtaccttacg cccggctgtg aaaatctgtc aggtaccttg ctcaagccga 25800
acggaatcga tcttgacgct cttgaaagtg gaagcccaca gttcctgggc agcctggccc 25860
agagcctccc aggctttgag tgttcgacag gacgggcaga gaccttctac gatgtctcgt 25920
gcaggggtgg gagagtcgca cttcttacaa cgaatctcag gacctagtgt acgcgtgatg 25980
ctgatatgca cttggaagct cctctgaggg gcctaggaga cgatttctcc cctaacccag 26040

gccgtgagtc aggggccggc ttaaaatgta agggcggggc tgggaggccc cttcaaatat 26100
ctagcccgaa atagggggcc agtcaaatat ctagagcaaa tatccccagc ccctcaaata 26160
tctacacccc tgaggacacg atagtcacac cttcgggaat ctcgcctgcc cgtaccaggt 26220
ctgttgccca tccctcgtac tggtaaggga tttcgacaat aacttcgaac tgcttcttca 26280
gcagggcgca gtctatggcc agggcactct cccttcccgg cagcgtcacc gagcgtagac 26340
ctgttagtcc gtgatatctg tctttgagga tcatcacatc taccacagac cgggtaaagc 26400
cagccagatc ggacaccagc cctgctcgac gataggagat gttcatccac acccggttgg 26460
caagccgcac cagttctcta tgatcgctta gcaggcctgg gccacggata gcaacaccac 26520
ccggctccac cccttggaca ggcccaaaca ctaccgaact gacgatgcct tgcagggtat 26580
ggtcgtcaca ccgaagggct gctaccagtt gatctttcaa cagttgagct ttgttcatac 26640
gtgatacccc ttattgcgaa ggatgccttt cgcttccgac cagaaaccct tagcaataga 26700
ttgggggcgc tcaacaaagt ccaggccttg agtgttcagg aaatccacca tagacaacag 26760
gaggcttacc ttcgagaatg atgggctttt agccactgtc cagtacatag acggctcatc 26820
atggtaaaac tgcaaagagt ccaacagtcg gcggcgaaca tcggagtctg cgatgaagaa 26880
cttctcgtca tagagagtct ccatacttcc atctttacgc cgcacccagt cgtcctccag 26940
gcctggtacg ccgccgtagt acttggaagc gaactcccac gactgagggc ttagcaggtg 27000
gccaacagcg cagtggcttc ctttgccatc tgcatagttg cagagaccat taataaggca 27060
tcggcttttc tgagtgtaca gacggtcagc gaggtgaatc aggatgtcag cgatagtttg 27120
agcagtgatc ataagtcctc caaagttggt ttagggctca gtgtataaga gccctccaag 27180
gttgtcaaga cagtttcgga ttgaacccta accaggaggg catccaatca ctaccgtaac 27240
gggtccaggc atactccatc tcctccatta aatccagagc tgcctcttca tcggctacct 27300
gtagagtagc agtgcaaccg ttgcagtcgt tgatcaactg aatctcgtac atcagagagg 27360
accctccaca ttaaacacaa agtgatcgaa cacatcgaac cgtgggcatc catcttcttc 27420
gggcaggata tccagggaaa acaggtgatc ccagccctca tcgtccacca cgtagaagga 27480
accgtccttg tacaggccgt ctgtgataac atcgtaggtt ttacccaacg tgaggtgggc 27540
taccgtaggg tcttcatcct tcgggctgga accctccatg tagagggtca ttacgtaggt 27600
agccttcagc tttcgcccct ctgcgatgag ggacttgaca gcggcacggt gttggctata 27660
aaggtcttgc atggaatcct cctaatcaat gcccagggag ttggccatta gtgaaatcag 27720
tttacctagt tgacgatgag tctcgatcag gacgccttct tcgtctcggc actcccagta 27780
cccgtcgtca tgctttttga aagagtaccc gcactcgacc atctcttcct ggcgtttagc 27840
tgcccaagtg ttcatagctt tcgttccata ctgaggggcg gttggaggtt atctccagga 27900
gcctcattgc ggagacaagg ttactattct ccacgtcaga aagcaacctc atcagacgat 27960
tttttgtcag ctcctggcgg acatcttttg gaggtaccgg acgttgaagc aacatctcct 28020
ccacctcctc ctggtgacgt ttcttcatcc tctgggcctg aacaatccag tcaagctcgt 28080
ccatggcgcc ggagcagatg caggtgtacc ccgtgacgga gtagtcgtac cctgcaaacc 28140

gatccccgct gttctggata gggagagcag cacgaccaca gaaggagcag gagtgggccc 28200
cgcgctgttg aagggtggaa gtaggttgaa ggtcggcgtg tcgggaattc caactgtagg 28260
tgtagccgta cttatccgca cccctgacgc tcttccctac gaaaacccaa gagaagaagc 28320
gttcagcccg ttcgtcgcaa tgggcgggga atttcccctc tgcatgaaaa tcgtagtttt 28380
gatcgttcat ctttacctcc cggttgaaat agggccagtt tacccggccc catacaacct 28440
gtcaacagct ttggccgtac ttatccagcc aaaagtcata gccttccggg gtgtcactcc 28500
acacaaaggc gctcttcaaa cccacgaaca taccttctcc cgtctgcaac cactccgcca 28560
tgtcatggtc agttcgctcc ctgaccagcc ggaggtcagc cttcattgcc agacggcgat 28620
gattgcccgt cagcccttgc cggtcgtagt gattgttgag ctgagcacgt atagtcttgt 28680
tcactttaca cctccttccc agccagttcg ggaatcttgc acacagccga gtgacgccga 28740
gaggtttgag caccccaggc agtcggatgt ttcaggatta ccgtcttcgc ccggacagct 28800
tccacagtgg tgactaccat ctcagtagcc ttccctgcgg tgctggcgat agccacctgg 28860
tcgccaacat gcagtcggcg gcctgccacg tcaaacgctt cgatttcttt actcatggaa 28920
tccaactcct cctttcgttg taaatgaggt gaacttgtct aggaaccctt cccgtaccca 28980
cacctcccga gcctccataa ggtccttcat ggcctcttgc tggcaaggga tgtcggtgta 29040
agcccgggaa gctccccggt ctgcctggat acgataccag attgtactcc cgtctgacca 29100
ccagttcaag gaaaatgtga tcttctcccc tttagggctc tctgcctgga aacgtagcag 29160
cttagtcgtc atacccttca atctcctccc atatcaaatc ttgcatgccc ttgttaatgg 29220
gcatcttcaa cacccggaga gcgttggcca ttttgaaggc ggcctgtttc cctcccagca 29280
ggacatcttc ctctttcatc tcgtaaatgc aaaggttggc ccctagcata cccatctcgt 29340
tgaaagcacg caagcctacc cactctcgtg cttgctccca ggaactgaag ccgaaatagt 29400
aagtgagcat ctctttttca tccttggcaa ggtcttccca cccggggatg tcttcctcgg 29460
ggactggatg aagttccttc tccttgtcta agcacatgca aagatcgata tagaggccag 29520
tagcctgcca ccgagggtat cccaggccct ccagggtctt ggattgcacc ttgctgctca 29580
gtgcgtcggc atacataccc ttgccacaca cggtttcgat tcgaagaatg aaaagagatg 29640
ccataaaatc ctccaggtac gaaaaagccc ccacggctat tcagctcatg ggggctatga 29700
taggtcaacc ggctcggcta gtcaacatga aattgtactt ggagtaatcc acatcaccgc 29760
tcagcttgga gtggctgtag tccgtcacct tcttctcgaa gaagttgtcg tgtcgtgcgg 29820
cgttgatcat ccactccatc cagggcaacg ggttctcacc cacctggtgc tccttgagaa 29880
gctcgcattc gaagagcctt aggcggcaca ggtagtcgat gtactcgaca gtctgctcga 29940
aggtcagatc atgctggggc cccttctcaa aagccaggac cagatacttg acctcggctt 30000
cccggtagtc ttcgatcagg tatcggattt cctggtccaa gatcgaattc tcttccgggg 30060
taaggtcctt acgcatggct ttcaggacgc ggatattctt ctccacgtgg tcggtctcat 30120
ccgccaggct ccactggttg atgtcgttga acccgatcag cttgccgcaa cgcttcatgt 30180

tcagcagcac agtgaacgca ccgaacaggc ctacaccttc gccggtcaga agttgggcca 30240
ggatgcaagc ggcctggagg ggctcggacc agcttgcgtc tatccgattc tctcccatca 30300
cctccagttt gtctcgcatc tccttgtagg aggcaaaagc tttccaatcg ccatcggtga 30360
acccgaactc ctcggctgcc agggcataag ctcgctggtg aaccacctca cgggaggcgg 30420
cagtaatgag ccagtttcga atctcattgt tcttgatgta cgggaggagc ttggtgtagc 30480
cctcgccgac tgtccggtcc atctcggtga acatggacag ggttttgtcc agcagccact 30540
tgtggttctc gtggggaacc tggttggtcc cgagccctga ggtgctgaaa tactgctgct 30600
tgtcgtctgt caggtctgcc tggtgaacat cccaatgcat atcgatggca tggacccgag 30660
ctgcctcgac tgcccaaggg taaacaaagg gacggtaact ggtgctttca acaaatacgc 30720
tcacaatctc tcctttcaaa gaaggggccc gaaggcccct ctgttttact gcccagtcca 30780
gatttcatgc ggccacatct ggccttctgg cgggggttgc atcgggttga ggatgatgtc 30840
tgcctcttcc acctcatggg ggatgaacag ctcgtttttt tcagcctccg caacgcccat 30900
gatgttccac tcatagctgg cggtgggcgg gaacaggaac cagcccgcaa tcagcagctt 30960
gagttgggaa cctccgtgag tggaggaatg gtccattgcg tcgatgagga agtaggtgtt 31020
gtatggtgga attggcacca cgaacatcat gtcttcgttc atcgtgatcg gataactgaa 31080
gttgacgtac ttgtactgca acgccagaca gttttccacg gttaacgcca tgtgtgctcc 31140
ttacccttgg caggacaggc aagtgtcctc gtccagggag attgtgtatc gcactgggag 31200
ggcattcaga ggctcagcgc ccccggtacc aaccttcatg ctcttcgccg ccttggagcg 31260
gcagtagtag aggctcttca gccccttgtg ccacgcatag atgtggatgt ccgacatctc 31320
ttgcagcgtg atgtccgggt gaacgtacaa gttgaccgac tgagactgac agatgaaggg 31380
ttgacgccct gctgctgcct caatcaacca cattgggcta atctcccgga tcgtcttgaa 31440
caccgctttc acctcatcgc tcaaaccttc gatgtgctgg caagagcctt cagcatcgtt 31500
gatcgaacgc caggtttcct tggtgtccag gccgactttt tccagctctc gccggaggta 31560
ttcgtttttg atcaggaagc tccccgcccg gccttgagcg ttgaagcagt tgcctgccca 31620
gggttcagtg gacggagata cgttgaccag gctggagctg ttggcattcg gagcaatcgc 31680
cagcaaatgg ctattccgac gcccgcttcc ctggcagtcc ggagcctccc cacgggagcg 31740
tcccagctcc aagctggcct gtacggcctg ctccttgatt tgcttgtaaa cctggtagtt 31800
gagctgctgc atgccaccca tgccaccaga ttcgaacggg atcatacgct gttgtagcag 31860
agtatggaag cccatggttc ccaagccgat tgcacgctcc ttggaggcgg agtgtacggc 31920
ccggtgcatg ctcggaggtg ccaggcggat gaagaactcc agcacgttgt ccaggaaccg 31980
aaccatgtct gcgataaaca gggggtcgtc cttccactca tcgtaggtgc ccaggttaac 32040
actcgacagg cagcacaccg cagtacgctt gaagctggtg tacagggtga tttcggagca 32100
caggttgctc tgtcgtaccc ggtacagcgg atgagtgatg cattccggta ggttacggtt 32160
caccgtgtcc acgaacaaca tgaacggctc gcccgtgtcc ttacgaacct gaagcatctc 32220
ttcccatagc tcacgtgccg gaaccttacg tccagtctta ccgtgtttgg gatcaacgaa 32280

ctcccaatcc tcacccttga tgaccgcctc catgaaggcg tccgggatgt tagcaccgtt 32340
gttcaggttg aagcgcttca gcttcgggtc accgcctacc gggttccgca tttggaggaa 32400
cacagggaac tcggggtgag ccacgtccaa gtacatggcc atgctgccac gacgagtacg 32460
ggtctgacgg taagcccggc acatgacatc gtagtcccca gcgtgctcaa tgacgcccgt 32520
agacagctca tccggggccc tgatgcctgc gtagacgcct accccaccac cagccatgct 32580
caactctttc gcttcaccag aggccgttgt gaggccttta cggctgtccg cgatcatcga 32640
taggaagcaa ctgatgggca tcccggtggt atcgatgttt tcttgaagcc agtcccctgc 32700
ttcgtcaaac tgctcttcgg tgaaattggg ccactcgatg tccagggcgt tggtttgtac 32760
cgggcttgca ggcactgccc actgccggct gaaatagcca tacatccgct gagcaaaatc 32820
gtagtcaccg aagcaatagc tcaccgctgc tcgtgacagc gtttgttgag gcgatgtctc 32880
gtgttccttc ttgtagaagc cgtccgaggt gagcatcgcc aatccttgtt ccgggatcgt 32940
ccggtccagg gaataatcaa tcagaatgcc gttgtacggc tgaacactct cactcattca 33000
ggaaatccac catagcctgt gcagggttta gtcgccgacc catatggatc agctcatcac 33060
ggaggctacc gtccttggca gaggcgatga ttgcctcctc gctcggtgcc cccttcttga 33120
tccagctctc gtccagtcgc tcatagcctt cgaaacgttc gccggagata tgttgcccac 33180
ggaggttccg gtgttcccgg tgaaccactt cgtagggctg tgtggtgtcc atgccgttgc 33240
ggtacagcag ggctgccaag accttggggt tacctgcctg gtaggcggcg aacgcctctt 33300
tgaaaccagt taccagccgc aggtccgaca cggacagcga aaactcgacc ttaccttgac 33360
tcacagttta ccccttcttg caattgttcg gaattagttg acgatgttga atccaacctt 33420
ttaggccgga ggaccagtaa caaccatcac ggtcctggtg ggtcacaccc ttctcccagc 33480
cacctgctat atgcatgggg gtggcctggt gctcggtagg actggcgtgg actggctcgc 33540
tattgatcag cttgtcgaag ataacttcgg ccttgtcctg gctcttgtca gtggcacggt 33600
agctggtctg agcacagcag cttgcagaaa gcttcaacgc ttgctctaca gagatgacct 33660
ccccgttctc atcgaagtat tgccggccca ggcccgggat gaactccgtt ttcacatagg 33720
gaacgtgcca ttgaccaggg accagcaatt caggggccac cttggcagtg gcctccttca 33780
tcattcgagc caggtgctgg aactccggct gggcgtcttc gtgatcacgg agccagtaga 33840
agttctccca ctcggtagcg gtcactaccg tcttcatcat ctggaaaggt tcaaggaccc 33900
ggttgacaat ctgcttgtgc agcccaacct tgtgcatagc cagggccatc ccagcggcgg 33960
ccttggcagc tttgatccag agatacttgg ctgccacctg aagaggcttc gacagctcct 34020
tgtgagcttg catgccggcc tggttagcac cccaatggac tggcatagct gggttattcc 34080
gcacctgctc aatgagcttc ttggttggga tagcccgact ggaggcacag ttcttgaaga 34140
gagcgtggtg cgtcatcagc tcggcgtgga tgaaccgggg atactccagc tcgaaggtgg 34200
tgattgttac cgggaactgt gatgtacctg ggtaggaaat gcttttggcg atcacgctcg 34260
ctttcattgg ttactccttc ttcgtttctg ctttggcacg ggtctgaatc agctcccgct 34320

gagagaagaa taccatcttc tcggcgtcat agacagcgtt gttgcccttc tttttcttac 34380
ccaaggtgcg ctcggctgct tttcgccaga ttgccttgaa cacgttcgct tcggcgaagg 34440
tcatgcccag tgcttcgata aaatcgttgc actcccctat gtagggctcc ccgccagagg 34500
tggggtttcg gacctctacc tggtagtagc tggaagaact tcccgtgtgt tccccctcct 34560
cttcggtagg ggctacaggg tttacccatc cagacgggct tccaatggtt ttgaacatct 34620
ctgccgcttc tttggaagcg acatactcgc tggcagtgat cattacagag ggtcccagtt 34680
tcacagcttg actccttcgt ccagggaggt ggaggccacc ttcatagttt cggcaatcat 34740
ctgcgtggca gccagcagag tgacatacac agtgatcatc ggggtgtcgg ggtagttctt 34800
ttccaaccac atcagcaggt cagcccccag ctcctgatcg atggacactt ccatctcagt 34860
ggtctcaggc ttctctgggg cgtcgagcag gtcgctcatg atcatgcgtg aattctcctt 34920
gtagatatga aagagcccac ccgaaggtgg gctttgtgtc agatgcccac taccaggtgt 34980
tcccggtcca ggacgttctg acgtgtgcgg gagtgcttcc cacagctatg gcagcggaga 35040
acctcgaagg ccgacactgc ggtgtagtac agcttgccag tgggacgaag atcgtccgaa 35100
cagcacacag ggcaaagagg agtgaccttc tgagggatca acgcagagag cgacgggtgg 35160
gtattatacc acgcacgcag cttcaagtaa acctcttcca ggacaatcac gtcgaagacg 35220
ttgtactcca tcatgtgatc ccaagcctcg gggtcaccat ccataaccct cgcccacagt 35280
tcgaaacccc cagtgtcgag tttatcgtgc tgacacttca ggaaaatcgc cagggacttc 35340
agcgaattgc tcgggaactt gaacaccttc ttggcgatgt cgcacgtgtc gatgatcttg 35400
tacggactgg gaggcatgat gtcgtgcttc accaggtgag cattgatcgt cggcaggtcg 35460
aacttacgcc cgttgtgggc cactacgaag tctgcttcct ccaacaggcc cactaggacc 35520
ttgagaagct ctcggtcgtt cttcggctcg gtgttgtaca ggtcgaagtc cgggagctta 35580
cactccagaa tatcatcact gcccagccac ttggccgaga aggtgagcaa gtaaccctct 35640
tgcaaaacct ggttctggcc cactgtggtc ttccagcggc tccagtagta gccgatagct 35700
ggggccgtct caatatcgag gatcagcact ttgggcatcg gtttgttgca ggtgatattt 35760
tgagttcgaa ggaagtcgct cacagtggac ttggccatgc ccagctccac ggcaatctga 35820
cgccaggact tcccggctgc tgccagttct agagcggtat tgtgccacgg tttcatgaaa 35880
tctccttagt gcgccgtgat gacgtaattg gacgggtgac cacagcggag gtggccatag 35940
ctgttgtcga actcgcagat ggcgtcgaac cactcttccg agtcctgctc taattccccg 36000
gtaatcaggg cacacagctc atcgaaccgc ttgcactctt cttccgggac caggtaccag 36060
tggcaggaat catcctgcct gaacatacac cgcttggtca tttgtacttc tccttcagga 36120
ccgtgaagat ttgatccctc cgtttagctg ctgtagtgtc tggctctacc ccgtgatctt 36180
tcaaccacgc cttgacaacg ttgacaccct tcttgccgat ggcgatggct tccttgtcag 36240
cctgggcttc tccccaggac tggaatccct gccgctccat gtgggttttg atttcatggc 36300
acggcttgca cagccaggcc aggtcgtcca tacctaccag caccactcct tcgatgaacc 36360
gttgtaggtc ctcaatagag cggaggctgt attcccctgt ccggtgatcg acttccatgt 36420

ccgctgcacg cttcacctcc ccacatttgg agcactgggc gccttttacg gttgggaacc 36480
gcttcatgct tctcgggttg gtgttcacca tctccacaga gttgttcttg atgaactcca 36540
gcttgaccgg gtgcctcatc cacatggcgc gacggatacc tcctcgcagc caggacagga 36600
atgaagcctc agtcttccaa acctctggaa gctcttccca gggtcggcga cgaacactca 36660
tctcccaccc cactggaaca acagccggtt gaacaggccc atgccgtaga tagcccccac 36720
cttggacagc ttcaggggtc ctagttggat cacgcagatg acacccgccc gaacgaagta 36780
caggtggggg aaccaccccc acaacttgcc tttctcggtg accgtctcca tcagccgaac 36840
tcctcttcga ctgcctcctc gggagtcagg ccttggtcgt agtagtcccc gtacaggcct 36900
tccaggaagt acgcatgggc tgacggcagg ccgcgacttt cgagaaccag gtccaactct 36960
tccagccaat ctgtaaggct tacttgctgc attctatcac tcccattttc tccagcgtct 37020
gccggacatt gattcggtcg ttttcccaac gctgcatgtg ggcgcagtcc acgtacatct 37080
gcatgatgtc aatggcgtcc tttcgcataa cctcgtcggt ccagctaacg tactccactt 37140
cctccggata ccagagcttg tattggtcgt acattgcctt ccaggcatcc cggtcgtcct 37200
ggcaatcctt caggacagag tagaccttct tctccccaaa gggctttgga gtgcctccgg 37260
cctggtaggt ggcccactcc aggatgtcac gggggttgta gttgtccgta ctgtctccca 37320
ggagaatctg gaagtacagc cacatccgac catgcccctt gatatcctta cccatctcga 37380
cgagctggcc aaaccccctg ttatccataa tgcccaggcc aggtttgttg gggttgtaga 37440
gacggctctc tactcccatc gcatccttgt cctgcgtgca agggataatc cattgagcag 37500
caccccttcag ctcggcaatg cgtccggcgt accctcgggt tgccagagca tcgtctgcct 37560
cgcagcggta agtggtcagg gccccgtgct tttgatcagg gtacttcttg gcgtcgctca 37620
agtgtaccgg acggagggaa tcccccgggg actctttgta gcgtcccgcc ttctcagtgc 37680
cgttcggcgc tcgcttgttg ggaggggtgc ggtagcgctt gggaaggtcg atgtcgagtc 37740
ggaagttgtc cgggcccgaa atcagaacct ggtagcccgt ggcattcacc tcagcacaca 37800
ccccggcaat catttgcttg agggtgaaca ggacgttctc gataggctca gggtactgga 37860
catcccgaat ttcgaactga tcctcagtga acttcccacc gtgctgggtc ttcaggaagt 37920
ctcggaattc cgtgcgatgg ttgaattcct tctccctacc gctggggatg tgcttcgcta 37980
tgatagcacg cccctctgcg gcagagcagg ccttaaaggc ccactcgtca ccgtcgatta 38040
cggcaattgt atctgccggt tgggacatta tgcgtcctcc gagagttcct cgtaaacctg 38100
acgaagctgg gagaactcat agtctttctc ctcgaagtcg cgcttcgcgt cgtgggctgc 38160
tgccttgtcg atcagcttga tacggtcctt gtccaggccg tgctccttgg catccttctt 38220
gagctgcgaa cggtcgtctg cgttggtcag cttttcacgg tgcagagtca ccaggcggtc 38280
gaacagttcc ttctcggtca tctgaccttc acgctcgtcg ttgtagtttt ccatttctct 38340
ctccttcagt tctggatgtt gatggtgctg ggggaaacct gctcagtttt ccgaggcagg 38400
atttggaagt cgaacagcgg ctgacgagat tccgggtcag ttgtgctggg aatcatcagc 38460

gtgcttttgg agccttgagc aataagatgt agctgctctt tccaatactg gaaatcctct 38520
ttcatggcta ccggaatgtc atggccaaaa taggcctgcg gcagccgtag gacgaagacg 38580
ttgtgctggt cattcacaac gagagtgttg ttctgtgcca tttactcttc ctccacccac 38640
gcgtgggcat cgatgtagtt gcttaccatg tcctgggcga acccctccag gaaccgctcc 38700
ctctctttct ctgacatctc ttcccactcg tctcgtggca tgtgctcttc atcttcatga 38760
acaccgcctg cgaaaccagt gttaacgcgg cacttgatca ccacgtaatc ttccattctc 38820
atctcctcct ttggagtaaa agaaagggcc ccgaagggcc ctgagggttc cgtcttagaa 38880
cggaatatcg tcgtcgaagc tgtccacgtc cgaagcagga ccctgagctg ggggagcgct 38940
ctgctgggtt gctacggggc gcggtgcggg agcttctttg gcttccggta ccttgtctcc 39000
gcgttccttg gccagttcac ggagaacggc ttccatgcgg gtgccagcca ggttgttagc 39060
ctgctgaatc ttgcgaatca catcgcggcg gagcaggcgg gcggtctcga tagtcacgtt 39120
gtcgaagtcg atcaggagcg gcttctgacg aagctcagcc acacctgcac gcatcatctt 39180
ggccgggacc tgggtcgcgc caccgaagct gacgttggta tagacgagcg ggttgccgtc 39240
tttgtccttc ttgtcgttct tcgccggggt cttcttgacg ttcacggtgc acatgaacgg 39300
ctggttgagc agcggctcga tatccaggtc ggtaccgtcc aggatcgcgg ttgctgcacc 39360
cggttgcatt gctttcgcca gcttggtcag cgggctgttc gggtggtagg tccagatgcc 39420
accttcgacg atacggcctt cgccgtcacg cggcggggtc gcctggaggg tgataccact 39480
cacttcacca cggaaggact tgttcagcat caggcggtac tgttgctcac cgatgtcacc 39540
accgtagtcc actacttctt ccaccaggtc tgcgaatact gccacctgat ccacaggctt 39600
ctgagctttg ctctcaccgt tctcatcaac gaagtcttcc cggttttgct cgcccaggta 39660
gacgatcagc gagacgcggg cttcgtggtt acccggggtc ggctcgggga ggttgacgaa 39720
ctcacgcggg gtgccggtgt tgccagtgct tttgggacgc agtgccatgt tttacttctc 39780
ctatcaatta ccctatcggg cggtagttgc ccctaatcgg ggggtttgtc gcttgttagc 39840
gaacttcttc ggtgacaatc ttgcgagtaa ttcgctcgat gatccaccct aactgcttgc 39900
cacccagttg agctttcatc ttccgggcgt cttcccgaag gctgaaggcg taggaccggc 39960
tggtcagatc gtacttgttg cagaccacat attccgtggt ctcctctacc tgaatagaca 40020
tgatgtcctc ctcatttgga acgctattga agcatataag ctattgaaag tcaaggactt 40080
ttatcaaaaa cttgcatact tctggttgat agctccccgc gcattattgc aacgaggagc 40140
ctattgaagc acaatccgat ggcaatgtca agtctctcgg ataccttgca ccgtcaggta 40200
ggccaactcc tgttcaaacc actccgcatc gttgggcatc tccaaaggga agtccctacc 40260
cttggcgtgg ttctctgtcc agtagaggag gaagtcctcc aactcctggt ggcgcatgtc 40320
cacgtaatcc atgatagcca cacctgttgg tgcggtattg cctttcttgc tcatatctcc 40380
tccaggcacc ttagtggcag gtgccccacg tggttcccaa catgtaccct gcggacaggt 40440
ctactcgcag attgaagtgc tcaccagcag ctttgacagc ctcgatagcc gtctcccctg 40500
cccgactgta gcctacgaac caaccgccct tcggggactc tccgatatcg ctccagattt 40560

gacccgagga ctcttccatc tcgtccttcc actgctggac cagggccttg catcgggcgt 40620
cttccttctt ctgaagcttc ttctgctcct ccgggtcctc gtgctgaatc tcttgccagc 40680
ccaaggcttc cttcgagaac atcttgaact tgaccaaatc cttggtcact tccaactggc 40740
tctcatcgtg gtacgcaatg agctgctggc aatacgactt attcttccaa tcatccttcc 40800
agaagtctac cagcagacct tcagccatca gcttctggtc gtgcatgatc atcgccacct 40860
tggcacagat aacacccgcc gactggaaca ggctgttaac cagggcatgt tcagagcgag 40920
tcggaatctg acggccatcg atcccaacga tgaacttctt gttccccttc tccttccaga 40980
agtcggtgag aaccgtcttc agcagcgcaa gggggctagc agcctcccag aaggcgtcga 41040
agatggcctg ggcctcctgc atagagcagc cgacgatctt cgctacacga gccacctgag 41100
ccccgtagga gcagccatac ttgaccgact tggcattggt acggctgaag tctcctccga 41160
tgatggtgga aatcttcgcc gccgtcaccg tgtgaatatc gttgggcttc tcagccacaa 41220
gcgctaccgc atactcttgc gctgcctgga tgcatgcttc ccggtacttg gcgaagttgg 41280
acccaaactt gatcttgagc ttctgtgccg cagcagggct gagacgcatt gccgtgctca 41340
ggcagtagtg ggcttcaatc ttcgcttcca gggagtcaaa gtcgtagcca atctgccaac 41400
acttgttggt gtccacgccg aacaggtttc gcatcggtac cccgtacagc gaggtcacac 41460
gaggaatgtt ggccacctgt ttgtgtttga accggctggt accagcacca caactgtctg 41520
ccggagttgc aatccggcca tcttcacgct gataggccat gtaccccttg acaggcatgc 41580
catcttcgtc caggtcgtcc gggtcatacc caccacccag gatcgagttc cgacgatggc 41640
gatacgtcag gtagttggtc acgtgccgaa cgtgagggaa cttctcggag attcgttcca 41700
gggcgggaca gaggtccttg tcctgaccta cggtgaactc cgggttggtc cacaccttca 41760
actgccggtc attgcctcgc tccaggatgt actccagaac gatgtctggg ttgtccatca 41820
tgaaggccag ggaccactta gcacgcagct tctctttcag gtgctccagt cggtacttct 41880
tgaacgggct tgcataggtt tgctcgatgt acgaggtcac caccttttcg aacttctcga 41940
aaggtagctg ggccttcgtc ttctgatccg acgaaagatc acgctccttc cacgaagtcg 42000
ggtcccatcc catgtccacc agccaaccct tgatgtgggt tgtatcgctg atcttggcag 42060
gcacctcagt gagcaagctt accgtatgat cgaacggtag gccatacgtc ctaccgttga 42120
tttccacgta gttgggagca tccggccccg aacgtggaag gtgcttaccg cccatcttct 42180
ccaggaactt ctccatgttg ctggagagtc ccagaccgcc cttgatcttc ttctcctggg 42240
tcttcgggaa gcaatacttg gcagcttccg tcttggtcgg cttcttcggg ggaaggatcg 42300
gttcaacaat ggcacgacac tcctccatca gttgatccag ctcccggacg ttggcctggg 42360
ccaggcgctc gtcgaagaag aaccccctgt gactttgacg ggtcaccacc caagccacgt 42420
agctttcggt ctggactgct ggggcccagt tccaggcgcc ccactctgcc atcagagctt 42480
tgtagaccag caggttgaca tccacgtctc ggacgttgta ctccagcatc tccgggtgat 42540
acgtcttgaa ctctgcacca cgaggggcgt tctcggtaat caatcccagt tcaacagcct 42600

tcgcacgcca atcgatcttg gccaggccca gcttgtcacc ccaggcccca atgccgtggc 42660
cagggcgacg gtcagggtta agggttttgg agattaccag ggtgtccacg atcttgacgg 42720
ggttaccgtt gatggtgtcc ttggtctctc cccacccgat ggtgtactcc agcccgcatg 42780
tcagcttcag agccagcaag tcgtagttga taatgttgtg gccaattacg accgagcact 42840
cttcacccag ccatttggcg aagtccatca cctcgtcagg accccacttg aacacctcgt 42900
cggtgtcaat gtcccgagct acagcacagt gtagcttgaa atcctctttc aggcgccagg 42960
gtgaggcggt gtaatccacc gtcccctccc cgatcagccc ggtagcctcg atatcgaata 43020
ctacccggcg tttctgtacc tcttccacgt tactcctcct ttcatccgtc agaacggaat 43080
atcatcgtcc ccggtcggcg gtgggccgtc caactgaggg tagacgggag ccacatcttc 43140
cggcgaatga acttgctcga cacacgctgc ttcagggtat tgctctaacc cttcagactc 43200
tccatgttcc tggttgtact cctccacctg ctggttgagg aagtccgtca gggaaatcaa 43260
ggtgtgtgtc ttgatgttat acaggaccct acccgctggt ccggtgtacc ctgtgtggcg 43320
gcatttggta agggtcacca gggtggtgtt ccgttccaaa tcgtcctccg cgtgcttgtt 43380
gcgcaccagg gtgatgttga tgttgcccga cttgtagatg gtcgaggagc caatgatgtc 43440
ttcctcgctc agctcagcgc cccggcttgc agcagtcttg ttgtccttgc ccttacgggt 43500
gtggttgacg ttgatgaaga ttacgtcgta agccttcacc atctgcttct gccacttcat 43560
gaacaacgcc tgttcacctg tgtccatccc gtcaatcagg tctgacattg ggtcaaggac 43620
gatcacctgg atgccccccg aaatgatcat ctgctcaatc ttctgcttga tcgcatcgtg 43680
gtccccccga tcatccacca gcaggaaccg gtcagaccca tcagcgttga ggaacagctc 43740
gttactcgcc tgctctaccc tgtcagattc gattgttgcc aggtactctt gagggtcctc 43800
aatcaaggcc agcttctggc ccaggtgccg ggacagcaga taacccccgt actctgcaat 43860
gtcggcctcc agggtgacga tgcccatctt gtaaggactg ttgaaaatcc aatggtacac 43920
cagttcgttg acccaggtgg tcttaccgat gccggagcct gcgatcaggt tgacgatgga 43980
gttcagcggg aaacctccag cggtcttccg agacagctcg tacaggaacg agggcagggg 44040
cagcttaggc gccattgcct tgttaagcat agcctctcga atctgacccg accccacgat 44100
gcctgccggg gatgccttgc gggcgtcgaa gaaggcattg atgaacgcct tggcctgctt 44160
gtcctccaac atcttgttgg ggtccttccc tgggaggttg gcaatccgta ccttccccat 44220
gggcagcact tccagcgccg cctgagcacc ctgctctccc ttctcatccg cgtcgaacat 44280
gaggatgatt tcgtcgaaag agttgatgta ctcatagttg gcagccaact ggctggctgc 44340
cgaggtttca cccacagtcg gacttacgac tggatacggc aacggtgcat ctgcgatgtc 44400
cgcgtaaggg ttcttcttct tccagcgctg gagctgatca actcggagca tctggtaggc 44460
tgcatgagca tcctcctctc cgcctacaat gaggaggtat ttgcctccgg cagggaacag 44520
gtgttgacca taaagctcac agtgtttgtc gttccgaccg atggcgtaga aggacttggg 44580
aatctgacga accttatatc ctaccagctc accatggtcg tggatcggat aataccgcac 44640
cacaggctcg ttgtctgcgt agtgcgtgat acacccgtac ttctcgttgg tctgccgggc 44700

tatgccccgg aacaaagtcg tgtcagcccc gccgttgcct tggattttct cccggtcttc 44760
ggcagtgatg acgggcttac gttgaatctc tctcacttct ttctcctctt tcctcctctg 44820
ggcatgggca cgtcgatcac ctttgatgat cactgctccc aactcttccc ccaggggaga 44880
ccttgccagg gtgttgttcg aaaccatccg atcacaactg aaacagcgtc cgccgaagac 44940
cccaggctca tcctcgtaga ccgccatagc gtcgctgcta ttacatcctg gcaggacaca 45000
ggcgaacttc ccaatcagca cttcctactc cttctgcaat aaaaagggcc caatcagggc 45060
cccttagggt tacgccagca ggagaggtgg tggctcggga ggcgggttgt tcgcctttcg 45120
ctccttctcc tgcttgtcat agaggtgctt gtagcttcca cgctccccct tgttcagagg 45180
gtaagacact tggaagactc caggaccaga ctgtaccaca gttacctggg taaagtcaac 45240
atccattcgg atacgttgct tcaccttctc gacggcctgg cccgccgtga ggtccattac 45300
ggtgaggata ttaccatcca gcttcagctt tgccattatt gatatccctc ctccggcatc 45360
cggtctgtga tggacatcct cagtccgatc acgttttcta caggtacctc ctcttcatct 45420
ccgtggggga gtaccgacac cagcagaagt ttcatgtctg aggagtagcc gtacaccgcc 45480
ccagttaaaa cggtgtcata ctgggctttc ttgtcattgc cgtctacccc attcacatac 45540
ccctcttcgc ggaagaggat gacttcccca ggaagaggga gttcagacca cgggaaatca 45600
tcctcctggc gaaggccttt gtagggtacc aggtcagttt cgggtacgcc gtacataacg 45660
cgaaggcgcc catcaacatg ccagactatc ccgtagcctt tttcattgca gaggttctta 45720
accttacctg ttgtgctgat cccgtgaggg atgtagaaca cgataccgat ggcacctttc 45780
tcccggaatt ccccgaatag cgtgcccttg ctcccagaca cttccgggtc cggatgaatg 45840
cacaccaggt caagcatttc aaatttgctc acggtacttc tcctgcgcaa tgcgttcttc 45900
gcgggtttgg cgtttgcaag cttccaggta cggcatcagc aggtgtttca gggagttccg 45960
ggctgggagt acactttgaa tctggtcata catgtcgctg gagccgggca tttcggactt 46020
gtagccttgc atgaagttgg cccattggtc catggcatgg accttgtggc ggctttccca 46080
tacgcactcc agaataccat gcatccccag gccttctcgc agacgttcac ctgtgtcagg 46140
gcagatgact cctcgaagct ccggaacggt catcagcagg acgatcttgg ccgcctgggt 46200
cagtccaggg taaatattct cagcaatgct gggataaacg ttgatttcgc tcatgtttcc 46260
tcctgtaagg cccggatgat ccgggccatg ttggtcagtg ttcgaagccg tgaggagtat 46320
actgcctgaa acgcccctgc atgtcaaccc agtagaacga ttcattcatg atgtcggcct 46380
gtaccatgcc cagctcgacc agtcgggcga actcttcatc gtctcgaatc tggcgagcga 46440
tttccaggga tttcaggatg ttttggaact gggactgcat ggactctcct tactcgtgtt 46500
tcgccagcca agattggctg atagctttga aatgggggta gtgaccgtag cggttcttgt 46560
ctaccgctgt gaaaacaagg ccttccctgt tggtggcagg attatagctc ttaccttcag 46620
ccatggcaag gatgtcggca taagtgacat cttcaaacac atgccgggtc gagagcacag 46680
gcacatgctg aatatcgaga gtttccgcca gcatcctgca atactctggg gactcgtaag 46740

caccctcctt gatgttgaag acactgaaca agaagaaggt gggtttctcc agtttctcct 46800
tgttgccctg gatagccggg ccacacacct cgccttgaat tgcgaacggc tgtttgctga 46860
gttcatagaa ctctgccaac ttgcgaggaa tgtcctgatc cagtacagcc tggccaaagt 46920
ggttgcaatc ctcgaaactg gggaaatcag tccaggtagg ctccaccagg gtttgattcc 46980
ggcttgccat gccaacaccg tcttcattta cccacacagt gcaactggag ccatccatct 47040
tgacggtcac ctggtacggc ccatctgcga tgatcttctc cagagggatg ttctgaaccc 47100
gttcctggtc cgtcttcggg atgtatcgag ggaacgtgct gacacgggag acattactgt 47160
tccctgccgc cacctcttca ggtttctcgt acttcagcac accgagcatt tcatccagcg 47220
gcatcttctc gttcagatcg tccaacacct cttgcacggg cgctttctgg agccggcgaa 47280
ggatgccagc ttccaactgg gccaggcggc ctgggaaggc tcgtagagga agggccaagc 47340
cttgtgaggt gacacctcgc attttcatgg tccgcagtcg ggagtatacc acctcctctt 47400
cgcctccaca ctcgctgggg cagccttgcg acacggcata acacactcct gccatctcac 47460
aatccatacc cagggagcag gcttctttct tccgggtaac cttcaccagg ttcttggtaa 47520
ggtgggagaa cgccgggtct gccactggca gcaggctgtc aatctcgaag tagacagcca 47580
ggtcgccggg caacagcact cctttcggga cgatggtggt ccagcctccg aagaaggcct 47640
tctcaatccg atctgcccca gcgatgggtt cgatccccga cacttgacga attgaagcca 47700
gtttacgcat atccgtttgc ctccttgaag tcttgaataa ggtccccgat tgaacctgtt 47760
ttgacggtgt acatcacctc agccggcgtc ttctggttga ggtcggcgat atattgtggc 47820
caatggaaga ggctgtgcaa ctcggcctcg gtcaacgtga ggacgtttcc tgcatccgtc 47880
accacaagga ataggtaaag ctcggggcag aatttccacg gcacagcaca gaagatcacc 47940
ccattcccag tgactcgtcc atccttagtc gccaactggg cccctatctg gtgctttcct 48000
tccgaagggt gaaactcgtc agcccactca ggcaggatat cagggaactc aaaatcgtcg 48060
tccatcacgt ccatcttact tctcccagtt tttgttggcc aaggccaggg cttcttttgc 48120
gggagcgagg aagctcttaa tgtccttttc ttcccagccc aaaaggataa ggatggactc 48180
actcatccga cacagacgct ccagctccgc tttcatctcc cgggcagcac acagggtagc 48240
agcatgctct tccttaacat cagaggcaat cacgtactcg atcaggtatc cacccctcac 48300
tttagggagg gcttctcgga tgtaaactcc gtcaaactcg ctatccacca cttcccacgt 48360
tctgtgttta acaccttcca acgctttatg ttccatgcat cctccttcaa aagaaatggg 48420
gccagcctgg ctggcccctg ttggtcactt gtagacgcgg ttctggaagc cggggctggt 48480
cagagcctcg tagggtacga acaccgcaga ggaattcttg gtcatttcca tctggacttc 48540
cagcgccttg tactggagca gccgctccga caggccacgg ttcgtgattt cgttctggtc 48600
agccagagta cgtgccttgg tcagttcgat ttcacgatcc gcttgggcca gcttcaggga 48660
gttctcacgc ttgaccatct cgatagcttg ttcgttctcg gcggtcttga tagccagctc 48720
tcgctcgttc tgagcttcga tagccttcgt gatcacttcc gggtattgca ggtcagccag 48780
tgtgatgttg ctcacctcca aagggctacc ctccattgct gctaccagtt gggagtgtag 48840

ggcttggttg atcttgtcga agttggctgc cacatcctgg gtgcggtact ttcccaggac 48900
cgagcggctg acacgttgta ccacatcctt accgtagacc ccatacacct gctggagagt 48960
tactcggtca ttctccaccc ggatatcgtt gaacatggca ttgatggttc gctcggtgcc 49020
tgcgattcga gtacggaagc gtaccacgaa cgtcaggtcc agattgtcgg ccatcttcac 49080
tttcaggggc tcggcgacgg tctgggtgga gacatccagc atcacgatac gagtccacgg 49140
ggcacgccag tgacggccag gttcgaggac attggtgctg tagccactac cgctgaggtg 49200
cttgcctttc atggcaggcg gtaccacatc agagcagcct gcaaccaggg ccagggccag 49260
gacaatggcg acgccacgca tcaggttctt gaacatcttt cctccttact tcaggttgtt 49320
tttcgtttcg atggcttcaa gggtcttgtt gaaccgctct tctgcctttt cacggtatgc 49380
acgatactcc tccttctccc ctgtgtcaac agttgtggga tgggattgga acgtgcccac 49440
cgggcgaatc tcttccacct tgtagccgaa gcccaggcac gataccgaga tgcagacgat 49500
agcccacacg gggtaccact tacgccacca ggacgcttgc ggcttggtca tgtttcctcg 49560
ttcaacctcg ctccgggtca tcttgatgat accccagtag accaggcagg cgactgcgat 49620
aaggcctgcc aagtggtaga cgctaacgct caggtagatc ggcagtccca tcacttcagc 49680
caccccaact cgatagcctt ctccagcgcc atgataagga agttggcgtg catcttgtcc 49740
tccaccaaaa gctcttgatg aacgatatct cctggcattc cgaatgtagc ttgtccttca 49800
tcggggtaca ccaagcgcac agctccggga ggggcctctt tgcacgtgat caggcacaca 49860
ttgctcatat cgtcgctgat atccgcaatc cggatggtat cgaagggagg gttgggttgg 49920
cggatgtcga gttccattgg tacctcctta gcaggcgaaa gtcagagttt cgaaggcttg 49980
tttagcatcc tgggcggtga ggaagatcag gataataacc ccacttgtga ggcctacccg 50040
gatttctttc ttcgatgcga ggatttccag agcagtgacg tgttgggcgt tgatagctac 50100
cttcaggcta ttggccaagt tattctgttc tttggtgaaa attaccatct ttactctcct 50160
ctcttgcaat agatgttggt gatcggacct acccgggtaa cttcaaaaac cccttgttgt 50220
acgaaacaca tcctggacag gtgggggtct tttaaagacc ttcgatcttg ttcgtagagg 50280
gctatcgctg cttggctaac cagggcagcc agtatcaaca gcttagtcct cttggaggct 50340
ttctggtaag cctccttgag gtctggaatc ataacagcta cttcgagaac ttgcaagtgt 50400
aggaagtcag ccggtctcct ttcagagtca cttccaacac tccagctccc gcctcgcatt 50460
tggtgattgc ctctttcacg gcttgcaccc gctcagattc gagcttcact tggttctctt 50520
ccaagcgctg catcatgccc aggaaggaaa acatcatggt gagtgccagg ataactgcta 50580
aaaccacgac agaccatgct ttcatatgtt gcaatttcaa atccctcgct agttcatcca 50640
aagtcgtccc ttctttcacc cacctcacag cagggcctgg aggaggcggt ggaggggcct 50700
cccggaccgt tcgaacatac tccactgtcg gaggcgggtt gaggtgttcg gtcacttctt 50760
acctccctta cgcgacttaa cagcttcagc ttgaagggcc ttcagtttct ttttcttggc 50820
cttcttcttg tcggtcgatt cctcctgggc cgggacttca acctcccacc ggctgttcag 50880

cgtagcaggg tacggggatg gcagggtgag cattgttgcc agcgcacatg ccagtccgag 50940
ggctgctgta ctgttcttca ttgttcctcc tttatattcc tgtgtcaacg gttccagtga 51000
ttctttcgca gttgagggtt cttgggggct gtacggtgct tcttggcgcg caggtggcgc 51060
tgatttttgg aaagcttgtc ccactcatcg aagatcacca cgagcttatc acgggggtcc 51120
tctacctggg cgccgttctg atcgtacagc ttaccatcct ctagaagcat aaccaaccct 51180
ccagaagtca gcatggctgc caatacacgc tgctccaggg ccgagaaatc catggccaag 51240
agagtcttcc acaccctgtt ggaatcccgt ttgggcatgt tcacacggag agtggcggtt 51300
actcctaagg gagtcccttt gtggtagtcc gagtacacta tcccatccgg ctcggcgatc 51360
accgattcaa tgtcaaatgt tgcgtcgatg taaaccttat ccatggaacc tcctcaagct 51420
gttgatgtgg ccactgtata gggtgttgtg ggggatgtca actacttttc tccgggcgtg 51480
aaaaagcccc accagcttgt ggccagtggg gctcatcttt tccgcttgag gttgtcctgc 51540
tcttcttgaa gcagccgcat ctgcttagac gtggtatact gcatggcatt gatttgctct 51600
tgcagtctgt ttgttcggat atcgaagtct cgccgcatct cttccaggct gagccggtac 51660
tccttccgga tgatgtcgaa ctggtactcg gtgctggtca cccgttgagc gtagtagacc 51720
agaactccca aaccgaccac cagagtcagc agtgtagcga agagcatccc caggacggtg 51780
atttcgtggg ccttcgtcat gaggacacct ctcgtttacc gctcctgctt catcatctgg 51840
atgatcattt cggagtattt ctgattgacc tccatcttct tgtccaggtc ccctagccga 51900
acatccaggt agttcaggat acggctttcc gtggcaacca gtttttgctc ggtcacagca 51960
acagcttgga gggcatacag cctatcctcc accttaccga tctgcgtctg ttgccaggtg 52020
gcccatgcca tcagcagggg taggaggaac tttgctgcca ggtccagtac atacttccac 52080
tgggagtctt cgttgttact gcgtgcggct gccatactgg gcctccaggt ctttgtacca 52140
ctgctggatg aagtggagtt tgttgttggc gcaacctgct gcaccagtgt tcaggacgta 52200
ccccttggcc aaggccttta ccgtgtcctc ttccaccggg aagattacgc agggggttgt 52260
ccactcattt gggagtacag gcaggatgac ctgcgggggc tgcggcacag aaggcttcgt 52320
tgagcaactc catagtccca ggagagagac gctcagtagc cacatgccca ggaacgctcg 52380
taggcgcatc tttcacctct ggtagggtca gtgtaggggt ccgatccaaa cgttgcacca 52440
cggagcccac agaggcctcc agggtcttgt tttgaatcac cagttcctcg ttcatcgaga 52500
acacggacag acattgcttt cggaaggatc ggtacgatgc cgatgcttct gcgtagctgg 52560
cctccagaga agccaggctc tgctccagcc tgtcgatttt ctgatgactc tcccggagaa 52620
gccaggagag ccccagaacg aggactacca acgctatccg catggcagtc atcagtttca 52680
tttttcctct tcctccttgg gaatctcatc cgatacttga actaccatgc cccggaggga 52740
ggtttggacg atgtaccggc ctgctgcaat cagcatggag acgcctgcca cgaccttggg 52800
gtataggtcg ggaccgagaa cacctgcaag cccgggaatc agcggctcga agatgatcag 52860
catcgagagg atgaagagga agatgctcag caccactgta ctgctacggt gcagcttggc 52920
cttttcagcc actagcttca gcatatgctt tctccaattt cacgtcatac ttgttcttgg 52980

cgtagcctgg accgttgtac agtctggcaa acttggccca atcatgggct ttcaaggcag 53040
catgcgtggt gggttgtgcc ttgatgaacc ggcagaaggc gtccatctgg gctccttcgc 53100
tggcgtacat ggcgtttacg aaagcttgaa gggtagggta ccccatcagc ttccagtggt 53160
agcccatgat ctggaacatc ccccaggagc aggactccag ggcgctgtcc ctgtcgatct 53220
ttacggcacg ggccagttta gcgtgttgct cgctgtattt tccataccca ccagctacct 53280
tatttaccag gtctggggga tgaccttccg ttgggagccc tttggcctgg agctgtcggt 53340
acatctggtg acgctcgtag aggatcgttg gctctcctgt aggctggaag ccccctaccg 53400
gggcctccac cttggtcact gccttgattg ctgggacatc tacccccaac atctcagcag 53460
cttgctgata ctctctgtcg gtgatcatac tacatcccgt agatggtggt gatagcgaac 53520
cagcagaacg gatacgccca gttgccccag ttgcacgaca cccggaactg agtattactc 53580
cactggtccg tcatgtaggc gcctggcccg atagaggcgt tgaaagtgcc catcacagtt 53640
acgctcacgt tcaggattcg agccgggcgt ggcagccgta caatcgtaga ccgatcatcg 53700
tacaatgcag gcccttgccc ccactgaagg atcaacccgt tcggaagagc tgcccagccg 53760
gatgcactgg aggattgttc cgattggaag tctaggccag ttgctccgtt cggtagaccc 53820
agagcgtcca gagtagcaca tcctttcaag cccaggttgt tccgggctgc ttgtgcatcc 53880
ctgacatctc caaggttgtt cgccgaggtt aggaagtctc ccgagttggc agttgccata 53940
gtccccaaac ccaggttagc ccgtgcttga gggacgttgg tcaaccctgc caggttctca 54000
ctcttgcgca ttacgtcgcc tacaggcagt gtagcagtgt ctgtgatccc caggttagcc 54060
cgggctgtgg gcttgttggg gatatcagcc aggttgctag cagtcttcaa ccaggtgctc 54120
tcagggagga gcgctgaaga cgtgatcccc aggttagccc gtgctgcggc tttgttggga 54180
acatccgcca ggttgtcatt acgcttgagc caggtgttct ccggcagcac agcagagctg 54240
gccaggccca ggttagaccg ggcaagctgc ttatccggga ggtccgccag attctggctc 54300
ttatacaggg tgagtgcttt caggacactt ggtgcaatcg ccttgttctc aacggtgcca 54360
gcgtaccctt ccgcctggct tgccaaagcg atgatgcctg gtgctgctcc agctcggggg 54420
tacaaggctg gcaactgatc ccttgggact gcatggcttc ctgctgtggc aggggctaca 54480
gcaaacacct gcccggctgc cccagccttg aaggcgtatc ggacatctgt ctcggctttg 54540
gaatataccg agaggttttg cctggccatg gcaatgttgc tgatattggc cagtgtttca 54600
taatcctttt tcaaggattg aagctgggaa tttactttcg cagcctcacc gtacctctcg 54660
aatgcgggtt cccagtccag gttgcttgcc ggagatacgg ggtcaacacc tcggctggac 54720
cgtttggcct tgtacaccac cccatctgac cctttggcca ggctggtacc cccttcgtaa 54780
gtggtggcgg catcccacat ggcgatgcct tgttggttga tgtgggccag ggccttatct 54840
accctctgga aataccagtt gtgccattgg tagggaggga tttcgttggt ccagcctgtc 54900
atgatcttgt ttggagcagg cttagtcgcc tgcccaatag atgcccagat tacggacagg 54960
tcgtcaggtt ttggaagcgg cataatcttt cctcaattct tggggcggcg cgggcggctc 55020

tttgcggaag ggaatcccgc cttttcaggc cactttctca gagctgagcg atactttcgc 55080
caggccttct ctttgaccgc atcttcatcg tctatggcca tttctacttg acgatctgca 55140
tctcgtagtt cattatctct ccagttacgg gcatcaatga ccaaatcccg gtcagacaca 55200
tcttccggga ctggaatgtc acccgcctca atggcagaca caatggcgtc ataaatcttt 55260
ttagcattct cgtcgtagtc gcaggcgaag tacggtgacc acccccattc cgggtgttta 55320
atctcacact cataccccat cttatacttt acaacgtttc tgaatttcat cctcaccttc 55380
caggtagaag taaggggccg aagccccttg tttggttagg aaatgcgttg ccacagggtg 55440
atctggttac caccccctgc aaacgctccc atgcaacggt actgcccggc tgccggaagt 55500
gcccctgcgg tgtaactgat ggtagagtac ggtttcagtt ggctccctgc cctaatggtg 55560
ccagggcctc ccgacccgcc tgactggtct cctgccagga tgtactgccc tacggcgtcc 55620
caggctgctc cagctcgggc aatgtcgtta ggaatctcac ccttcttggc atataccgaa 55680
tccacccagt tggtcaggga attgtacgcc ccgtaggcag gacccgtgat gttacctgcc 55740
gtattgaggg tagcacttcc cacgttcacg ttgcctgaaa cgttagcgtt tccggaggcg 55800
ttgagcgtgg ctgcgttgac ccttccaact acagtcaagt cttgacctgt aagagtcatg 55860
cctgtcgagc cacccagcgt ccggaagttc atggacccgt tggacggagc accccaaatc 55920
aggcccaact cactcccatc tgccttctgg aaaccgatgt gggcattccc tgtaccagag 55980
gagcggctga aaatgttcga tccatacccg ataatgcttc cgtaggcgtt gatgcctacc 56040
gcactaaagc tcccgcttga atccctttga accagggagt tagactgggc agtagttacg 56100
gcaggaatct gagcagagtt tgccaggccg gacactcttg caactggtac cgtcccgtgg 56160
gtcagtgtcc cagtttcatc gaatcggaac atccaagtct gcgttccccc attaaaacct 56220
ccaacagagg aaggttggac ggtcaaatac ggaccgttat tcaacgtgct agcgaatacc 56280
acggtatcgg acgacccttg ggacactcgg tcaagtccta gacgactacg ggcatcgctt 56340
agggttggtc caagggagaa ctggttccct gtgtggagaa cgacttctcc cccaatctgt 56400
acgatggggt tcgaagtgtt tcggaaagca aactgaagga tgttatcttc tattacagcc 56460
agactgatcc aatccgtgtt agacttgttt cggagtgcca actgggttgt tttatcagtc 56520
cctgacatgt tcgccccgtt gatgtggagg cgggccacta tacctgcgta atcggctgtc 56580
agaggtggga gcgtgatatc ctgggttccg tcaaaaagga caccgttgat gtttctcccg 56640
gtttgcagtc gagtcgcagt tgaagcgtta cctgtcaggc taccaatcac cgtcccgatg 56700
gcggcagttc ctgtagaatc cctggcaaca actgtgctcg cctgattaat agccgtggcc 56760
gtcatgacag cagcgctgcc aagttgaagc tgcgacctac ccgttgcggc atctgctacg 56820
ccgatccagt ttcgaccaaa cggggtcgtt gccaggttgg ccatggaggt agccgtgttg 56880
aaatacggga tagctccaac cacaggtgtc aggttggaca gccaagtgag ctgggaagca 56940
ctcgtttgag ctttcagctc tgcccgccag gctgcatcgg agctaagacc catgagccct 57000
acagtgtagg aggtgatgcc acccagggtt gcttgtcccg aagcgttgta gaacgggaat 57060
ctccacgcag tgggggtaag cgaggtcagg gactccagag ctggagacca ctcttggaaa 57120

gcccggttcc agttggtgtt tcccgcagtc gtgccaggct cactgttggt atttgtccga 57180
gaggccttgt acactacacc gttgcgttgt acataggagg tgtttgcctg atactcggtc 57240
tcactgtccc actcaggcag gcctttctgg agcaggtagg ccacgttggt gtccaaccgg 57300
ttttgcaggt agttcaacca ttgccggggg actacctcca cctcccagcc cttgttcatc 57360
ttttccggac ctggggcggt gatatcacca gagacgcccc aaatcaggtt catactgatc 57420
ttgttgatat tggccatttt gctctcttac gttagaaggt tcgacagaat accacctacc 57480
gaggggtcgt tcaggtcgcc tgtgccaaac cctccctgga agccttcagt ggcaaacggg 57540
cggtcagcag agaattccga gtaggtgtag ttcacaccta aggtcttcgg caacaggcta 57600
gccacgttgc ccagctcgaa cagcaaaccc ctctccacgg tagaaagaat cttcccgatg 57660
ccgatacggc agttggcgtt tcccagttcg tcgatgatga tctggctagc cccaaacagg 57720
aacttgtagg cttcgatcac atcctccggg gtagatagcg tccggttctt cagaatcttc 57780
gccttgatca tcaggcggta ctcttcgtcc gttgcctccc gggaaatctc agtagcttgc 57840
cccaaacccc accaaggggc cccgactgaa gggtcgttca aagaaccgta aggtccagcg 57900
ttgtgcgggg taggtagcgg ggttggattg tcaggacgat ctttgtagcc gaagaatttg 57960
aatatctcgg cagtgacaag gctccgggga agacctacaa tctcccccag aacgtccaat 58020
tgagccccta ctgctgtgtc cagggtccgc tcttgctgga cctggcgcat aacctcttgc 58080
agttcaattt gagcctgaag aagcagctct acgaacttat cgaagatcgg cttcttcttg 58140
aattgctctg tgtaccggct ttgagcgact ttcagaaagt caatcatccg gaactggttc 58200
ttttccagca tatcaggtca ccgtgattgc aatacgggtg gcatcgaaaa tagccacctc 58260
gtcataccct acagggatgt tcgccatggc ctgggtgccc cctttctttc caatggtcaa 58320
gctgttaacc tggaaccctg gaacgctgtt aatcggagtg tatagtctag agtataccac 58380
ctctttacca attttgtcca gcccatcttg gtacgccagg atggcagcag tgacctgctc 58440
ctttacatcc ccgggaagaa cctgacctgc aatggcagag atgttcaggt tgacctggac 58500
ctcaatctct ttggctctgc ggaatccaat ggagtgaacc ttgccctggg agtccgcaat 58560
ctgcaccacc gtatccccga aggcttggat acctgtaggc ttcttctgcc aaatcatctt 58620
gccgatgtca gagtcgtcac ccccatgagt gatcaccgtg aaggcgtggg gaggaacgtt 58680
atgcaccgag tcatggttgt tggtgtcatt ctcaatgatg gtcacatccc gcaccccagg 58740
aacctggtgc atgccgtcca gcatcgcttc caagatggca gcggcctggt agaacttaga 58800
attcctccac cgttcccgga gttcgacatc cgtctccctg aacctgccgg atacggcgtt 58860
ggttgggttg tacaccgaat cccagcccag gatcggcacc gagatggtgt caatcgtccc 58920
tggaagttga gtcgatggcc ccatttgggt atcctctact acccctggtt tggccacctt 58980
gattggggtg atgcgggggt ccaggtcgaa gtggacaatc tggagacgat tcctgcgacg 59040
agcccagttg tagttgttct tggcatcagt ctgtgtttcg aagctggtcc acgactgcga 59100
taccttttga cccagcatgg tctgaataga cgttgcagtg tccgtgggtg tcgcggtgta 59160

cgtggcatat acaaagttgc ttccaccatc cgtcgagtaa ccgatcttat actcgtgtcc 59220
agcttcaaca gccgtgaccc tccaggctac ccctgaagcc tgttgaaggt tcatcaatac 59280
cggggtaatc acccggaact gcctcaaggt gacgctggag gatacagagc ccaccgagct 59340
gaccacgatg ttgtagttgc cctcaaacac tacttgagcc ctggtgggct cttcagcgta 59400
tcggatgata cccgatagag caacttcgtt atccagagcg tagccggttg ccacgttggg 59460
gttgaaactg tcatgcacct cttgcagggc ttcccacagc tcagccacgc ttggggctac 59520
tacgccaatc atgcggccca gggcagagtt ctcactggta tctactacat caccaggttc 59580
aacttcgtca gagaagatgt cctgggctcg gttgtggtac tgctccagta cctcaggaag 59640
gtaccggatt tccagccctt tatcagtcaa tccagccatt tatacctcca gcggcagacg 59700
cacggctact tcttcgttgg agtaagtccg cactttgaat tctaaggtga agcgccgagc 59760
gggcgcgttg tattcgcttc ggtattccag gatttccttc accccctcat cctcagccaa 59820
gaggcgttga aagatgatgt ccaccgtcgc cttgtgggcc accttggtga ggattctttg 59880
acggtagggc acccctacgt ccaggttcaa gaaccactca cccaggaacg tccagagctt 59940
gattttgagc ctttgagcta caatctggac ttgcccctga gttacagggg tctgcccatt 60000
cacgaacacg atgtcgtgac tattttcatc cagtagaagg tccataatcc ctcacgcttg 60060
tgggacggca gtagtaccac cgccaggcat aacccctcca tgcacgtggg tgtggaacgg 60120
aatgccgttg aatgtcgata cacccgtctg ggtgtagttt ccagagtggg tgatgttccc 60180
tgtccaggtg gtattctgtg cctggacttt gagggccggt gtgttcaggg taatcccttc 60240
ggacccttcc aaagtgatgt tccgggcctt gacgctcacg tctaggtccg tcaggagttc 60300
gatgttccca ttctccttca gccttacctc gcactccgtt gcagtggcca ggttgtgtgc 60360
taccaccagg tcgtaggtgc tgtgggggaa cttcctgtgg gccgggttat tggggctgac 60420
tgcgaagggg aagaccccag gaatagccac agcatcacgt ccactgaaag ttcgagaccc 60480
cgtgggcttg gtggggttcc cggtaccggc tttaaagtta tcaatgcctc gctcgcagaa 60540
gaccagtaga acctcatctc cggggaacaa ggggaagctg accaaagtcc ttcgagttcc 60600
cggcatttgc acaggcacgg aaagaatggt aggctgctcc tctgaagtcc cgtcatcgta 60660
cagccggtta actaggggct gagcatcaat catcaacgtc tccaggtctt tgtagactcg 60720
gatgaccctc cccgggatgg ctgttctcac attgttcagt tgttgctcga tcagcgtgtt 60780
caccacgctg ctgaaactta gttcaatcat ctgaggtctt cctcgataat gatgccacag 60840
aagcaatcaa cgtaccattc ctggtcatcg tagcccccgt agtacctggc tgagttgatc 60900
cgataccagc ctgtcacttc ttttgactcg atacggacca cttgcccagg agcaaagtca 60960
gcgttcaaca gggctttgaa ttgaacccct tgcgtccgcc tcttgtctcc cttctgccga 61020
ttaccttcag aggatgttcg gtaggggata ccgatcaggc cagtagattc gttcagtacc 61080
ggggccgact tgatgttttt gtcgtgaatc ccgttgacat cgttgacgta gagggcgcct 61140
gcatcacacc gccactccat attgttgtcc ctgcaaatgt cgttcaagat ttcctttgca 61200
ctcccgtcca tgggataccc gaacaccacg gggttgttga tattggtccc tgaaaaaaca 61260

ccccgtacca cgccgggtag ttgccccata gcagcttcaa tgacatcctt ggcagtctgc 61320
cccggaggaa tcgtctgctt gagccggatg tggtttaggt cggtgtaccc ctcacccagg 61380
atgagctgag tcaccatgtc cgggccctgc tgggcagtcc ggcgttcaac cacgttgccc 61440
tccagtacca cccgggttcc ggctgccgta taccctactt ccagtttgac cgacaaatag 61500
tccgtatcaa gcattgctcg ggtgcttgct gagaggttgt acagttcgac cgtggcactg 61560
ttggacttct tcttgttgtc tgccgacttg ctgatgtcga accgcatgtg tacatccttg 61620
atcaggactc ccttgcctgt ccggtaatct ccaacggtga gcaagtacac ccgttcgaac 61680
tgttccattt acacctcctc gctatcgtcg tattcctcaa tctccacgta gtacagttcg 61740
aagaactcgt tgagagtaga gatatcttca cagatgtcgt tcttgtggtt gtcggggaga 61800
actaggaaat acccgttcag cccgtacctg gcccatccgt agtcatagcc tagagggtag 61860
ttcggaacca acttcatgtt ccgaatgata acctctccgt ccatgttttc tacggtcatg 61920
tgccaatatc gttgcctatc cgaccaagaa aagctgaggt accggatttg gtcccccagc 61980
ttcatttggt aggcgtattg tgggtctcgg taagtgggta gctccaccag cctggcccgc 62040
tctcggatag ccactatcgc acctcccggt tagtcatcct gtgcaatacg gtttcccctt 62100
tcgggttctt cccgtctgca tccgttacct tctcatcttt cggggcttgc ttgcccttgg 62160
cctgcttctc gctagccttg ttcttgatcg attcgctcac cttcttagga atggctgccg 62220
tctggaaggt ggttgtcctg atcacctcaa atgccaggtc cacatacaac gactcaccgc 62280
tctgcgggtc ttccttgaag ctgatcgagg tgatcaccac gttctggaag atggtcctca 62340
cagcagagcc ttcgaagtcc agcaggacaa tcctctcacg gtccctggtg atcctgtcca 62400
gttcttctcg gatcaggttt gcccccttct ccttgggagc cgctggaacc accacggtgg 62460
gcctgtccag gtccaagaac tggttgaccg tctccgggag gaagttcgtc caggccgtgt 62520
ctgcccccgg ggtgatgatg gggcccgatg tcactggcgt actattgaca aaaggcttag 62580
gggtgatccc gaaagcctgt gcgtcagcag ccgtaataac tggcctgtcc aggttgaagt 62640
cagcgtcaga gacgaccccc gaaagggtca tcttctgatt ctgagcaatc acatggtcgg 62700
taaccgaagc tcccgattct accacgtgct gagtcacctg gttagcccgg gagatgttca 62760
ggttcagaat cgcatcgaac cagatgatat ccccggactc tcgttgaata gctaccgtca 62820
tttcttgttc tccgatgcat ccaccatggc ctggcgaaga gtgtcatgga agctgtccat 62880
gattcgggtt tgaagcatct cgctgaactc cttttcatcc tggatttgag gagcgctctc 62940
aatctggatg gggatgttca gggtaatctg ccctacactg ggcataccac ccaccccgtt 63000
ctccgctttg aggtactcag ggactcggcg aacaacgtct tcatctatgt tccgccccat 63060
gtcattctga gcacgtcggg ctcgggcctc aaggtcctgc cggaggtcat cacggaggag 63120
catggttacc gggctgttcg acagcgctac ggccccggcg tctccagcaa gcttcgcagc 63180
cttgccaaaa tccttattct tcagggcttc agcagcctga accagcttgg tgatgactgt 63240
cagcacggcc agaatcttcg acgtgaagaa gtccagcgca gtggagaaca taccaccctc 63300

agcgaaccct cccagcagct ctcccatggc ttcagccagg cgaccaaccg cctctgctgt 63360
ctggactact gccccaccga tcttcagcca cacagcttgc tgctctggcg tcagttggtt 63420
gaagaagtcg ccaattacgc tatccttccc ttgcaggaag ccgacgaaat cctcgaccac 63480
caggaacagg ccgccgatag ccaaggcaat ctgccccaac gggaatagcg caaggccgat 63540
agctccagcc aaagccatca actgtccaga tgtcagtccc agcttgctgg ccaggtcggg 63600
tagttgttgc agcatctgac gaatcaagat agcggctgcc tcaaacgggg aaaggagcca 63660
ctccatcgca gcacccaggc cttttacagc ctcctcgtta tcattcagcg tccgagccat 63720
ctccttgaac aggttggcca tgccctggcc catgccgttg ttgtagatca gccccacagc 63780
gtccgaccag gcgttgttaa agcgctgctg ttgggcagca gtggagttct gcgccctgga 63840
caacgcaccg ccttctcggg ccttctcggc aatgatacga gcgaactcgg gaagcacgtc 63900
cgttcggacc aaccccttcg ccatcatatc accgagttct ttggtgctaa tctctctgcc 63960
ttctttcgca gaaacagcct gggccatgag gctcatagct gccgggaacc gttcacccaa 64020
ctggagcttc agttcctcag acatcacctg ttctttgttc atcatctgtt cgacggcgcg 64080
caaagaacct ttcatggact cggtatccaa gcccattacg gtaccatact ctgtcatgga 64140
acggaagatg ctgtcaacaa catctttgtc cattccggcg ccctgtccag aggccagcat 64200
cttggtgtag gaaggtgcaa cagctcgcca gtcgaagccg acctcgttgg ccatctgctt 64260
gaactgtgcc atagtggctt gaccagcttc ctggctacca cctaccgcag ccatggcgtt 64320
ctgctgggcg atcatttgct gacccatctg gttcatctgg gagatggcaa acgcgcctcc 64380
caggccaggg ataaagcctc gtccccagtg ggctacagca gcccctgcac cagcaccagc 64440
catacctccc acagcatggg cattcctacc ccttacctgg ttgacatggc cttgcaaatc 64500
gattctaatt ctagcctctc ggcgaatctg ggccaggaca tccgcaatct cctgccggag 64560
gtgagccctg tcaaccgaca gcctgatatt tggcgtccgc agagtgatag tgcctagcct 64620
ctgaatcact gcgtcgatgg tccgacgagc atttcggtag aacttggact gactgatgtc 64680
ggcatttagg tgaagcgtga agcgttgctc agtctgccgc ttccatgcca acagggcttg 64740
ctctgccttc ctcgctcgaa tagagacggg aatttccaga gccaagcctt gcataaacgt 64800
gcgaaggaat cgcttctgtt gggcgatatc cttgatgttg aaacgaacat tgctcagcgt 64860
gactggagtg tttgccagtc gctcactgag cttcttgacc atcgttccca gggtgtcctg 64920
ggcgatgtcc acattcttga gacgaatctg ggcattggta gccattttga tcttggctgc 64980
caggcttcga ccatcaaggt caaccctcat cgtcactttc ttgttggcca ggccagccac 65040
ttcgttgagg cggtctctga cctgttccag gttcttcttg aacgtcagca gggggcggtt 65100
atccgccctc cactggatcg tcccgacgag ctttgtaatc tcttcagttg ccatcttgtt 65160
gcctcagctt ggcgatttct cgggcgatgt ctgcggcttc gtcgtgagct gcctggtact 65220
ctaggaggtc atataaatcc tcggtatcgt acagcgtttg cagctcgtga aggctcgcca 65280
ggcgcagaga gtgcgtcaac acctgataga cctcgtagtc cttttggaac tccttcatga 65340
gccgctttag gcgggggtgt accatctggt cacccccctc cccacttact gttgaccgtc 65400

gaaaccgagt gcttgaaaaa ccgagccgaa gttcagcttc actacctcag tcaccaactg 65460
gaacagcttg ccaaagtcag ccgagaactc ctcgttgaag ttgatttcca tgttgttctt 65520
ggacacactg gtcatcatgc cgatgatcat cttttccacc tcaacctcgt cgatccgctc 65580
aaccaccttg ctgaccaggg tggcgatgtc catcgaacct gcaccctctt tcgagcctac 65640
gcctgcctct gcaagggccg ggccgagaag cttggtgagc atcttcaggt gagacaggcc 65700
agtggtagcc ggcatggagg tgatcaggaa acggtcaccg ttgatttcga tggaatgttg 65760
cttgcgtgcc atttgggctc tccttttaga attggtctaa gaccgagttg attgcattgc 65820
ccgcagccgt cttcagcccg tcgaagctga agaagtccag ggggttgttc gaaccagaca 65880
gatcacggtc cgttactcgg gcaatctcaa tcgtccacac agggttgtca aaccccaggc 65940
tcctacgggt ctgggggtga cctgaaatcc aggcgttgtt ggtgtagatg atggtatcgc 66000
cgttgttgtc agccagggcc aggtctagcc tgccggactg gttaacgcgg tcctgtgtca 66060
ggatgtcgaa caagacatcg tttgtgtccg aggtctgaag aagctctact cgcatacgtg 66120
cagattggtt gaggtttcga actcgggtca cctgaccccg gattcccacc actttacggt 66180
agacctccat gtcccaggtg acctcaaccg agactacacc tacgcattca aactccgaga 66240
ttatcagtct aacatcctcg ggggcgtaag tcaacacacg ctttgtcatc agataagtcc 66300
gtactgtcga aggattggaa ggatggctgc gccctgactc aggtagttct cgaagtcagt 66360
aaggtcgctg ttcccgccga tccggagggt agcgtcatag caagcgaatc gccacacgtt 66420
ttctcgaaga gtacccccaa accctacagt cggaggggtc tcaatccagg cagtgcctgc 66480
gaagaagttt gtgtttccca gggtgtcttt aaccaccagc gggaacttcc ccttgcgggt 66540
agctgcgtcc aggttgtaaa cagcgcccag gaagtcgttt gtaggcgagg actgcgccaa 66600
ggtgagggtc attcggtagc cgtcatcctt agtgtagagg cgggccactg atccatccat 66660
agctcgctga gtctgggtcg cttgaacctc tttctggatc gtgacgaact ccccctccgc 66720
gtatccggtc acttcatggg ctccggcgat caggatcgta acctctcttg gagagtaagt 66780
caaggtgttc atatttcctc caggtacaaa aaaggccgcc cacgaggggc ggccaggttg 66840
tcgcttacag ccaacgggcg tcgaggttca caccaagagc cgtgagggtg tctgcggtag 66900
cggggctcac cttctggttg ccgccatgtt ggaacgtggt gttggtcatg gaaatgaccc 66960
aatcacgggt gttcatctgg ttgctgaagc caccctgcgg gatcactgca atgtacgcct 67020
cgtccgagaa gtacagggag cggcccgagt tgtccttgat cagcatctcg aacatgccgt 67080
ctgcggacat cgcttcacgg tcaaactcgt gaaggaaggt aaggacatcg ttcgactccg 67140
aggtctgggc caggcttacg gtagcccgtg cgccggagtt ggcgttgaac acccgggtgt 67200
gagtgtcatc agcaccaaca tactcggtga acgtgtccac caggcgttca acggagacaa 67260
tgctgtcttc gctgaagccg gtcagggtgt ggctgatacc cgaagccgcg tggttgatta 67320
cgatagtaac ctggttaggg gcgtaagtgg aagtacggcc tgccattgtc tactccttaa 67380
gccgtgacta cgccacggat accgatgatg tggatggcac cagcgagctg agcttcgaac 67440

tcgataccgt catagaaccg gcgagcccga tcattcggag acatactgtt tgcatcccgt 67500
acacggaccc ggggagccgg ggtgtcggtg aacagttgga ctcgaatgcc ctcttggagc 67560
tgtgctcgaa catccgtttc gaggatggtg gtaccctgct tggtgtacgg gattttcttg 67620
ctgttccgct gacggaacca caggcgctcc cggaggcgag cttcaaacca gtcgatgcct 67680
accatggtgt cgatccactc gccagacacc atgctaccga acgtggtgct gttggcatcc 67740
ccaacgaact cgtaggtgtt acccttgtgg tcgtggatgt ttgccgactc ttggtcgtcc 67800
agctcatcgt aggtagctcc agcgatgccc ttgaagctcc aggtgttgct gcccggctgc 67860
tcttgcagtt ggctcgacat ccagcccagc tccgggaagt cctggtcagc tcgaccattc 67920
caaagcagga aggtacgatg gtagcctgcc ttgctcaggg tttccaggat gttgccagca 67980
gtggcgttct tcacagccac ggagctggta gaggtgccga agattttctt cgcactcatc 68040
gactcgatgg tagcagccac ttccaggaca tctgccggga cgtgagattc gatggtagca 68100
ccgtaccact tgctgttcgc agcgcttact gccaggatag cggcacccca gccctcggtg 68160
gacggcatgt tcgacttcga caggtttgcc gaggtagtca gcgaccagga ggtgctggag 68220
ctgcgaacgg tgatggtacc gtcggtattg tcggtcagag tgatgccaac gaccgggctg 68280
acgttgaaag ctgctaccag gcctgcgcag atggttactg ccgtgtctgc cggctcgtca 68340
tcttcatcca caggttccgg tttcgacagg tagctcaccg gaataccgtt cagggtcatg 68400
gtgtaggtgg tctgcggtgc aacctctaca atggagacag tggcgcctgg cacgttgcgg 68460
cgaccaacaa ccaggcggtt ggggcgcggg ttctgtgcga aggccatacg agctgcctgc 68520
attacggagc tattgttcgg gaagtctgcc atgacagctt ccgtcgatgc atattcccgc 68580
gcccgctcgg agaagttgga gtgggtagcc aggaacatcg gcacgttgaa cgcagtggtg 68640
gctaccggct gcgtttgccg atcaatggtg atctggatga tgtcggtaag acgggtcatc 68700
ttcagggttc ctcgttgagt gagatatggt tcttgataac gtggtcaggc tctcgtccgg 68760
cgttggtgta aaccgcgtcc agctccaaag cttcgatcca ggtcacctct tctttccgtg 68820
ttatggcgta ggacagatga acatcgtacc ggaaatgctg gtacagctca ttgggacgtt 68880
gatccctgat ccgacgaatt ggattggccc cattctttga aaacccgttg gattccaaag 68940
cctggtaacc ctcctcagtg ctgaggaaga attccaaatg ctggactgct gccgctgcca 69000
tggcgtggtt gtgtggactt cccaggcctg taaaacgggc tttaagggtg taccacttgg 69060
tgacctcttg tacagccctc tccgggtctg ttgggtcaat ctccagagtg cctgctacct 69120
gctcggtgaa taccgatttg atgtccatcg gctcgatgta cagtgcaggc gtctgggtct 69180
gttggttatc acagtagctt agcaccagag ggaaatcccc caaagcagcc tcagccacgt 69240
tgtagagggc atcttccaac tgggtgtaaa tgtcctctat gccggggtat ttcgccctca 69300
gctcgtccat ggtcagggcc atcaggtacc ctccacacga acacacatgg ctttgacgtg 69360
atccaggacc ctcatgtcgt aggaagcggt cttcacgact tcgtacagct cgcccttcca 69420
ccagaactgg tccgcatccc agccatcctc agcctcgttg gcttctcgca gggtggtgtg 69480
gttcctggta taaaccttca aagccttctt ggtccgttca ccctcgggga gttgcatcgt 69540

gtccgtgctt ttcagcacag gctggaagct tccctcgaca atgtaaatct ccggagggtc 69600
ttgaacaggc cgtcctcgga ccaggcgctt cctgggccgg cgaatcacct cgttcttgac 69660
gaagtgagtg cttagcatgg gtggcgtgag catcacttcc tcctcacgat cttgtacttg 69720
acgctatcct gcatcttgcc cgtatgagtc agggggtcgt caaagccttt cacctgagcc 69780
cagcgagcgc tgttatggcc tgggaagtcg tcaatgttga cctggataat ctccaccatg 69840
gtccggccca tcttgttgag ctgacccttc tgggttcgcc catctcgaat ggtagcctct 69900
accacctgct tcatggcttt ccggtagtag ttcaccgtgg cccagtcact gaagctggga 69960
gtcatgaagt ctcgttccgg gttggtttcc gtaccgaaaa cgttgtaggc ggccacctgg 70020
gcgactggga ggttaccatt ctctggaccg taccggtctt cagggaagaa gcctccttcc 70080
agcgtcagac cgtccaaggc ctccatccgg gcaatcagag cgtctagctt ccgaaagtca 70140
gcagagattc ggctgggcat ctagggcctt cccgattctc ggcatgtcat tgtaccccac 70200
gttctgctgc tcgtaaagcc gcaaatcgcg attgaatccg atgaaaatct tccgtgccac 70260
attgtctgga ttggcgttgt tcatggccat atctttggct gacacaccac caacgtaagg 70320
cagagcaatc acggttgtca ggttcgggtc cttcaggata tcctggagag tttgcctata 70380
ggccttccac cagtcgtttc cgtagacctc aatatcacca gtccgttccc tgacgtaacg 70440
ggccagcttg aagaggattg accgtacaac gtccaggaaa gccctgcgga tgttgccatt 70500
gtacatgtcg ataaaatatt ggtaatcttc gtccgtcaag atttcaatgt cgtcccagat 70560
atcccccacc atcaggcgga gctgatcgac cacattgttc ttcggatcgc ctgtgtaggg 70620
catatcctac tccttttctc gggaccaaaa aaggggcccc gaaggccccc gttttcttag 70680
gccgctttgc ctttcacgac cagttgcggg tacatcagga cgttgatgaa gttggtctcg 70740
gagttaatct ccaggaactc acctttcggg tccacgtact cgaacgcgta gctttcttcg 70800
ccgggggtgt tgacgtgttc gaacttgctc gccggagcga agaaggtctt gaacaggtct 70860
tcggtgccgc tgccggggat gaagacagct tcaccagccg gaacgtaacg gcgcttctcg 70920
ccacccggac ggccttggaa gccaccacgg tactcgatgt acagcacgtt gccgaagtag 70980
aactcacggt aacgggcgtc gaagccacgg gcacgcaggc gttcacggag aatctgcggc 71040
tggctggcgt agtatttgta cgcctcaacc acggtcgggt ggctgatcag cttgctgaag 71100
aactccggag agcagatagc gataacatcg ccaactacgc cgccagtgta ggcctcgtcc 71160
tgcatgtgag cgtaaaccag ctcgctcttc tccaggatgt tggtggtcgg ggtgtccagc 71220
tcgaagttga cttcgaaacg ggtcttaccg aactcttggt accagtcgta accggtagca 71280
ccaccaacgg tgttgttggg ggtgtagggc ttgccggtaa ccagggtgtg catacgagca 71340
aactccatgg tagcggcgtg agtgccacgc agacgctcca gcttgcgcat acgaacctgg 71400
tccagggttt ccagctcttg cttgccgtag gcacgcttgc cctgaatgtc gcgcggggtg 71460
atgtaatcgt cgtaggtgaa gtgcggaacc gcgaaggtgc gcatttgacg atcatagtca 71520
cgaccaacct ggtggcgagc accacgatgg atatcttcca tcaggccgaa cgacttggtg 71580

atttcttcca gagtcacgga ctcttgggtg gtacggtcaa caccgaagac gccgagctgt 71640
tgggtcagac cccacacgtt cgggatcagg atcatcggtt cggtcaggtc ggtgtattcg 71700
aagttgttct gaagcgaggg aatcggtcgc atggttcctt ccttatacct ggtgtacagc 71760
gaagatgttc ttcttcgcca gttctgcgaa agcggtttct ttgtctgcgg cttctacatc 71820
gtccgcaaag atcacacctt cgtcggcgat cttggcgtag ccacgtgcca gaagcaggac 71880
ctgagtaaac tggtcagcag cggcgcggtg ctcgcaatcg atcaggatgt aggcagcgtt 71940
ggcagcggtc gccttggtga ccagcttgcc gtcagcgtcc agaaccgagc cggtctcttt 72000
cacagccggg tccagccagg ttttcggcat gacttcgcgg gttacgccgg aggccgggtc 72060
gtattcgtac ttgagccagt tgctcaggcg ctgatggtcg gtagcgataa cgggcatgat 72120
gggctcctat accttattgc ttggtgtact tggccagcat cagagccttg agggtctcta 72180
cctggctagc gttttcagcg tcagatgcac cgctcagacc gacttcacgg tccagaccag 72240
ccggaacttc ttttgctttg gtgatgtgct tgagaacgac agcgaagctt tcatcgctca 72300
ggctttccat ggctttcatc agctcaacct gagcttcagc gtcagcaccg gaaccggcta 72360
cagcggcctt acgagcttct accttagcgg ccttctggat ggcttccatg gcggcgattt 72420
gttcggtagc agccttcagc aggtcggctt gcttcttgag ttcatcttgc agctttgctt 72480
cggctgcctg tacagctttg tcgatgagtt cttgactcac agggctttcc tctttgttgg 72540
tgtggtcggc cccagaagcg gtggccatat ggggttccag caggtcacga gctttcgcga 72600
tgctgacgat tgccgcgtcg tcaagcttat tcaagccggc aacggggtcc tcgctcttgt 72660
acaggacttt cagaatctcg atgccaccta cgcggcgttc gatgtactct tggtacgact 72720
ctccgtccat ctgccaatct ttcccttcag tggtgccgta ccccagggca gcagacaaca 72780
tctctgcatc gctgtagtac atgttgaaga acttccggag gaactccggg aactccagct 72840
ccactttgac cttggtggct ttcagcacgt cttgcgggat tcccgcgtca agtgccttgg 72900
ttaccagggt cgtataccca ttcgcagggc caccttgtcc cttatggacc agcgccagat 72960
gagcgccttc atggtcaaaa gagatgtcgg tcagccggcg tttggctttt cgatcactca 73020
tcggtgaggt cctctacagt tgctcgggcg ccgatactga cgccgttgaa cgatccatcc 73080
aacacccctg cccacaggtc atctgccaga gcatcacctt ccgggaagtg catccaagcg 73140
caccacgtcc ctttggtaat ggtccgtcca tcgtcggtgt cgaagtcggc caggttgatg 73200
aagctttgct cgatctgggc agcctcggtc tcgacggcgt ggaacaggtt ggctttcatg 73260
cagtgccggt tgaactgaat gcatgccttc tccacctcga ctgcggagta gatgtccccg 73320
tgaaggtcca cctcatccgg agccaggacg acaaagagtg cccggcgctc ggccttgtca 73380
acactcttca ccacgtcctc cttgggagtc ggctggtttt cttcagccat ttcagttatc 73440
ctggtttgcg gttgaatcat cgcgggcgct ggggctctta gaagtccctt cacccgccgt 73500
cttcatcccc tctcccgcct tgctgtcgtt tgccaagggc agagcctctc ggtcgatagg 73560
ctgagtggca ggaagctccg gtaggccaat ggccttgcgg tttcggttgg ccacatcacg 73620
agtaaactct aggccaccaa cggacatcat tcgctggatc gccttggagt aaacatccaa 73680

gtcaatctcg tcgaggtcgc cgaactcgaa ggtggggaac tcttcatcgg gtcgatatcc 73740
gtttaaccgg aacagttgag gtaccaggtc gttgttcagc acgtcccgga tcatctgcaa 73800
acgtgcttca atggccatag ccatcaggtt ggtcttagca ccagccaggc tgtaggagcc 73860
aacgttgtcc tggcccatct tcagaacatc tgcgaacagg acctggagaa tcttgttatc 73920
ccaacgcttg atgattgcgt cggtatcgta catcttaccg ccctggctgc tagtcagctc 73980
gaacttgaac agcgggaggc gagcttccgg gtcaaactgc tgcggaagaa tgagcgacgt 74040
ttgctcattc atggtgatgt tccgcatggc gttcttgtaa tactcgtaga tcgccttctc 74100
ttcgtcggaa gcgtccgggg acatgtaccg aggcgggatg tacagggtag gcataccatt 74160
catatctctg gtaatgccta cggcttcctg ctcttccagg gcagtccggt aactccaggc 74220
gaagtatacg tttttcagag ggctattgcc ctcggggttg tcacggcgag ggtcgatacg 74280
gaagttcagg aacttctctt tcgggatgaa aatctcatca ccaccgttgg gcaggttctt 74340
cagataggtt ccataccgct gctcccatcc actgatcagt ttgggagact gcacagcacc 74400
gaggaagtcc ctaccatcct cgctgaaccg ccacttcctg atggtctcct gagaacggac 74460
agggagacgc ttcaggccaa tcttgttgtc attgtacttg gagccatttg cccacgtccg 74520
tttccggtac accttctcat ggatcgagaa gccgtaagtg aagcaactgg tcacctcttt 74580
gatgaagtcg taccatgtgt ggtccatatc gtgcatgacc tgggagagga atttaacctt 74640
ctcctgcatg gcagcgtcag gctctgtacc gcccttgaca ctccattcta cacggcttac 74700
catcaattcg aacaatgcca gggcacttgc aatggtggcg tctcgggcca tcaccttgaa 74760
ggtgttcagg ttctcgggga accgtagctc agcccgagtt tcctcgtaga ttgcaccacc 74820
gctttgcttt aggcccgtgt gtccgatttc gcccagccga aggcgggggg ccgatggcac 74880
ttctgccgcc tttctgacgt tcctctttct ccgagccaca aagggcctcc tatcgttcaa 74940
tggccagcgg gtttggtcgg gtcaggtccg gagggatgaa gttgggaatg tggatgtcgg 75000
atgccaaata agtgtaggcg tccgaggtgg catccacctg gtcgtcctta acattccggc 75060
ttccatcaaa cacctccagc tccgtcaggt aagcctcgtt ccaggggcct tctactacct 75120
gaacacctcc agcctctgcc atactggcaa aaggaccgaa ccgggtgacc tttgatttgt 75180
tggttggacg ggtcctggca tggaacccgt gctctgccag gcggcgaaca aagtcccatg 75240
caaggtgctg tcctgccgct cctggatcac gtggaatcag gatcgtagtg tcagctccgt 75300
catgtcttgc tgtctcaagg atcagttcaa aaaccccgcc aatccggcgt cggtccctaa 75360
ccacatcctc gattgtgtat agcgtgctcc ggcctttgct catcaacacg cccgctgtcc 75420
agtctggctc tttgttggag tcagatggca gcgtaccgct gatatcccag gctcttacct 75480
gtctcacggt atctgtaggc ctaagcggaa tgattggagt ccattctcgc ttgaagtagc 75540
cgcttgcttc ttctcgggca aaccagctac catggagcag tcgaagtttc tcaacccggt 75600
tcaaaccttc aagccaggca acgtactggg ggttaatctc cattacgaca gggttgtcgt 75660
atacgttggc ggagatgaac ttgaaagtca ggggaggaga cttcaggttg tacttttcaa 75720

tcagctcttc cgctgtgtca gcgaactcca tgtctccgtt ttggatagtg aagaaccgag 75780
tgattccgtc cctctcgggg agcgggatac ctgtctcagg gtccaggtac caagtcagcc 75840
acttacgcag cggactgtta taatcagggt tgcaggtgat cttcatgtga ggttcaacct 75900
cgggacactt cggaccacgc atacgagaca tcaagtaaag caacatgtcc agggtgtatt 75960
gctgtccttc gtcaacgagg aattcgttga tttccagacc ctggtagttg tccttatcgt 76020
cgatgtgggc aaagtgtcga aggtgaatct cagcccccga ggagaagacg aatttcccgt 76080
ccttgtcctt ccatttgacc ttcttgtcaa actgcttgta caactgaagg cagttatcca 76140
agatgccgcc aggacctttg atctgaggag tagtccgtcg agtaatcagg ccccggaaca 76200
tcgggtgctt cacatgcttt agaaagtcca tcactccgag gtaggattta ccagccccgg 76260
cagctccacc gaacacggtg atgttcgcag tggagttgat aaacgcttcc tgcttcggag 76320
acttaggacc gatgactgtc ttagtcattt gtcaagccct caaagtttct cttgcttata 76380
tggtcagtat atgctagggg cttggctttg tcaagcccct agcatgctct ttatgctttg 76440
agtgcgttaa tcagagcatt gagcttggag gttacggcgg tcaggtcaga atccgcagga 76500
atagaggtga tgagggacac cttatccttg atggctgcaa ggacagcagc atcagcccca 76560
ctcacatctg cccaggccgg cttccagtta ccagccttgg cggtgcttga gctggtgccg 76620
atggtcagat tggaagtgcc tgcaccaata gcagttcgtg cagcagcagc atccgcagct 76680
ttcatcactg ccttgccagt gactccagca ccttccaggg tatctacggt cggtgccggt 76740
gcaggctccc cgccgtcccc tgccttcatc aggcctgcct ggatagctcc caggacgccg 76800
attcgagcga ttgcatcgta gaggacctta tcctctgggg tcatttcata agccatggta 76860
ggctccttgt tgcattgccc ggggccaatc cctgggcaca aaaaagcccc gctatgcggg 76920
gctattcgtc acccccagta gccagtcatc tgaacactta aaggtgatgc gagggttttc 76980
agatgagatt cgtactcggg gtggagttct ttcacaaccg ggtgaacgtc cggctggaag 77040
tacagggtat atgggatgag gttggtttct tctggctctt gccctgtgtg gcactccaag 77100
aggtctatca gagactcctg gttctttgcg acaaccggca acccccgtaa aaaggtgagc 77160
cacctggtct gaccactctt ctttggatca atcctcccta ccccaccgtg tcctcgtggg 77220
ctatcccctt tttcaatgaa gggtcgcaca agggcgtcta ggaatgtgct ggaataatct 77280
ccgtgagaga atgtcatcaa aacctgggga gttatctcag gacattggga gttcctgagt 77340
ctgctcaata ggtacgtcag agcgctgctg tctacctggt tggcttcgtc aactgctgcc 77400
ctttgaaata cgaatcctcc caggcttcta ggagcttcct cgtttgagat gcctcgaaga 77460
actatggagg acctgcgatc tggagtcctt accatcaaat ctcttcgaaa gaaccgtaga 77520
gttgggtcaa catccactat ctgttgcgcc aggcctccag ggcccgtaag atatggaatg 77580
gttctccggg ctgccaagcc tcgatatcct tccaagtgca tgggttcggt gagccacttg 77640
gctacgaggt gggacttacc tccgcctgcg tgggaggcgt acaggatgat actgtgctta 77700
tcgtcagaca tcacgcttgc ttgtgcggga gtgaattgga acgtcatttg caacctccat 77760
tacgggttga gaaagtaaaa agccccagcg aagatcgccg gggctaaggg tcacttctga 77820

gtcgctgacc cggagcgaca agacccgatt agcatcgggt agattggcag acgtggtggg 77880
attcgaaccc acaaatgctg gagtcaaagt ccagtgcgtt aaaccttttc gccacacgcc 77940
tgattgtaag tgagaggttt tggaaaaacg ggctactacc tctcaagcct agcctagctg 78000
aactgcctgg agcagttttg gggctgtgtt gaggaatcga acctcctgtt gctcagcggt 78060
gggagccctc tatccccacg ttaccggctt acgatccggg gagcctatcg cttcactgca 78120
acagtccaac ttacacagca tttggtggag ggggctggat ttgaaccagc gatgcccggt 78180
cggacgccgg atttacagtc cggtgcatta aaccgctctg ccacccctcc gttgaaacta 78240
acctcctggt gcgcagtgca aggccttatg cgtggaggtt actcttttgg ctccgctccc 78300
tggaatcgaa ccaggcacgt cttgattaac agtcaagctc cctcaccttg agggaccgaa 78360
cggaatgtag gagggtcact tggaccctgg ccccgcagga actacccttg ctgtcgggct 78420
atgaaattag cgggttggta acccggaccc gtccgggaga tgctggatca cctcctctgc 78480
acatcagctt cgtcggtccc acatctgaat ctcttaaatc cactttacca cattttcaga 78540
taaagtcaag agattttggc gggcagtctc agattcgaac tgagctgggt tgaatttgga 78600
gtccaacctg gtcgccaaga cccctaccct aaaacctcac gtttgacaga accatccctt 78660
ccttcaggat tgtgtagatc gtcgagtcat cgtgaacgaa tacgaaattc cgaaccttgg 78720
aaacccttac gaccctgccg tgctttacgc agagcgcgtt atagtttcct atccgcatac 78780
gaagctcaaa atcacagtat ttcgcttcag cgaaaagctt ccggatgacg tagatgtctc 78840
taccgtcctc gaagaccgag gggtggaact ggagccggaa ccgctcacga aagcgtttcc 78900
tggcatgggg cgtaacgaac atgtttctca tggcacctcc tgtttcttcg tggagggtat 78960
taaaccgtgt tcagtacgcc gtgtcaagag tgccggtgga ctcccatcct caaaggtccg 79020
gctgacactt ctccaggact ttagagcctg gaggacctac cccggcaacc tccggggcgt 79080
agggggttcg tgtggtgaac cttcaacccc tcccgccata cggctccggg aacgcctagt 79140
ggggctggga ggcccctaca ccacaagagc cgaggacata gccctcaatc tcatggatac 79200
tttgctcagc cagccaaagc tctcggtact ccggagagcc tggaaccttg aggcccatct 79260
tacggcttag gctgatcctg tgattcacca ggtcttccag ggtttggcaa tcagccagaa 79320
ctgattccac atacaccctt gactccggga acacccggtc cattacttct actgggactt 79380
gcatgctatg ctcctctctt ggcaggcata gtgagcgaga attctacgac ctgggcctga 79440
ggttcatcct cctgctcagg aacaccgtca gacttgttct ttactgcgtc aatggacgcc 79500
tgtgccctga gggctgccag gtaggcagcg aacaccttgt cagccatctt gagacgtact 79560
ggagatgtct gggacgcatt ggtcatttcg tcgatcatga gctgaatagc cttggcactg 79620
gcaggcttca gctttttgag gatgtgatca agctctccgg cagacatcgc cttgagggcc 79680
tgagcttcct tggtgggccg accacgaggg ttgccactct ggccctttac gaacttgctc 79740
atacttactc cttggttggg tgcctgagag gggaaggagg aggaaccctc ccaggtctta 79800
aactggtgta tctatagata ctatagcatc ctgtaggtgt gtttgtcaat ccctagtcac 79860

attgacggac tggtgtcaag ccctgcttcg catgagggaa tgcgtagttc tcagtatggc 79920
ctaccatctt ggcacggcct tgacacaggg agcagaggtc ttcatgctta cctgatactg 79980
aattcttact aggatactta attatcctat tacagccttt acatctcata aaaattaacc 80040
tatagtaatt aatattaatt atatacttag tacaagggag ggggcacttg cccactccct 80100
cccttgtata ttatatataa tatatttaat aatattacta taaattcttt agcttgtcaa 80160
gccttgactt ctgcttcaga tggtggtata gtgtactctc aatccaagga ggaatcaaaa 80220
atgtccgtaa aacctgctta tccagcggat cgtaagcgta tccgtgtagt cgaacaaaac 80280
cccatcagct tcctgcaagc cgtggaagat gccatcgctg aaggctaccg tgttgagaac 80340
accatggctg gattcccagt ggtcagctct gcttcgggtg ttcgtaccat caccctcttc 80400
cacgaggaag tgtgtggccc gactgaagtc gccagcccgg aggccgtaaa gagtctctct 80460
gagaacgccg ttgaggtcaa agactacaac ctcatgaatt tcctgcttgc attccaatcc 80520
caggttctgg caggatacac cctggacacc tcccgtgtgt ccatgttgtc cagtagcctg 80580
cactgggctg tcctggagaa ggggcaagat gagaacgttg tgttcgactt cggctcttcc 80640
gagcccgaag ctgaagctga gtcacaacct gaagccgagc cccaggctac tgagaaaccg 80700
gcccgtaaaa agcggcagcc aaagccgaaa cctaagcagc aggatgatgc tgagcaagaa 80760
caaattgaaa aaggagagaa tgaatgactg agaaagcaac tcaactgaac cgccaggacc 80820
tgttcaagca aatggcacat gacgccctca tgagtgttct cgtaccgatt atgccggacg 80880
aagatactct ggatcgactg taccaggctg tgcaggtggt caacctcgaa cccctggcca 80940
aagagttcga ccgagtgatg acggcagacg gtaccgactg gaagaaaatc cgcaaggtgg 81000
actccttcct gcgttccaag gactacatgg aagtgaatgg ccgctacgcc gtggtgcaag 81060
ccgagctaca ggaccaggta ggcctgatgc tccaggctct gtacagcgtt gtagtgccgg 81120
aagagaaacg ccccaagcaa gaggagaccg ctgaagtctc cgaagaagcg gtggaaagct 81180
gataacctaa gcccgcccag tgtggcgggc ttttttgcgc ctgaaataag ttgacagagc 81240
aggagagtct gtgtaggatg cccctgcaaa agtaataccg caaggagtaa acatgagcag 81300
taccccaaga actaccgtgg tgaccttcca ggacttctcg gctttggact tcgctccgcc 81360
agccacctgg tacatccaga acgctatggg tgattatgta ttcgtacaca cccgagacag 81420
gatgaaggct caaacgttcg tagacgaaga gtacggcaag ggtcgataca cggtgaaggc 81480
cagcaagatg cagcaggcca aaggagacct cacctgccgg gggacagcaa ccagaaaagg 81540
ccaacgaaag ccatcctagg aggtgctatg aagacaatga ttgtggtaga ggaactgaac 81600
aacggtctaa aggtttaccg cccaaaggtc aagatgtatc agcccctcct ggatcgccta 81660
ttctcctggc atccgaacaa gtggcgggac tgccacctta tctggcccaa cactgtgaga 81720
aaggttgagg tgtgggttat ggaccctact gacccagacg tgcatattcc cgagggacac 81780
ctagaaaaag ctatcattgc tcagcaggag atttacgagg gggcagtcgc gcttaaaagt 81840
cggtgcccga atatcgaaga cctgacagga ctggacctat caaaatcctt tgctgagctg 81900
gttctggaca tgtgcatcga ctcgtacaac gccatcaacg gctgccgggt ggaaaacagg 81960

tatgtgttgg aatacccaga gaaaaagcgg gggtattccc tgtgaaacag aagcataaag 82020
aggcttttat gcggtgcgcc gagtccttcg ctgaatgctc tgtgggtgtt cgtgcaaagg 82080
ttggtgcagt gattgtccag ggaaacaaga tcatcagtca ggggtataac ggcctcccaa 82140
gcggcctgga aggccctctg gagaacgaag agggggttac taggccagag gtacggcacg 82200
ccgagaaaaa cgccctgttg tggctctcgt gcagccagga gagtgctcaa ggtgcctgga 82260
tgttcgccac aatgtccccg tgtgagtatt gtgctcacga tattgttgac gcaggcatca 82320
agcgggtgta ctatcgccat cagtaccgac tgacagcagg aattgattac ctgctgaaga 82380
acggagttga agtcatccac atgccaaaag gagattaaac atgtacgcac aagactgtaa 82440
gaaacgcgaa cgggtgttgc ttgagctgtc ttacgaagag acaaagatgc ttgcagctct 82500
tgtaggcaag gtgtccgcag tggcggcaac aactcccctg ggagccttca tggatgagct 82560
gtatctgact actgtgcatg ttgctgacca acatggaagc aacccgtatg aagagcagtt 82620
tggagtacaa gcgatcggtg aagtggatga gtttgccatt ctggtaaaaa gctatgacgg 82680
tcaattccgt taataaggag ggtttatgca aaaagtgacg atgtaccaaa aggacaaggg 82740
cggcaacttc aagcagtgga gctgctggag tgaaggtgac aaggtattcg tggagcatgg 82800
aaaggagggt ggaaagctga ctgtcaaggt gaccacctcc aagccgaaga acgtaggccg 82860
tgcaaacgag accactggcg agcagcaggc tcacctggaa gccacttcca aggtagagaa 82920
gcagttcaag gcaggttacc gcgaagataa gagtgatctg gacaatgttc cgatcctccc 82980
gatgctggcc gaggactacc tgaagcgggc caagtccatc accttccctg ccatctacag 83040
cccaaagatg gatggtgtac gatgcctggc tcttcgacat gaagatggtc gtgtggagct 83100
tcgtagccgt ggtggggagc agtacatcct gccacacgtt gccgaggctc tggcccagat 83160
catggagatt ggcgacattt ttgatggtga gatttaccta catggtgagt ccctggaaga 83220
catccagtcg gcagttaagc gtactgacac cctgaaagag gtggagaaag ctgagaagaa 83280
gcttatgaag tacgcggacg acgagttttc cgatgattct gaactccagg aagcccaaga 83340
cgccttggac ctggcaatcc acatcgctgc tctgcgtccg gcgttgcaat accacatctt 83400
cgatgtcgtg agttgtaaag cgttgggtgt taccgttgat acccaattct cttggcgtct 83460
agacgccctt cgtcgaattg tctatagcgc agaccttatg gggaacccca ccctgtttgt 83520
agtagatcag gacgtaatct tcttccggga ggatgtagac aagtaccacg ataaggttgt 83580
catggaaggg tacgaaggca tcatgcttcg gaacatgacg gggctttacg aagcatctca 83640
gcgatcttcc gacctccaga agtacaagaa attcctcgac gaggagtttg aggttgttga 83700
cgtggtggaa ggcaaagacg ggaacgcagt tctccgcgtc ttctgcccca gagtgaagga 83760
gaaagcagac ggtcgtggac agtactccac ggggattttc gatacaacct tcggagatca 83820
tgcggctcgc cgggatcaac tggccaatcc ggagaaatac atcgggcgtt acctgaaggt 83880
gaaataccaa ggccgtttca agaagaccct ggttccccag ttcccagtcg gcctgtgctg 83940
gcgggaaatg gacgaaaacg gcaatcccgt agactaaggt tgacaacccc aaggaggcca 84000

ggcatactgg cctccagttc acagaaggag tttactatga aaatgctgtt gcgattcacc 84060
catcgtacaa ctttcgactc ggcgaccgcc aagaacactg catggcttca aatggacagc 84120
ctgatccgtt tcatgggcag cctgggctat ggtttcctgg cagagtcggg cagcttctat 84180
acgagcattc ccgagctgaa gagcaagggc ctacaaacac tgtctttcca gaccgcagtc 84240
gatcttcaca acgagccgaa gagcttgtgg ctggaaactg gtcgaggcag cggcaccttg 84300
gcccctgcta atcccaaact gaaaggcctg gagctgaccc cttacggctt ccagttgagc 84360
caggctacga agatcgtcaa acgagtgttc ctgactcgga ctcggaagag caagcaagca 84420
aacttcgtca tcttctgcta taagcatgaa gtgaagttct gccaagaaaa cttcagccgg 84480
gaattcctga gtaccgcctt ccctgtgttc cggaacggcc tggcctcgtt cctgtaatcc 84540
gtgccggcgc aatgccggca taccacccac ctgatagaaa ggagcccgaa tgaagaccct 84600
agaagcgctc tgtctgagca ctctattggc cgcgtccgga ctactgttcg agatcatggt 84660
aggcattgag acttctcgct tccatcttct tgagaagcga gcggtcctca gcagtcaacc 84720
tctggatgaa gtccagaaaa gagaaaccta caaggtattg tgcgagcgtc agaagtcctg 84780
tagaaccctg gcagaggcga tctacttcga agcccggggc gagagctgga aagggaagga 84840
aggtgtagcg cacgtcatca tcaacagagt gaacaaccca gaccgatggc cttcaacagt 84900
tccaggggtg gtgtacttcc gttgccagtt ctcgtacact tgtgatggaa gccgaaaggc 84960
aggaatccaa gagaaggccc agtgggatca atctaaggta gtggcctgga gggctttaaa 85020
aggtctgtcc aaagacccca cggggggtgc tgatcactac ctgaaccctg cgaaggtcaa 85080
atatatgccc cgctggacga aaaccatgaa aaagacgctc agaatcgagc ggcacgtgta 85140
ctacaaatca taaggaggaa gtatggaact ggtaatcgaa gccaagaagg tcgaggttga 85200
agcaacgtac agtccgtatt ccggacctga agtgactgtc gagctatcag gtgtaaagtc 85260
tacggtcgat gctcttctgg atcaactgga tgatgatgat atcttggatt acgtcatctc 85320
caatatccat ccccgggaca ttttgggaga gatggaggta tacgacatca cgtccttcct 85380
ggaatctaaa gggttcgagg taaccacatg agtctgttct acacaggtgt agggtcacgg 85440
gagactcctg ggtatattct tgacatcatt gtcaagctgg ggtactacct ttcggccgct 85500
ggggtgaccc tccgtacagg tggagcgcca ggtgcagacc aagcctttga ggaaggctgg 85560
tggaagcatc agatttggga tggagatggt acttctaagg ggcgaatcta tatcccgtgg 85620
aacggtttct atgacaggct gcaccacgga gatgcctatg gcgccgtagt atccctgtcc 85680
cacatctcct acggcttgga agtcagagcc cagcagattg cccagcagtt ccatcctgca 85740
tgggaacatc tcagcatcgg agcgaagaag ctccacacca ggaatgtctt ccaggtgctg 85800
ggggatgatc tggagactcc cagtgagttc ctaatatgct tcgctattcc gaacaaggac 85860
ggaagtgtcc agggaggcac aaacaccgct gtcaagctgg ctaaggcgca cggcgttcgg 85920
gtgacgaacc ttcacaacga ggaatcgaga ggttgggcaa cagaatggat caagagaatg 85980
gaggaccgat acgggactat tcccgaatag aagcccgggc aaatgagctg attaagcggg 86040
ctaatcggct cagggctgac aaagaacgca aggggaacaa gaaggggcgc cagttggaaa 86100

accaactgat gcccttgttc tcgttcatcg cgtttgagag ggatgatccc tggccgttgg 86160
agatggataa catcttccac ggggttgcca acctggattg gtaccggtcc agtgccgggg 86220
gtaaggaggg tgtgagcggg acgacaggca actacagaca ggtgcctctg tcgaaatcgg 86280
ctctgcgtaa aatatttacc ttcctcccct tgatttccac agaggaagtc cgtaaactgc 86340
tcctcatcga cgacaggcaa gccagtcggt acatgaaggc ggccttgttg gcatacccca 86400
agctttgtga atttaaggag aatttttatg agtttcaccc cgaccagtga gcagtgggac 86460
atcatggaag ctgccgaaga cctggcagcc gagcaacgtc cgaatcgccg cctgaaaatc 86520
aatgctggtg ccggtgcagc gaagaccacc accatgagcc tggtctctca acgcctccag 86580
gttacctccc tgtacctggc cttcaacaag tccatggcag aagaggccaa gaacaagttc 86640
ccgcagtggg tggtaaagaa gacgacccac ggcctggcat atggggtgtt cggggccaag 86700
ctcagcgaga aacttcgccg ccccaaaggg ggctacgtca acgtcctggg gaccggggca 86760
gagattgccc gctacttcaa gatcgccagc atggaatcgg acatgggggt caccgtcacg 86820
gcttccagca ttggccaggc catccgcgat acggtgaacc gcttcgagta ttcggcagac 86880
gaggagcttg aggtcaagca catcagctac atgcccctgg caaagctttc cgagaaggac 86940
cgtgtagacc ttccctacta tgaggccctg gttctccgct atgcaaaggc cttgtggaag 87000
aagcgtattg acccgaacga gcagacgctg atcaaccacg acacctacct gaagctgtac 87060
cagctctcca agccggacct cagccagtac aaggtgatct atctggacga ggcccaagac 87120
accaacgatt gcgtcctgga catcgtgctg cgtcagcagg actccctgct ggtcatggtc 87180
ggcgaccctc ggcagcagat ttacagcttc cgtggcagcg tgaacgctat ggccaagatt 87240
aaggccccag agtgcaagct caccaccagc ttccgtttcg gtgagaaagt ggctgaagtg 87300
gcgaacatca tcctacagaa cagcaaagcc caagccctgc acgggtggga aaagctggaa 87360
tcgaaggtat gggagagcaa cggcttcctg gcggacggtg acgctgaagg ggtacctgaa 87420
atcccggagc agcatgcccg gctctatcgg accaacgccg ccttggtgtg ggacgctgta 87480
aacctgattg gtcagggtaa gagtgtccga ctggagattg acacccgaga ctttatccgt 87540
ctgctggaga gtgctaccgc cctgtaccag ggtgacgaga agaaggtgaa gcacgaaaac 87600
atcctgtgct acacttcttg gcaagagttc cgggacgaat acgcagagag cccgggcgaa 87660
ctgggcaggg tgtgccggat catcgaaggg gatgaccagt ggaagatttt caaggccctc 87720
cgtgaccatg agaatgatcc ctcggcagac atcgtactga cgactgcaca caagtccaaa 87780
ggtcgggagt ttgatgtggt tgtcctggct gacgactatc cgagtgggta cgatgacaag 87840
aagcggtggg taggtttgaa cgagatggag cagaatctcc tgtacgttgc cgccacccgc 87900
tgcaagaagc acctatacgg gaatacaacc ctggacaata tcatggtgga aacccgtgcc 87960
atgtggcttg aagcgaagga ggaagaatga agcaggtgaa tttcgaggag gcaatctcga 88020
acctagagcg agggatcagt gtcgtgctgt accttaaagg attggagggg tttgctgatg 88080
acctgattta ccgcacatac ctattcaagg gaggtttcgg tcatgtcaaa gattcctact 88140

ccgacggtac tgcgatgact gcgctgcttc cgttggaggg ctccgaacgg atgggatcgg 88200

tcctgaaaga ttgccttgtc tgggacctgt cctcagccca ctcggtgagc tataatgggg 88260

tttgactggc aaaaggttcc cttacacctc cgagaggtgt tgtgggaaaa tgaaatacaa 88320

aacggaatgg tagaagaagt agactgtgcg gg 88352

## Description

The present invention relates to isolated phage specific to PAK and CHA strains of *Pseudomonas aeruginosa* and therapeutic compositions comprising said phages. The invention is also directed to their use for the treatment or prevention of bacterial diseases caused by *P. aeruginosa,* in particular for phage therapy of lung infections. The present invention also relates to human or pet food products prepared thereof.

*Pseudomonas aeruginosa* causes acute pneumonia with a high mortality rate in immuno-compromized patients, while in cystic fibrosis patients, it triggers chronic inflammation leading to destruction of the lungs (*1*). This bacterium as well as an increasing number of opportunistic pathogens is now resistant to multiple antibiotics, urging us to look for other therapeutic approaches, among which phage therapy has been proposed (*2*). Phage therapy has been considered since the late 80s (*3-5*) mainly because: 1) It has been used for decades in Eastern Europe and is still used to treat humans infections (*6*); 2) Phage biology is much better understood today than it was in the mid-1900s when it was overlooked in favour of antibiotics (*7-9*); 3) Phages are known to be the most specific bacterial killer. Curiously, a large number of *P. aeruginosa* bacteriophages have been characterised (*10*), but few papers report on using these phages in therapy experiments, and none test them on a lung infection model (*11, 12*).

There is a need to develop compositions including phage therapeutics in acceptable vehicles that can be administered to subjects infected with bacterial microorganisms.

It is therefore an object of the present invention to develop new and useful phage therapeutics which can be used to prevent or to treat infectious disorders caused by pathogenic bacteria, preferably by *Pseudomonas aeruginosa* infections, in particular lung infection diseases in humans or animals.

With the use of a bioluminescent *P. aeruginosa* strain the inventors have monitored and quantified the efficacy of a phage treatment in live animals using phages specific to the PAK strain of *P. aeruginosa.* For example, they have demonstrated that an intranasal phage treatment is effective in saving animals. It has also been shown that phages given 24 hours (24H) before the initiation of the infection are able to prevent the development of this infection demonstrating the use of phages for a preventive treatment. Both curative and preventive treatments were also demonstrated as active against the CHA strain.

Thus the present invention is directed to isolated and/or purified bacteriophages that are specific to PAK and CHA strains of *Pseudomonas aeruginosa.*

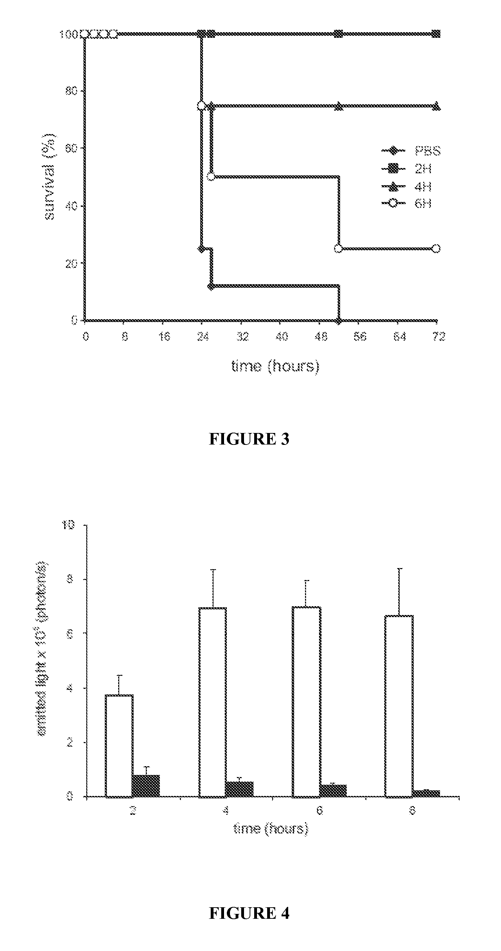

In a preferred embodiment, said isolated and/or purified bacteriophages according to the invention are bacteriophages of the Myoviridae family, more preferably capable to reach 100 % survival of infected mice with *Pseudomonas aeruginosa* bacteria when administering said bacteriophages 2 hours (2H) post-infection, as shown for example in figure 3 herein.

Preferably, said bacteriophages are isolated from sewage water.

In a more preferred embodiment, said isolated and/or purified bacteriophages according to the invention are selected from the group of bacteriophages consisting of:
- PAK-P1 which has been deposited at the Institut Pasteur, 28 rue du Docteur Roux, F-75024 Paris Cedex 15, FRANCE, on July 11, 2008, under the deposit number CNCM I-4040;
- PAK-P2 which has been deposited at the Institut Pasteur, 28 rue du Docteur Roux, F-75024 Paris Cedex 15, FRANCE, on July 11, 2008, under the deposit number CNCM I-4041;
- PAK-P3 which has been deposited at the Institut Pasteur, 28 rue du Docteur Roux, F-75024 Paris Cedex 15, FRANCE, on July 11, 2008, under the deposit number CNCM I-4042;
- PAK-P4 which has been deposited at the Institut Pasteur, 28 rue du Docteur Roux, F-75024 Paris Cedex 15, FRANCE, on July 11, 2008, under the deposit number CNCM I-4043;
- PAK-P5 which has been deposited at the Institut Pasteur, 28 rue du Docteur Roux, F-75024 Paris Cedex 15, FRANCE, on July 11, 2008, under the deposit number CNCM I-4045; and
- PAK-P3 CHA which has been deposited at the Institut Pasteur, 28 rue du Docteur Roux, F-75024 Paris Cedex 15, FRANCE, on March 30, 2009 under the deposit number CNCM I-4139.

Are also more preferred isolated and/or purified bacteriophages according to the present invention that are selected from the group of bacteriophages consisting of the bacteriophages having the sequences SEQ ID NO: 1 , SEQ ID NO: 2, and SEQ ID NO: 3, or a specific fragment thereof capable to reach 100% survival of infected mice with *Pseudomonas aeruginosa* bacteria when administering said specific sequence fragment 2 hours (2H) post-infection as shown in figure 3.

SEQ ID NO: 1 corresponds to the complete nucleic sequence of the PAK-P1 phage which has been deposited at the Institut Pasteur, 28 rue du Docteur Roux, F-75024 Paris Cedex 15, FRANCE, on July 11, 2008, under the deposit number CNCM I-4040;

SEQ ID NO: 2 corresponds to the complete nucleic sequence of the PAK-P2 phage which has been deposited at the Institut Pasteur, 28 rue du Docteur Roux, F-75024 Paris Cedex 15, FRANCE, on July 11, 2008, under the deposit number CNCM I-4041; and

SEQ ID NO: 3 corresponds to the complete nucleic sequence of the PAK-P3 phage which has been deposited at the Institut Pasteur, 28 rue du Docteur Roux, F-75024 Paris Cedex 15, FRANCE, on July 11, 2008, under the deposit number CNCM I-4042;

In a second aspect, the invention is directed to a pharmaceutical composition comprising isolated and/or purified bacteriophage according to the present invention.

In a preferred embodiment, the present invention comprises the use of isolated and/or purified bacteriophages according to the invention as a medicament.

In a more preferred embodiment, the present invention relates to a composition comprising isolated and/or purified bacteriophages according to the present invention as a medicament or pharmaceutical composition for the prevention or the treatment of an infectious disease caused by bacteria in a mammal, preferably in human.

In a more preferred embodiment, said composition of the present invention is intended for the prevention or the treatment of lung infection, preferably caused by pathogenic *Pseudomonas aeruginosa.*

In a particular aspect, said composition according to the invention is an aerosol formulation for administration to the lungs of a mammal.

For the composition of the invention, said bacteriophages can be initially prepared in lyophilized form.

In one embodiment, said pharmaceutical composition contains at least one of the bacteriophages of the present invention in an amount sufficient to achieve the desired effect in an animal or human individual. This composition may be a tablet, a liquid, a dried oral supplement, a wet oral supplement, dry tube-feeding, wet tube-feeding etc. The pharmaceutical composition will further contain carriers and excipients that are suitable for delivering the respective active molecule of different nature to the target tissue. The kind of the carrier/excipient and the amount thereof will depend on the nature of the substance and the mode of drug delivery and/or administration contemplated. It will be appreciated that the skilled person will, based on his own knowledge, select the appropriate components and galenic form.

The routes of administration include but are not limited to: oral, aerosol or other device for delivery to the lungs, nasal spray, intravenous, intramuscular, intraperitoneal, intrathecal, vaginal, rectal, topical, lumbar puncture or intrathecal. Excipients which can be used as a vehicle for the delivery of the phage will be apparent to those skilled in the art. For example, the free phage could be in lyophilized form and be dissolved just prior to administration. The dosage of administration is contemplated to be in the range of 1 to 10 ml of 10³ to about 10¹³ pfu/per ml, twice a day. The phages are administered until successful elimination of the pathogenic bacteria is achieved.

With respect to the aerosol administration to the lungs, the isolated phages of the present invention can be incorporated into an aerosol formulation specifically designed for administration to the lungs by inhalation. Many such aerosols are known in the art, and the present invention is not limited to any particular formulation. An example of such an aerosol is an aerosol wherein the propellant of which contains trichloromonofluoromethane, dichlorodifluoromethane and oleic acid. The concentrations of the propellant ingredients and emulsifiers are adjusted if necessary based on the phage being used in the treatment. The number of phages to be administered per aerosol treatment will be in the range of 10³ to 10¹³ pfu.

In another aspect, the present invention is directed to a food or pet food product, a nutritional composition or a supplement for nutritional composition which comprises at least one isolated and/or purified bacteriophage of the invention.

Finally, the present invention is directed to the use of a least one isolated and/or purified bacteriophage of the invention for the preparation of a food or pet food product, a nutritional composition or a supplement for nutritional composition for the prevention or the treatment of infections caused by pathogenic bacteria, preferably by pathogenic *Pseudomonas aeruginosa* in humans or animals, or for decreasing the degree of contamination of food product contamination.

For example, a usual food product may be enriched with at least one isolated and/or purified bacteriophage according to the present invention, particularly food products or nutritional supplements for clinical nutrition of animals or human.

In another embodiment, said at least one isolated and/or purified bacteriophage of the present invention may be prepared so as to improve pet food quality. As dietary adjuncts, they may be encapsulated or may be provided in powder form and packaged in conjunction with or separately from a main meal, be it wet or dry. By way of example, a powder containing bacteriophages according to the invention may be packed in sachets in a powder form or in a gel or lipid or other suitable carrier.

The nutritional formula is preferably enterally administrable. For example in the form of a powder, a liquid concentrate, or a ready-to-drink beverage. If it is desired to produce a powdered nutritional formula, the homogenized mixture is transferred to a suitable drying apparatus such as a spray drier or freeze drier and converted to powder.

The following examples preceded by the legends of the figures are given by way of illustration only and in no way should be construed as limiting the subject matter of the present application.

### Figures legends

### Figures 1A-1C: A phage treatment cure deadly infected animal.

Figure 1A: Survival curves of infected animals treated by PBS or phages at indicated phage/bacterium ratios.
Figure 1B: Example of time-course pictures of mice infected by bioluminescent *P. aeruginosa* and treated by PBS (left) or treated by the PAK-P1 phage at a ratio phage/bacterium of 10/1 (right).
Figure 1C: Quantification of emitted light given as a ratio between two time points (4H vs 2H and 6H vs 4H). The chest area from infected mice (n=12 from two independent experiments) treated by PBS (white) or treated by the PAK-P1 phage at a ratio 10/1 (black) was quantified.

### Figure 2: A phage treatment reduces inflammation.

Cytokine levels were measured in BAL 24H after instillation of PBS (black), or PBS+ PAK-P1 phage (white), or bacteria + PBS 2H later ( 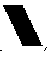 ), or bacteria + PAK-P1 phage 2H later (grey)

### Figure 3: Time-course of a phage treatment.

Survival curves of infected mice treated by PBS (◆) or by the PAK-P1 phage at 10/1 ratio at, 2H (■), 4H (▲) or 6H (○) after the infection was initiated.

### Figure 4: Efficiency of a 24H phage pre-treatment.

Time-course of light emitted (photons/s) from the chest area of mice pre-treated by PBS (white) or PAK-P1 phage (black) 24H before the infection by *P. aeruginosa* (n=4 for each group).

### Figures 5A-5C: Phages characterisation

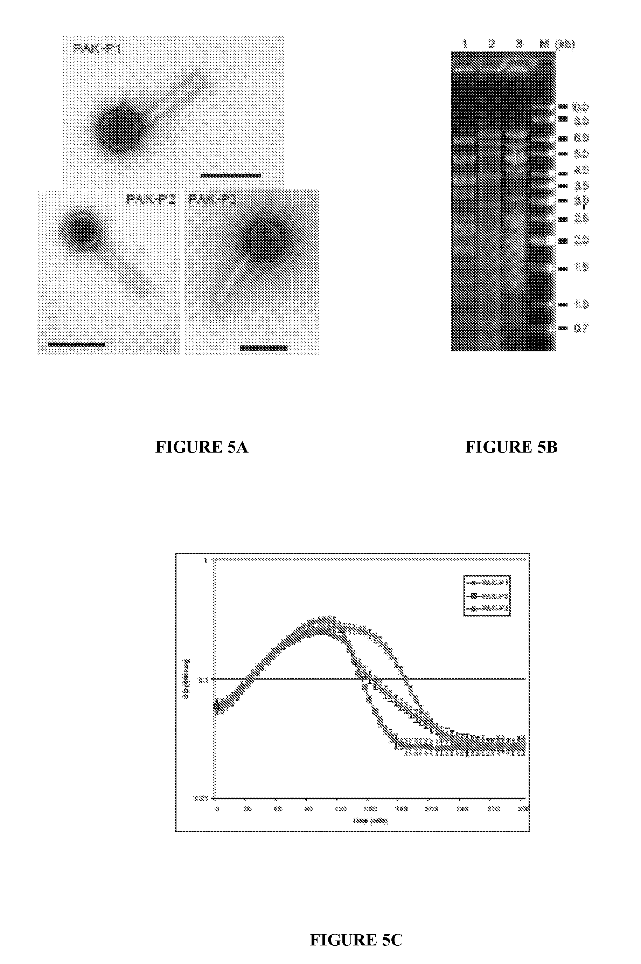

Figure 5A: Electron microscopy of phage PAK-P1 (scale bar = 100 nm).
Figure 5B: Restriction profile of phages PAK-P1, PAK-P2 and PAK-P3.
Figure 5C: Lysis kinetics of phages PAK-P1, PAK-P2, PAK-P3 (average of 7 measures with s.d.).

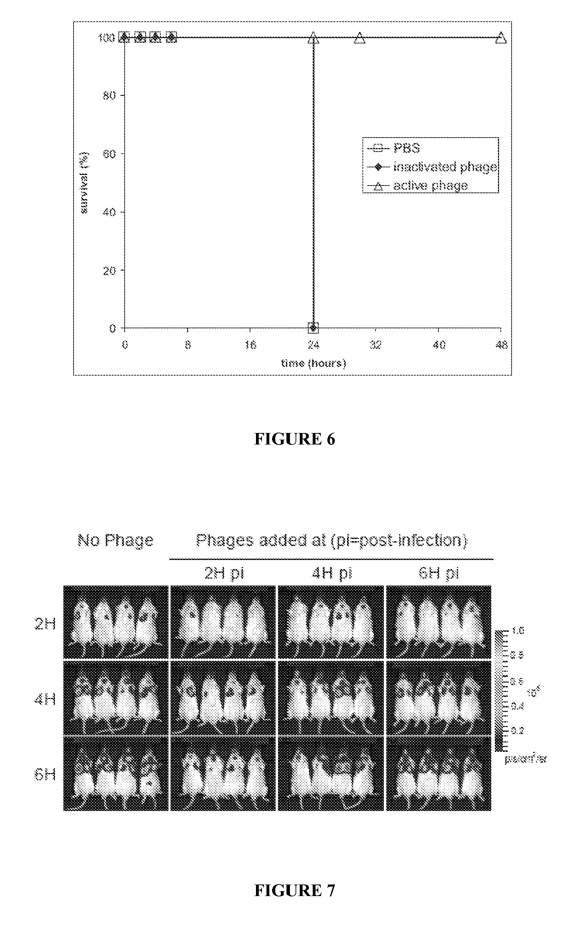

### Figure 6: An active phage is required for treating infected mice.

Survival curves of mice infected by P. aeruginosa and treated by PBS ( ), by inactivated PAK-P1 phage (◆), or by active PAK-P1 phage (Δ), the two latest at the 10/1 ratio.

### Figure 7: Determination of the optimal delay before phage treatment

Pictures of animals treated by the 10/1 ratio of the PAK-P1 phage at 2, 4 or 6 H post-infection by bioluminescent *P. aeruginosa.*

Supplementary Figure 4: Comparison of three phage efficiencies
A) Survival chart of infected mice treated with phages PAK-P1, PAK-P2 and PAK-P3 at 3 phage/bacterium ratios (1/10; 1/1; and 10/1).
B) Comparison of the efficiencies of phages PAK-P1, PAK-P2 and PAK-P3 at the ratio 1/10 determined by the ratio of light emitted between 2H and 6H post-infection (ie 4H after phages were added).

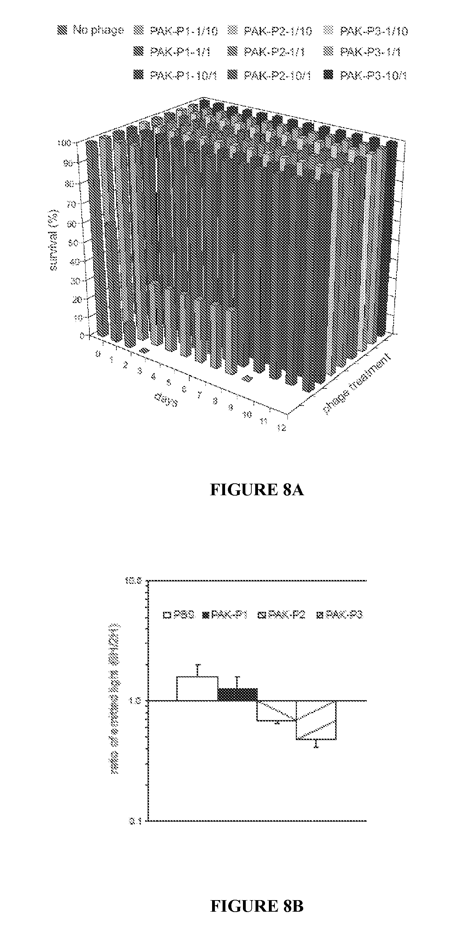

### Figures 8A-8B: Comparison of three phage efficiencies

Figure 8A: Survival chart of infected mice treated with phages PAK-P1, PAK-P2 and PAK-P3 at 3 phage/bacterium ratios (1/10; 1/1; and 10/1).
Figure 8B: Comparison of the efficiencies of phages PAK-P1, PAK-P2 and PAK-P3 at the ratio 1/10 determined by the ratio of light emitted between 2H and 6H post-infection (ie 4H after phages were added).

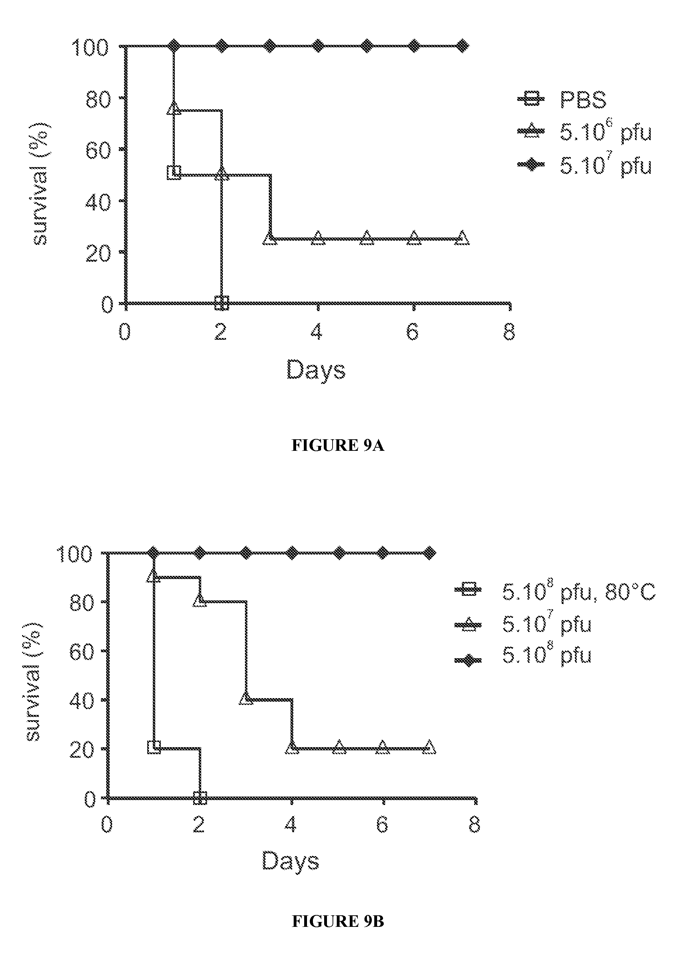

### Figures 9A-9B: A treatment with the PAK-P3-CHA phage cure deadly infected animals by the CHA strain.

Figure 9A: Survival curves of infected animals treated 2H later by PBS or by the PAK-P3-CHA phage at indicated amounts.
Figure 9B: Survival curves of animals pretreated by the PAK-P3-CHA phage 4 days before to be infected by the CHA strain.

### EXAMPLES

### Materials and Methods

### P. aeruginosa strains

The bioluminescent PAK strain (PAK lumi) was obtained as described in the publication Ramphal. et al, page 587, First column, paragraph " Bacterial Strains" of Materials and Methods ( 21: J Immunol 181, 586-592, 2008 "Control of Pseudomonas aeruginosa in the Lung Requires the Recognition of Either Lipopolysaccharide or Flagellin") (*21*). 4 serotyped clinical *P. aeruginosa* strains were used to determine phage host range. 10 primo-colonization and 10 chronic clinical strains of *P. aeruginosa* were obtained from the French center of cystic fibrosis strain collection (P. Plésiat, Grenoble, France).

### Phages isolation, preparation and characterisation

Phages were isolated from sewage water (provided by the Laboratoire d'Hygiène de la Ville de Paris) as follows. First, sewage water was filtered at 0.2 µm and mixed with an equal volume of 2X Luria-Bertani (LB) medium. Second, this mixture was inoculated with a fresh culture of PAK lumi and incubated on a shaker at 30°C overnight. The next day chloroform (1/10 volume) was added to the flask and placed on a shaker for one more hour. The medium was then centrifuged at 10,000 g for 10 min. 1ml of the supernatant was collected and 1/10 vol. of chloroform was added. After a brief mix by vortex, the eppendorf tube was centrifuged at 7,500 g for 5 min. To determine if phages were present in this extract, a drop (10 µl) of the supernatant was applied on a LB agar plate and let dry. Using a palatine wire, the plate was streaked from the drop through the rest of the plate to isolate individual phages. Then, 1 ml of a growing culture of PAK lumi was applied to cover the entire plate; the excess was removed and the plate was incubated at 37°C overnight. Most of the time a drop from a 100 fold dilution of the original supernatant was enough to obtain isolated plaques. The following day, one or two plaques were picked up and resuspended in 200 µl of SM buffer (10mMTri HCl pH7, NaCl 200 mM, gelatine 0.03%). 20 µl of chloroform was added in each tube and tubes were briefly mixed by vortex and centrifuged at 7,500 g for 5 min. Afterwards, 10 µl of the supernatant was applied on a LB plate and let dry and the previous procedure was repeated at least three times. Once the majority of plaques were homogenous, 10 µl of the last resuspended plaque were added to 1 ml of growing culture of PAK lumi at OD 0.1 at 600 nm. This culture tube was incubated at 37°C for 3 to 4 hours until lysis occurred. After addition of 1/10 vol. of chloroform, the culture was transferred to an eppendorf tube, centrifuged at 7,500 g for 5 min and put at 4°C. This was considered as the primary stock. Several dilutions of this stock were kept at 4°C and used to infect larger volume of culture in order to prepare larger amounts of phages.

Large preparations of phages were performed using cesium chloride ultracentrifugation technique as described in Boulanger (22).

Plaque assay (from not diluted to 10⁻⁶ dilution) were performed to determine host range and activity levels in comparison with the PAK lumi strain. A high activity was defined as an activity equivalent to the PAK lumi strain; a weak activity as 10² to 10⁴ less active than the PAK lumi strain and a null activity when no lysis was observed with the not diluted phage solution.

Lysis kinetics were performed at 37°C in 96 wells microplates in a final volume of 100 µL with a ratio of 1 phage for 1000 bacteria. OD was automatically recorded every 5 minutes with a microplate reader (Biotek, USA).

Phage DNA was prepared using the Qiagen DNA lambda mini-prep kit from 5ml fresh liquid lysate. Restriction profiles by AccI enzyme were performed using manufacturer's recommendations (NEBioloabs, USA).

Electron microscopy was performed from cesium chloride phage preparations (23). Observations were performed on a Jeol 1200 EXII.

For animal experiments, phages prepared by cesium chloride ultracentrifugation were diluted in PBS.

### Animal infections

Mice (8 weeks old Balb/c males) were supplied by the Centre d'Elevage R. Janvier and housed in animal facility following Institut Pasteur guidelines in agreement with European recommendations. Food and drink were provided ad libitum. Mice were anesthetized by intra peritoneal injection of a mixture of ketamine-xylazine before infection. Then, 1 10⁷ luminescent bacteria resuspended in 50 µl of PBS were instilled intranassaly. Two hours after bacterial instillation, bioluminescence was recorded and, while mice were still asleep (isofluorane inhalation), phages were applied intranasally in a final volume of 30 µl.

### Luminescence measurements

Photon emission of the luminescent bacteria in the lungs of infected mice was quantified using the IVIS 100 system (Xenogen Biosciences). After infection, luminescence record was performed in mice under isoflurane inhalation in an IVIS charge-coupled device camera coupled to the LivingImage software package (Xenogen). A digital false-colour photon emission image of the mice was generated, and photons were counted within a constant defined area corresponding either to the surface of the chest encompassing the whole lung region or to the entire animal. Photon emission was expressed as photons per second emitted.

### Bronchoalveolar lavage and inflammation analyses

Bronchoalveolar lavage (BAL; 4x0.5ml) were performed at the indicated time periods after infection following pentobarbital euthanasia. The BAL fluids were diluted and plated on LB agar plates to obtain viable bacterial counts in the BAL fluid. BAL were centrifuged 10 min at 1,400 rpm and then murine cytokine concentrations were determined using DuoSet ELISA kits obtained from R&D Systems.

### Statistical analysis

P values were calculated with unpaired t-test using XLStat software (France). Error bars are s.e.m.

### Results

New phages specific to the PAK strain of *P. aeruginosa* were isolated from sewage water. 3 phages named PAK-P1, PAK-P2 and PAK-P3 were chosen for this study. Electron microscopy observations revealed that these three phages are part of the *Myoviridae* family (*10*). By studying their lysis kinetics in liquid culture, their host range against a panel of 4 clinical *P. aeruginosa* strains and their DNA restriction profiles, It has been established that these three phages are genetically distinct (see Figures 5A-5C and Table 1 below).

**Table 1: Phages host range was determined against a panel of 4 clinical strains serotypes O:4; O:9; O:10; O:11) and compared to the PAK strain (serotype O:6). N = no activity; W = weak activity; H = high activity. D)**

| Serotype | PAK-P1 | PAK-P2 | PAK-P3 | | | |
|---|---|---|---|---|---|---|
| O: 4 | | W | | W | | W |
| O: 6 | | H | | H | | H |
| O: 9 | | W | | N | | N |
| O: 10 | | N | | N | | N |
| O: 11 | | W | | W | | W |

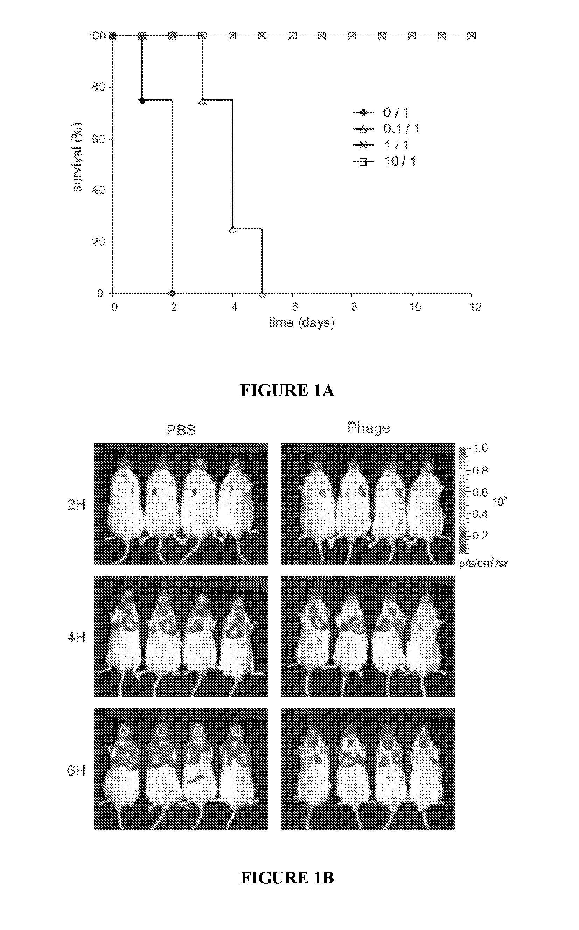

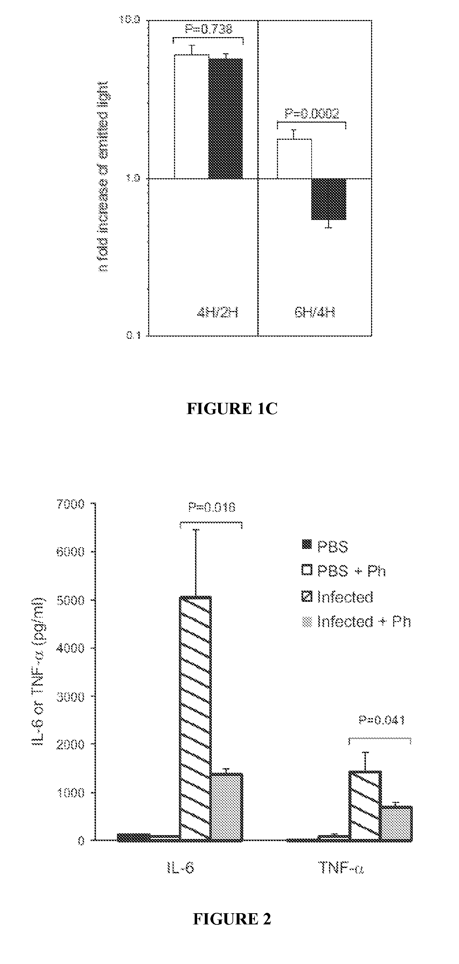

To ascertain the affect of phage on infection in a live animal lung infection model, mice were both infected by the bioluminescent PAK strain and treated with phage by intranasal instillation. After a preliminary experiment which showed that the PAK-P1 phage was active *in vivo* by delaying the death of highly infected animals (data not shown), we designed an experiment to determine the amount of this phage required to fully cure infected mice (see Figures 1A-1C). While non phage-treated mice (NPT-mice) died within 48H after the PAK inoculation (the majority were still alive after 24H), phage-treated mice (PT-mice) using a ratio of 1 phage/10 bacteria, died within 5 days. PT-mice using higher phage to bacterium ratios (1/1 and 10/1), survived until the end of the experiment (12 days). In two independent experiments, 100% of animals treated by the dose 10/1 survived, while only 80% of animals survived among those treated by the 1/1 ratio, leading us to choose the ratio 10/1 as a standard condition for future experiments.

An active phage was required to cure animals since giving infected mice a heat-killed phage PAK-P1 solution (shown to be inactive on a bacterial lawn) did not cure them when given 2H after infection, animals dying at the same rate as untreated animals (see Figure 6). Moreover, to determine if PAK-P1 phage treatment was harmless to animals, we applied intranasally a dose 10 times higher than that previously defined as a standard dose, and followed the behaviour of a group of mice (n=8) for 10 days. These mice did not show erratic behaviour, their fur stayed regular and they gained weight, suggesting the phage did not have an adverse affect on these animals.

The rate at which the PAK-P1 phage was able to eliminate bacteria *in vivo,* was estimated by quantifying the light emitted by the bioluminescent bacteria in live animals during the first hours of infection (see Figures 1A-1C). Animals were first inoculated with PAK and then with PAK-P1 phage 2H later. Between 2H and 4H post-bacterial infection, the variation of light emitted from PT-mice by 10/1 dose and NPT-mice showed no significant difference, showing that the initial evolution of the infection is similar in both groups during the first 4 hours. However, at the 6H time point (ie 4H after phage was added), the variation of light emitted from phage treated mice was highly significantly reduced compared to NPT-mice, suggesting a rapid killing of bacteria by phages. 24H after the infection started, PT-mice presented none or weak spots of light, while NPT-mice were dead or highly luminescent (data not shown), suggesting that phage treated animals can survive the lethal bacterial challenge.

It is hypothesised that if phages are able to kill bacteria *in vivo* leading to a reduction in the amount of these bacteria in the lungs, then the inflammatory response (the first line of host defence against invading pathogens) should be diminished. By performing broncho-alveolar lavages (BAL) on NPT-mice and PT-mice, we evaluated the levels of two inflammatory markers, TNF-α and IL-6 (known to be induced by a bacterial challenge (*13-15*)) 24H after phage addition (see Figure 2). Both IL-6 and TNF-α increased in PAK infected animals (either with or without phage) compared to non PAK infected controls confirming that the infection had started in both groups. These levels were significantly reduced however, in the phage-treated group in comparison to the untreated group confirming that bacteria/ml (n=4). In contrast only 1 10reducing the number of bacteria by phage resulted in an attenuation of the host inflammation response. 48H after the infection IL-6 and TNF-α levels returned to basal values in the PT group (data not shown). We also measured the bacterial load and phage amounts in BAL after 24H of infection. As expected in the BAL of NPT-mice no phage was detected and the average amount of bacteria was 1 10⁸ bacteria/ml (n=4). In contrast only 1 10² bacteria/ml were recovered from BAL of PT-mice together with 1 10⁷ phages/ml (n=4). In comparison, in BAL from uninfected PT-mice, PAK-P1 phage was still present at a concentration of 5 10⁵ phages/ml (n=2), which confirmed that phages did multiply inside the lungs of infected and phage treated animals.

Next, the maximum possible delay of phage treatment has been determined to maintain a 100% survival rate of animals (2H, 4H and 6H after the infection). While in the PT-mice 2H post-infection group 100% of mice survived, at 24H only 75% of mice were still alive in the 4H and 6H groups. At 72H, survival was close for the 2H (100%) and 4H (75%) groups but dropped to 25% for the 6H group. These results could actually be anticipated when looking at the bioluminescence pictures at early time points (see Figure 7). In such experiment giving phage 2H post-infection was then required to reach 100% survival of infected animals (see Figure 3).

This set of experiments demonstrates that non-invasive bioluminescence technology is remarkably useful to assess the efficiency of a phage treatment and especially to study the kinetics of infection at early time points without sacrificing animals. For example, in the time-course experiment to determine the optimal time to give phage after the infection was started, bioluminescence images helped us to understand why early phage inoculation (2H) was so important in resolving a PAK infection (see Figure 7). It is during this early time after infection that the multiplication of bacteria is most rapid. In such conditions bacteria susceptibility to phage infection is the highest. Then, rapidly the infection will be reduced and consequently, the host will diminish its inflammatory response which is a benefit since an excess of inflammatory response can be deleterious (*13-15*).

Results obtained with the PAK-P1 phage encouraged us to examine the ability of other PAK-killing phages to cure infected animals or to prevent their infection. Next, 2 new phages PAK-P2 and PAK-P3 have been compared with the PAK-P1 by varying phage/bacterium ratios 10/1, 1/1 and 1/10 (see Figure 7). In terms of survival, animals of the three groups treated by phages at the ratio of 10/1 or 1/1 survived to the end of the experiment (12 days). However, at the dose of 1/10 of PAK-P1 phage 66% of mice died on the 3^{rd} day and 100% on the 9^{th} day, while a ratio of 1/10 for PAK-P2 and PAK-P3 allowed the survival of 100% of infected animals. Examination of the bioluminescent data of the 1/10 dose between 2H and 6H time points, revealed that the ratio of the light emitted from animals is more reduced by phages PAK-P3 and PAK-P2, than by phage PAK-P1 (see Figures 8A-8B). A similar efficiency is observed both *in vivo* and *in vitro* (PAK-P3 and PAK-P2 faster than PAK-P1) suggesting that inside the lungs there is no specific cellular factor (proteases for example) which could have enough activity to prevent phages from infecting bacteria.

The efficiencies of our phages were also studied against a panel of twenty *P. aeruginosa* clinical strains from cystic fibrosis patients. Ten strains from primo-colonisation and ten strains from chronically colonized patients were also studied. Our three phages were able to efficiently lyse up to 40% of primo-colonisation strains but only moderately lyse 30% of chronic ones (see Table 2).

**Table 2: Phages efficacies on P. aeruginosa clinical strains Numbers represent the percentage of strains sensitive to phages based on plaque assays from non diluted to 10⁻⁶ dilution.**

| Strains / Phages | PAK-P1 | | | PAK-P2 | | | PAK-P3 | | |
|---|---|---|---|---|---|---|---|---|---|
| *efficacies* | *H* | *W* | *N* | *H* | *W* | *N* | *H* | *W* | *N* |
| Primo-infection | 40% | 20% | 40% | 10% | 50% | 40% | 0% | 40% | 60% |
| Chronic-infection | 0% | 10% | 90% | 0% | 20% | 80% | 0% | 30% | 70% |

This is in accordance with the fact that the phages were isolated from planktonic cultures, a bacterial life-style closer to the primo-colonisation state than the chronic state. In the case of chronic infection, phages described here are most likely not the most appropriate. For these situations more adequate phages should be specifically isolated (*16, 17*) or existing phages could be "selected", by cultivating them on bacteria growing in biofilms, to become more efficient. Actually, Hanlon *et al.* (*18*) demonstrated at least for one case that a *P. aeruginosa* phage was active on biofilms.

As phages given in uninfected animals persisted in reasonable amounts inside the lungs at least for 24H, we tested whether a phage pre-treatment would prevent a subsequent infection. Two groups of mice which were administered either 1 10⁸ PAK-P1 phage or buffer intranasally, were infected 24H later by 1 10⁷ bacteria. Quantifications of light emitted from these 2 groups of mice, showed that 2H after bacteria inoculation the light emitted in the phage pre-treated group was approximately 5 times lower than the buffer pre-treated group (see Figure 4). Following time points confirmed that the bacterial load decreased in phage pre-treated animals and increased in the buffer pre-treated animals (see Figure 4). Finally, 100% of phage pre-treated animals survived while 100% of untreated animals died. This full protective effect was also observed when a 1000 fold dilution of phages (i.e 10⁴ of PAK-P1 phage alone or a cocktail of the three phages) was given 24H before infection (data not shown).

This last experiment demonstrates that low doses of phages can actively prevent an infection from occurring and provide 100% protection against a deadly bacterial challenge. Such an effect was not anticipated since it is generally reported that phages are rapidly eliminated from the body (*19*). This does not seem to be the case for the lungs since we could still detect traces of phage on lungs of uninfected mice 5 days after instillation (data not shown). Recently Golshahi et al. (*20*) provided evidences that phages given by nebulization should be efficiently distributed in lungs. Taken together, these results demonstrate the possibility of using phages in prevention of bacterial lungs infection. For example one could propose to pre-treating populations at risk (immuno-compromized or cystic fibrosis patients) with moderate dosage of phage in order to decrease the probability of infection when going to places where patients are more likely to be infected by bacteria (care centres, hospitals etc). This is particularly relevant in situation where an epidemic has been identified where a preventive treatment with specific phages could be used to limit the spread of the epidemic strain.

### References

1. G. Pier, R. Ramphal, in Principles and Practice of Infectious Diseases, 6th Ed G. L. Mandell, J. E. Bennett, R. Dolin, Eds. (Elsevier Press, New York, NY, 2004) pp. 2587.
2. K. Thiel, Nat Biotechnol 22, 31 (Jan, 2004).
3. C. R. Merril, D. Scholl, S. L. Adhya, Nat Rev Drug Discov 2, 489 (Jun, 2003).
4. H. Brussow, Microbiology 151, 2133 (Jul, 2005).
5. P. A. Barrow, J. S. Soothill, Trends Microbiol 5, 268 (Jul, 1997).
6. A. Sulakvelidze, E. Kutter, in Bacteriophages: biology and applications Kutter, S. A. E, Eds. (CRC Press, Boca Raton, FL, 2005) pp. 381-436.
7. G. F. Hatfull, Curr Opin Microbiol 11, 447 (Oct, 2008).
8. A. Campbell, Nat Rev Genet 4, 471 (Jun, 2003).
9. W. C. Summers, Annu Rev Microbiol 55, 437 (2001).
10. H. W. Ackermann, Arch Virol 152, 227 (Feb, 2007).
11. J. Wang et al., Int J Mol Med 17, 309 (Feb, 2006).
12. R. Watanabe et al., Antimicrob Agents Chemother 51, 446 (Feb, 2007).
13. S. Mukhopadhyay, J. R. Hoidal, T. K. Mukherjee, Respir Res 7, 125 (2006).
14. S. A. Nonas, J. H. Finigan, L. Gao, J. G. Garcia, Proc Am Thorac Soc 2, 188 (2005).
15. M. Adib-Conquy, J. M. Cavaillon, FEBS Lett 581, 3723 (Jul 31, 2007).
16. P. Knezevic, O. Petrovic, J Microbiol Methods 74, 114 (Aug, 2008).
17. S. Sillankorva, P. Neubauer, J. Azeredo, BMC Biotechnol 8, 79 (2008).
18. G. W. Hanlon, S. P. Denyer, C. J. Olliff, L. J. Ibrahim, Appl Environ Microbiol 67, 2746 (Jun, 2001).
19. M. Skurnik, E. Strauch, Int J Med Microbiol 296, 5 (Feb, 2006).
20. L. Golshahi, K. D. Seed, J. J. Dennis, W. H. Finlay, J Aerosol Med Pulm Drug Deliv 21, 351 (Dec, 2008).
21. R. Ramphal et al., J Immunol 181, 586 (Jul 1, 2008).
22. P. Boulanger, Methods Mol Biol 502, 227 (2009).
23. H. W. Ackermann, Methods Mol Biol 501, 113 (2009).

## Claims

1. Isolated and/or purified bacteriophages that are specific to PAK and/or CHA strains of *Pseudomonas aeruginosa.*

2. Isolated and/or purified bacteriophage according to claim 1 of the Myoviridae family.

3. Isolated and/or purified bacteriophages according to claim 1 or 2, selected from the group of bacteriophages consisting of:
- PAK-P1 which has been deposited at the Institut Pasteur, 28 rue du Docteur Roux, F-75024 Paris Cedex 15, FRANCE, on July 11, 2008, under the deposit number CNCM I-4040;
- PAK-P2 which has been deposited at the Institut Pasteur, 28 rue du Docteur Roux, F-75024 Paris Cedex 15, FRANCE, on July 11, 2008, under the deposit number CNCM I-4041;
- PAK-P3 which has been deposited at the Institut Pasteur, 28 rue du Docteur Roux, F-75024 Paris Cedex 15, FRANCE, on July 11, 2008, under the deposit number CNCM I-4042;
- PAK-P4 which has been deposited at the Institut Pasteur, 28 rue du Docteur Roux, F-75024 Paris Cedex 15, FRANCE, on July 11, 2008, under the deposit number CNCM I-4043;
- PAK-P5 which has been deposited at the Institut Pasteur, 28 rue du Docteur Roux, F-75024 Paris Cedex 15, FRANCE, on July 11, 2008, under the deposit number CNCM I-4045; and
- PAK-P3 CHA which has been deposited at the Institut Pasteur, 28 rue du Docteur Roux, F-75024 Paris Cedex 15, FRANCE, on March 30, 2009 under the deposit number CNCM I-4139.

4. Isolated and/or purified bacteriophages according to one of claims 1 to 3, selected from the group of bacteriophages consisting of the bacteriophages having the sequences SEQ ID NO: 1, SEQ ID NO: 2, and SEQ ID NO: 3., or a specific fragment thereof

5. Pharmaceutical composition comprising at least one isolated and/or purified bacteriophage according to one of claims 1 to 4.

6. Composition according to Claim 5 capable to reach 100 % survival of infected mice with *Pseudomonas aeruginosa* bacteria when administering said specific sequence fragment 2 hours (2H) post-infection as shown in figure 3.

7. Composition according to claim 6 for the prevention or the treatment of an infectious disease caused by bacteria in a mammal, preferably in human.

8. Composition according to claim 6 or 7 for the prevention or the treatment of lung infection.

9. Composition according to one of claims 6 to 8 for the prevention or the treatment caused by pathogenic *Pseudomonas aeruginosa.*

10. Composition according to one of claims 6 to 9, wherein said composition is an aerosol formulation for administration to the lungs of a mammal.

11. Composition according to claim 10, wherein said composition contains bacteriophages which have been initially prepared in lyophilized form.

12. A food or pet food product, a nutritional composition or a supplement for nutritional composition comprising at least one phage isolate according to one of claims 1 to 4.

13. The use of a least one phage isolate preparation according to one of claims 1 to 4, for the preparation of a food or pet food product, a nutritional composition or a supplement for nutritional composition for the prevention or the treatment of infections caused by pathogenic bacteria, preferably by pathogenic *Pseudomonas aeruginosa* in humans or animals.
